# EUROPEAN PATENT APPLICATION

(11) **EP 4 361 148 A1**
(43) Date of publication of application: **01.05.2024**
(21) Application number: 22828544.1
(22) Date of filing: 24.06.2022
(51) Int. Cl.: C07D 413/04, A61K 31/4245, A61K 31/454, A61P 31/12, C07D 413/14

(54) **HETEROCYCLOALKYL-SUBSTITUTED POLYHETEROAZOLE DERIVATIVE AS MEDICAL DRUG FOR TREATMENT AND/OR PREVENTION OF RS VIRUS INFECTIOUS DISEASE**

(30) Priority: 25.06.2021 JP 2021106034
(71) Applicant: Albius Sciences Alpha Private Limited, People's Park Centre 058357 (SG)
(72) Inventor: MATSUOKA, Shigeru, Yufu-shi, Oita 879-5503 (JP); TSUCHIKAWA, Hiroshi, Yufu-shi, Oita 879-5503 (JP); YAMADA, Kentaro, Yufu-shi, Oita 879-5503 (JP); KATO, Akira, Yufu-shi, Oita 879-5503 (JP)
(74) Representative: Bird & Bird LLP
(86) International application number: PCT/JP2022/025361
(87) International publication number: WO 2022/270628

(57) **Abstract**

The present invention provide compounds useful for treating and/or preventing RS virus infections.

The present invention relates to compounds represented by formula (I), its enantiomer, or a pharmaceutically acceptable salt thereof: 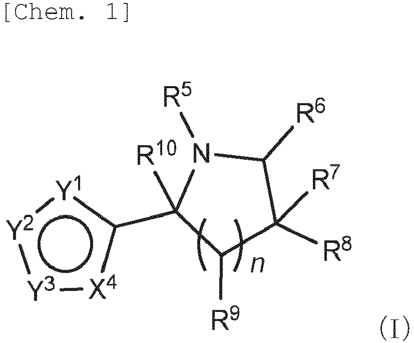 wherein Y¹, Y², Y³ and X⁴ and so on are as defined in the specification;
as well as a use of the compounds for treating and/or preventing RS virus infections, and pharmaceutical compositions comprising those compounds.

## Description

### BACKGROUND TECHNOLOGY

The present application claims the priority under the Paris Convention with respect to the Japanese Patent Application No. 2021-106034 filed on June 25, 2021, which is incorporated herein by reference in its entirety.

Respiratory Syncytial (RS) virus occurs worldwide, and causes a respiratory infection that has no regional or climatic bias, and is universally endemic and recurrent. It is estimated that almost 100% of children are infected by the age of two, and it is believed to be an infectious disease with a higher mortality rate than influenza in newborns because of the higher rate of infection. In fact, because there is no effective treatment for RS virus, approximately 30 million cases occur worldwide each year, and about 100,000 people die. In recent years, advances in diagnostic technology have made it possible to identify RS virus infection with a high degree of accuracy, and there is an urgent need to develop effective therapeutic pharmaceuticals (Non-Patent Document 1).

VIRAZOLE (registered trademark), Ribavirin for Inhalation Solution, USP, currently approved in the United States for the treatment of patients with severe RS virus infection, has an uncertain therapeutic effect in clinical practice, and has side effect problems such as teratogenicity (Non-Patent Document 2). The mechanism of action is also unknown (Non-Patent Document 3). Meanwhile, SYNAGIS (registered trademark), palivizumab, which is a monoclonal antibody drug against the surface protein of RS virus, was also approved in Japan in 2001, but it requires monthly intramuscular injection for about six months from before to during the epidemic period, which imposes a heavy burden on patients. It is also known as an expensive drug because it is a biopharmaceutical, and thus, insurance coverage is limited to high-risk children, who are particularly susceptible to severe disease (Non-Patent Document 4, 5).

### CITATION LIST

### Non-PATENT DOCUMENTS

Non-Patent Document 1: Barr, R.; Green, C. A.; Sande, C. J.; Drysdale, S. B. Respiratory syncytial virus: diagnosis, prevention and management, Ther. Adv. Infectious Dis. 2019, 6, 1-9.
Non-Patent Document 2: Domachowske, J. B.; Anderson, E. J.; Goldstein, M. The Future of Respiratory Syncytial Virus Disease Prevention and Treatment, Infect. Dis. Ther. 2021, 10, S47-S60.
Non-Patent Document 3: Aljabr, W.; Touzelet, O.; Pollakis, G.; Wu, W.; Munday, D. C.; Hughes, M.; Hertz-Fowler, C.; Kenny, J.; Fearns, R.; Barr, J. N.; Matthews, D. A.; Hiscox, J. A. Investigating the Influence of Ribavirin on Human Respiratory Syncytial Virus RNA Synthesis by Using a High-Resolution Transcriptome Sequencing Approach, J. Virol. 2015, 90, 4876-4888.
Non-Patent Document 4: Hiroyuki Tsutsumi, Clinical Significance of Palivizumab Administration, Infectious Agents Surveillance Report (IASR) 2018, 39, 219-220
Non-Patent Document 5: K. Okada, M. Mizuno, H. Moriuchi, S. Kusuda, I. Morioka, Y. Mori, K. Okamoto, K. Okada, S. Yoshihara, T. Yamagishi, U. Yokoyama, T. Kubota, H. Kudo, M. Takagi, S. Ito, Y. Kanamori, Y. Sasahara, Japanese Society of Paediatrics Committee on Immunization and Countermeasures against Infectious Diseases, Working Group for Revision of "Guidelines for the Use of Palivizumab in Japan, The Japanese Society of Pediatrics, The Japanese Society of Neonatal and Child Health Care Medicine, The Japanese Society of Pediatric Infectious Diseases, The Japanese Society of Pediatric Respiratory Medicine, The Japanese Society of Pediatric Cardiology, The Japanese Society of Pediatric Rheumatology, The Japanese Society of Pediatric Hematology and Cancer, The Japanese Society of Pediatric Nephrology, The Japanese Society of Pediatric Surgery, The Japanese Society for Immunodeficiency and Autoinflammation. Consensus Guidelines for the Use of Palivizumab in Japan, Japanese Journal of Pediatrics 2019, 123, 807-813

### SUMMARY OF THE INVENTION

### PROBLEMS TO BE SOLVED BY THE INVENTION

Under the circumstances, the inventors of the present application investigated a new therapeutic agent for RS virus infection, and found small molecule compounds that exhibits an inhibitory activity on RS virus proliferation at the cellular experimental level among the compounds originally designed and synthesized by the inventors. The development of therapeutic agents from these compounds could save a lots of the newborns who would die each year from RS virus.

### MEANS TO SOLVE PROBLEMS

Accordingly, the present invention encompasses the following aspects:

### <Compounds>

[1] A compound represented by formula (I), its enantiomer, or a pharmaceutically acceptable salt thereof: 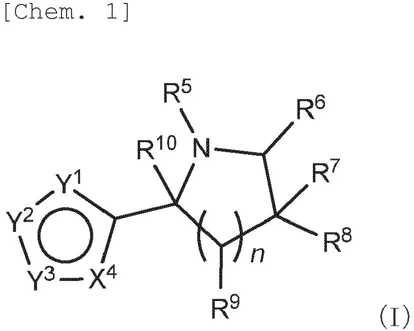 wherein
   Y¹, Y², Y³ and X⁴ are each independently -O-, -N=, -S-, -NR¹-, or -CR²=;
      in which at least one of Y¹, Y², Y³ and X⁴ is -N= or -NR¹-;
      R¹ is hydrogen, an optionally substituted C1-6 alkyl, an optionally substituted C6-10 aryl, or an optionally substituted C6-10 heteroaryl;
      R² is hydrogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl;
   R⁵ is an optionally substituted C1-6 alkyl, an optionally substituted C6-10 aryl, an optionally substituted C6-10 heteroaryl, an unsubstituted or substituted carbonyl, an unsubstituted or substituted sulfonyl, an unsubstituted or substituted sulfinyl, an unsubstituted or substituted acyl, or an unsubstituted or substituted thioacyl;
   R⁶, R⁷, R⁸, R⁹ and R¹⁰ are each independently hydrogen, an optionally substituted C1-6 alkyl, an optionally substituted C6-10 aryl, an optionally substituted C1-6 alkoxy, hydroxy, amino, cyano, carbamoyl or a halogen, or
   R⁷ and R⁸ are cross-linked together with the carbon to which they are attached, to form a C3-6 spiro ring,
   R⁶ and R⁹ are cross-linked together to form -CH₂- or - CH₂-CH₂-, or
   R⁸ and R¹⁰ are cross-linked together to form -CH₂- or - CH₂-CH₂-;
   n is an integer of 1 or 2;
   in which the substituent in "optionally substituted" is selected from the following:
      hydroxy, a halogen, cyano, carbamoyl, amino, an amidinoamino, a carboxy, a C6-10 aryl, a C1-4 alkoxycarbonyl-substituted 5- to 10-membered heteroaryl, a C1-4 alkylsubstituted C6-10 aryl , a hydroxy-substituted C6-10 aryl, a halogen-substituted C6-10 aryl, a C1-4 alkoxy-substituted C6-10 aryl, a (an optionally substituted amino)-C6-10 aryl, a C1-4 alkoxycarbonyl, a C1-4 alkoxycarbonylamino, a 5- to 6-membered heterocycloalkyl, a C3-6 cycloalkyl, a 5- to 10-membered heteroaryl, a (a halogen-substituted C1-6 alkyl)-substituted C6-10 aryl, and a trialkylsilyloxy, an alkylarylsilyloxy, a triarylsilyloxy, or a protecting group;
      provided that, when the aromatic 5-membered ring comprising Y¹, Y², Y³ and X⁴ is a 1,2,3-triazole substituted with phenylmethyl, R⁵ is not tert-butoxycarbonyl.
[2] The compound according to [1], its enantiomer, or a pharmaceutically acceptable salt thereof,
   wherein the compound is represented by formula (I): 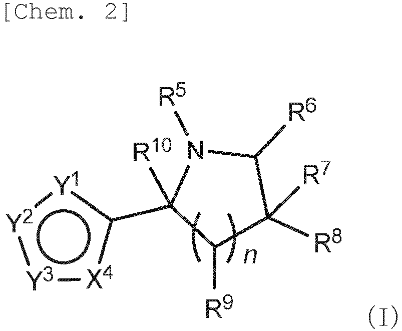
   wherein
      Y¹ is -O-, -N=, -S-, or -NR¹-;
      Y² is -O-, -N=, -NR³-, or -CR²=;
      Y³ is -N=, -NR⁴- or -CR¹⁸=;
      X⁴ is -N= or -CH=;
      in which at least one of Y¹, Y², Y³ and X⁴ is -N=, -NR²- or -NR³-;
      R¹, R³ and R⁴ are each independently hydrogen, an optionally substituted C1-6 alkyl, an optionally substituted C6-10 aryl, or an optionally substituted C6-10 heteroaryl;
      R² and R¹⁸ are each independently hydrogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl.
[3] The compound according to [1] or [2], its enantiomer, or a pharmaceutically acceptable salt thereof,
   wherein the compound is represented by formula (I): 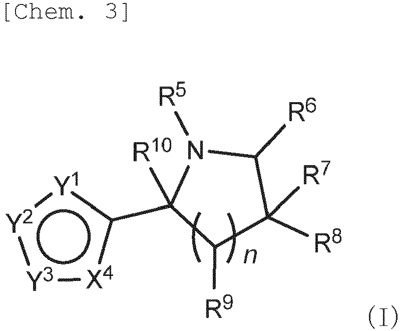
   wherein
      the group represented by the formula: 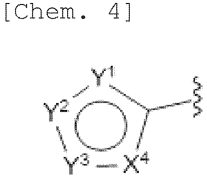 is the group represented by the formula: 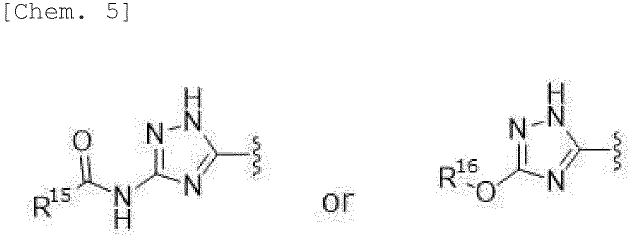
   wherein
      R¹⁵ is an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl; and
      R¹⁶ is an optionally substituted C1-6 alkyl, an optionally substituted C6-10 aryl, or an optionally substituted C6-10 heteroaryl.
[4] The compound according to [1] or [2], its enantiomer, or a pharmaceutically acceptable salt thereof,
   wherein the compound is represented by formula (I): 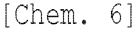 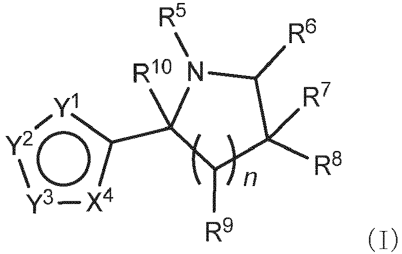
   wherein
      the group represented by the formula: 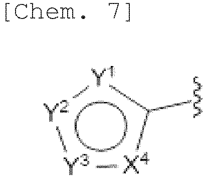 is the group represented by the formula: 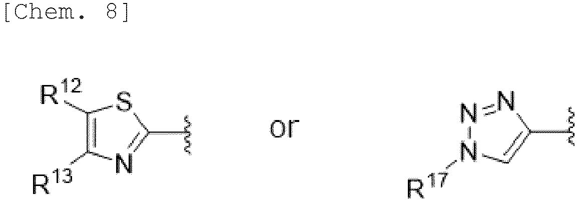
   wherein
      R¹² and R¹³ are each independently hydrogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl; and
      R¹⁷ is an optionally substituted C1-6 alkyl, an optionally substituted C6-10 aryl, an optionally substituted C6-10 heteroaryl.
[5] The compound according to [1] or [2], its enantiomer, or a pharmaceutically acceptable salt thereof,
   wherein the compound is represented by formula (I): 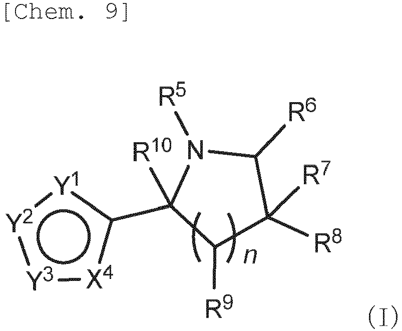
   wherein
      the group represented by the formula: 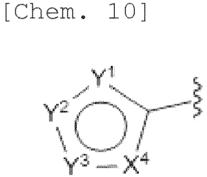 is the group represented by the formula: 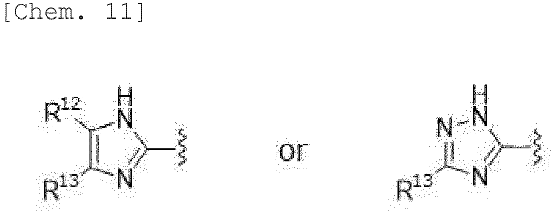
   wherein
      R¹² and R¹³ are independently hydrogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl.
[6] The compound according to [1] or [2], its enantiomer, or a pharmaceutically acceptable salt thereof,
   wherein the compound is represented by formula (I): 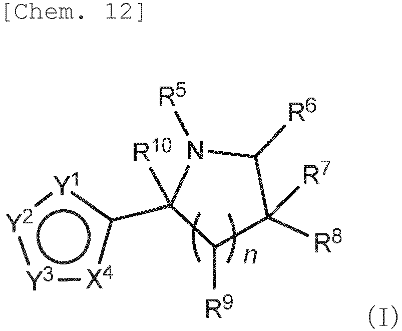
   wherein
      the group represented by the formula: 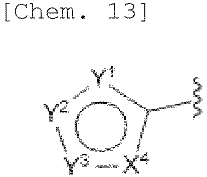 is the group represented by the formula: 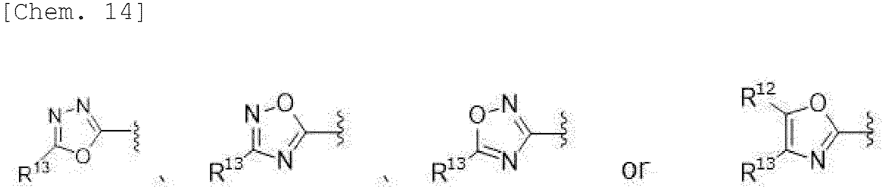
   wherein
      R¹² and R¹³ are each independently hydrogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl.
[7] The compound according to [6], its enantiomer, or a pharmaceutically acceptable salt thereof,
   wherein
   the group represented by the formula: 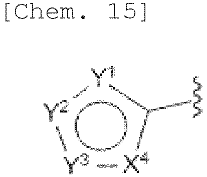 is the group represented by the formula: 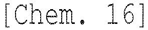 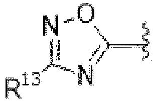 wherein
   R¹³ is hydrogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl.
[8] The compound according to [1] or [2], its enantiomer, or a pharmaceutically acceptable salt thereof,
   wherein the compound is represented by formula (I): 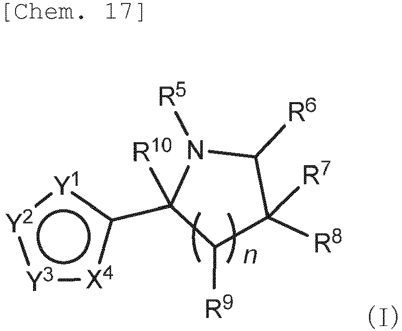
   wherein
      the group represented by the formula: 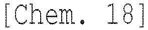 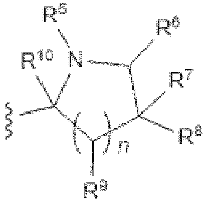 is the group represented by the formula: 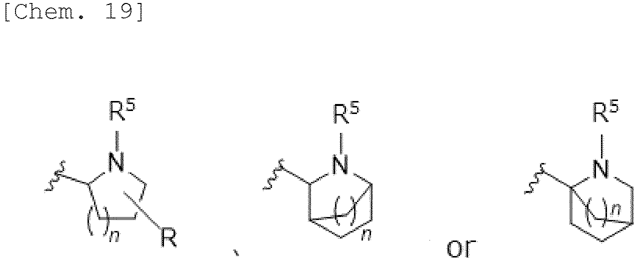
   wherein
      R is hydrogen, an optionally substituted C1-6 alkyl, an optionally substituted C6-10 aryl, an optionally substituted C1-6 alkoxy, hydroxy, amino, cyano, carbamoyl or a halogen;
      R⁵ is an optionally substituted C1-6 alkyl, an optionally substituted C6-10 aryl, an optionally substituted C6-10 heteroaryl, an optionally substituted carbonyl, an optionally substituted sulfonyl, an optionally substituted sulfinyl, an optionally substituted acyl, or an optionally substituted thioacyl;
      n is an integer of 1 or 2.
[8-2] The compound according to any one of [3] to [7], its enantiomer, or a pharmaceutically acceptable salt thereof, wherein the compound is represented by formula (I): 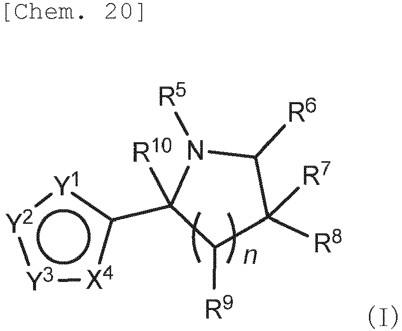 wherein
   the group represented by the formula: 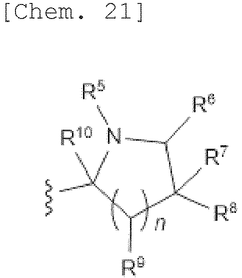 is the group represented by the formula: 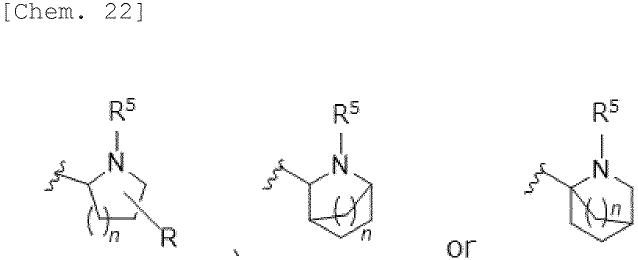 wherein
   R is hydrogen, an optionally substituted C1-6 alkyl, an optionally substituted C6-10 aryl, an optionally substituted C1-6 alkoxy, hydroxy, amino, cyano, carbamoyl or a halogen;
   R⁵ is an optionally substituted C1-6 alkyl, an optionally substituted C6-10 aryl, an optionally substituted C6-10 heteroaryl, an optionally substituted carbonyl, an optionally substituted sulfonyl, an optionally substituted sulfinyl, an optionally substituted acyl, or an optionally substituted thioacyl;
   n is an integer of 1 or 2.
[9] The compound according to [8], its enantiomer, or a pharmaceutically acceptable salt thereof,
   wherein the compound is represented by formula (I): 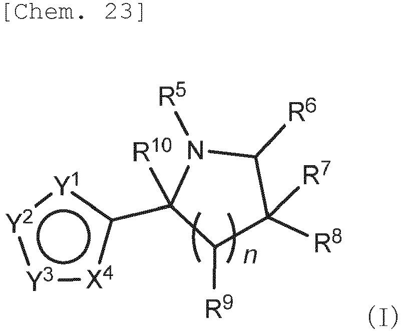
   wherein
      the group represented by the formula: 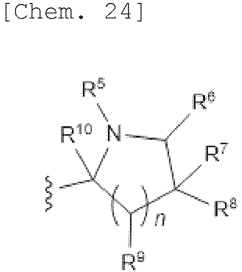 is the group represented by the formula: 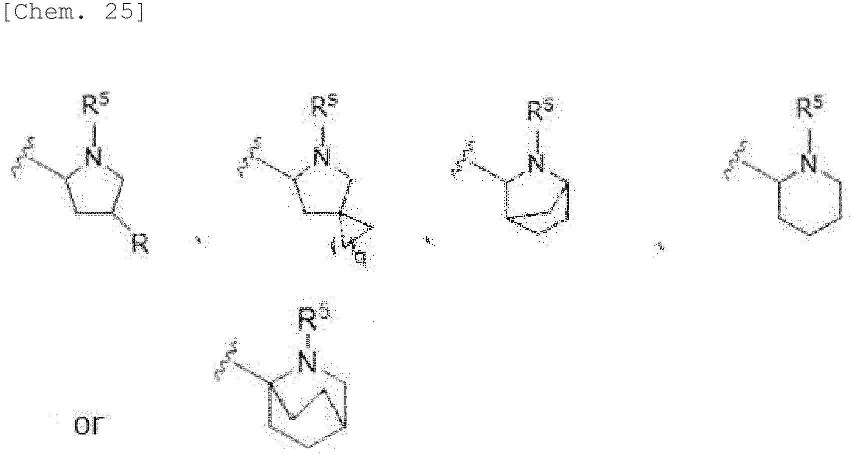
   wherein
      R is hydrogen, an optionally substituted C1-6 alkyl, an optionally substituted C6-10 aryl, an optionally substituted C1-6 alkoxy, hydroxy, amino, cyano, carbamoyl or a halogen;
      R⁵ is an optionally substituted C1-6 alkyl, an optionally substituted C6-10 aryl, an optionally substituted C6-10 heteroaryl, an optionally substituted carbonyl, an optionally substituted sulfonyl, an optionally substituted sulfinyl, an optionally substituted acyl, or an optionally substituted thioacyl; and
      q is an integer from 1 to 4.
[9-2] The compound according to any one of [1] to [7], its enantiomer, or a pharmaceutically acceptable salt thereof, wherein the compound is represented by formula (I): 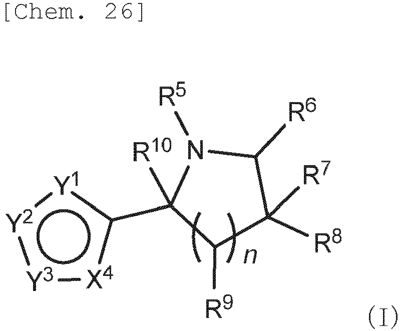 wherein
   the group represented by the formula: 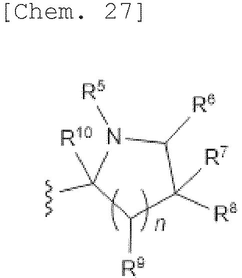 is the group represented by the formula: 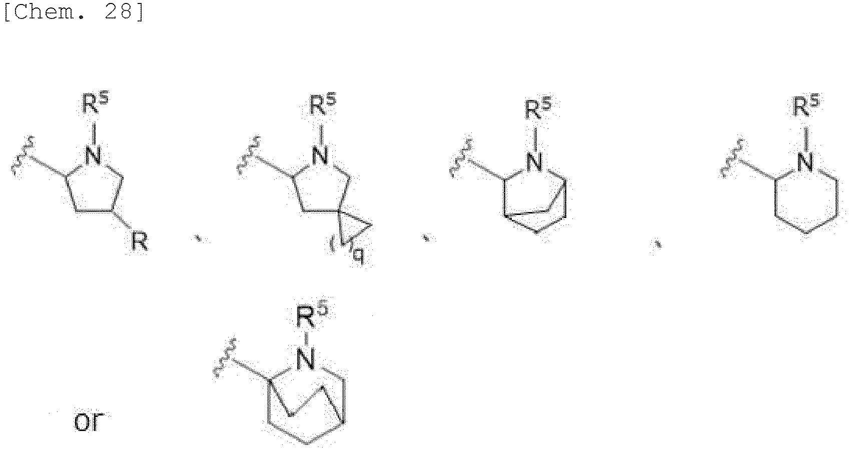 wherein
   R is hydrogen, an optionally substituted C1-6 alkyl, an optionally substituted C6-10 aryl, an optionally substituted C1-6 alkoxy, hydroxy, amino, cyano, carbamoyl or a halogen;
   R⁵ is an optionally substituted C1-6 alkyl, an optionally substituted C6-10 aryl, an optionally substituted C6-10 heteroaryl, an optionally substituted carbonyl, an optionally substituted sulfonyl, an optionally substituted sulfinyl, an optionally substituted acyl, or an optionally substituted thioacyl; and
   q is an integer from 1 to 4.
[10] The compound according to [9], its enantiomer, or a pharmaceutically acceptable salt thereof,
   wherein
   the group represented by the formula: 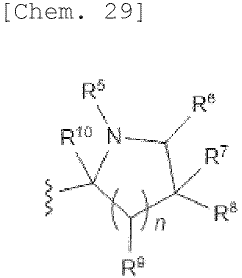 is the group represented by the formula: 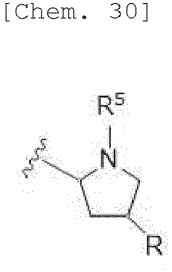 wherein
   R is hydrogen, an optionally substituted C1-6 alkyl, an optionally substituted C6-10 aryl, an optionally substituted C1-6 alkoxy, hydroxy, amino, cyano, carbamoyl or a halogen;
   R⁵ is an optionally substituted sulfonyl.
[10-2] The compound according to any one of [1] to [7], its enantiomer, or a pharmaceutically acceptable salt thereof, wherein
   the group represented by the formula: 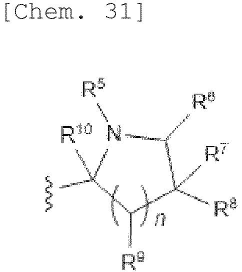 is the group represented by the formula: 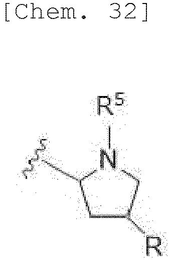 wherein
   R is hydrogen, an optionally substituted C1-6 alkyl, an optionally substituted C6-10 aryl, an optionally substituted C1-6 alkoxy, hydroxy, amino, cyano, carbamoyl or a halogen;
   R⁵ is an optionally substituted sulfonyl.
[11] The compound according to [7], its enantiomer, or a pharmaceutically acceptable salt thereof,
   wherein the compound is represented by the formula: 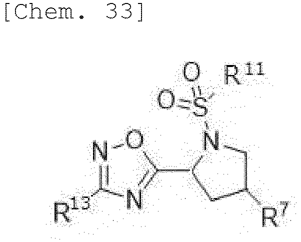
   wherein
      R¹³ is the formula: 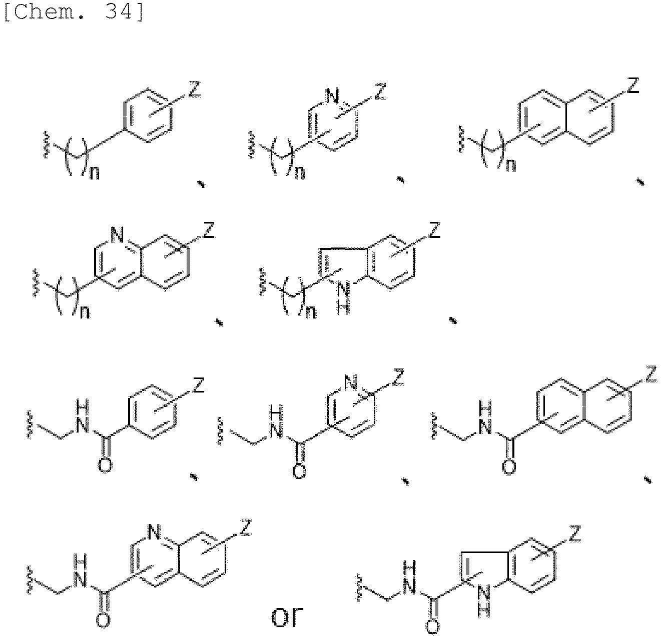
      in which
         Z is hydrogen, amino, dimethylamino, a halogen, hydroxy, cyano, carbamoyl, an optionally substituted C1-6 alkyl, or an optionally substituted C1-6 alkoxy;
         n is an integer from 1 to 5;
      R⁷ is the group represented by the formula: 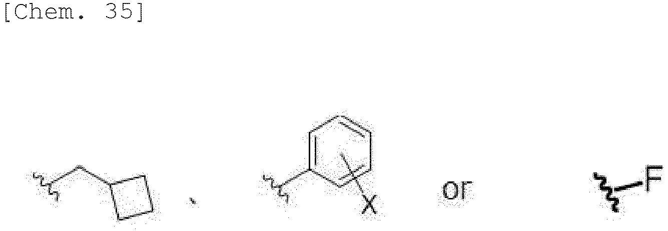
      in which
         X is hydrogen, amino, a halogen, hydroxy, methoxy, or an optionally substituted C1-4 alkyl;
      R¹¹ is methyl, or the group represented by the formula: 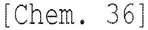 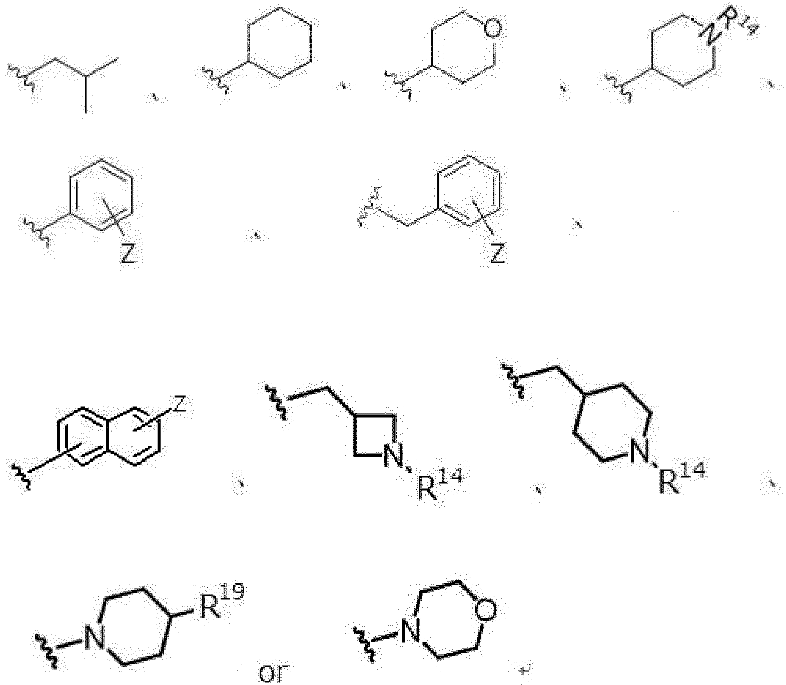
      in which
         R¹⁴ is each independently hydrogen, an optionally substituted C1-6 alkyl, an optionally substituted C6-10 aryl, an optionally substituted C6-10 heteroaryl, an optionally substituted carbonyl, an optionally substituted sulfonyl, an optionally substituted sulfinyl, an optionally substituted acyl, or an optionally substituted thioacyl;
         R¹⁹ is hydrogen, an optionally substituted C1-6 alkyl, an optionally substituted C6-10 aryl, an optionally substituted carbonyl, hydroxy, an alkoxy, or an alkoxymethyl;
         Z is the same as defined above.
[11-2] The compound according to any one of [8] to [10-2], its enantiomer, or a pharmaceutically acceptable salt thereof, wherein the compound is represented by the formula: 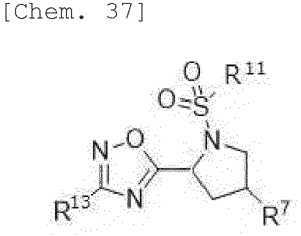 wherein
   R¹³ is the formula: 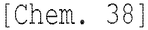 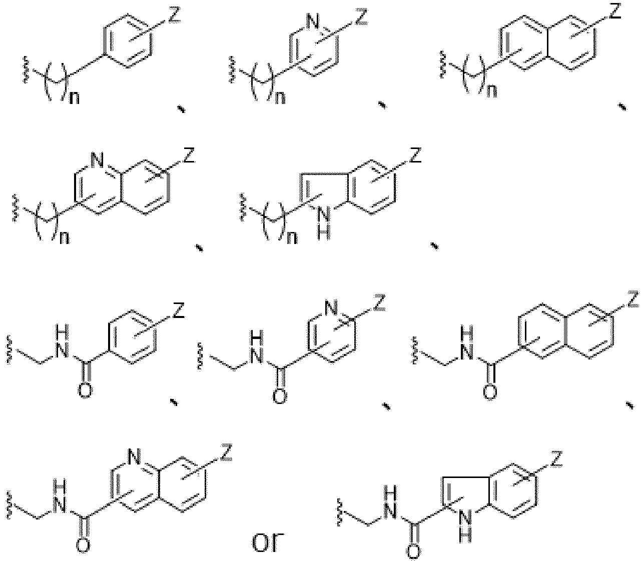
   in which
      Z is hydrogen, amino, dimethylamino, a halogen, hydroxy, cyano, carbamoyl, an optionally substituted C1-6 alkyl, or an optionally substituted C1-6 alkoxy;
      n is an integer from 1 to 5;
   R⁷ is the group represented by the formula: 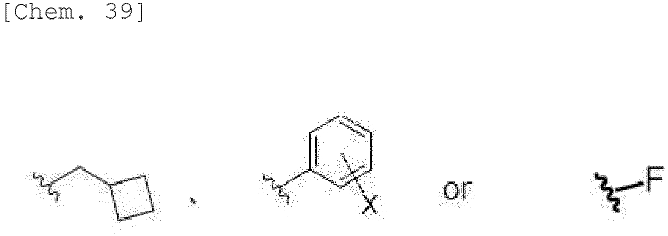
   in which
      X is hydrogen, amino, a halogen, hydroxy, methoxy, or an optionally substituted C1-4 alkyl;
   R¹¹ is methyl, or the group represented by the formula: 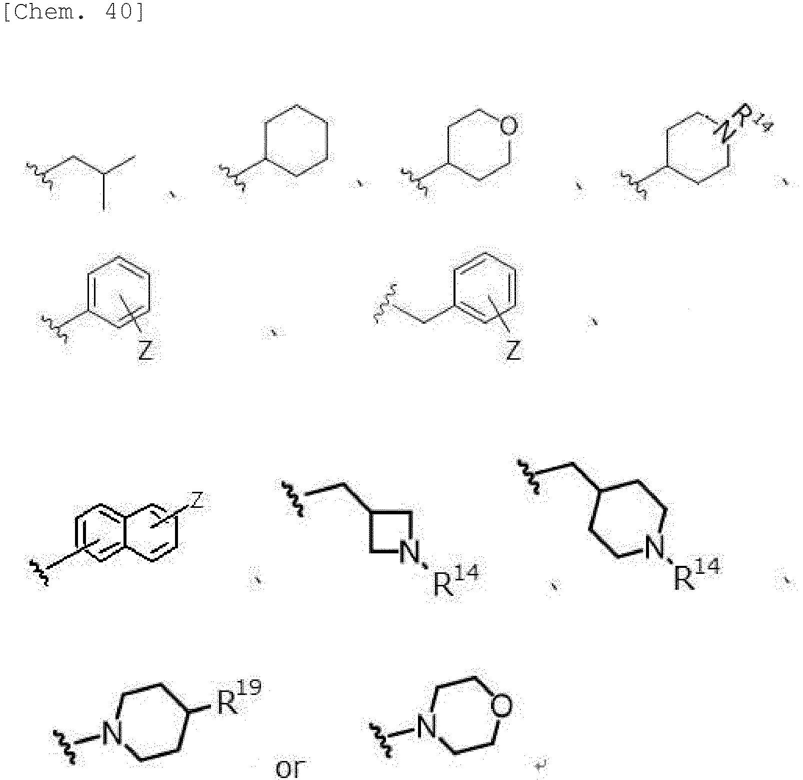
   in which
      R¹⁴ is each independently hydrogen, an optionally substituted C1-6 alkyl, an optionally substituted C6-10 aryl, an optionally substituted C6-10 heteroaryl, an optionally substituted carbonyl, an optionally substituted sulfonyl, an optionally substituted sulfinyl, an optionally substituted acyl, or an optionally substituted thioacyl;
      R¹⁹ is hydrogen, an optionally substituted C1-6 alkyl, an optionally substituted C6-10 aryl, an optionally substituted carbonyl, hydroxy, an alkoxy, or an alkoxymethyl;
      Z is the same as defined above.

### <Pharmaceutical compositions>

A pharmaceutical composition, which comprises the compound of any one of [1] to [11], its enantiomer, or a pharmaceutically acceptable salt thereof.

The pharmaceutical composition according to [12], for treating or preventing an RS virus infection.

### EFFECT OF INVENTION

According to the present invention, small molecular compounds that are expected to have a good therapeutic effect on RS virus infections are provided. Those compounds lead to inexpensive, stable, small-molecule pharmaceuticals, which are expected to be widely available, including in developing countries, and to provide a large ripple effect from a public health perspective, the small-molecule pharmaceuticals being clearly different from current drugs for preventing RS virus infections, which are biopharmaceuticals.

### EMBODIMENTS FOR CARRYING OUT INVENTION

Hereinafter, the present invention is described in more detail. Terms as used herein have the meanings normally used in the art, unless otherwise specified. Therefore, unless otherwise defined, all technical and scientific terms as used herein have the same meaning as generally understood by those skilled in the art to which the invention belongs.

### <Definition>

The term "group" as used herein means a monovalent group unless otherwise specified. Specific examples of a non-monovalent group include alkylene groups (divalent). The term "group" may also be omitted in the description of substituents, etc., below.

The number of substituents in the definitions herein of "optionally substituted" or "substituted" is one or more unless otherwise specified, and there is no particular limit on the number as long as they can be substituted. Unless otherwise indicated, the description of each substituent also applies when that substituent is a part of or a substituent of another substituent.

Groups herein modified as "optionally substituted" or "substituted" may be substitited on any portion of the groups. For example, "an optionally substituted arylalkyl" and "a substituted arylalkyl" may be substituted on the aryl moiety, may be substituted on the alkyl moiety, or may be subtituted on both of the aryl moiety and alkyl moiety.

Substituents in the definition of "optionally substituted" herein may be also selected from Substituent α consisting of the followings, and may be sustituented wiht 1 to 5 substituents that are identical or different: Substituent a: hydroxy, a halogen, cyano, carbamoyl, amino, amidinoamino, carboxy, a C6-10 aryl, a 5- to 10-membered heteroaryl substituted with C1-4 alkoxycarbonyl, a C6-10 aryl substituted with C1-4 alkyl, a C6-10 aryl substituted with hydroxy, a C6-10 aryl substituted with C1-4 alkoxy, (an optionally substituted amino)-C6-10 aryl, a C1-4 alkoxycarbonyl, a C1-4 alkoxycarbonylamino, a 5- to 6-membered heterocycloalkyl, a C3-6 cycloalkyl, a 5- to 10-membered heteroaryl, a C6-10 aryl subsituted with (C1-6 alkyl substituted with halogen), and a trialkylsilyloxy, an alkylarylsilyloxy, a triarylsilyloxy, or a protecting group.

The Substituent α may be optionally substituted with one to five substituents selected from Substituent β, which are identical or different:
Substituent β: a halogen, hydroxy, carboxy, cyano, a C3-10 alicyclic group, a C1-6 alkoxy, a C3-10 alicyclic oxy, a C1-6 alkylthio, a 5- or 6-membered heteroarylthio, a C6-10 aryl, a 5- or 6-membered heteroaryl, a 4- to 10-membered non-aryl heteroring, a C1-6 alkylcarbonyl, a C3-10 alicyclic carbonyl, a C6-10 arylcarbonyl, a 5- or 6-membered heteroarylcarbonyl, a 4- to 10-membered non-aryl heterocyclic carbonyl, and a Protecting group.

As used herein, "C1-6" means that the number of carbon atoms is from 1 to 6. The same applies to other numbers, and for exmaple, "C1-4" means the number of carbon atoms is from 1 to 4, and "C1-3" means the number of carbon atoms is from 1 to 3.

As used herein, "heteroatom" means an atom other than carbon and hydrogen atoms, and includes oxygen, nitrogen, and sulfur atoms.

As used herein, "hydroxy" is a monovalent group of -OH. This group may be also referred to as a "hydroxy group" or "hydroxy".

As used herein, "halogen" means an atom belonging to the halogen groups, such as a fluorine, chlorine, bromine or iodine atom. Preferably, it is fluorine atom or chlorine atom. More preferably, it is a fluorine atom. Halogen may be also referred to as "halogen atom" or "halo".

As used herein, "carboxy" is a monovalent group of - COOH. This group may be also referred to as a "carboxy group," "carboxy", "carboxyl," or "carboxylic acid group.

As used herein, "cyano" is a monovalent group of -CN.

As used herein, "amino" is a monovalent group of -NH2. This group may be also referred to as "amino group".

As used herein, "alkyl" means a linear or branched, saturated aliphatic hydrocarbon group. "C1-6 alkyl" is an alkyl group with 1 to 6 carbon atoms, and preferably it includes "C1-4 alkyl", more preferably "C1-3 alkyl", and even more preferably "C1-2 alkyl". Specific examples of "C1-4 alkyl" include methyl, ethyl, propyl, isopropyl, n-butyl, isobutyl, tert-butyl, sec-butyl, and the like. Specific examples of "C1-6 alkyl" include, but are not limited to, a C1-4 alkyl, n-pentyl, isopentyl, neopentyl, tert-pentyl, 1,2-dimethylpropyl, and n-hexyl.

As used herein, "alkenyl" means a straight or branched, unsaturated aliphatic hydrocarbon group containing at least one carbon-carbon double bond. "C2-6 alkenyl" is an alkenyl group with 2 to 6 carbon atoms, and a preferred example includes "C2-4 alkenyl". Specific examples of "C2-6 alkenyl" include, but are not limited to, vinyl, 1-propenyl, 2-propenyl, 1-butenyl, 2-butenyl, 3-butenyl, 2-methyl-1-propyleneyl, and 2-methyl-2-propyleneyl.

As used herein, "alkynyl" means a straight or branched, unsaturated aliphatic hydrocarbon group containing at least one carbon-carbon triple bond. "C2-6 alkynyl" is an alkynyl group with 2 to 6 carbon atoms, and a preferred example includes "C2-4 alkynyl". Specific examples of "C2-6 alkynyl" include, but are not limited to, ethynyl, 1-propynyl, 2-propynyl, 1-butynyl, 1-methyl-2-propynyl, 3-butynyl, 1-pentynyl, and 1-hexynyl.

As used herein, "aryl" means a monovalent group of a monocyclic or bicyclic aromatic hydrocarbon ring, and "C6-10 aryl" means an aryl group with 6 to 10 carbon atoms. Specific examples of "aryl" include, but are not limited to, a C6 aryl and a C10 aryl. Specific examples of C6 aryl include, but are not limited to, phenyl. Specific examples of C10 aryl include, but are not limited to, 1-naphthyl and 2-naphthyl.

As used herein, "arylalkyl" means an alkyl substituted with at least one aryl. "C6-10 aryl C1-6 alkyl" means a C1-6 alkyl substituted with at least one C6-10 aryl. Specific examples of C6-10 aryl C1-6 alkyl include, but are not limited to, benzyl (phenyl-CH₂-), phenethyl (phenyl-CH₂CH₂-), naphthalen-1-ylmethyl, naphthalen-2-ylmethyl, 2-(naphthalen-1-yl)ethyl, and 2-(naphthalen-2-yl)ethyl.

As used herein, "(optionally substituted amino)-arylalkyl" means an arylalkyl substituted with an amino group that may be an optionally substituted, wherein the alkyl or aryl group, or both, are substituted with an amino group. The amino group of said arylalkyl group may be unsubstituted or substituted with one, two, or three substituents, e.g., an optionally substituted alkyl (for example, an unsubstituted C1-6 alkyl, a C3-6 cycloalkyl-C1-6 alkyl, a C3-6 cycloalkylcarbonyl, and the like). Specific examples of (optionally substituted amino)-C6-10 aryl C1-6 alkyl include, but are not limited to, 4-(dimethylamino)benzyl, 4-((cyclopentylmethyl)amino)benzyl, 4-((cyclopentylcarbonyl)amino)benzyl, and 4 -((2-carbamoylethyl)carbonylamino)benzyl.

As used herein, the C6-10 aryl moiety of "C6-10 arylthio" is synonymous with C6-10 aryl above. "C6-10 arylthio" is preferably "C6 or C10 arylthio". Specific examples of "C6-10 arylthio" include, but are not limited to, phenylthio, 1-naphthylthio, and 2-naphthylthio.

As used herein, "C6-10 arylsulfonyl" means a sulfonyl substituted with "C6-10 aryl" above. "C6-10 arylsulfonyl" is preferably "C6 or C10 arylsulfonyl". Specific examples of "C6-10 arylsulfonyl" include, but are not limited to, phenylsulfonyl, 1-naphthylsulfonyl, and 2-naphthylsulfonyl.

As used herein, "heteroaryl" means a monovalent group of monocyclic or bicyclic aromatic heterocyclic rings containing from 1 to 4 heteroatoms selected from the group consisting of oxygen, nitrogen and sulfur atoms, which are identical or different.

As used herein, "5- or 6-membered heteroaryl" means a monovalent group of a monocyclic aromatic heterocyclic ring containing 5 to 6 atoms including 1 to 4 heteroatoms selected from the group consisting of oxygen, nitrogen and sulfur atoms, which are identical or different. Specific examples of "5- or 6-membered heteroaryl" include, but are not limited to, pyrrolyl, furyl, thienyl, pyrazolyl, imidazolyl, oxazolyl, isoxazolyl, oxadiazolyl, thiazolyl, isothiazolyl, thiadiazolyl, triazolyl, tetrazolyl, pyridyl, pyridazinyl, pyrimidinyl, and pyrazinyl.

As used herein, "5- to 10-membered heteroaryl" means a monovalent group of a monocyclic or bicyclic aromatic heterocyclic rings consisting of 5 to 10 atoms including 1 to 4 heteroatoms selected from the group consisting of oxygen, nitrogen and sulfur atoms, which are identical or different. Specific examples of "5- to 10-membered heteroaryl" include, but not limited to, a 5- or 6-membered heteroaryl, quinolyl, isoquinolyl, naphthyridinyl, quinoxalinyl, quinolinyl, quinazolinyl, phthalazinyl, imidazopyridyl, imidazothiazolyl, imidazoxazolyl, benzothiazolyl, benzoxazolyl, benzoimidazolyl, indolyl, isoindolyl, indazolyl, pyrrolopyridyl, thienopyridyl, flopyridyl, benzothiadiazolyl, benzoxadiazolyl, pyridopyrimidinyl, benzofuryl, benzothienyl, benzo[1,3]dioxol, thienofuryl, chromenyl, chromanyl, coumarinyl, and quinolonyl.

As used herein, "heteroarylalkyl" means an alkyl substituted with at least one heteroaryl. "5- to 10-membered heteroaryl C1-6 alkyl" means a C1-6 alkyl optionally substituted with at least one 5- to 10-membered heteroaryl. Specific examples of 5- to 10-membered heteroaryl C1-6 alkyl include, but are not limited to, pyridin-2-ylmethyl, pyridin-4-ylmethyl, 2-(quinolin-8-yl)ethyl, 2-(quinolin-5-yl)ethyl, 2-(quinoxalin-5-yl)ethyl, and 2-(1H-indol-3-yl)ethyl.

As used herein, "cycloalkyl" means a non-aromatic saturated hydrocarbon ring group, and includes those with partially bridged structures, partially spiroted and those with one or two carbonyl structures. "C3-20 cycloalkyl" means a monocyclic or bicyclic cycloalkyl with 3 to 20 carbon atoms. "C3-6 cycloalkyl" means a monocyclic cycloalkyl with 3 to 6 carbon atoms. Specific examples of C3-6 cycloalkyl include, but are not limited to, cyclopropyl, cyclobutyl, cyclopentyl, and cyclohexyl.

As used herein, "cycloalkylalkyl" means an alkyl substituted with at least one cycloalkyl. "C3-6 cycloalkyl C1-6 alkyl" means a C1-6 alkyl substituted with at least one C3-6 cycloalkyl. Specific examples of C3-6 cycloalkyl C1-6 alkyl include, but not limited to, cyclopropyl methyl, cyclobutyl methyl, cyclopentyl methyl, cyclohexyl methyl, 2-cyclopropyl ethyl, 2-cyclobutyl ethyl, 2-cyclopentyl ethyl, 2-cyclohexyl ethyl, 3- cyclopropyl propyl, 3-cyclobutyl propyl, 3-cyclopentyl propyl, and 3-cyclohexyl propyl.

As used herein, "heterocycloalkyl" means a non-aromatic saturated heterocyclic ring containing one or more heteroatoms that are the same or different, which is selected from the group consisting of oxygen, nitrogen and sulfur atoms, and includes those with partially bridged structures and those partially spirocheted.

As used herein, "4- to 20-membered non-aryl heterocyclic ring" means a monocyclic or bicyclic non-aromatic heterocyclic ring consisting of 4 to 20 atoms containing one or more heteroatoms selected from the group consisting of oxygen, nitrogen and sulfur atoms, which are identical or different, and includes those having partially unsaturated bonds, those with partially cross-linked structures and those with partially spiroted structures. The non-aryl heterocycle may form a fused ring with an aryl or a heteroaryl. For example, the hetero rings also include the condensation with a C6-10 aryl or a 5- or 6-membered heteroaryl. Said non-aryl heterorings may also contain one or two carbonyls, thiocarbonyls, sulfinyls or sulfonyls, and contain a cyclic ring such as a lactam, a thiolactam, a lactone, a thiolactone, a cyclic imide, a cyclic carbamate, a cyclic thiocarbamate. In this regardd, the number of 4-to 20-members (ring size) and the number of heteroatoms comprised in the ring do not include the oxygen atoms of carbonyl, sulfinyl and sulfonyl, and the sulfur atom of thiocarbonyl.

As used herein, "4- to 10-membered non-aryl heterocyclic ring" means a substituent in which the "4- to 10-membered non-aryl heterocyclic ring" of "4- to 20-membered non-aryl heterocyclic ring" above is a monovalent group.

As used herein, the 4- to 10-membered non-aryl heterocyclic portion of "4- to 10-membered non-aryl heterocyclic oxy" is synonymous with "4- to 10-membered non-aryl heterocyclic ring" above. "4- to 10-membered non-aryl heterocyclic oxy" is preferably "4- to 6-membered non-aryl heterocyclic oxy". Specific examples of "4- to 10-membered non-aryl heterocyclic oxy" include, but are not limited to, tetrahydrofuranyl oxy, tetrahydropyranyl oxy, azetidinyl oxy, pyrrolidinyl oxy, and piperidinyl oxy.

As used herein, the 4- to 10-membered non-aryl heterocyclic portion of "4- to 10-membered non-aryl heterocyclic thio" is synonymous with "4- to 10-membered non-aryl heterocyclic ring" above. "4- to 10-membered non-aryl heterocyclic thio" is preferably "4- to 6-membered non-aryl heterocyclic thio". Specific examples of "4- to 10-membered non-aryl heterocyclic thio" include, but are not limited to, tetrahydropyranyl thio and piperidinyl thio.

As used herein, "4- to 10-membered non-aryl heterocyclic carbonyl" means a carbonyl group substituted with "4- to 10-membered non-aryl heterocyclic ring" above. "4- to 10-membered non-aryl heterocyclic carbonyl" is preferably "4- to 6-membered non-aryl heterocyclic carbonyl". Specific examples of "4- to 10-membered non-aryl heterocyclic carbonyl" include, but are not limited to, azetidinyl carbonyl, pyrrolidinyl carbonyl, piperidinyl carbonyl, and morpholinyl carbonyl.

As used herein, "4- to 10-membered non-aryl heterocyclic sulfonyl" means a sulfonyl group substituted with "4- to 10-membered non-aryl heterocyclic ring" above. "4- to 10-membered non-aryl heterocyclic sulfonyl" is preferably "4- to 6-membered non-aryl heterocyclic sulfonyl". Specific examples of "4- to 10-membered non-aryl heterocyclic sulfonyl" include, but are not limited to, azetidinyl sulfonyl, pyrrolidinyl sulfonyl, piperidinyl sulfonyl, and morpholinyl sulfonyl.

As used herein, "5- to 6-membered heterocycloalkyl" means a heterocycloalkyl consisting of 5 to 6 ring atoms, including one or more heteroatoms selected from oxygen, nitrogen and sulfur atoms, which are identical or different.

As used herein, "heterocycloalkylalkyl" means an alkyl substituted with at least one heterocycloalkyl. 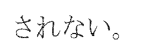

As used herein, "alkylcarbonyl" is a monovalent group of -C(=O)-alkyl. Preferred examples of alkylcarbonyl include a C1-6 alkylcarbonyl. Examples of C1-6 alkylcarbonyl include, but are not limited to, acetyl (CH₃C(=O)-), n-propanoyl (CH₃CH₂C(=O)-), n-butanoyl (CH₃CH₂CH₂C(=O)-), n-pentanoyl (CH₃(CH₂)3C(=O)-), n-hexanoyl (CH₃(CH₂)₄C(=O)-), and n-Heptanoyl (CH₃(CH₂)₅C(=O)-).

As used herein, "alkoxy" is a monovalent group of -O-alkyl. Preferred examples of alkoxy include a C1-6 alkoxy (that is a C1-6 alkyl-O-) and a C1-4 alkoxy (that is a C1-4 alkyl-O-). Specific examples of C1-4 alkoxy include methoxy (CH₃O-), ethoxy (CH₃CH₂O-), n-propoxy (CH₃(CH₂)₂O-), isopropoxy ((CH₃)₂CHO-), n-butoxy (CH₃(CH₂)₃O-), isobutoxy ((CH₃)₂CHCH₂O-) tert-butoxy ((CH₃)₃CO-), and sec-butoxy ((CH₃CH₂CH(CH₃)O-). Specific examples of C1-6 alkoxy include, but are not limited to, a C1-4 alkoxy, n-pentyloxy (CH₃(CH₂)₄O-), isopentyloxy ((CH₃)₂CHCH₂CH₂O-), neopentyloxy ((CH₃)₃CCH₂O-), tert-pentyloxy (CH₃CH₂C(CH₃)₂O-), and 1,2-dimethylpropoxy (CH₃CH(CH₃)CH(CH₃)O-).

As used herein, "alkoxycarbonyl" is a monovalent group of -C(=O)-O-alkyl. Examples of alkoxycarbonyls include, but are not limited to, a C1-6 alkoxycarbonyl, preferably a C1-4 alkoxycarbonyl. Specific examples of C1-4 alkoxycarbonyl include methoxycarbonyl, ethoxycarbonyl, n-propoxycarbonyl, isopropoxycarbonyl, n-butoxycarbonyl, sec-butoxycarbonyl, tert-butoxycarbonyl, and isobutoxycarbonyl. Examples of C1-6 alkoxycarbonyl include, but are not limited to, a C1-4 alkoxycarbonyl, n-pentyloxycarbonyl, isopentyloxycarbonyl, neopentyloxycarbonyl, tert-pentyloxycarbonyl, 1,2-dimethylpropyloxycarbonyl, and n-hexyloxycarbonyl.

As used herein, "alkoxycarbonylamino" is a monovalent group of -NH-C(=O)-O-alkyl. Examples of alkoxycarbonylamino include, but are not limited to, a C1-6 alkoxycarbonyl amino, preferably a C1-4 alkoxycarbonyl amino. Specific examples of C1-4 alkoxycarbonylamino include methoxycarbonylamino, ethoxycarbonylamino, n-propoxycarbonylamino, isopropoxycarbonylamino, n-butoxycarbonylamino, sec-butoxycarbonylamino, tert-butoxycarbonylamino, sec-butoxycarbonylamino, and isobutoxycarbonylamino. Specific examples of C1-6 alkoxycarbonylamino include a C1-4 alkoxycarbonylamino, n-pentyloxycarbonylamino, isopentyloxycarbonylamino, neopentyloxycarbonylamino, tert-pentyloxycarbonylamino, 1,2- dimethylpropyloxycarbonylamino, and n-hexyloxycarbonylamino.

As used herein, "C1-6 alkylsulfonyl" means a sulfonyl group substituted with "C1-6 alkyl" above. "C1-6 alkylsulfonyl" is preferably "C1-4 alkylsulfonyl". Examples of "C1-6 alkylsulfonyl" include, but are not limited to, methylsulfonyl, propylsulfonyl, and butylsulfonyl.

As used herein, the C1-6 alkyl moiety of "C1-6 alkylthio" is synonymous with the C1-6 alkyl above. Examples of "C1-6 alkylthio" include "C1-4 alkylthio", preferably "C1-3 alkylthio". Examples of "C1-6 alkylthio" include, but are not limited to, methylthio, ethylthio, propylthio, butylthio, isopropylthio, isobutylthio, tert-butylthio, sec-butylthio, isopentylthio, neopentylthio, tert-pentylthio, and 1,2-dimethylpropylthio.

As used herein, "arylcarbonyl" is a monovalent group of -C(=O)-aryl. Preferred examples of arylcarbonyl include a C6-10 arylcarbonyl. Examples of C6-10 arylcarbonyl include, but are not limited to, benzoyl (i.e., phenyl-C(=O)-), 1-naphthylcarbonyl, and 2-naphthylcarbonyl.

As used herein, the C6-10 aryl moiety of "C6-10 aryloxy" is synonymous with the C6-10 aryl above. "C6-10 aryloxy" is preferably "C6 or C10 aryloxy". Examples of "C6-10 aryloxy group" include, but are not limited to, phenoxy, 1-naphthyloxy, and 2-naphthyloxy groups.

As used herein, "heteroarylcarbonyl" is a monovalent group of -C(=O)-heteroaryl.

As used herein, "5- or 6-membered heteroarylcarbonyl group" means a carbonyl group substituted with "5- or 6-membered heteroaryl" above. Specific examples of "5- or 6-membered heteroarylcarbonyl group" include, but are not limited to, pyrazoylcarbonyl, triazoylcarbonyl, thiazoylcarbonyl, thiadiazoylcarbonyl, pyridylcarbonyl, and pyridazoylcarbonyl groups.

As used herein, the 5- or 6-membered heteroaryl moiety of "5- or 6-membered heteroaryloxy group" is synonymous with "5-membered heteroaryl" or "6-membered heteroaryl" above. Specific examples of 5- or 6-membered heteroaryloxy group include, but are not limited to, pyrazoyloxy, triazoyloxy, thiazoyloxy, thiadiazoyloxy, pyridyloxy and pyridazoyloxy groups.

As used herein, the 5- or 6-membered heteroaryl moiety of "5- or 6-membered heteroarylthio group" is synonymous with "5-membered heteroaryl" or "6-membered heteroaryl" above. Specific examples of "5- or 6-membered heteroarylthio groups include, but are not limited to, pyrazoylthio, triazoylthio, thiazoylthio, thiadiazoylthio, pyridylthio, and pyridazoylthio groups.

As used herein, "5- or 6-membered heteroaryl sulfonyl group" means a sulfonyl group substituted with the "5- or 6-membered heteroaryl" above. Specific examples of "5- or 6-membered heteroarylsulfonyl group" include, but are not limited to, pyrazoylsulfonyl, triazoylsulfonyl, thiazoylsulfonyl, thiadiazoylsulfonyl, pyridylsulfonyl and pyridazoylsulfonyl groups.

As used herein, "carbamoyl" is a monovalent group of - C(=O)-NH₂.

As used herein, "amidinoamino" is a monovalent group of -NH-C(=NH)-NH₂.

As used herein, the description "a group substituted with a substituent" means that the group is substituted with at least one substituent. For example, "a hydroxy-substituted C1-6 alkyl" means that the C1-6 alkyl is substituted with at least one hydroxy.

As used herein, "a carbamoyl-substituted C1-6 alkyl" is a C1-6 alkyl substituted with at least one -C(=O)-NH₂ group. Examples of "carbamoyl-substituted C1-6 alkyl" include, but are not limited to, a C1-4 alkyl substituted with carbamoyl. Specific examples of "carbamoyl-substituted C1-4 alkyl" include 2-amino-2-oxoethyl (i.e., H₂NC(=O)-CH₂- or carbamoylmethyl), 3-amino-3-oxopropyl (i.e., H₂NC(=O)-CH₂CH₂-, or carbamoylethyl), 4-amino-4-oxobutyl (i.e., H₂NC(=O)-(CH₂)₃-, or carbamoylpropyl), and 5-amino-5-oxopentyl (i.e., H₂NC(=O)-(CH₂)₄-, or carbamoylbutyl). Examples of "carbamoyl-substituted C1-6 alkyl" include, but are not limited to, a C1-4 alkyl substituted with carbamoyl, 6-amino-6-oxohexyl (i.e., H₂NC(=O)-(CH₂)₅-, or carbamoylpentyl), and 7-amino-7-oxoheptyl (i.e., H₂NC(=O)-(CH₂)₆-, or carbamoylhexyl).

As used herein, "amidinoamino-substituted C1-6 alkyl" is a C1-6 alkyl substituted with at least one -NH-C(=NH)-NH₂ group, wherein the nitrogen atom of the amidinoamino group may be protected with a nitrogen-protecting group (e.g. tert-butoxycarbonyl group). Examples of "amidinoamino-substituted C1-6 alkyl" include, but are not limited to, an amidinoamino-substituted C1-4 alkyl. Examples of "amidinoamino-substituted C1-4 alkyl" include, but are not limited to, (aminoamino)methyl, 2-(amidinoamino)ethyl, 3-(amidinoamino)propyl, and 4-(amidinoamino)butyl. Examples of "amidinoamino-substituted C1-6 alkyl" include, but are not limited to, an amidinoamino-substituted C1-4 alkyl, 5-(amidinoamino)pentyl and 6-(amidinoamino)hexyl.

As used herein, "carboxy-substituted C1-6 alkyl" is a C1-6 alkyl substituted with at least one -COOH group. Examples of "carboxy-substituted C1-6 alkyl" include, but are not limited to, "carboxy-substituted C1-4 alkyl". Examples of "carboxy-substituted C1-4 alkyl" include, but are not limited to, carboxymethyl, 2-carboxyethyl, 3-carboxypropyl, and 4-carboxybutyl. Examples of "carboxy-substituted C1-6 alkyl" include, but are not limited to, a carboxy-substituted C1-4 alkyl, 5-carboxypentyl, and 6-carboxyhexyl.

As used herein, "unsubstituted carbonyl" in "unsubstituted or substituted carbonyl" means a carboxylic acid group, and "substituted carbonyl" means an ester of a carboxy or an amide thereof, a hydrocarbonyl group, an optionally substituted lower alkylcarbonyl group, an optionally substituted arylcarbonyl group, and the like.

As used herein, "unsubstituted sulfonyl" in "unsubstituted or substituted sulfonyl" means a sulfonic acid group, and "substituted sulfonyl" means an ester of a sulfonic acid group or an amide thereof, a hydrosulfonyl group, an optionally substituted lower alkylsulfonyl group, an optionally substituted arylsulfonyl group, and the like.

As used herein, "unsubstituted sulfinyl" in "unsubstituted or substituted sulfinyl" means a sulfinic acid group, and "substituted sulfinyl" means an ester of a sulfinic acid group or an amide thereof, a hydrosulfinyl group, an optionally substituted lower alkylsulfinyl group, an optionally substituted arylsulfinyl group, and the like.

As used herein, "unsubstituted acyl" in "unsubstituted or substituted acyl" means an acyl group such as an esterified carboxy, and "substituted acyl" means a carbamoyl, an optionally substituted lower alkylcarbamoyl, an optionally substituted lower alkanoyl, an aroyl, an optionally substituted heteroarylcarbonyl, and the like. Specific exmaples include trifluoroacetyl.

As used herein, "unsubstituted thioacyl" in "unsubstituted or substituted thioacyl" means thioacyl and "substituted thioacyl"

"Protecting group" means to a group of atoms that, when bound to a reactive functional group in a molecule, shield, reduce, or prevent the reactivity of the functional group. Typically, the protecting group can be selectively removed during the synthetic process if desired. Exmaples of the protecting group may be described in Peter G. M. Wuts, "Greene's Protecting groups in Organic Synthesis", 5th Ed., John Wiley & Sons, Inc, Hoboken, New Jersey (2014) and Harrison, et al., Compendium of Synthetic Organic Methods, 1-8 vol., John Wiley & Sons, NY, and the like, As used herein, "protecting group" can fall under the definition of Substituent a. Typical exmaples of a protecting group for nitrogen include, but not limited to, formyl, acetyl, trifluoroacetyl, benzyl, benzyloxycarbonyl ("CBZ"), tert-butoxycarbonyl ("Boc"), trimethylsilyl ("TMS"), 2-trimethylsilylethanesulfonyl (" TES"), trityl and a substituted trityl group, allyloxycarbonyl, 9-fluorenylmethyloxycarbonyl ("FMOC"), and nitro-veratryloxycarbonyl ("NVOC"). Typical examples of a protecting group for hydroxyl include, but not limited to, those in which the hydroxyl group is acylated (esterified) or alkylated, such as benzyl and a trityl ether, as well as alkyl ethers, tetrahydropyranyl ethers, trialkylsilyl ethers (e.g., TMS, triethylsilyl, t-butyldimethylsilyl (TBDMS), triisopropylsilyl (TIPS)), alkylarylsilyl ethers (e.g., t-butyl diphenylsilyl (TBDPS)), triarylsilyl ethers (e.g., triphenylsilyl), glycol ethers (e.g., ethylene glycol ether, propylene glycol ether, and the like), and allyl ethers.

### <Preferred Embodiments>

The followings are descriptions of preferred embodiments of the present invention. The embodiments provided below are intended to offer a better understanding of the present invention and not to limit the scope of the invention to the following descriptions. Therefore, it is clear for those skilled in the art to make modifications as appropriate within the scope of the invention, taking into account the description herein.

### <Compounds and compositions of the present invention>

In an aspect, the present invention provides a compound represented by formula (I), its enantiomer, or a pharmaceutically acceptable salt thereof: 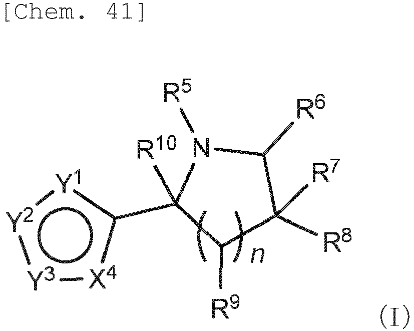 wherein
Y¹, Y², Y³ and X⁴ are each independently -O-, -N=, -S-, -NR¹-, or -CR²=;
   in which at least one of Y¹, Y², Y³ and X⁴ is -N= or -NR¹-;
   R¹ and R² are each independently an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl;
R⁵ is an optionally substituted C1-6 alkyl, an optionally substituted C6-10 aryl, an optionally substituted C6-10 heteroaryl, an unsubstituted or substituted carbonyl, an unsubstituted or substituted sulfonyl, an unsubstituted or substituted sulfinyl, an unsubstituted or substituted acyl, or an unsubstituted or substituted thioacyl;
R⁶, R⁷, R⁸, R⁹ and R¹⁰ are each independently hydrogen, an optionally substituted C1-6 alkyl, an optionally substituted C6-10 aryl, an optionally substituted C1-6 alkoxy, hydroxy, amino, cyano, carbamoyl or a halogen, or
R⁷ and R⁸ are cross-linked together with the carbon to which they are attached, to form a C3-6 spiro ring,
R⁶ and R⁹ are cross-linked together to form -CH₂- or - CH₂-CH₂-, or
R⁸ and R¹⁰ are cross-linked together to form -CH₂- or - CH₂-CH₂-;
n is an integer of 1 or 2;
in which the substituent in "optionally substituted" is selected from the following:
   hydroxy, a halogen, cyano, carbamoyl, amino, an amidinoamino, a carboxy, a C6-10 aryl, a C1-4 alkoxycarbonyl-substituted 5- to 10-membered heteroaryl, a C1-4 alkylsubstituted C6-10 aryl , a hydroxy-substituted C6-10 aryl, a halogen-substituted C6-10 aryl, a C1-4 alkoxy-substituted C6-10 aryl, a (an optionally substituted amino)-C6-10 aryl, a C1-4 alkoxycarbonyl, a C1-4 alkoxycarbonylamino, a 5- to 6-membered heterocycloalkyl, a C3-6 cycloalkyl, a 5- to 10-membered heteroaryl, a (a halogen-substituted C1-6 alkyl)-substituted C6-10 aryl, and a trialkylsilyloxy, an alkylarylsilyloxy, a triarylsilyloxy, or a protecting group;
   provided that, when the aromatic 5-membered ring comprising Y¹, Y², Y³ and X⁴ is a 1,2,3-triazole substituted with phenylmethyl, R⁵ is not tert-butoxycarbonyl.

In an embodiment of the present invention, R¹ and R² in formula (I) are preferably, each independently, an optionally substituted C1-6 alkyl, an optionally substituted C1-6 alkoxy, an optionally substituted C1-6 alkylthio, or an optionally substituted C1-6 alkylamino groups, wherein the substituent is each independently an optionally substituted C6-10 aryl or an optionally substituted C6-10 heteroaryl, and thus, R¹ and R² are more preferably, each independently, an optionally substituted C6-10 arylalkyl or an optionally substituted C6-10 heteroarylalkyl.

Compounds wherein the aromatic 5-membered ring comprising Y¹, Y², Y³ and X⁴ is a 1,2,3-triazole substituted with phenylmethyl and R⁵ is tert-butoxycarbonyl are described in Tetrahedron Asymmetry_2008_19_495-499. As described in Reference Example 1 herein, the reaction conditions are different from those described in the literature.

In a preferred embodiment, the present invention includes a compound represented by formula (I), wherein R¹ and R² are each independently a group of the formula: 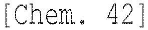 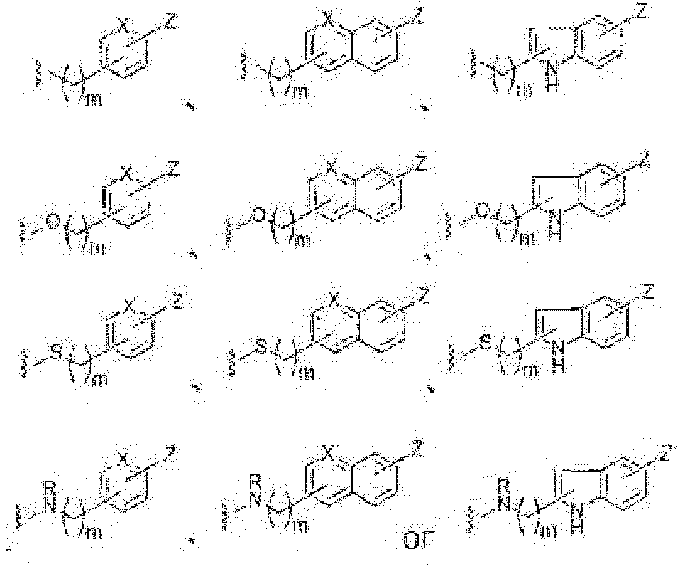 wherein
Z is hydrogen, amino, a halogen, hydroxy, cyano, carbamoyl, an optionally substituted C1-6 alkyl, or an optionally substituted C1-6 alkoxy; and
R is an optionally substituted C1-6 alkyl, or an optionally substituted C1-5 alkylcarbonyl;
X is N or C; and
m is an integer of 1 to 7.

In such aspect, preferred embodiments also include a compound represented by formula (I) wherein R¹ and R² are each independently a group of formula: 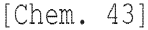 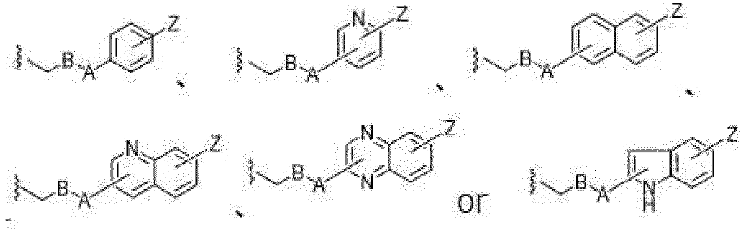 wherein
Z is hydrogen, amino, a halogen, hydroxy, cyano, carbamoyl, an optionally substituted C1-6 alkyl, or an optionally substituted C1-6 alkoxy; and
A is an unsubstituted carbonyl or an unsubstituted amino;
B is an unsubstituted carbonyl or an unsubstituted amino;
wherein A and B are not the same group at the same time; and the position of the substituent: -A-B-methylene- is not limited.

In another embodiment, the present invention provides a compound, its enantiomer, or a pharmaceutically acceptable salt thereof according to the present invention, wherein the compound is represented by formula (I): 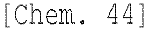 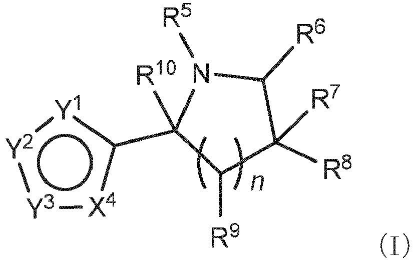 wherein
Y¹ is -O-, -N=, -S-, or -NR¹-;
Y² is -O-, -N=, -NR³-, or -CR²=;
Y³ is -N=, -NR⁴-, or -CR¹⁸=;
X⁴ is -N=, or -CH=;
   in which at least one of Y¹, Y², Y³ and X⁴ is -N=, -NR¹-, -NR³-, or -NR⁴-;
   R¹, R³ and R⁴ are each independently hydrogen, an optionally substituted C1-6 alkyl, an optionally substituted C6-10 aryl, or an optionally substituted C6-10 heteroaryl; and
   R² and R¹⁸ are each independently hydrogen, an optionally substituted C1-6 alkyl, C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl.

In another embodiment, the present invention provides a compound, its enantiomer, or a pharmaceutically acceptable salt thereof according to the present invention, wherein the compound is represented by formula (I): 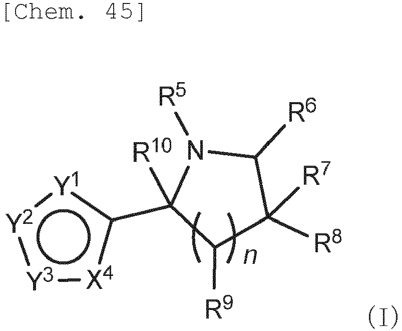 wherein
the group represented by the formula: 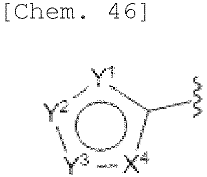 is the group represented by the formula: 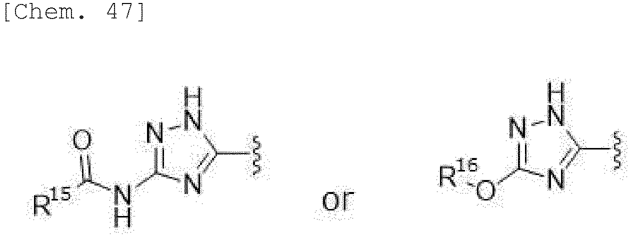 wherein
R¹⁵ is an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl; and
R¹⁶ is an optionally substituted C1-6 alkyl, an optionally substituted C6-10 aryl, or an optionally substituted C6-10 heteroaryl.

In another embodiment, the present invention provides a compound represented, its enantiomer, or a pharmaceutically acceptable salt thereof according to the present invention, wherein the compound is represented by formula (I): 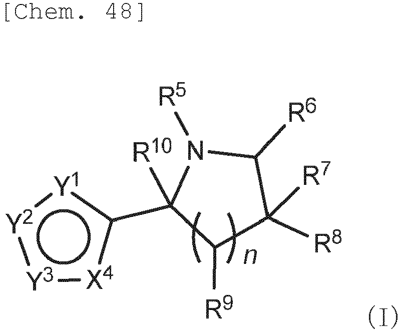 wherein
the group represented by the formula: 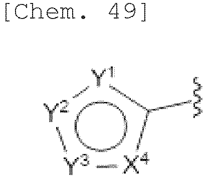 is the group represented by the formula: 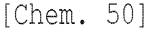 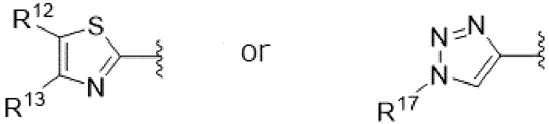 wherein
R¹² and R¹³ are each independently hydrogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl; and
R¹⁷ is an optionally substituted C1-6 alkyl, an optionally substituted C6-10 aryl, an optionally substituted C6-10 heteroaryl.

In another embodiment, the present invention provides a compound, its enantiomer, or a pharmaceutically acceptable salt thereof according to the present invention, wherein the compound is represented by formula (I): 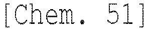 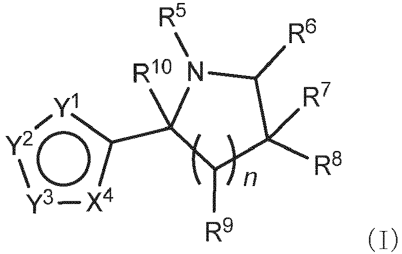 wherein
the group represented by the formula: 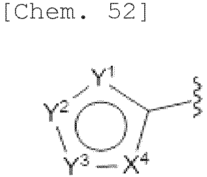 is the group represented by the formula: 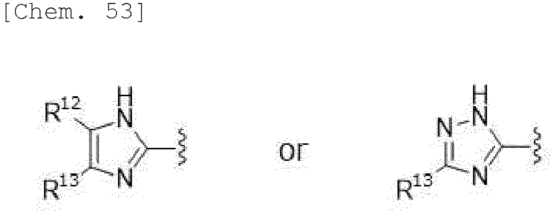 wherein
R¹² and R¹³ are independently hydrogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl.

In another embodiment, the present invention provides a compound, its enantiomer, or a pharmaceutically acceptable salt thereof according to the present invention, wherein the compound is represented by formula (I): 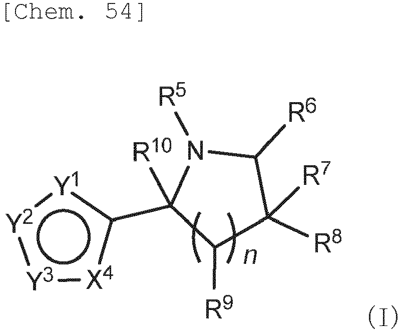 wherein
the group represented by the formula: 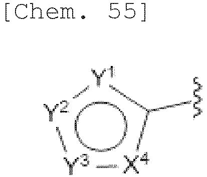 is the group represented by the formula: 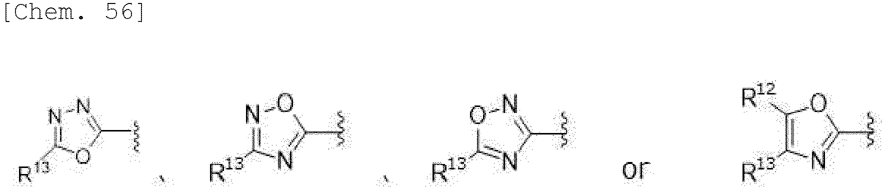 wherein
R¹² and R¹³ are each independently hydrogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl. In this regard, specific examples include a compound, its enantiomer, or a pharmaceutically acceptable salt thereof according to the present invention, wherein the group represented by the formula: 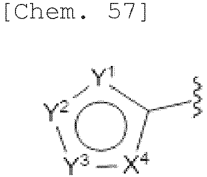 is the group represented by the is the group represented by the formula: 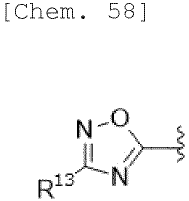 wherein
R¹³ is hydrogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl.

In another embodiment, the present invention provides a compound, its enantiomer, or a pharmaceutically acceptable salt thereof according to the present invention, wherein the compound is represented by formula (I): 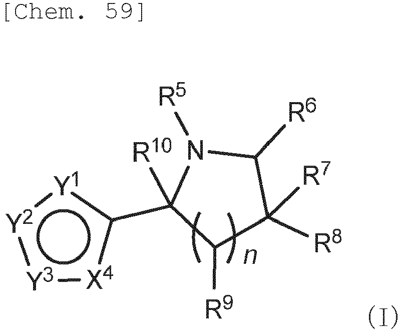 wherein
the group represented by the formula: 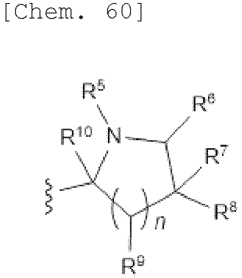 is the group represented by the formula: 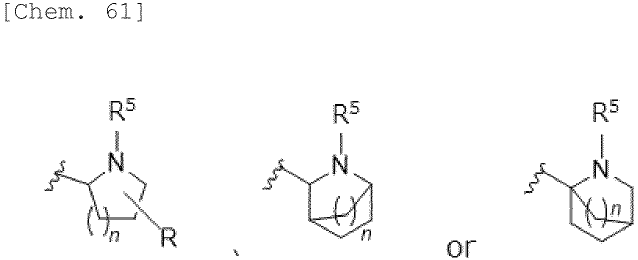 wherein
R is hydrogen, an optionally substituted C1-6 alkyl, an optionally substituted C6-10 aryl, an optionally substituted C1-6 alkoxy, hydroxy, amino, cyano, carbamoyl or a halogen;
R⁵ is an optionally substituted C1-6 alkyl, an optionally substituted C6-10 aryl, an optionally substituted C6-10 heteroaryl, an optionally substituted carbonyl, an optionally substituted sulfonyl, an optionally substituted sulfinyl, an optionally substituted acyl, or an optionally substituted thioacyl;
n is an integer of 1 or 2.

In another embodiment, the present invention provides a compound, its enantiomer, or a pharmaceutically acceptable salt thereof according to the present invention, wherein the compound is represented by formula (I): 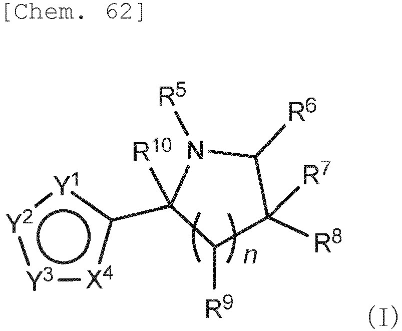 wherein
the group represented by the formula: 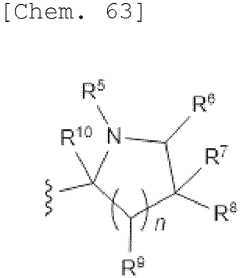 is the group represented by the formula: 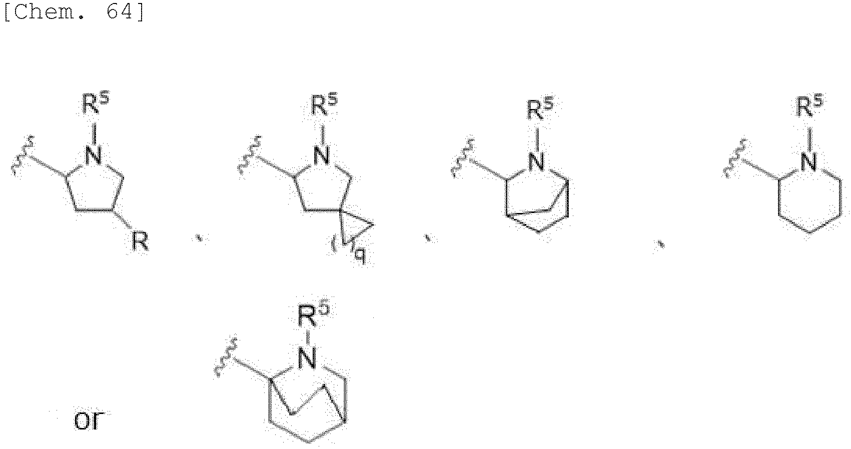 wherein
R is hydrogen, an optionally substituted C1-6 alkyl, an optionally substituted C6-10 aryl, an optionally substituted C1-6 alkoxy, hydroxy, amino, cyano, carbamoyl or a halogen;
R⁵ is an optionally substituted C1-6 alkyl, an optionally substituted C6-10 aryl, an optionally substituted C6-10 heteroaryl, an optionally substituted carbonyl, an optionally substituted sulfonyl, an optionally substituted sulfinyl, an optionally substituted acyl, or an optionally substituted thioacyl; and
q is an integer from 1 to 4. In this regard, specific examples include a compound, its enantiomer, or a pharmaceutically acceptable salt thereof according to the present invention, wherein the group represented by the formula: 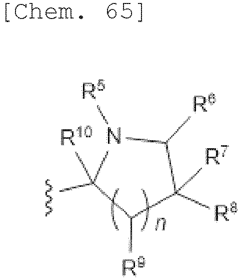 is the group represented by the formula: 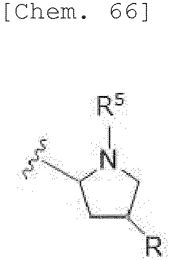 wherein
R is hydrogen, an optionally substituted C1-6 alkyl, an optionally substituted C6-10 aryl, an optionally substituted C1-6 alkoxy, hydroxy, amino, cyano, carbamoyl or a halogen;
R⁵ is an optionally substituted sulfonyl.

In another embodiment, the present invention provides a compound, its enantiomer, or a pharmaceutically acceptable salt thereof according to the present invention, wherein the compound is represented by formula (I): 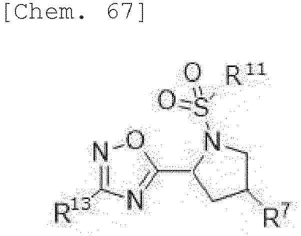 wherein
R¹³ is the formula: 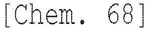 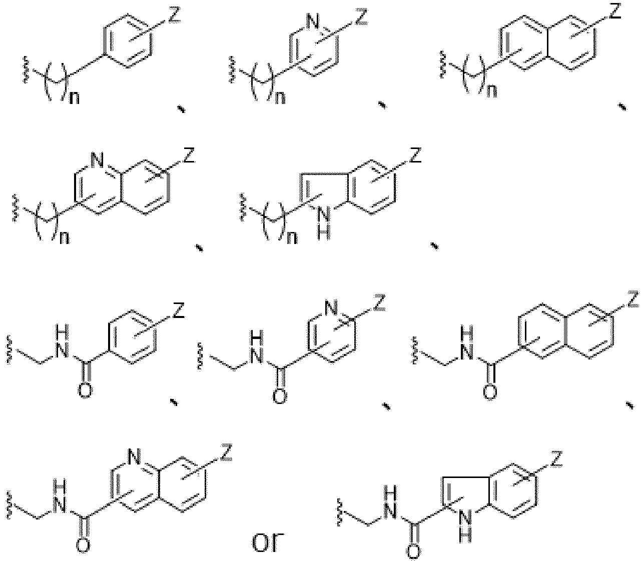 in which
   Z is hydrogen, amino, dimethylamino, a halogen, hydroxy, cyano, carbamoyl, an optionally substituted C1-6 alkyl, or an optionally substituted C1-6 alkoxy;
   n is an integer from 1 to 5;
R⁷ is the group represented by the formula: 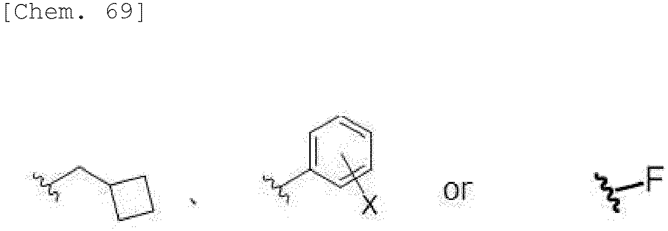
in which
   X is hydrogen, amino, a halogen, hydroxy, methoxy, or an optionally substituted C1-4 alkyl;
R¹¹ is methyl, or the group represented by the formula: 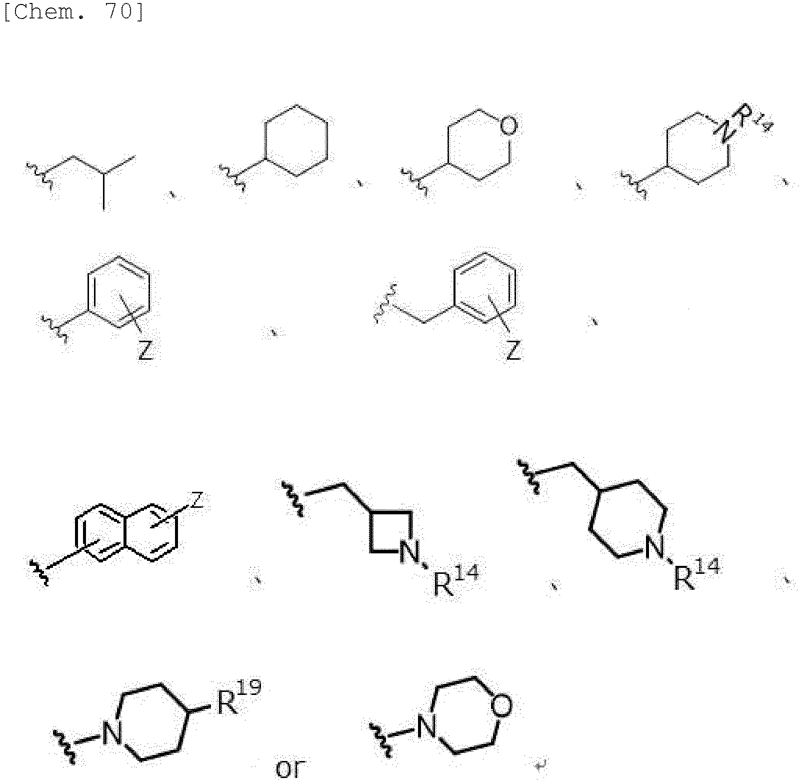 in which
   R¹⁴ is each independently hydrogen, an optionally substituted C1-6 alkyl, an optionally substituted C6-10 aryl, an optionally substituted C6-10 heteroaryl, an optionally substituted carbonyl, an optionally substituted sulfonyl, an optionally substituted sulfinyl, an optionally substituted acyl, or an optionally substituted thioacyl;
   R¹⁹ is hydrogen, an optionally substituted C1-6 alkyl, an optionally substituted C6-10 aryl, an optionally substituted carbonyl, hydroxy, an alkoxy, or an alkoxymethyl;
   2 is the same as defined above.

The detailed descriptions about the compounds of the present invention are further provided below.

The compounds of the invention exist in stereoisomers and optical isomers, such as tautomers and geometric isomers, depending on the type of substituent, and the present invention encompasses all of them, as well as mixtures of them. Specifically, the compounds of the invention exist in diastereomers and enantiomers when they contain one or more chiral carbon atoms, and the compounds of the invention encompass mixtures of these diastereomers and enantiomers, as well as those isolated from each ones.

In another embodiment, the present invention encompasses various hydrates, solvates and crystalline polymorphs.

In yet another embodiment, the present invention also encompasses prodrugs corresponding to the compounds of the invention. As used herein, prodrugs are derivatives that are degraded in vivo by acid hydrolysis or enzymatically to give compounds represented by formula (I). For example, in case that a compound represented by formula (I) has a hydroxy, amino or carboxy group, these groups may be modified according to conventional methods to make a prodrug. For example, C.-GWermuth, "The Practice of Medicinal Chemistry" , 4th Ed.,Academic Press, (2015), Chapter 28 describes prodrug technologies. Examples for compounds with a carboxy group includes those in which the carboxy is modified to an alkoxycarbonyl group, an alkylthiocarbonyl group, or an alkylaminocarbonyl group. Examples for compounds with an amino group include those in which the amino group is modified to an alkanoylamino group by substituting with a alkanoyl group, those in which the amino group is modified to an alkoxycarbonylamino group by substituting with an alkoxycarbonyl group, those with an alkanoyloxymethylamino group, or those with hydroxylamine. Examples for compounds with hydroxy include those in which the hydroxy is modified to an alkanoyloxy group by substituting with an aforementioned alkanoyl group, those with a phosphate ester, or those with an alkanoyloxymethyloxy group.

As used herein, "pharmaceutically acceptable salts" mean acid addition salts and base addition salts that are acceptable for pharmaceutical use. Specific examples of "pharmaceutically acceptable salts" include, but not limited to, acid addition salts such as acetate, propionate, butyrate, formate, trifluoroacetate, maleate, fumarate, tartrate, citrate, stearate, succinate, ethyl succinate, malonate, lactobionate, gluconate, glucoheptate, benzoate acid salt, methanesulfonate, benzenesulfonate, paratoluenesulfonate (tosylate), lauryl sulfate, malate, ascorbate, mandelate, saccharinate, xinafonate, pamoate, silicate, adipate, cysteine salt, N-acetylcysteine salt, hydrochloride, hydrochloride, hydrobromide, phosphate, sulfate, hydroiodide, nicotinate, oxalate, picric acid, thiocyanate, undecanoate, acrylic acid polymer salt, carboxyvinyl polymer, and the like; inorganic base addition salts such as lithium salt, sodium salt, potassium salt, calcium salt, and the like; organic base addition salts such as morpholine, piperidine, and the like; addition salts with amino acids such as aspartic acid, glutamic acid, and the like.

### <Preparation method for the compounds of the present invention>

The specific descriptions of general preparation methods for the compounds of the present invention are provided below together with examples, but those descriptions do not limit the invention in any aspect.

The compounds of the present invention can be prepared by the preparation methods described below. These preparation methods can be modified accordingly based on the knowledge of those skilled in synthetic organic chemistry. In the following preparation methods, the compounds used as raw materials may be used in the form of their salts, as long as the salts do not interfere with the reaction.

In the following preparation methods, even if the use of a protecting group is not specifically indicated, a functional group other than the reaction point can be protected as necessary and deprotected after the reaction is completed or a series of reactions is carried out to obtain a target compound, in case that any functional group other than the reaction point is changed under reaction conditions or that it is inappropriate to carry out post-reaction processing. The protecting groups as used in these processes can be found in the literature (Peter G. M. Wuts, "Greene's Protecting groups in Organic Synthesis", 5th Ed., John Wiley & Sons, Inc., Hoboken, New Jersey (2014), and the like. The introduction and removal of the protecting groups can be performed by methods commonly used in organic synthetic chemistry (e.g., methods described in the literature above) or by methods similar thereto.

The starting materials and the intermediates in the following preparation methods can be purchased commercially, or obtained by synthesis according to methods described in the public literature or known methods from known compounds. These starting materials and intermediates may also be used in their salts as long as they do not interfere with the reaction.

Intermediates and target compounds in the following preparation methods can also be converted to other compounds encompassed in the present invention by converting their functional groups as appropriate. The conversion of the functional groups in the process can be carried out by methods commonly used in synthetic organic chemistry (e.g., the method described in in RC. Larock, "Comprehensive Organic Transformations", 2nd Ed., John Wiley and Sons, Inc., New York (1999)) or by methods similar thereto.

An inert solvent in the following preparation method means a solvent that does not react with raw materials, reagents, bases, acids, catalysts, ligands, and the like as used in the reaction (hereinafter may be referred to as "raw materials and the like as used in the reaction"). Even if the solvents as used in each step react with the raw materials and the like as used in the reaction, they can be used as inert solvents as long as the desired reaction proceeds and the target compounds are obtained.

The compounds of the present invention are represented by heterocycloalkyl-substituted polyheteroazole derivatives. Thus, the preparation of the present compounds is based on heteroazole ring formation reactions on heterocycloalkyl compounds.

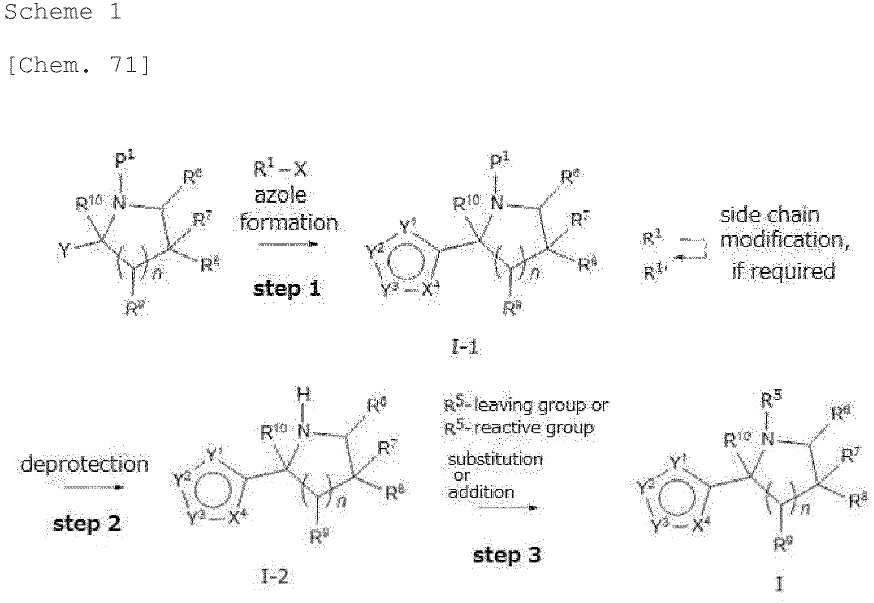

Alternatively, 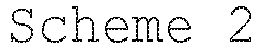 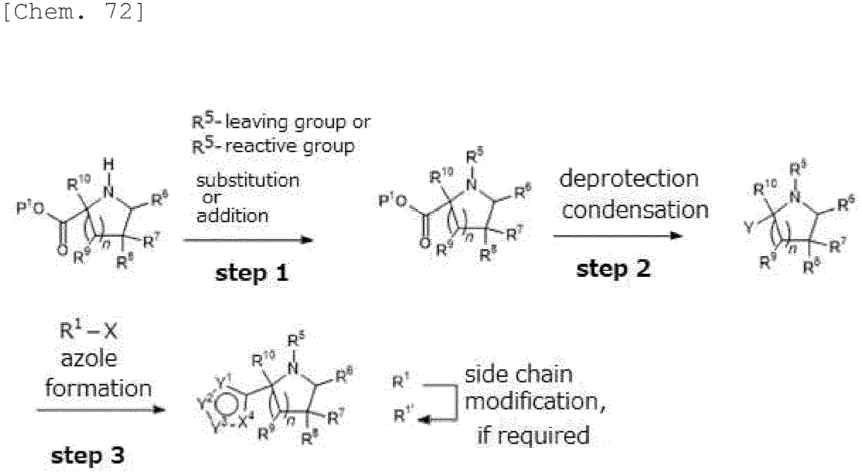 wherein
Y¹, Y², Y³ and X⁴ are each independently -O-, -N=, -S-, -NR¹-, -CR²=, -NR¹'-, or -CR²'=;
   in which at least one of Y¹, Y², Y³ and X⁴ is -N=, -NR¹- or -NR¹'-;
   R¹ and R¹' are hydrogen, an optionally substituted C1-6 alkyl, an optionally substituted C6-10 aryl, or an optionally substituted C6-10 heteroaryl;
   R² and R²' are hydrogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl;
R⁵ is an optionally substituted C1-6 alkyl, an optionally substituted C6-10 aryl, an optionally substituted C6-10 heteroaryl, an unsubstituted or substituted carbonyl, an unsubstituted or substituted sulfonyl, an unsubstituted or substituted sulfinyl, an unsubstituted or substituted acyl, or an unsubstituted or substituted thioacyl;
R⁶, R⁷, R⁸, R⁹ and R¹⁰ are each independently hydrogen, an optionally substituted C1-6 alkyl, an optionally substituted C6-10 aryl, an optionally substituted C1-6 alkoxy, hydroxy, amino, cyano, carbamoyl or a halogen, or
R⁷ and R⁸ are cross-linked together with the carbon to which they are attached, to form a C3-6 spiro ring,
R⁶ and R⁹ are cross-linked together to form -CH₂- or - CH₂-CH₂-, or
R⁸ and R¹⁰ are cross-linked together to form -CH₂- or - CH₂-CH₂-;
n is an integer of 1 or 2;
in which the substituent in "optionally substituted" is selected from the following:
   hydroxy, a halogen, cyano, carbamoyl, amino, an amidinoamino, a carboxy, a C6-10 aryl, a C1-4 alkoxycarbonyl-substituted 5- to 10-membered heteroaryl, a C1-4 alkylsubstituted C6-10 aryl , a hydroxy-substituted C6-10 aryl, a halogen-substituted C6-10 aryl, a C1-4 alkoxy-substituted C6-10 aryl, a (an optionally substituted amino)-C6-10 aryl, a C1-4 alkoxycarbonyl, a C1-4 alkoxycarbonylamino, a 5- to 6-membered heterocycloalkyl, a C3-6 cycloalkyl, a 5- to 10-membered heteroaryl, a (a halogen-substituted C1-6 alkyl)-substituted C6-10 aryl, and a trialkylsilyloxy, an alkylarylsilyloxy, a triarylsilyloxy, or a protecting group; and
   p¹ is a protecting group.

In some embodiments of the aspect, the present invention includes the following types:

### Table 1

**[Table 1]**

| Azole types | X | Y | | | | |
|---|---|---|---|---|---|---|
| 1.2,4. oxadiazole | -C(NOH)NH₂ | -COON | -O- | -N= | =CR¹- | -N= |
| 1.2.4. oxadiazole | -COOH | -C(NOH)NH₂ | =N- | -O- | -CR¹= | =N- |
| 1,2,4-oxadiazole | -CONNNH₂ | -COOH | =N- | -N= | =CR¹- | -O- |
| oxadiazole | -C(NH₂)-CR₂OH | -COOH | -O- | -CR²= | =CR¹- | -N= |
| 1*H*-imidazole | -C(NH₂)CR₂O | -COOH | -NH- | -CR²= | =CR¹- | -N= |
| thiazole | -C(NH₂)CR₂O | -COOH | -S- | -CR²= | =CR¹- | -N= |
| 1*H*-1,2,3-triazole | -N₃ | -C≡CH | -N= | =N- | -NR¹- | -CH= |
| 1*H*-1,2,4-triazole | -C(NH)OEt | -CONHNH₂ | =N- | -NH- | -CR¹= | =N- |

| | | | | | | |
|---|---|---|---|---|---|---|
| * In case that R¹ has a reactive functional group such as amine, alcohol, carboxylic acid, thiol, and the like, the side chain can be modified. (e.g., compound 11) | | | | | | |

### Step 1 of Scheme 1 and Step 3 of Scheme 2: Azole formation

In some embodiments of the aspect, the present invention includes the following reaction types:

### Table 2

**[Table 2]**

| | Reaction types of Azol formation |
|---|---|
| 1,2,4- oxadiazole | amidation and cyclodehydration |
| 1,2,4- oxadiazole | amidation and cyclodehydration |
| 1.2,4- oxadiazole | amidation and cyclodehydration |
| oxadiazole | amidation and oxidative aromatization |
| 1*H*- imidazole | amidation, ion formation and cyclodehydration |
| thiazole | amidation, thiocarbonylation and cyclodehyd ration |
| 1*H*-1,2,3- triazole | Huisgen cycloaddition |
| 1*H*=1,2,4- triazole | addition and cyclodehydration |

### Step 2 of Scheme 1 and Scheme 2: Deprotection

Step 2 comprises a deprotection reaction to remove protecting group P¹. The deprotection reaction is well known to those skilled in the art. De-tert-butoxycarbonyl reaction, and de-9-fluorenylmethyloxycarbonyl reaction are performed.

### Step 3 of Scheme 1 and Step 1 of Scheme 2: Substitution or addition

Step 3 of Scheme 1 and Step 1 of Scheme 2 comprises substitution (or addition) reactions to introduce R⁵. In the step, sulfinylation reactions, sulfonylation reactions, carbonylation reactions, thiocarbonylation reactions, reactions forming carbamates, acylation reactions, carbonation reactions, or alkylation reactions, all of which are well known to those skilled in the art, are performed.

The intermediates and target compounds in the above preparation methods can be isolated and purified by subjecting them to purification methods commonly used in organic synthetic chemistry (e.g., neutralization, filtration, extraction, washing, drying, concentration, recrystallization, various chromatographic techniques, and the like). Each intermediate can be also used in the next reaction without any particular purification.

Optically active compounds of the present invention can be prepared by using optically active starting materials or intermediates, or by optical resolution of racemic forms of intermediates or final products. Examples of methods for optical resolution include, but are not limited to, separation methods using optically active columns, and fractional crystallization methods. Diastereomers of the compounds of the present invention can be prepared by separation methods including, but are not limited to, column chromatography and fractional crystallization methods,.

Pharmaceutically acceptable salts of the compounds represented by Formula (I) can be prepared by mixing a compound represented by Formula (1) with a pharmaceutically acceptable acid or base in a solvent including, but are not limited to, water, methanol, ethanol, 2-propanol, ethyl acetate, or acetone.

### <Pharmaceutical compositions>

In one embodiment, the compounds of the present invention are those having an antiviral activity against RS virus. Specifically, the compounds of the present invention exhibit an inhibitory activity on RS virus proliferation, thereby enabling the treatment or prevention of RS virus infection (respiratory syncytial virus infection).

Accordingly, the present invention, in another aspect, provides a pharmaceutical composition comprising a compound, its enantiomer, or a pharmaceutically acceptable salt thereof according to the invention, and specifically a pharmaceutical composition comprising the same for treating or preventing RS virus infection.

RS virus, also called respiratory syncytial virus, occurs worldwide, and causes a respiratory tract infection that has no regional or climatic bias, and is universally endemic and recurrent. It is estimated that almost 100% of children are infected by the age of two, and the mortality rate in newborns is thought to be higher than that of influenza due to the high chance of infection. In fact, because there is no effective treatment for RS virus, approximately 30 million cases occur worldwide each year, and about 100,000 people die. Recent advances in diagnostic technology have made it possible to identify RS virus infection with a high degree of accuracy, and there is an urgent need to develop effective therapeutic pharmaceuticals.

As used herein, "treatment" means a method or process aimed at (1) delaying or preventing the onset of a disease or condition; (2) slowing down or halting the progression, aggravation or exacerbation of the onset of a disease or condition; (3) inducing remission of the onset of a disease or condition; or (4) facilitating the cure of a disease or condition. Treatment may be given as a prophylactic measure before the onset of the disease or the condition, or treatment may be given after the onset of the disease.

According to the present invention, "prevention" means a prophylactic action on a onset of RS virus infection.

According to the present invention, a pharmaceutical composition usually means a drug agent for treatment or prevention, or for examination/diagnosis for diseases or pathophysiology.

In one embodiment, the compounds of the invention can be administered by oral or parenteral administration as a formulation, medicine or pharmaceutical composition, either directly or by using an appropriate dosage form. Examples of these dosage forms include, but are not limited to, tablets, capsules, dispersions, granules, liquids, suspensions, injections, patches, and poultices. These formulations can be manufactured by known methods using additives that are used as ordinary pharmaceutical additives.

As these additives, excipients, disintegrants, binders, fluidizers, lubricants, coating agents, dissolving agents, dissolution aids, thickeners, dispersants, stabilizers, sweeteners, flavoring agents and the like can be used depending on the purpose. Specific examples of these additives include, but are not limited to, lactose, mannitol, crystalline cellulose, hydroxypropyl cellulose with low substitution degree, corn starch, partially pregelatinized starch, calcium carmellose, croscarmellose sodium, hydroxypropyl cellulose, hydroxypropyl methylcellulose, polyvinyl alcohol, magnesium stearate, sodium stearyl fumarate, polyethylene glycol, propylene glycol, titanium dioxide, and talc.

Dose of the compounds of the invention is appropriately selected according to the subject to be administered, the route of administration, the disease, and the subject's age, body weight and symptoms. For example, for oral administration, the lower limit is 0.01 mg (preferably 100 mg) and the upper limit is 1000 mg (preferably 6000 mg) per day for adults, and this dose can be administered once a day or divided into several doses.

In another aspect, the present invention relates to a method for treating or preventing RS virus infection, comprising administering to a subject in need of such treatment or the like a compound, its enantiomer, or a pharmaceutically acceptable salt thereof according to the present invention, preferably administering to such subject an effective amount of a compound, its enantiomer, or a pharmaceutically acceptable salt thereof according to the present invention.

Furthermore, in yet another aspect, the invention relates to a compound, its enantiomer, or a pharmaceutically acceptable salt thereof according to the present invention for treating or preventing RS virus infection.

In yet another aspect, the invention further relates to the use of a compound, its enantiomer, or a pharmaceutically acceptable salt thereof according to the present invention, for the preparation of a medicament for treating or preventing RS virus infection.

Patent documents such as a patent or a patent application and reference documents such as a non-patent literature including an academic literature, cited herein are incorporated herein by reference to the same extent as if they were each specifically disclosed in their entirety.

As described above, the preferred embodiments are shown to facilitate understanding of the invention. Hereinafter, the present invention will be further described in detail by way of the working examples, but it should be noted that these would not limit the scope of the present invention and merely illustrate the invention.

### EXAMPLES

### Example 1

### 3-(3-phenylpropyl)-5-[(2S)-1-tert-butoxycarbonylpyrrolidin-2-yl]-1,2,4-oxadiazole 1-1

### Example 1-1: N'-hydroxy-4-phenylbutanimidamide

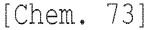

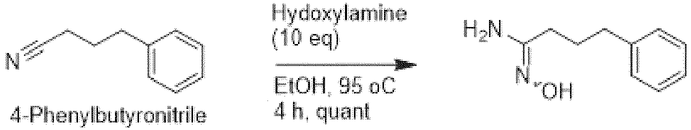

4-Phenylbutyronitrile (500 mg, 3.44 mmol) and 50% hydroxylamine aqueous solution (2.03 ml, 34.4 mmol) were added to an eggplant-shaped flask, and were dissolved in anhydrous ethanol (13.8 ml), followed by heating the solution to reflux at 95°C for 4 hours. After distilling off the solvent, the product was dried in vacuo to afford the title compound (oil, 614 mg, yield: 100%).

### Example 1-2: 3-(3-phenylpropyl)-5-[(2S)-1-tert-butoxy carbonylpyrrolidin-2-yl]-1,2,4-oxadiazole 1-1

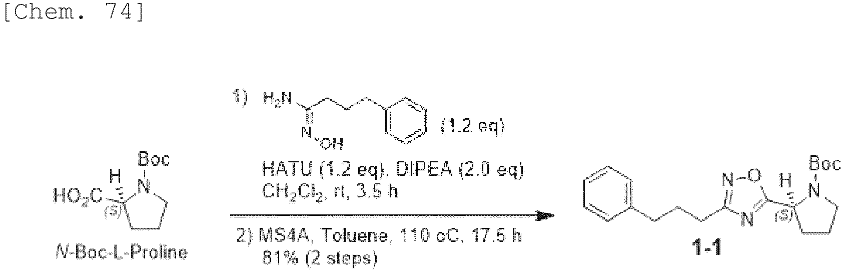

N-Boc-L-proline (100 mg, 0.465 mmol) was added to a 25 ml eggplant-shaped flask and was dissolved in dichloromethane (3.15 ml). HATU (1-[bis(dimethylamino) methylene]-1H-1,2,3-triazolo[4,5-b]pyridinium 3-oxidehexa fluorophosphate) (212 mg, 0.558 mmol) and diisopropyl ethylamine (0.162 ml, 0.929 mmol) were then added thereto and the mixture was stirred at room temperature under a nitrogen atmosphere for 10 minutes. Then, N'-hydroxy-4-phenylbutanimidamide (99 mg, 0.557 mmol) was added thereto while washing with dichloromethane (1.5 ml), and the mixture was stirred at room temperature for 3.5 hours.

After removing the stirrer bar and distilling off the solvent, the residue was purified on a silica gel column Q-Pac SI30 size 20 (Q-pack SI30 size 20) (hexane:ethyl acetate = 83:17 to 40:60). After distilling off the solvent, the imidamide intermediate was obtained as a mixture with a urea compound. The intermediate was added with pre-dried molecular sieve 4Å (MS4Å) (905 mg), and was then dissolved in an Aultra-dehydrated toluene (4.821 ml). The mixture was stirred at 110°C for 17.5 hours with a Dimroth condenser attached. After removing MS4Å by celite filtration, the solvent was distilled off and the resulting residue was purified on silica gel column Q-Pac SI30 size 20 (hexane:ethyl acetate = 91:9 to 66:34). After distilling off the solvent, 140 mg (0.391 mmol, 81% (after 2 steps)) of the desired compound 1-1 (oil) was obtained.

### Example 2

### 3-(3-phenylpropyl)-5-[(2S)-1-isobutylsulfonylpyrrolidin-2-yl]-1,2,4-oxadiazole 1A

### Example 2-1: 3-(3-phenylpropyl)-5-[(2S)-pyrrolidin-2-yl]-1,2,4-oxadiazole TFA salt 1-2

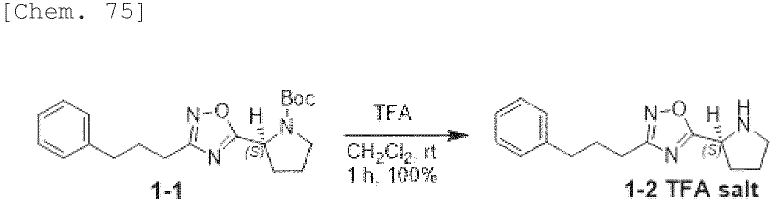

Compound 1-1 (115 mg, 0.324 mmol) prepared in Example 1 was added to a 10 ml eggplant-shaped flask, and dichloromethane (0.85 ml) and TFA (0.15 ml) were added thereto, followed by stirring the mixture at room temperature for 1 hour. After removing the stirrer bar and distilling off the solvent, the process of dissolving the residue in chloroform (1 ml) and conducting the distillation was repeated three times to remove the TFA. Then, the residue was dried in vacuo for 3 hours to afford the desired compound 1-2 (amorphous solid) as a TFA salt 120 mg (0.324 mmol, 100%).

### Example 2-2: 3-(3-phenylpropyl)-5-[(2S)-pyrrolidin-2-yl]-1,2,4-oxadiazole 1-2

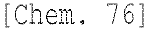

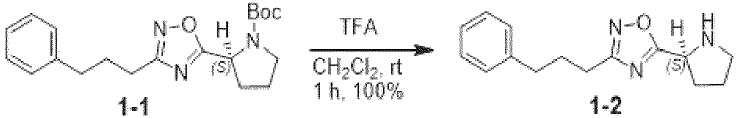

Compound 1-1 (323 mg, 0.903 mmol) prepared in Example 1 was added to a 25 ml eggplant-shaped flask, dichloromethane (2.37 ml) and TFA (0.418 ml) were added therein and the mixture was stirred at room temperature for 1 hour. After removing the stirrer bar and distilling off the solvent, saturated sodium bicarbonate aqueous solution (5 ml) was then added thereto to make it basic, and then the mixture was extracted with ethyl acetate. The solvent was distilled off and the resulting residue was purified on a silica gel column Q-Pac SI30 size 20 (hexane:ethyl acetate = 50:50 to 0:100). After distilling off the solvent, 127 mg (0.494 mmol, 55%) of the desired compound 1-2 (oil) was obtained.

### Example 2-3: 3-(3-phenylpropyl)-5-[(2S)-1-isobutylsulfonyl pyrrolidin-2-yl]-1,2,4-oxadiazole 1A

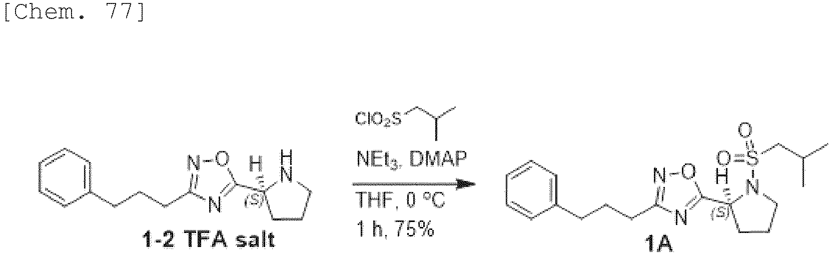

To a 10 ml eggplant-shaped flask, the TFA salt (22 mg, 0.059 mmol) of compound 1-2 prepared in Example 2-1 was added, then ultra-dehydrated THF (0.916 ml), triethylamine (0.051 ml, 0.366 mmol) and DMAP (2.2 mg, 0.018 mmol) were added thereto sequentially, and the mixture was cooled to 0°C, which was added with isobutylsulfonyl chloride (0.0185 ml, 0.137 mmol), followed by stirring the mixture at 0°C for 1 hour. The reaction was quenched by adding 0.1 M hydrochloric acid aqueous solution (1 ml) and the mixture was extracted with ethyl acetate (1 ml x 2). After distilling off the solvent, the residue was purified on a silica gel column Q-Pac SI20 size 10 (hexane:ethyl acetate = 90:10 to 66:34. After distilling off the solvent, 16.7 mg (0.044 mmol, 75% (after 2 steps)) of the desired compound 1A (oil) was obtained.

### Example 3

### 3-(3-phenylpropyl)-5-[(2S)-1-cyclohexylsulfonylpyrrolidin-2-yl]-1,2,4-oxadiazole 1B

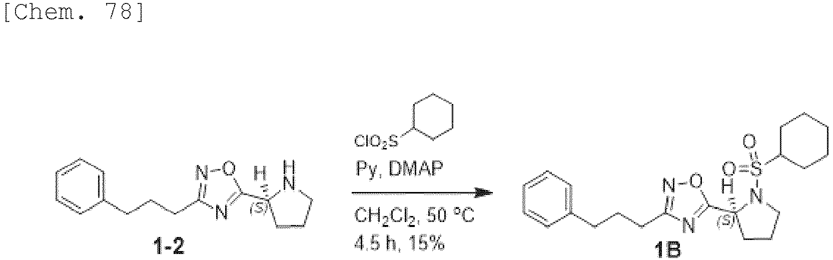

To a 10 ml eggplant-shaped flask, compound 1-2 (16.5 mg, 0.064 mmol) prepared in Example 2-2 was added, and then CH₂Cl₂ (0.916 ml), pyridine (20.7 pl, 0.256 mmol), cyclohexanesulfonyl chloride (20.7 pl, 0.128 mmol) and DMAP (2.4 mg, 19 pmol) were added sequentially thereto, followed by stirring the mixture at 50°C for 4.5 hours. The reaction was quenched by adding 0.5 M hydrochloric acid aqueous solution (0.8 ml) and the mixture was extracted with ethyl acetate (1 ml x 2). After the extract was dried over magnesium sulfate, which was then filtered out, followed by distilling off the solvent, the residue was purified on a silica gel column Q-Pac SI20 size 10 (hexane:ethyl acetate = 90:10 to 66:34). After distilling off the solvent, 3.8 mg (9.4 µmol, 15%) of the desired compound 1B (oil) was obtained.

### Examples 4 and 5

### 3-(3-phenylpropyl)-5-[(2S)-1-methanesulfonylpyrrolidin-2-yl]-1,2,4-oxadiazole 1C, and

### 3-(3-phenylpropyl)-5-[(2S)-1-trifluoroacetylpyrrolidin-2-yl]-1,2,4-oxadiazole 1C'

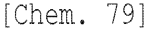

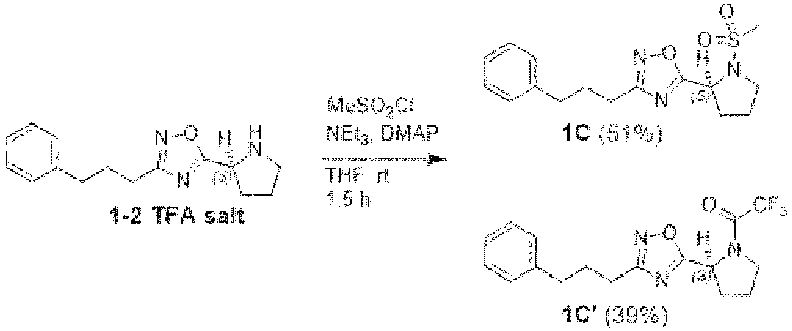

To a 10 ml eggplant-shaped flask, the TFA salt (20 mg, 0.054 mmol) of compound 1-2 prepared in Example 2-1 was added, and then ultra-dehydrated THF (1.08 ml), triethylamine (67.5 pl, 0.487 mmol) and DMAP (2.6 mg, 22 µmol) sequentially and finally methanesulfonyl chloride (12.7 pl, 0.162 mmol) were added thereto, followed by stirring the mixture at room temperature for 1.5 hours. The reaction was quenched by adding 0.5 M hydrochloric acid aqueous solution (1.2 ml) and the mixture was extracted with ethyl acetate (1 ml x 2). After distilling off the solvent, the residue was purified on a silica gel column Q-Pac SI20 size 10 (hexane: ethyl acetate = 90:10 to 50:50). After distilling off the solvent, 9.3 mg (27.7 µmol, 51%) of the desired compound 1C (oil) was obtained. Also, 7.4 mg (20.9 µmol, 39%) of compound 1C' (oil) was obtained as a byproduct.

### Example 6

### 3-(3-phenylpropyl)-5-[(2S)-1-benzenesulfonylpyrrolidin-2-yl]-1,2,4-oxadiazole 1D

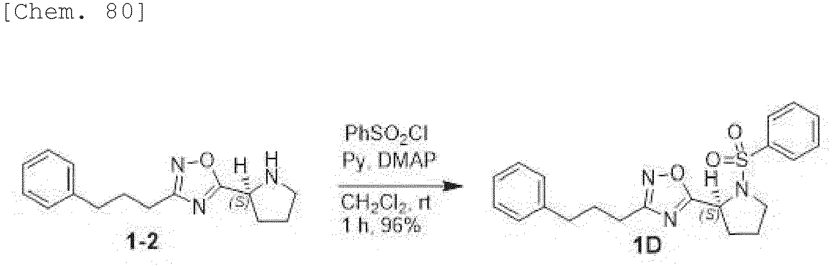

To a 10 ml eggplant-shaped flask, compound 1-2 (18.3 mg, 0.071 mmol) prepared in Example 2-2 was added, and CH₂Cl₂ (1.42 ml), pyridine (23.µl, 0.284 mmol), benzenesulfonyl chloride (18.4 µl, 0.142 mmol) and DMAP (2.6 mg, 21 pmol) were added thereto sequentially, followed by stirring the mixture at room temperature for 1 hour. The reaction was quenched by adding 0.5 M hydrochloric acid aqueous solution (0.8 ml) and the mixture was extracted with ethyl acetate (1 ml x2). After the extract was dried over magnesium sulfate, which was then filtered out, followed by distilling off the solvent, the residue was purified on a silica gel column Q-Pac SI20 size 10 (hexane:ethyl acetate = 90:10 to 70:30). After distilling off the solvent, 27.3 mg (68.7 µmol, 96%) of the desired compound 1D (oil) was obtained.

### Example 7

### 3-(3-phenylpropyl)-5-[(2S)-1-benzylsulfonylpyrrolidin-2-yl]-1,2,4-oxadiazole 1E

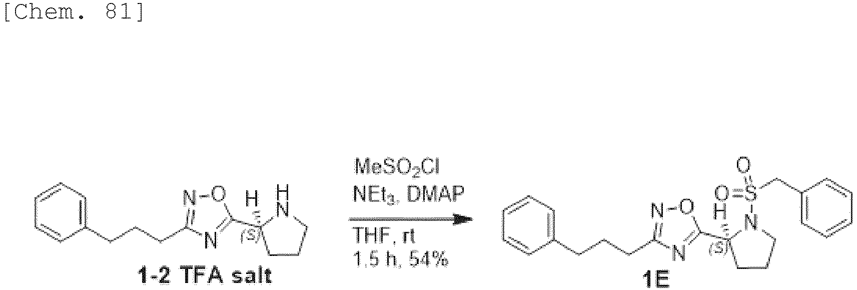

To a 4 ml vial was added, the TFA salt (20 mg, 0.055 mmol) of compound 1-2 prepared in Example 2-1 was added, then ultra-dehydrated THF (1.1 ml), triethylamine (68.9 pl, 0.497 mmol) and DMAP (2.7 mg, 22 µmol) were added sequentially, and finally benzylsulfonyl chloride (32.5 mg, 0.166 mmol) was added thereto, followed by stirring the mixture at room temperature for 1.5 hours. The reaction was quenched by adding 0.5 M hydrochloric acid aqueous solution (1.2 ml) and the mixture was extracted with ethyl acetate (1 ml x 2). After distilling off the solvent, the residue was purified on a silica gel column Q-Pac SI20 size 10 (hexane: ethyl acetate = 90:10 to 75:25). After distilling off the solvent, 12.2 mg (29.6 µmol, 54%) of the desired compound 1E (oil) was obtained.

The compounds prepared in Examples 1-7 are described in Table 3 along with their physical property data. In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acid-acetonitrile;
0-0.9 min linear gradient A:B (95:5)-A:B (10:90), 0.9-3 min A:B (10:90)

### Table 3

**[Table 3]**

| Ex | Cn | Chemical Structure | MW | ¹H-NMR δ ppm | [M + H] | RT :Min |
|---|---|---|---|---|---|---|
| 1 | 1-1 | 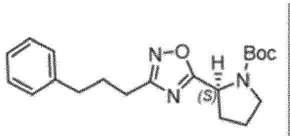 | 357.45 | 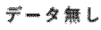 | 358 | 1.86 |
| 2 | 1A | 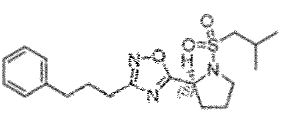 | 377.50 | (COCl3) δ: 7.32-7.16 (5H, m). 5.26 (1H, dd, J = 8.4, 3.2 Hz), 3.72-3.62 (1H, m), 3.55-3.45 (1H. m), 3.02-2.90 (2H, m), 2.80-2.67 (4H, m). 2.45-2.34 (1H, m), 2.30-2.01 (6H, m) 1.07 (3H. d. J = 5.8 Hz), 1.03 (3H, d, J = 6.8 Hz) | 378 | 1.77 |
| 3 | 1B | 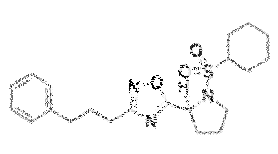 | 403.54 | (CDCl3) δ: 7.31-7.17 (5H, m), 5.30 (1H, dd, J = 8.4, 3.2 Hz), 3.78-3.71 (1H, m), 3.51-3.45 (1H, m), 2.96-2.86 (1H, m), 2.78-2.67 (4H. m), 2.45-2.33 (1H, m), 2.22-2.02 (7H, m), 1.86-1.79 (2H, m), 1.68-1.61 (1H, m), 1.52-1.39 (2H, m), 1.27-1.08 (3H, m) | 404 | 1.83 |
| 4 | 1C | 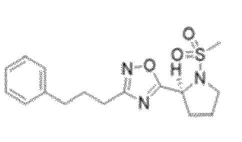 | 335.42 | (CDCl3) δ: 7.33-7.17 (5H, m), 5.23 (1H, dd, J = 8.4, 3.2 Hz), 3.68-3.60 (1H, m), 3.58-3.52 (1H, m), 2.98 (3H, s), 2.78-2.67 (4H, m), 2.47-2.36 (1H. m), 2.27-2.18 (1H, m), 2.17-2.03 (4H, m) | 336 | 1.64 |

**Table 3 continued**

| [Table 4] | | | | | | |
|---|---|---|---|---|---|---|
| 5 | 1C' | 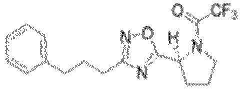 | 353.35 | (CDCl3) δ: 7.33-7.18 (5H, m). 5.11 (0.4H, d. J = 7.2 Hz), 4.98 (0.6H, dd, J = 8.0, 3.5 Hz) 3.72-3.41 (2H, m), 2.78-2.67 (4H. m), 2.42-2.25 (1H, m), 2.13-1.92 (5H, m) | - | - |
| 6 | 1D | 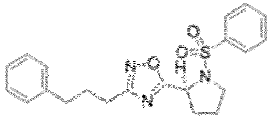 | 397.49 | (CDCl3) δ: 7.82 <2H, d. J - 6.8 Hz), 7.60-7.43 (3H. m), 7.32-7.17 (5H. m), 5.05 (1H, dd, J = 8.0, 3.6 Hz), 3.65-3.58 (1H, m), 3.50-3.45 (1H, m), 2.73-2.66 (4H, m), 2.21-2.00 (5H, m), 1.97-1.87 (1H. m) | 398 | 1.77 |
| 7 | 1E | 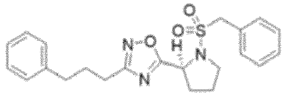 | 411.52 | (CDCl3) δ**:** 7.48-7.15 (10H, m), 5.12 (1H, dd, J = 8.4. 3.6 Hz). 4.41 (1H, d, J - 13.6 Hz), 4.31 (1H, d, J - 13.6 Hz), 3.39 (1H, dt, J = 9.6, 7.2 Hz). 3.07-3.01 (1H, m), 2.80-2.69 (4H, m), 2.33-2.23 (1H, m), 2.16-2.06 (3H, m), 2.03-1.92 (2H, m) | 412 | 1.77 |

| | | | | | | |
|---|---|---|---|---|---|---|
| Ex: Example Number Cn: Compound Number MW: Molecular Weight | | | | | | |

The following compounds can be prepared substantially according to the methods described in Examples 1-7.

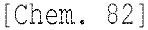

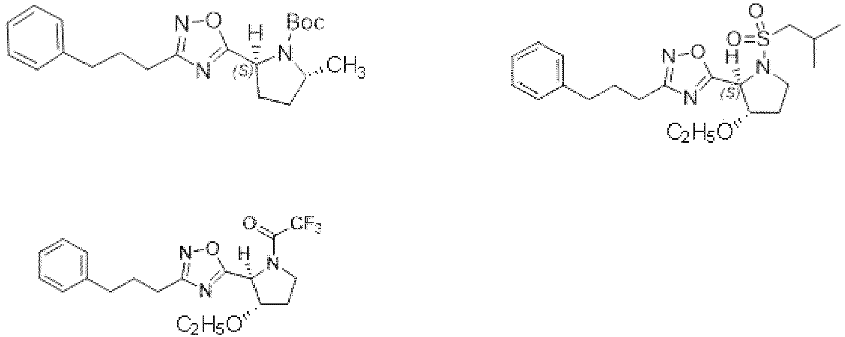

### Example 8

### 3-(3-phenylpropyl)-5-[(2S)-1-tert-butoxycarbonylpiperidin-2-yl]-1,2,4-oxadiazole 2-1

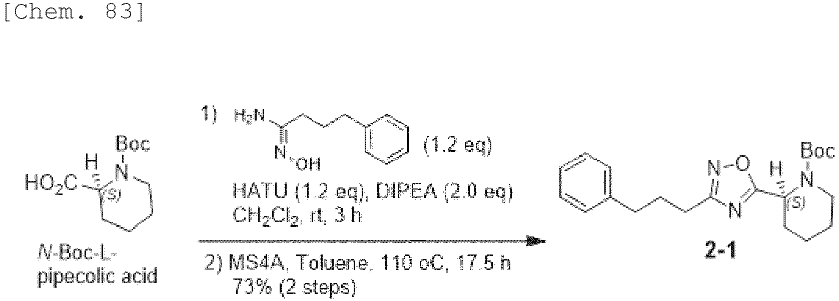

N-Boc-L-pipecolinic acid (100 mg, 0.436 mmol) was added to a 25 ml eggplant-shaped flask and was dissolved in dichloromethane (3.36 ml). HATU (199 mg, 0.523 mmol) and diisopropylethylamine (0.113 ml, 0.872 mmol) were then added thereto and the mixture was stirred for 10 minutes at room temperature under a nitrogen atmosphere. Then, N'-hydroxy-4-phenylbutanimidamide (93.3 mg, 0.523 mmol) was added while washing with dichloromethane (1.0 ml) and the mixture was stirred at room temperature for 3 hours.

After removing the stirrer bar and distilling off the solvent, the residue was purified on a silica gel column Q-Pac SI30 size 20 (hexane:ethyl acetate = 84:16 to 50:50). After distilling off the solvent, the imidamide intermediate was obtained. The intermediate was added with pre-dried MS4Å (850 mg), and was then dissolved in Aultra-dehydrated toluene (4.37 ml). The mixture was stirred at 110°C for 17.5 hours with a Dimroth condenser attached. After removing MS4Å by celite filtration, the solvent was distilled off and the resulting residue was purified on silica gel column Q-Pac SI30 size 20 (hexane:ethyl acetate = 93:7 to 83:17). After distilling off the solvent, 119 mg (0.320 mmol, 73% (after 2 steps)) of the desired compound 2-1 (oil) was obtained.

### Example 9

### 3-(3-phenylpropyl)-5-[(2S)-1-isobutylsulfonylpiperidin-2-yl]-1,2,4-oxadiazole 2A

### Example 9-1: 3-(3-phenylpropyl)-5-[(2S)-piperidin-2-yl]-1,2,4-oxadiazole TFA salt 2-2

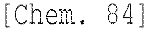

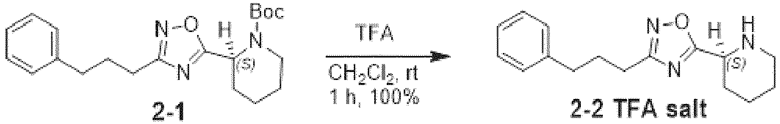

To a 10 ml eggplant-shaped flask, compound 2-1 (20.4 mg, 0.057 mmol) prepared in Example 8 was added, and dichloromethane (0.48 ml) and TFA (0.080 ml) were added thereto, followed by stirring the mixture at room temperature for 1 hour. After removing the stirrer bar and distilling off the solvent, the process of dissolving the residue in chloroform (1 ml) and conducting the distillation was repeated three times to remove the TFA. The product was then dried in vacuo for 3 hours to afford the desired compound 2-2 (white solid) as a TFA salt 22 mg (0.057 mmol, 100%) .

### Example 9-2: 3-(3-phenylpropyl)-5-[(2S)-piperidin-2-yl]-1,2,4-oxadiazole 2-2

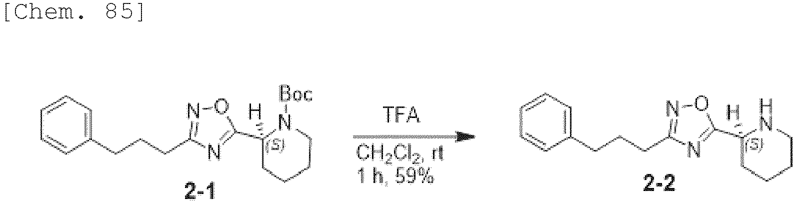

Compound 2-1 (224.8 mg, 0.605 mmol) prepared in Example 8 was added to a 25 ml eggplant-shaped flask, and dichloromethane (1.59 ml) and TFA (0.280 ml) were added thereto, followed by stirring the mixture at room temperature for 1 hour. After removing the stirrer bar and distilling off the solvent, saturated sodium bicarbonate aqueous solution (5 ml) was then added thereto to make it basic, and then the mixture was extracted with ethyl acetate. The solvent was distilled off and the resulting residue was purified on a silica gel column Q-Pac SI30 size 20 (hexane:ethyl acetate = 50:50 to 0:100). After distilling off the solvent, 96.9 mg (0.357 mmol, 59%) of the desired compound 2-2 (oil) was obtained.

### Example 9-3: 3-(3-phenylpropyl)-5-[(2S)-1-isobutylsulfonyl piperidin-2-yl]-1,2,4-oxadiazole 2A

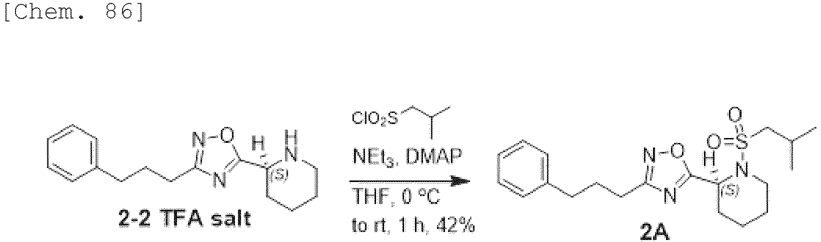

To a 10 ml eggplant-shaped flask, the TFA salt (22 mg, 0.057 mmol) of compound 2-2 prepared in Example 9-1 was added, then ultra-dehydrated THF (0.732 ml), triethylamine (45.7 pl, 0.329 mmol) and DMAP (1.3 mg, 0.011 mmol) were added thereto sequentially, and the mixture was cooled to 0°C, which was added with isobutylsulfonyl chloride (14.8 µl, 0.110 mmol), followed by stirring the mixture for 30 minutes at 0°C, and then at room temperature for 1 hour. The reaction was quenched by adding 0.1 M hydrochloric acid aqueous solution (1 ml) and the mixture was extracted with ethyl acetate (1 ml x 2). After the extract was dried over magnesium sulfate, which was then filtered out, followed by distilling off the solvent, the residue was purified by preparative TLC (hexane:ethyl acetate = 83:17). After washing the excised silica gel with ethyl acetate, the solvent was distilled off to afford 9.0 mg (0.023 mmol, 42%) of the desired compound 2A (oil).

### Example 10

### 3-(3-phenylpropyl)-5-[(2S)-1-cyclohexylsulfonylpiperidin-2-yl]-1,2,4-oxadiazole 2B

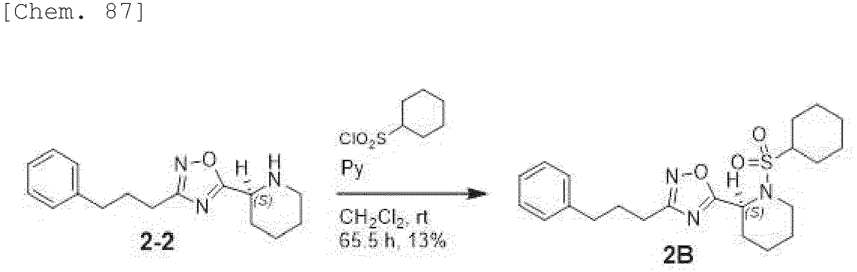

To a 10 ml eggplant-shaped flask, compound 2-2 (25 mg, 0.092 mmol) prepared in Example 9-2, CH₂Cl₂ (0.3 ml) and pyridine (29.7 pl, 0.369 mmol) were added sequentially, and the mixture was ice-cooled, which was added finally with cyclohexanesulfonyl chloride (35.4 mg, 0.184 mmol) while washing with CH₂Cl₂ (0.6 ml), followed by stirring the mixture at room temperature for 65.5 hours. The reaction was quenched by adding 0.5 M hydrochloric acid aqueous solution (1.2 ml) and the mixture was extracted with ethyl acetate (1 ml x 2). After the extract was dried over magnesium sulfate, which was then filtered out, followed by distilling off the solvent, the residue was purified on a silica gel column Q-Pac SI20 size 10 (hexane:ethyl acetate = 95:5 to 75:25). After distilling off the solvent, 4.9 mg (12.0 µmol, 13%) of the desired compound 2B (oil) was obtained.

### Example 11

### 3-(3-phenylpropyl)-5-[(2S)-1-methanesulfonylpiperidin-2-yl]-1,2,4-oxadiazole 2C

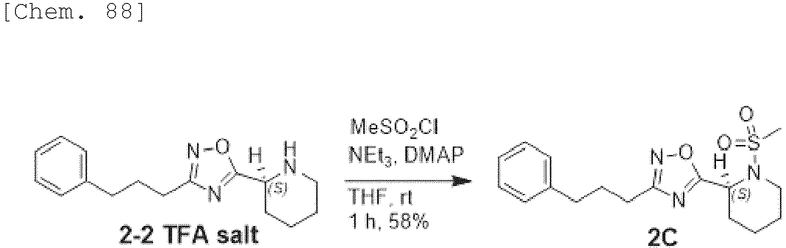

To a 4 ml vial, the TFA salt (21 mg, 0.055 mmol) of compound 2-2 prepared in Example 9-1 was added, then ultra-dehydrated THF (1.09 ml), triethylamine (68.0 pl, 0.490 mmol) and DMAP (2.7 mg, 22 µmol) were added sequentially, and finally methanesulfonyl chloride (12.8 pl, 0.163 mmol) was added thereto, followed by stirring the mixture at room temperature for 1 hour. The reaction was quenched by adding 0.5 M hydrochloric acid aqueous solution (1.2 ml) and the mixture was extracted with ethyl acetate (1 ml x 2). After the extract was dried over magnesium sulfate, which was then filtered out, followed by distilling off the solvent, the residue was purified on a silica gel column Q-Pac SI20 size 10 (hexane:ethyl acetate = 90:10 to 75:25). After distilling off the solvent, 11.1 mg (31.8 µmol, 58%) of the desired compound 2C (oil) was obtained.

### Example 12

### 3-(3-phenylpropyl)-5-[(2S)-1-benzenesulfonylpiperidin-2-yl]-1,2,4-oxadiazole 2D

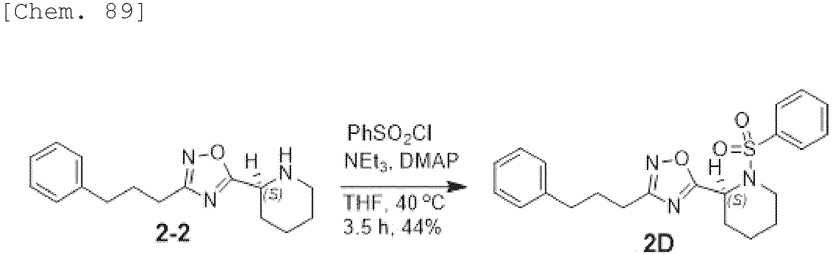

To a 4 ml vial, compound 2-2 (20.3 mg, 0.075 mmol) prepared in Example 9-2 was added, then ultra-dehydrated THF (0.997 ml), triethylamine (0.187 ml, 1.347 mmol) and DMAP (4.6 mg, 37 µmol) were added sequentially, and finally benzenesulfonyl chloride (29 pl, 0.224 mmol) was added thereto, followed by stirring the mixture at 40°C for 3.5 hours. The reaction was quenched by adding 0.5 M hydrochloric acid aqueous solution (1.2 ml) and the mixture was extracted with ethyl acetate (1 ml x 2). After the extract was dried over magnesium sulfate, which was then filtered out, followed by distilling off the solvent, the residue was purified on a silica gel column Q-Pac SI20 size 10 (hexane:ethyl acetate = 90:10 to 83:17). After distilling off the solvent, 13.7 mg (33.3 µmol, 44%) of the desired compound 2D (oil) was obtained.

### Example 13

### 3-(3-phenylpropyl)-5-[(2S)-1-benzylsulfonylpiperidin-2-ylJ-1,2,4-oxadiazole 2E

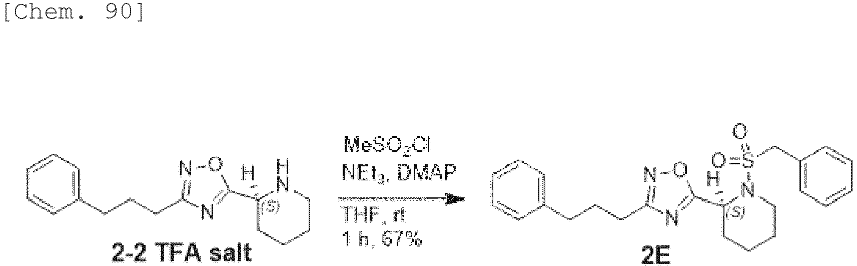

To a 4 ml vial, the TFA salt (21 mg, 0.055 mmol) of compound 2-2 prepared in Example 9-1 was added, then ultra-dehydrated THF (1.09 ml), triethylamine (68.0 µl, 0.490 mmol) and DMAP (2.7 mg, 22 µmol) were added sequentially, and finally benzylsulfonyl chloride (32.1 mg, 0.163 mmol) was added thereto, followed by stirring the mixture at room temperature for 1 hour. The reaction was quenched by adding 0.5 M hydrochloric acid aqueous solution (1.2 ml) and the mixture was extracted with ethyl acetate (1 ml x 2). After distilling off the solvent, the residue was purified on a silica gel column Q-Pac SI20 size 10 (hexane:ethyl acetate = 90:10 to 75:25). After distilling off the solvent, 15.5 mg (36.4 µmol, 67%) of the desired compound 2E (oil) was obtained.

The compounds prepared in Examples 8-13 are described in Table 4 along with their physical property data.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acid-acetonitrile;
0-0.9 min linear gradient A:B (95:5)-A:B (10:90), 0.9-3 min A:B (10:90)

**Table 4**

| [Table 5] | | | | | | |
|---|---|---|---|---|---|---|
| Ex | Cn | Chemical Structure | MW | ¹H-NMR & ppm | [[M+H] | RT :Min |
| 8 | 2-1 | 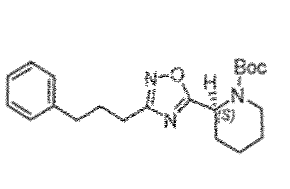 | 371.48 | 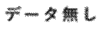 | 372 | 2.02 |
| 9 | 2A | 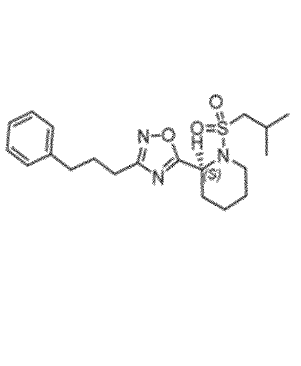 | 391.53 | (COCl3) δ: 7.32-7.15 (5H. m), 5.41 (1H, d, J - 4.8 Hz), 3.80 (1H, dt, J = 12.8, 1.2 Hz). 3.18 (1H, td, J = 12.8. 3.2 Hz), 2.98-2.88 (2H, m), 2.80-2.70 (4H. m), 2.34-2.20 (2H, m), 2.13-1.96 (3H, m), 1.83-1.57 (3H, m), 1.46-1.33 (1H, m), 1.08 (3H, d, J = 6.8 Hz). 1.06 (3H, d, J - 6.8 Hz) | 392 | 1.88 |
| 10 | 2B | 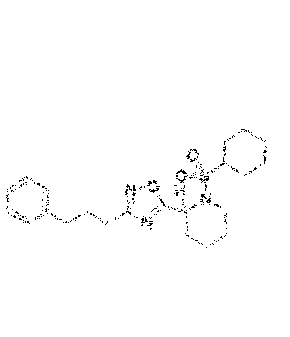 | 417.57 | (CDCI3) δ: 7.32-7.17 (5H, m), 5.34 (1H, d, J = 5.2 Hz), 3.82 (1H, br-d, J = 13.2 Hz), 3.27-3.17 (1H, m), 2.96 (1H, tt, J = 12.0, 3.2 Hz). 2.81-2.69 (4H, m), 2.30 (1H, dd, J = 14.0, 2.8 Hz), 2.20-1.96 (4H, m), 1.89-1.75 (3H, m), 1.69-1.38 (5H, m). 1.29-1.13 (2H, m), | 418 | 1.95 |

**Table 4 continued**

| [Table 6] | | | | | | |
|---|---|---|---|---|---|---|
| 11 | 2C | 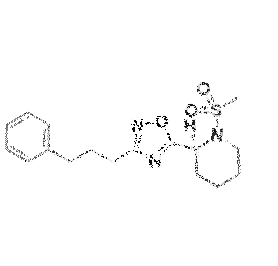 | 349.45 | (CDCl3) δ: 7.32-7.17 (5H, m), 5.44 (1H. d, J - 4.0 Hz), 3.81 (1H. dt, J = 12.4, 1.2 Hz), 3.17 (1H, td, J = 12.8, 3.2 Hz), 2.97 (3H, s). 2.79-2.68 (4H, m), 2.34 (1H, br-d, J = 14.0 Hz). 2.13-1.96 (3H, m), 1.82-1.60 (3H, m), 1.40-1.26 (1H, m) | 350 | 1.73 |
| 12 | 2D | 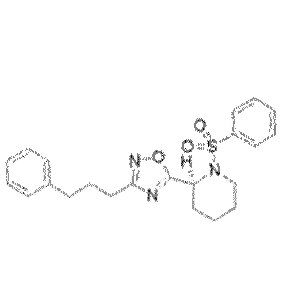 | 411.52 | (CDCl3) &: 7.67 (2H, d, J = 6.8 Hz). 7.41-7.13 (8H, m), 5.49 (1H, dd, J = 5.2, 2.0 Hz). 3.89 (1H, br-d, J = 12.8 Hz), 3.30 (1H, td, J (= 12.8, 3.2 Hz), 2.66-2.52 (4H, m), 2.10-1.86 (4H. m), 1.77-1.55 (3H, m). 1.51-1.37 (1H, m) | 412 | 1.87 |
| 13 | 2E | 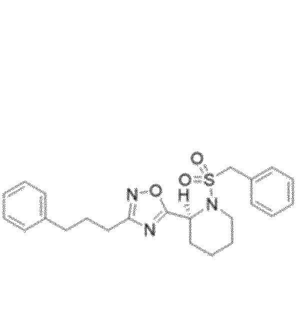 | 425.55 | (CDCl3) δ: 7.43-7.16 (10H, m), 5.30 (1H, br-d, J = 4.4 Hz), 4.37-4.27 (2H, m), 3.49 (1H. br-d. J = 13.2 Hz), 3.06 (1H, td, J = 12.8, 3.2 Hz), 2.82-2.70 (4H. m), 2.21 (1H, br-d. J = 12,4 Hz), 2.16-2.07 (2H, m), 1.92-1.81 (1H, m), 1.76-1.68 (1H, m), 1.62-1.43 (2H, m), 1.43-1.29 (1H, m) | 426 | 1.84 |

| | | | | | | |
|---|---|---|---|---|---|---|
| Ex: Example Number Cn: Compound Number MW: Molecular Weight | | | | | | |

The following compounds can be prepared substantially according to the methods described in Examples 8-13.

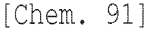

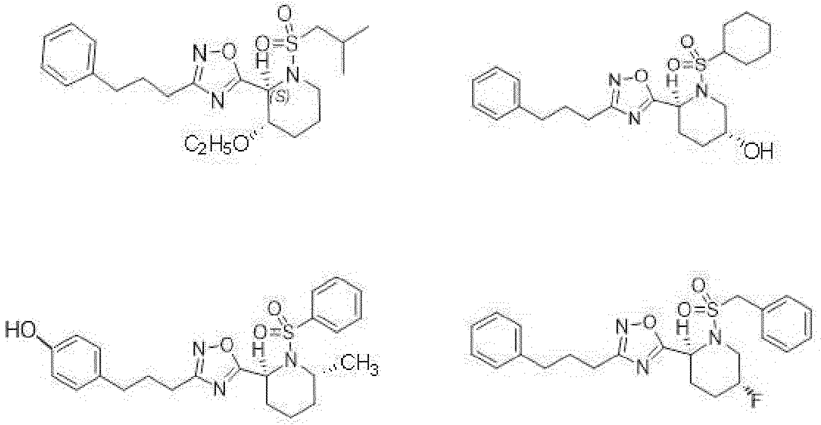

### Example 14

### 3-(3-phenylpropyl)-5-[(2S,4R)-1-tert-butoxycarbonyl-4-hydroxypyrrolidin-2-yl]-1,2,4-oxadiazole 3-1

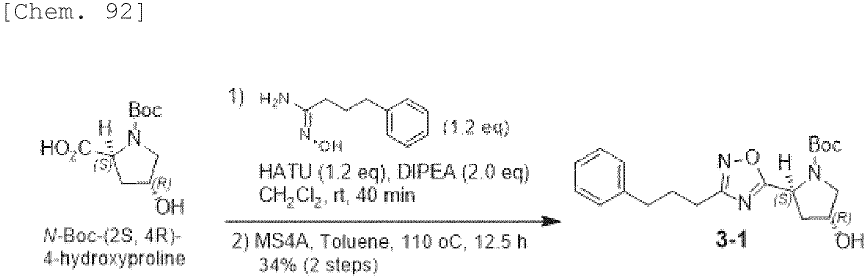

To a 50 ml eggplant-shaped flask, N-Boc-(2S,4R)-4-hydroxyproline (500 mg, 2.163 mmol) and N'-hydroxy-4-phenylbutanimidamide (463 mg, 2.595 mmol) were added while washing with dichloromethane (10.8 ml). Then, diisopropylethylamine (0.753 ml, 4.325 mmol) and HATU (987 mg, 2.595 mmol) were added thereto and the mixture was stirred for 40 minutes at room temperature under a nitrogen atmosphere. After removing the stirrer bar and distilling off the solvent, saturated sodium bicarbonate aqueous solution (5 ml) was then added thereto to make it basic, and then the mixture was extracted with ethyl acetate. The organic layer was washed with saturated brine, dried over magnesium sulfate, which was then filtered out, and the solvent was distilled off. The residue was purified on silica gel column Q-Pac SI30 size 60 (chloroform:methanol = 100:0 to 95:5) to afford the imidamide intermediate. The intermediate was added with pre-dried MS4Å (4.233 g), and was then dissolved in ultra-dehydrated toluene (10.8 ml). The mixture was stirred at 110°C for 12.5 hours with a Dimroth condenser attached. After removing MS4Å by celite filtration, the solvent was distilled off and the resulting residue was purified on a silica gel column Q-Pac SI30 size 60 (chloroform:methanol = 95:5). After distilling off the solvent, 278 mg (0.744 mmol, 34% (after 2 steps)) of the desired compound 3-1 (oil) was obtained.

### Example 15

### 3-(3-phenylpropyl)-5-[(2S,4R)-4-hydroxy-1-isobutylsulfonyl pyrrolidin-2-yl]-1,2,4-oxadiazole 3A

### Example 15-1: 3-(3-phenylpropyl)-5-[(2S,4R)-4-hydroxy pyrrolidin-2-yl]-1,2,4-oxadiazole 3-2

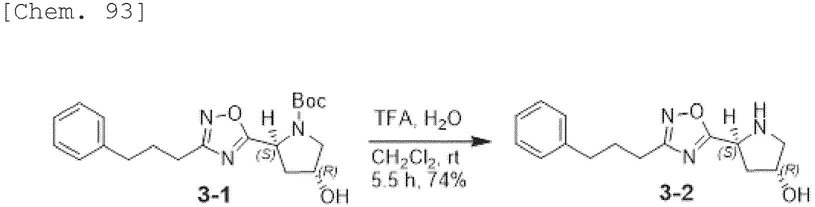

To a 25-ml eggplant-shaped flask, compound 3-1 (270 mg, 0.723 mmol) prepared in Example 14 was added, and then dichloromethane (1.90 ml), TFA (0.670 ml) and water (33.5 µl) were added thereto, followed by stirring the mixture at room temperature for 5.5 hours. After removing the stirrer bar and distilling off the solvent, saturated sodium bicarbonate aqueous solution (5 ml) was then added thereto to make it basic, and then the mixture was extracted with ethyl acetate. After the extract was dried over magnesium sulfate, which was then filtered out, followed by distilling off the solvent, the residue was purified on a silica gel column Q-Pac SI30 size 20 (chloroform:methanol = 100:0 to 90:10). After distilling off the solvent, 146 mg (0.535 mmol, 74%) of the desired compound 3-2 (white solid) was obtained.

### Example 15-2: 3-(3-phenylpropyl)-5-[(2S,4R)-4-hydroxy-1-isobutylsulfonylpyrrolidin-2-yl]-1,2,4-oxadiazole 3A

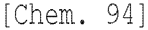

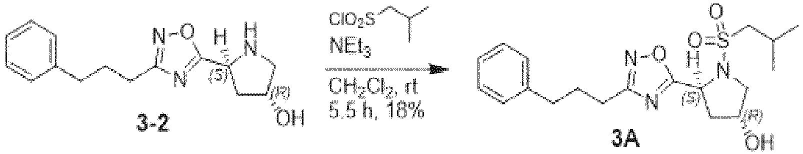

To a 10 ml eggplant-shaped flask, compound 3-2 (15.2 mg, 0.056 mmol) prepared in Example 15-1 was added, then dichloromethane (0.556 ml) and triethylamine (18.5 pl, 0.133 mmol) were added thereto, and the mixture was cooled to 0°C, which was added with isobutylsulfonyl chloride (8.8 µl, 0.067 mmol), followed by stirring the mixture at room temperature for 5.5 hours. The reaction was quenched by adding 0.25 M hydrochloric acid aqueous solution (1 ml) and the mixture was extracted with ethyl acetate (1 ml x 2). After the extract was dried over magnesium sulfate, which was then filtered out, followed by distilling off the solvent, the residue was purified by silica gel column Q-Pac SI20 size 10 (chloroform:methanol = 100:0 to 95:5) and preparative TLC (chloroform:methanol = 95:5). After distilling off the solvent, 4.0 mg (0.010 mmol, 18%) of the desired compound 3A (oil) was obtained.

### Example 16

### 3-(3-phenylpropyl)-5-[(2S,4R)-1-cyclohexylsulfonyl-4-hydroxypyrrolidin-2-yl]-1,2,4-oxadiazole 3B

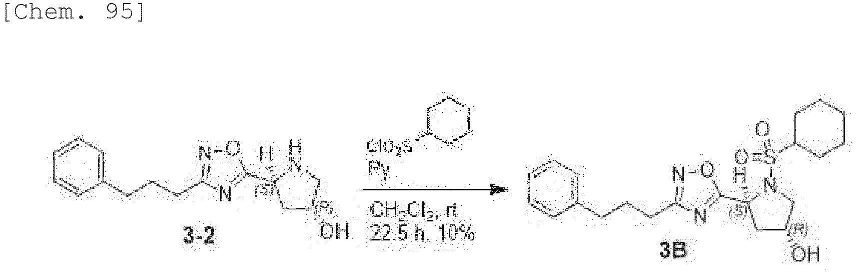

To a 10 ml eggplant-shaped flask, compound 3-2 (18.2 mg, 0.067 mmol) prepared in Example 15-1 was added, then CH₂Cl₂ (0.666 ml) and pyridine (12.9 µl, 0.160 mmol) were added thereto, and the mixture was cooled to 0°C, which was added with cyclohexanesulfonyl chloride (15.4 mg, 0.080 mmol), followed by stirring the mixture at room temperature for 22.5 hours. The reaction was quenched by adding 0.25 M hydrochloric acid aqueous solution (1.2 ml) and the mixture was extracted with ethyl acetate (1 ml x 3). After the extract was dried over magnesium sulfate, which was then filtered out, followed by distilling off the solvent, the residue was purified on a silica gel column Q-Pac SI20 size 10 (chloroform:methanol = 100:0 to 98:2). After distilling off the solvent, 2.7 mg (6.44 µmol, 10%) of the desired compound 3B (oil) was obtained.

### Example 17

### 3-(3-phenylpropyl)-5-[(2S,4R)-4-hydroxy-1-methanesulfonyl pyrrolidin-2-yl]-1,2,4-oxadiazole 3C

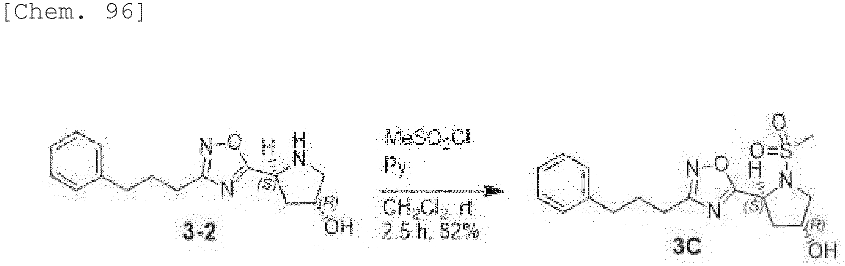

To a 10 ml eggplant-shaped flask, compound 3-2 (15.6 mg, 0.057 mmol) prepared in Example 15-1 was added, then CH₂Cl₂ (0.571 ml) and pyridine (11.1 pl, 0.137 mmol) were added thereto, and the mixture was cooled to 0°C, which was added with methanesulfonyl chloride (5.35 pl, 0.068 mmol), followed by stirring the mixture at room temperature for 2.5 hours. The reaction was quenched by adding 0.25 M hydrochloric acid aqueous solution (1.0 ml) and the mixture was extracted with ethyl acetate (1 ml x 2). After the extract was dried over magnesium sulfate, which was then filtered out, followed by distilling off the solvent, the residue was purified using preparative TLC (chloroform:methanol = 95:5). After distilling off the solvent, 16.4 mg (0.047 mmol, 82%) of the desired compound 3C (oil) was obtained.

### Example 18

### 3-(3-phenylpropyl)-5-[(2S,4R)-1-benzenesulfonyl-4-hydroxy pyrrolidin-2-yl]-1,2,4-oxadiazole 3D

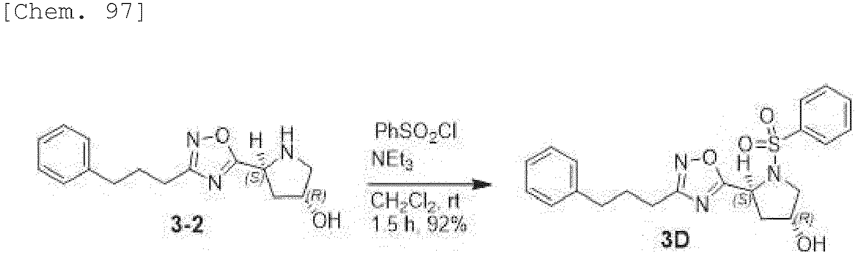

To a 10 ml eggplant-shaped flask, compound 3-2 (16.5 mg, 0.060 mmol) prepared in Example 15-1 was added, then CH₂Cl₂ (0.604 ml) and triethylamine (18.4 µl, 0.133 mmol) were added thereto, and the mixture was cooled to 0°C, which was added with benzenesulfonyl chloride (8.58 µl, 0.066 mmol), followed by stirring the mixture at room temperature for 1.5 hours. The reaction was quenched by adding 0.25 M hydrochloric acid aqueous solution (1 ml) and the mixture was extracted with ethyl acetate (1 ml x 2). After the extract was dried over magnesium sulfate, which was then filtered out, followed by distilling off the solvent, the residue was purified on a silica gel column Q-Pac SI20 size 10 (hexane:ethyl acetate = 90:10 to 34:66). After distilling off the solvent, 22.9 mg (0.055 mmol, 92%) of the desired compound 3D (oil) was obtained.

### Example 19

### 3-(3-phenylpropyl)-5-[(2S,4R)-1-benzylsulfonyl-4-hydroxy pyrrolidin-2-yl]-1,2,4-oxadiazole 3E

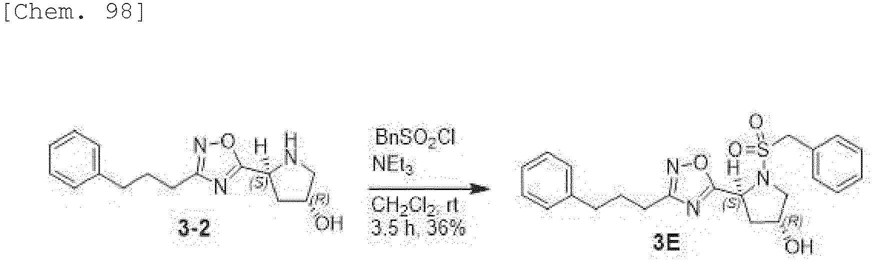

To a 10 ml eggplant-shaped flask, compound 3-2 (15.3 mg, 0.056 mmol) prepared in Example 15-1 was added, then CH₂Cl₂ (0.560 ml) and triethylamine (17.1 µl, 0.123 mmol) were added thereto, and the mixture was cooled to 0°C, which was added with benzylsulfonyl chloride (12.1 mg, 0.062 mmol), followed by stirring the mixture at room temperature for 3.5 hours. The reaction was quenched by adding 0.25 M hydrochloric acid aqueous solution (1.0 ml) and the mixture was extracted with ethyl acetate (1 ml x 2). After the extract was dried over magnesium sulfate, which was then filtered out, followed by distilling off the solvent, the residue was purified using preparative TLC (chloroform:methanol = 95:5). After distilling off the solvent, 8.5 mg (0.020 mmol, 36%) of the desired compound 3E (oil) was obtained.

The compounds prepared in Examples 14-19 are described in Table 5 along with their physical property data.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acid-acetonitrile;
0-0.9 min linear gradient A:B (95:5)-A:B (10:90), 0.9-3 min A:B (10:90)

**Table 5**

| [Table 7] | | | | | | |
|---|---|---|---|---|---|---|
| Ex | Cn | Chemical Structure | MW | ¹H-NMR δ ppm | [M+H] | RT :Min |
| 14 | 3-1 | 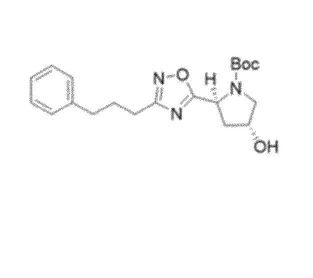 | 373.45 | (CDCI3) δ: 7.30-7.15 (5H. m), 5.27-5.12 (1H. m), 4.61 (1H. br-d. J - 2.8 Hz), 3.77 (1H, dd, J = 12.0, 4.4 Hz), 3.67 (0.7H, br-d, J = 12.0 Hz), 3.55 (0.3H, brad. J = 12.0 Hz), 2.77-2.68 (4H, m), 2.47-2.35 (1H. m). 2.26-2.18 (1H, m), 2.12-2.03 (2H. m), 1.88-1.80 (1H, m), 1.44 (2.7H. s). 1.28 (6.3H, s) | 374 | 1.67 |
| 15 | 3A | 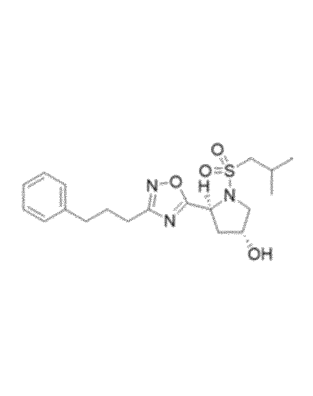 | 393.50 | (CDCI3) δ: 7.30-7.16 (5H. m), 5.36 (1H, t, J - 7.6 Hz), 4.61 (1H. br-s), 3.75 (1H, dt, J - 11.6, 2.0 Hz), 3.62 (1H, dd, J - 12.0, 3.6 Hz), 2.98 (2H, d, J = 6.4 Hz), 2.78-2.67 (4H. m), 2.53 (1H. ddt. J = 13.6, 8.0, 2.0 Hz), 2.37-2.20 (2H, m), 2.12-2.03 (2H, m), 1.08 (3H, d, J = 6.8 Hz), 1.04 (3H. d, J - 6.8 Hz) | 394 | 1.66 |
| 16 | 38 | 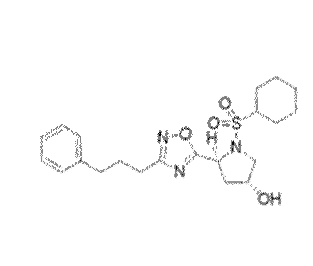 | 419.54 | (CDCI3) δ: 7.30-7.16 (5H, m), 5.50 (1H, t. J = 7.6 Hz), 4.59 (1H. br-s), 3.81 (1H, dt, J = 11.6, 2.0 Hz), 3.55 (1H, dd, J = 11.6. 3.6 Hz). 2.99-2.90 (1H. m), 2.78-2.67 (4H, m), 2.59-2.52 (1H, m), | 420 | 1.71 |

**Table 5 continued**

| [Table 8] | | | | | | |
|---|---|---|---|---|---|---|
| | | | | 2.36-2.27 (1H, m), 2.18-2.02 (4H, m), 1.87-1.80 (2H, m), 1.68-1.60 (1H, m), 1.53-1.38 (2H, m), 1.27-1.08 (3H, m) | | |
| 17 | 3C | 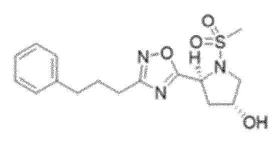 | 351.42 | (CDCI3) δ: 7.32-7.15 (5H, m), 5.27 (1H, t, J - 8.0 Hz), 4.62 (1H, br-s). 3.75-3.66 (2H, m). 2.99 (3H, s), 2.78-2.68 (4H, m), 2.54 (1H, ddt, J = 13.6, 7.6, 1.6 Hz), 2.34 (1H. ddd, J = 13.2, 8.4, 4.0 Hz), 2.13-2.03 (2H, m), 2.02 (1H. d, J = 3.6 Hz) | 352 | 1.48 |
| 18 | 3D | 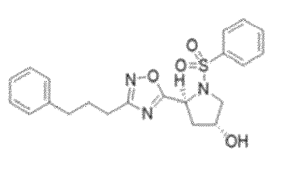 | 413.49 | (CDCI3) δ: 7.87-7.83 (2H, m), 7.59-7.46 (3H, m), 7.31-7.17 (5H, m), 5.11 (1H, t, J - 7.6 Hz), 4.56 (1H, br-s), 3.76 (1H, dd, J = 11.6. 4.0 Hz), 3.55 (1H, dt, J = 11.6, 2.0 Hz), 2.75-2.67 (4H. m), 2.39-2.25 (2H. m). 2.11-2.02 (2H, m), 1.61 (1H, d, J = 3.6 Hz) | 414 | 1.64 |
| 19 | 3E | 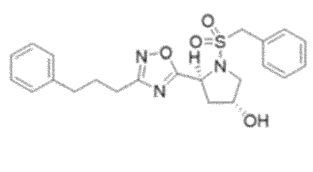 | 427.52 | (CDCI3) δ: 7.49-7.17 (10H, m). 5.38 (1H, t, J = 7.6 Hz), 4.46 (1H, br-s), 4.41 (1H, d. J = 13.6 Hz), 4.32 (1H, d, J - 13.6 Hz). 3.39 (1H, dt, J = 11.6, 2.0 Hz), 3.11 (1H, dd, J = 11.6. 4.0 Hz), 2.81-2.69 (4H, m), 2.52-2.45 (1H, m), 2.27-2.18 (1H, m), 2.16-2.07 (2H, m), 1.85 (1H, d, J = 3.6 Hz) | 428 | 1.66 |

| | | | | | | |
|---|---|---|---|---|---|---|
| Ex: Example Number Cn: Compound Number MW: Molecular Weight | | | | | | |

The following compounds can be prepared substantially according to the methods described in Examples 14-19.

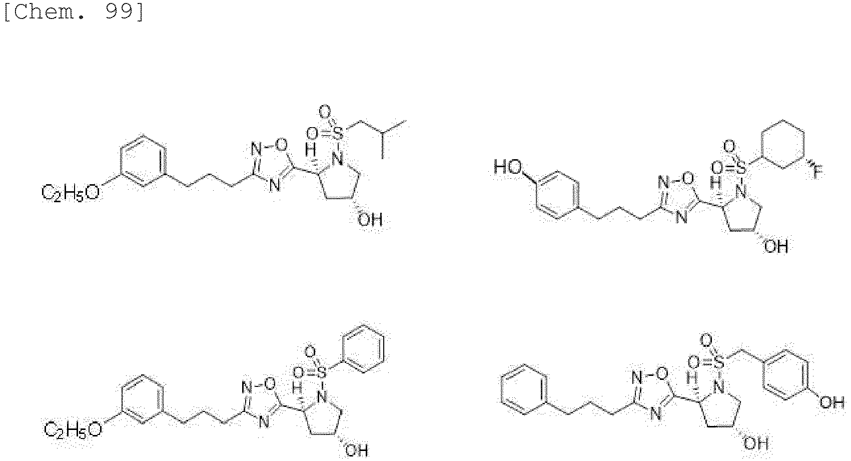

### Example 20

### 3-(3-phenylpropyl)-5-[(2S,4R)-4-tert-butoxy-1-cyclohexyl sulfonyl-pyrrolidin-2-yl]-1,2,4-oxadiazole 4B

### Example 20-1: 3-(3-phenylpropyl)-5-[(2S,4R)-1-[(9H-fluoren-9-yl)methoxycarbonyl]-4-tert-butoxypyrrolidin-2-yl]-1,2,4-oxadiazole 4-1

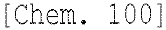

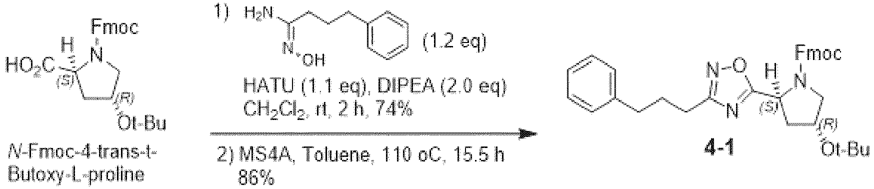

To a 50 ml eggplant-shaped flask, N-Fmoc-4-trans-1-butoxy-L-proline (1000 mg, 2.442 mmol) and N'-hydroxy-4-phenylbutanimidamide (479 mg, 2.686 mmol) were added while washing with dichloromethane (12.2 ml). Then, diisopropylethylamine (0.851 ml, 4.884 mmol) and HATU (1022 mg, 2.686 mmol) were added thereto and the mixture was stirred for 2 hours at room temperature under a nitrogen atmosphere. After removing the stirrer bar and distilling off the solvent, saturated sodium bicarbonate aqueous solution (5 ml) was then added thereto to make it basic, and then the mixture was extracted with ethyl acetate. The organic layer was washed with saturated brine, dried over magnesium sulfate, which was then filtered out, and the solvent was distilled off. The residue was purified on silica gel column Q-Pac SI30 size 60 (hexane:ethyl acetate = 90:10 to 50:50) to afford 1.032 g (1.811 mmol, 74%, and) of the imidamide intermediate. The intermediate was added with pre-dried MS4Å (5.16 g), and was then dissolved in ultra-dehydrated toluene (10.66 ml). The mixture was stirred at 110°C for 15.5 hours with a Dimroth condenser attached. After removing MS4Å by celite filtration, the solvent was distilled off and the resulting residue was purified on a silica gel column Q-Pac SI30 size 60 (hexane:ethyl acetate = 80:20). After distilling off the solvent, 857 mg (1.553 mmol, 86%) of the desired compound 4-1 (oil) was obtained.

### Example 20-2: 3-(3-phenylpropyl)-5-[(2S,4R)-4-tert-butoxy pyrrolidin-2-yl]-1,2,4-oxadiazole 4-2

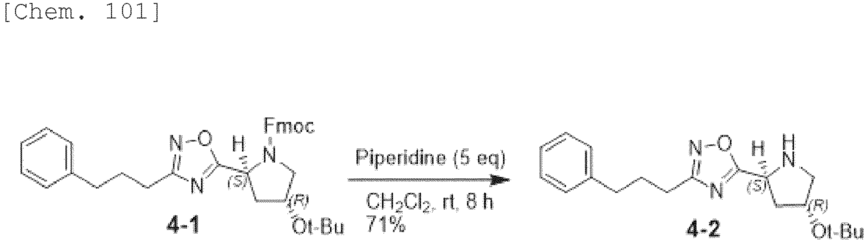

To a 25 ml eggplant-shaped flask, compound 4-1 (852 mg, 1.544 mmol) prepared in Example 20-1 was added, then dichloromethane (7.72 ml) and piperidine (0.787 ml, 7.72 mmol) were added thereto, and the mixture was stirred at room temperature for 8 hours. After removing the stirrer bar and distilling off the solvent, the residue was purified on a silica gel column Q-Pac SI30 size 20 (hexane:ethyl acetate = 80:20, and chloroform: methanol = 100:0 to 95:5). After distilling off the solvent, 362 mg (1.10 mmol, 71%) of the desired compound 4-2 (oil) was obtained.

### Example 20-3: 3-(3-phenylpropyl)-5-[(2S,4R)-4-tert-butoxy-1-cyclohexylsulfonyl-pyrrolidin-2-yl]-1,2,4-oxadiazole 4B

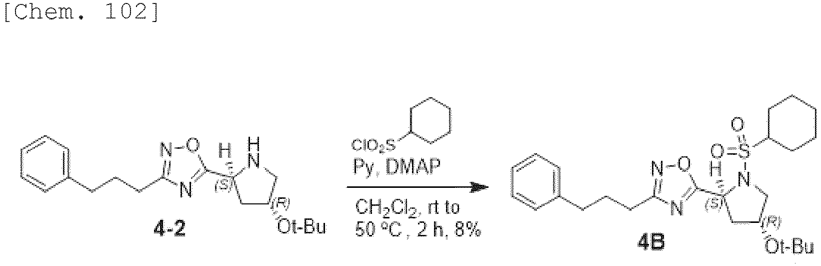

To a 10 ml eggplant-shaped flask, compound 4-2 (38.5 mg, 0.141 mmol) prepared in Example 20-2, CH₂Cl₂ (1.41 ml) and pyridine (45.5 pl, 0.563 mmol) were added, and the mixture was cooled to 0°C, which was added with cyclohexanesulfonyl chloride (54.2 mg, 0.282 mmol), followed by stirring the mixture at room temperature for 30 minutes and at 50°C for 2 hours. The reaction was quenched by adding 0.25 M hydrochloric acid aqueous solution (4 ml) and the mixture was extracted with ethyl acetate. After distilling off the solvent, the residue was purified on a silica gel column Q-Pac SI20 size 10 (hexane:ethyl acetate = 95:5 to 75:25). After distilling off the solvent, 5.5 mg (11.6 µmol, 8%) of the desired compound 4B (oil) was obtained.

### Examples 21 and 22

### 3-(3-phenylpropyl)-5-[(2S,4R)-4-tert-butoxy-1-(6-carbamoyl-2-naphthyl)-pyrrolidin-2-yl]-1,2,4-oxadiazole 4-3, and Naphthalene-2,6-diylbis(((2S,4R)-4-(tert-butoxy)-2-(3-(3-phenylpropyl)-1,2,4-oxadiazol-5-yl)pyrrolidin-1-yl) methanone) 4-4

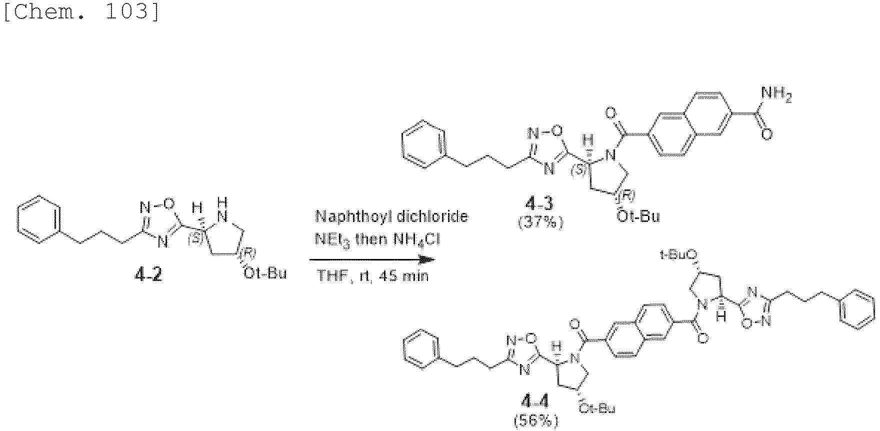

To a 10 ml eggplant-shaped flask added with compound 4-2 (10 mg, 0.030 mmol) prepared in Example 20-2, naphthoyl dichloride (23.5 mg, 0.091 mmol) separately prepared from 2,6-naphthalenedicarboxylic acid and oxalylchloride were added while washing with ultra-dehydrated THF (1.21 ml). Triethylamine (42.1 pl, 0.304 mmol) was added to this solution and the mixture was stirred at room temperature for 15 minutes. Ammonium chloride (16.2 mg, 0.304 mmol) was then added and the mixture was stirred at room temperature for 45 minutes. The reaction was quenched by adding water (0.5 ml) and the mixture was extracted with ethyl acetate. After the extract was dried over magnesium sulfate, which was then filtered out, followed by distilling off the solvent, the residue was purified on a silica gel column Q-Pac SI20 size 10 (hexane:ethyl acetate = 80:20 to 20:80). After distilling off the solvent, 5.9 mg (0.011 mmol, 37%) of the desired compound 4-3 (colorless, amorphous solid) and 7.1 mg (8.46 µmol, 56%) of compound 4-4 (colorless, amorphous solid) were also obtained as a byproduct.

### Example 23

### 3-(3-phenylpropyl)-5-[(2S,4R)-4-hydroxy-1-(6-carbamoyl-2-naphthyl)pyrrolidin-2-yl]-1,2,4-oxadiazole 4-5

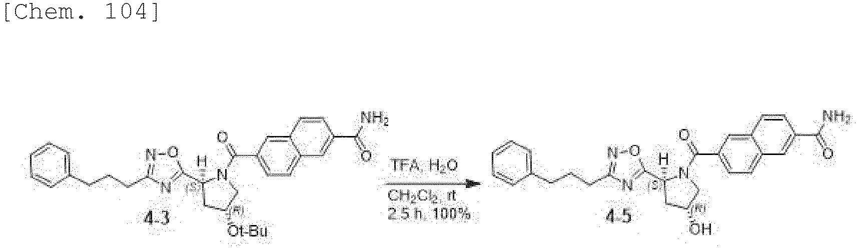

To a 4 ml vial, compound 4-3 (3.7 mg, 7.0 umol) prepared in Example 21 was added, and dichloromethane (0.14 ml), TFA (70 µl) and water (3.5 µl) were added thereto, followed by stirring the mixture at room temperature for 2.5 hours. After distilling off the solvent, the process of dissolving the residue in chloroform (1 ml) and conducting the distillation was repeated three times to remove the TFA. The product was then dried in vacuo for 20 hours to afford 3.4 mg (7.0 µmol, 100%) of the desired compound 4-5 (amorphous solid).

### Example 24

### Naphthalene-2,6-diylbis(((2S,4R)-4-hydroxy-2-(3-(3-phenyl propyl)-1,2,4-oxadiazol-5-yl)pyrrolidin-1-yl)methanone) 4-6

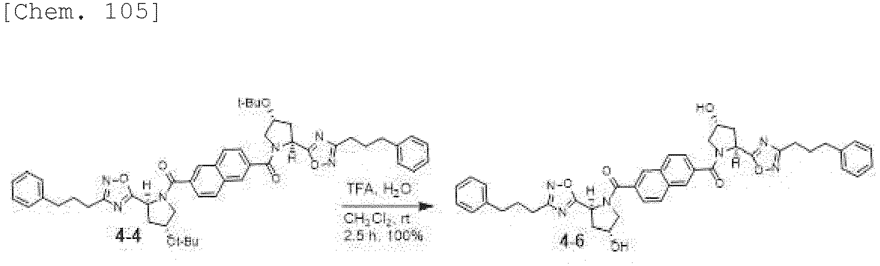

To a 4 ml vial, compound 4-4 (4.7 mg, 5.6 umol) prepared in Example 22 was added, and dichloromethane (0.14 ml), TFA (70 µl) and water (3.5 µl) were added thereto, followed by stirring the mixture at room temperature for 2.5 hours. After distilling off the solvent, the process of dissolving the residue in chloroform (1 ml) and conducting the distillation was repeated three times to remove the TFA. The product was then dried in vacuo for 20 hours to afford 4.2 mg (5.6 µmol, 100%) of the desired compound 4-6 (amorphous solid).

The compounds prepared in Examples 20-24 are described in Table 6 along with their physical property data.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acid-acetonitrile;
0-0.9 min linear gradient A:B (95:5)-A:B (10:90), 0.9-3 min A:B (10:90)

**Table 6**

| [Table 9] | | | | | | |
|---|---|---|---|---|---|---|
| Ex | Cn | Chemical Structure | MW | ¹H-NMR δ ppm | [M+H] | RT :Min |
| 20 | 4B | 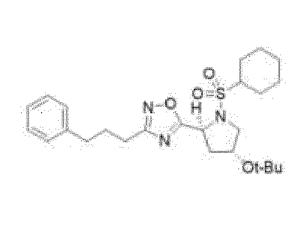 | 475,65 | (CDCI3) δ: 7.3,2-7.15 (5H, m), 5.29 (1H, dd, J = 8.0, 5.6 Hz). 4.48-4.41 (1H, m), 3.67 (1H, dd, J = 10.0, 5.6 Hz), 3.51 (1H, dd, J = 10.0, 4.0 Hz), 3.01-2.92 (1H. m), 2.79-2.67 (4H, m), 2.41-2.31 (1H, m), 2.30-2.22 (1H, m), 2.18-2.03 (4H, m), 1.87-1.80 (2H, m), 1.67-1.62 (1H, m), 1.57-1.44 (2.H, m), 1.27-1.12 (2H, m), 1.21 (9H, s) | 476 | 1.99 |
| 21 | 4-3 | 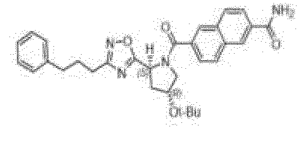 | 526.64 | (CDCl3) δ: 8,34 (1H, s), 8.07 (1H, s). 7.97-7.86 (3H, m), 7.72-7.66 (1H. m), 7.32-7.14 (5H, m), 6.34 (1H, br-s). 5.79 (1H, br-s), 5.63 (1H, t, J - 6.8 Hz). 4.41 (1H, br-s). 3.95 (1H, dd, J = 10.4, 5.2 Hz), 3.49 (1H, dd, J = 10.8. 3.6 Hz). 2.81-2.68 (4H, m), 2.53-2.38 (2H, m), 2.31-2.22 (1H, m), 2.16-2.03 (1H, m), 1.12 (9H, s) | 527 | 1.68 |
| 22 | 4-4 | | 839.05 | (CDCl3) δ: 8.09 (1H. s). 7.92 (1H, d, J = 8.4 Hz), 7.70 (1H, d, J = 8.4 Hz), 7.31-7.12 (5H. | 839 | 2.16 |

**Table 6 continued**

| [Table 10] | | | | | | |
|---|---|---|---|---|---|---|
| | | 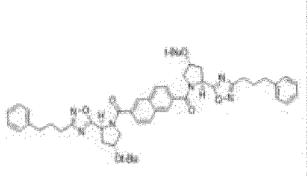 | | m), 5.63 (1H, t, J = 6.8 Hz), 4.41 (1H, br-s). 3.96 (1H, dd, J = 10.4. 4.8 Hz), 3.49 (1H, br-d, J = 8.0 Hz). 2.80-2.69 (4H, m), 2.46-2.37 (1H, m), 2.31-2.23 (1H, m), 2.16-2.06 (2H, m), 1.12 (9H, s) | | |
| 23 | 4-5 | 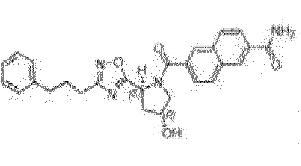 | 470.53 | 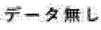 | 471 | 1.47 |
| 24 | 4-6 | 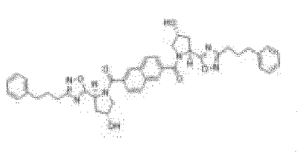 | 726.83 | 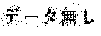 | 727 | 1.66 |

| | | | | | | |
|---|---|---|---|---|---|---|
| Ex: Example Number Cn: Compound Number MW: Molecular Weight | | | | | | |

The following compounds can be prepared substantially according to the methods described in Examples 20-24.

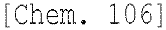

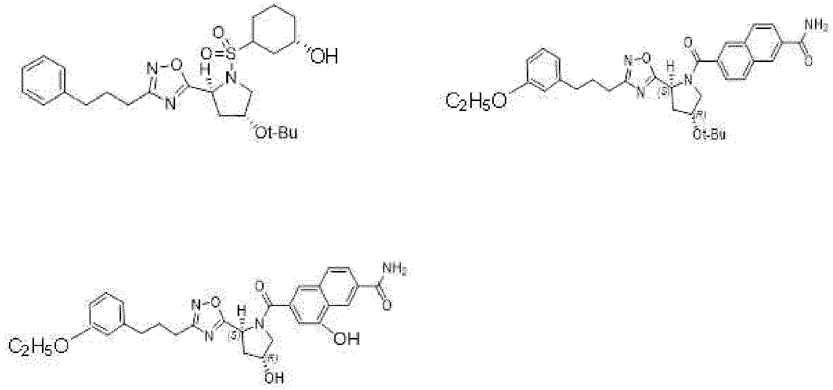

### Example 25

### 3-(3-phenylpropyl)-5-{ (6S)-5-isobutylsulfonyl-5-azaspiro [2.4]hept-6-yl}-1,2,4-oxadiazole 5A

### Example 25-1: 3-(3-phenylpropyl)-5-{(6S)-5-tert-butoxy carbonyl-5-azaspiro[2.4]hept-6-yl}-1,2,4-oxadiazole 5-1

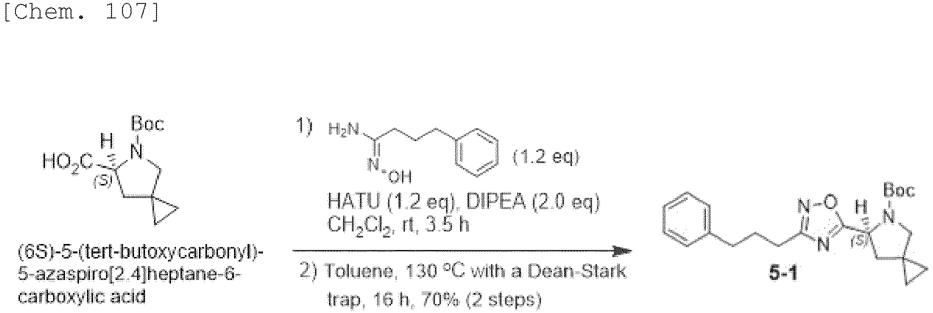

(6S)-5-(tert-butoxycarbonyl)-5-azaspiro[2.4]heptane-6-carboxylic acid (241.3 mg, 1.0 mmol) was added to a 25 ml eggplant-shaped flask and dissolved in dichloromethane (7.0 ml). Then, HATU (456.3 mg, 1.2 mmol) and diisopropylethylamine (0.34 ml, 2.0 mmol) were added thereto and the mixture was stirred for 10 minutes at room temperature under a nitrogen atmosphere. N'-hydroxy-4-phenylbutanimidamide (213.9 mg, 1.2 mmol) was then added while washing with dichloromethane (3.0 ml) and the mixture was stirred at room temperature for 3.5 hours.

The stirrer bar was removed and dichloromethane (20.0 ml) and 5% sodium bicarbonate aqueous solution (10.0 ml) were added to the reaction mixture. The separated organic layer was washed with distilled water (10.0 ml) and dried over anhydrous magnesium sulfate. After filtration, the solvent was distilled off to afford a mixture (686.0 mg) of the imidamide intermediate and urea compound. The mixture (686.0 mg) was dissolved in toluene (18.0 ml) in a 25 ml eggplant-shaped flask, which was placed in a Dean-Stark trap, and the mixture was heated to reflux at an oil bath temperature of 130°C for 16 hours. The solvent was distilled off under reduced pressure and the resulting residue was purified on silica gel column Q-Pac SI30 size 20 (hexane:ethyl acetate = 90:10 to 65:35). After distilling off the solvent, 269.2 mg (0.702 mmol, 70% (after 2 steps)) of the desired compound 5-1 (oil) was obtained.
Molecular weight: 383.49;
1H-NMR δ ppm (CDCl3: a mixture of the rotational isomers) δ: 7.33-7.13 (5H, m), 5.27-5.08 (1H, m), 3.55-3.30 (1H, m), 2.75 (2H, t, J = 7.6 Hz), 2.70 (2H, t, J = 7.6 Hz), 2.50-2.35 (1H, m), 2.08 (2H, quint, J = 7.6 Hz), 1.98-1.78 (1H, m), 1.45 and 1.31 (9H, s).
[M+H]; 384
RT (minute); 1.91.

### Example 25-2: 3-(3-phenylpropyl)-5-{(6S)-5-azaspiro[2.4] hept-6-yl}-1,2,4-oxadiazole TFA salt 5-2

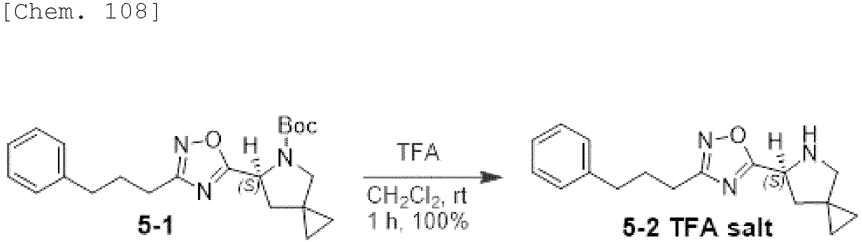

To a 4.0 ml screw-tube vial, compound 5-1 (40.0 mg, 0.104 mmol) prepared in Example 25-1 was added, and dichloromethane (1.0 ml) and TFA (0.16 ml) were added thereto, followed by stirring the mixture at room temperature for 1 hour. After removing the stirrer bar and distilling off the solvent, the process of dissolving the residue in chloroform (2 ml) and conducting the distillation was repeated three times to remove the TFA. The material was then dried in vacuo for 3 hours to afford the desired compound 5-2 (amorphous solid) as a TFA salt (41.5 mg, 0.104 mmol, 100%).

### Example 25-3: 3-(3-phenylpropyl)-5-{(6S)-5-isobutyl sulfonyl-5-azaspiro[2.4]hept-6-yl}-1,2,4-oxadiazole 5A

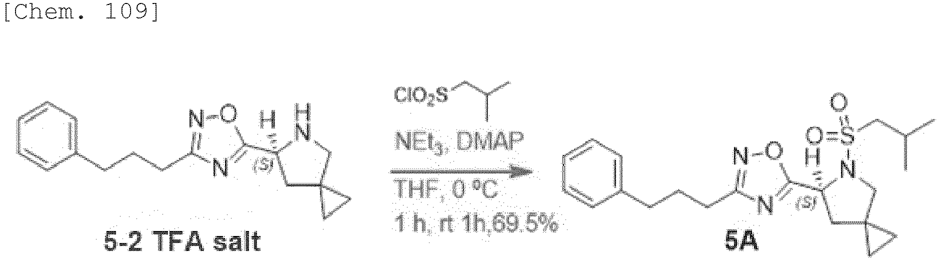

Ultra-dehydrated THF (1.5 ml), triethylamine (0.073 ml, 0.522 mmol) and DMAP (3.8 mg, 0.031 mmol) were added to a 4.0 ml screw-tube vial containing the TFA salt (41.5 mg, 0.104 mmol) of compound 5-2 prepared in Example 25-2, sequentially, and the mixture was cooled to 0°C, which was added with a solution of isobutylsulfonyl chloride (0.027 ml, 0.137 mmol) in ultra-dehydrated THF (0.5 ml), followed by stirring the mixture at 0°C for 1 hour and at room temperature for 1 hour. The reaction was quenched by adding 0.2 M hydrochloric acid aqueous solution (1 ml) and the mixture was extracted with ethyl acetate (6 ml). After distilling off the solvent under reduced pressure, the residue was purified by Si02-PTLC (PLC Silica Gel 60 F254, 0.5 mm layer thickness, 20 x 20 cm; developing solvent: hexane:ethyl acetate = 3:2). The silica gel of the target portion was scraped off and eluted with chloroform containing 10% MeOH (20 ml). The solvent was distilled off under reduced pressure to afford 29.3 mg (0.073 mmol, 69.5% (after 2 steps)) of the desired compound 5A (oil).

### Example 26

### 3-(3-phenylpropyl)-5-{ (6S)-5-cyclohexylsulfonyl-5-azaspiro [2.4]hept-6-yl}-1,2,4-oxadiazole 5B

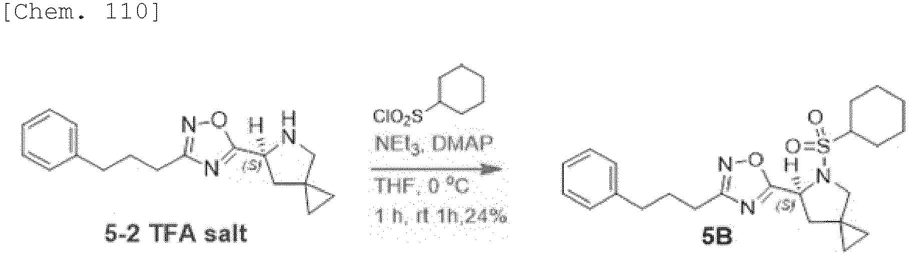

Ultra-dehydrated THF (1.5 ml), triethylamine (0.073 ml, 0.522 mmol) and DMAP (3.8 mg, 0.031 mmol) were added sequentially to a 4.0 ml screw-tube vial containing the TFA salt (41.5 mg, 0.104 mmol) of compound 5-2 prepared in Example 25-2, and the mixture was cooled to 0°C, which was added with a solution of cyclohexanesulfonyl chloride (0.034 ml, 0.128 mmol) in ultra-dehydrated THF (0.5 ml), followed by stirring the mixture at 0°C for 1 hour at room temperature. The reaction was quenched by adding 0.2 M hydrochloric acid aqueous solution (1 ml) and the mixture was extracted with ethyl acetate (6 ml). After distilling off the solvent under reduced pressure, the residue was purified by Si02-PTLC (PLC Silica Gel 60 F254, 0.5 mm layer thickness, 20 x 20 cm; developing solvent: hexane:ethyl acetate = 3:1). The silica gel of the target portion was scraped off and eluted with chloroform containing 10% MeOH (20 ml). The solvent was distilled off under reduced pressure to afford 10.8 mg (0.025 mmol, 24% (after 2 steps)) of the desired compound 5B (oil).

### Example 27

### 3-(3-phenylpropyl)-5-{ (6S)-5-methanesulfonyl-5-azaspiro [2.4]hept-6-yl}-1,2,4-oxadiazole 5C

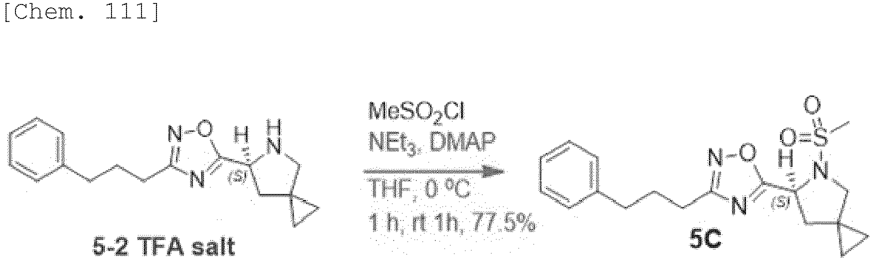

Ultra-dehydrated THF (1.5 ml), triethylamine (0.073 ml, 0.522 mmol) and DMAP (3.8 mg, 0.031 mmol) were added to a 4.0 ml screw-tube vial containing the TFA salt (41.5 mg, 0.104 mmol) of compound 5-2 prepared in Example 25-2, sequentially, and the mixture was cooled to 0°C, which was added with a solution of methanesulfonyl chloride (0.016 ml, 0.209 mmol) in ultra-dehydrated THF (0.5 ml), followed by stirring the mixture at 0°C for 1 hour and at room temperature for 1 hour. The reaction was quenched by adding 0.2 M hydrochloric acid aqueous solution (1 ml) and the mixture was extracted with ethyl acetate (6 ml). After distilling off the solvent under reduced pressure, the residue was purified by Si02-PTLC (PLC Silica Gel 60 F254, 0.5 mm layer thickness, 20 x 20 cm; developing solvent: hexane:ethyl acetate = 3:2). The silica gel of the target portion was scraped off and eluted with chloroform containing 10% MeOH (20 ml). The solvent was distilled off under reduced pressure to afford 29.2 mg (0.081 mmol, 77.5% (after 2 steps)) of the desired compound 5C (oil).

### Examples 28 and 29

### 3-(3-phenylpropyl)-5-{ (6S)-5-benzenesulfonyl-5-azaspiro [2.4]hept-6-yl}-1,2,4-oxadiazole 5D, and

### 3-(3-Phenylpropyl)-5-{ (6S)-5-trifluoroacetyl-5-azaspiro [2.4]hept-6-yl}-1,2,4-oxadiazole 5D'

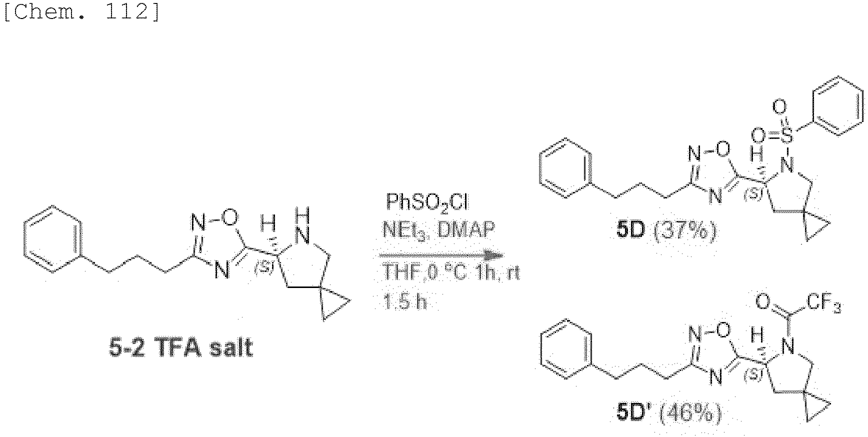

Ultra-dehydrated THF (1.5 ml), triethylamine (0.073 ml, 0.522 mmol) and DMAP (3.8 mg, 0.031 mmol) were added to a 4.0 ml screw-tube vial containing the TFA salt (41.5 mg, 0.104 mmol) of compound 5-2 prepared in Example 25-2, sequentially, and the mixture was cooled to 0°C, which was added with a solution of benzenesulfonyl chloride (0.027 ml, 0.128 mmol) in ultra-dehydrated THF (0.5 ml) solution, followed by stirring the mixture at 0°C for 1 hour and at room temperature for 1 hour. The reaction was quenched by adding 0.2 M hydrochloric acid aqueous solution (1 ml) and the mixture was extracted with ethyl acetate (6 ml). After distilling off the solvent under reduced pressure, the residue was purified by Si02-PTLC (PLC Silica Gel 60 F254, 0.5 mm layer thickness, 20 x 20 cm; developing solvent: hexane:ethyl acetate = 2:1). The silica gel of the target portion was scraped off and eluted with chloroform containing 10% MeOH (20 ml). The solvent was distilled off under reduced pressure to afford 16.5 mg (0.039 mmol, 37% (after 2 steps)) of the desired compound 5D (oil). Also, 18.3 mg (0.048 mmol, 46%) of compound 5D' (oil) was obtained as a byproduct.

### Example 30

### 3-(3-Phenylpropyl)-5-{ (6S)-5-benzylsulfonyl-5-azaspiro [2.4]hept-6-yl}-1,2,4-oxadiazole 5E

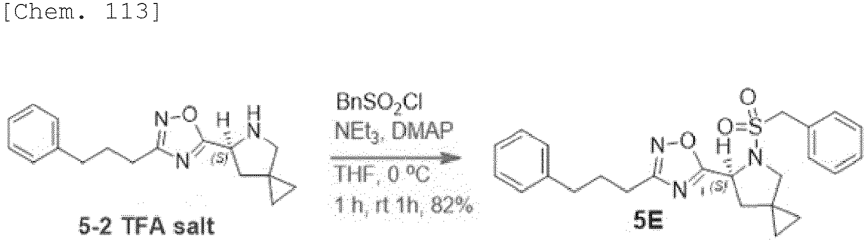

Ultra-dehydrated THF (1.5 ml), triethylamine (0.073 ml, 0.522 mmol) and DMAP (3.8 mg, 0.031 mmol) were added sequentially to a 4.0 ml screw-tube vial containing the TFA salt (41.5 mg, 0.104 mmol) of compound 5-2 prepared in Example 25-2, , and the mixture was cooled to 0°C, which was added with a solution of benzylsulfonyl chloride (39.8 mg, 0.209 mmol) in ultra-dehydrated THF (0.5 ml), followed by stirring the mixture at 0°C for 1 hour and at room temperature for 1 hour. The reaction was quenched by adding 0.2 M hydrochloric acid aqueous solution (1 ml) and the mixture was extracted with ethyl acetate (6 ml). After distilling off the solvent under reduced pressure, the residue was purified by Si02-PTLC (PLC Silica gel 60 F254, 0.5 mm layer thickness, 20 x 20 cm; developing solvent: hexane:ethyl acetate = 3:2). The silica gel of the target portion was scraped off and eluted with chloroform containing 10% MeOH (20 ml). The solvent was distilled off under reduced pressure to afford 37.4 mg (0.085 mmol, 82% (after 2 steps)) of the desired compound 5E (oil).

The compounds prepared in Examples 25-30 are described in Table 7-1 along with their physical property data.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acid-acetonitrile;
0-0.9 min linear gradient A:B (95:5)-A:B (10:90), 0.9-3 min A:B (10:90)

**Table 7-1**

| [Table 11] | | | | | | |
|---|---|---|---|---|---|---|
| Ex | Cn | Chemical Structure | MW | ¹H-NMR δ ppm | [M+H] | RT :Min |
| 25 | 5A | 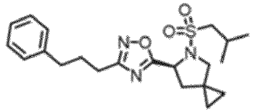 | 403.54 | (CDCl3) *δ*: 7.30-7.15 (5H. m), 5.38 and 5.36 (1H, dd, J = 8.4, 3.6 Hz), 3.69 (1H, d, J = 9.2 Hz), 3.25 (1H, d, J = 9.2 Hz), 3.01 (2H, d, J = 6.4 Hz), 2.76 (2H, t. J = 7.2 Hz). 2.70 (2H, t, J = 7.2 Hz), 2.52 and 2.48 (1H, d, J = 12.4, 8.2 Hz), 2.32-2.29 (1H, m), 2.08 (2H, quint, J = 7.2 Hz), 2.05 and 2.02 (1H, dd, J = 12.4, 3.6 Hz), 1.08 (3H. d, J = 6.4 Hz), 1.45 (3H, d, J = 6.4 Hz), 0.75-0.50 (4H, m) | 404 | 1.82 |
| 26 | 58 | 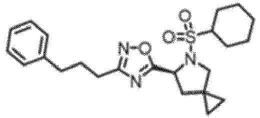 | 429.58 | (CDCI3) *δ*: 7.30-7.15 (5H, m), 5.43 and 5.41 (1H. dd, J = 8.0, 3.6 Hz), 3.74 (1H, d, J = 9.2 Hz), 3.26 (1H, d, J = 9.2 Hz), 2.95-3.03 (1H, m), 2.76 (2H. t, J = 7.6 Hz), 2.70 (2H, t, J = 7.6 Hz), 2.50 and 2.47 (1H, dd, J = 12.4, 8.0 Hz), 2.20-2.12 (2H, m), 2.10 (2H, quint, J = 7.6 Hz), 2.05 and 2.02 (1H, dd, J = 12.4, 3.6 Hz), 1.90-1.80 (2H, m), 1.58.1.42 (2H, m), | 430 | 1.91 |

**Table 7-1 continued**

| [Table 12] | | | | | | |
|---|---|---|---|---|---|---|
| | | | | 1.35-1.15 (4H, m), 0.75-0.50 (4H. m) | | |
| 27 | 5C | 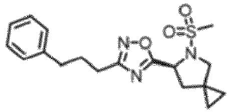 | 361.46 | (CDCI3) *δ*: 7.35-7.15 (5H. m), 5.35 and 5.33 (1H. dd, J = 8.0, 3.6 Hz). 3.66 (1H. d. J = 9.2 Hz), 3.28 (1H, d, J = 9.2 Hz), 3.03 (3H, s), 2.76 (2H, t, J = 7.6 Hz), 2.70 (2H, t, J = 7.6 Hz). 2.54 and 2.51 (1H, dd, J = 12.8, 8.0 Hz), 2.08 (2H, quint, J = 7.6 Hz). 2.04 and 2.01 (1H, dd, J = 12.8, 3.6 Hz), 0.50-0.75 (4H. m) | 362 | 1.69 |
| 28 | 5D | 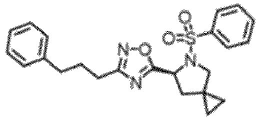 | 423.53 | (CDCl3) *δ*: 7.90-7.85 (2H, m), 7.60-7.45 (3H, m), 7.30-7.18 (5H, m), 5.17 and 5.15 (1H, dd, J = 8.0 and 4.8 Hz). 3.42 (2H. s), 2.73 (2H. t. J = 7.2 Hz), 2.69 (2H, t, J = 7.2 Hz), 2.22 and 2.19 (1H, dd. J = 12.8, 8.0 Hz), 2.06 (2H, quint, J = 7.2 Hz), 1.99 and 1.96 (1H, dd, J = 12.8, 4.8 Hz). 0.7-0.4 (4H. m) | 424 | 1.81 |
| 29 | 5D' | 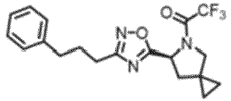 | 379.38 | (CDCl3) *δ*: 7.35-7.15 (5H, m), 5.49 and 5.47 (1H, dd, J - 8.0 and 3.6 Hz), 3.80 (1H. d. J = 10.4 Hz). 3.72 (1H, d, J = 10.4 Hz). 2.75 (2h, t, J = 7.2 Hz), 2.69 (2H, t, J = 7.2 Hz), 2.48 and 2.45 (1H, dd, J = 12.8, 8.0 Hz), 2.09 (2H, quint, J = 7.2 Hz). 1.99 and 1.96 (1H, dd, J - | 380 | 1.81 |

**Table 7-1 continued 2**

| [Table 13] | | | | | | |
|---|---|---|---|---|---|---|
| | | | | 12.8, 3.6 Hz), 0.8-0.5 (4H, m) | | |
| 30 | 5E | 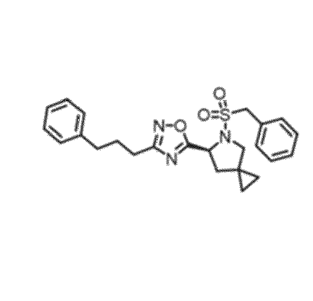 | 437.56 | (CDCI3) *δ*: 7.51-7.18 (10H, m), 5.28 and 5.26 (1H, dd, J = 8.0 and 3.6 Hz), 4.51 (1H, d, J = 13.6 Hz), 4.34 (1H, d, J = 13.6 Hz). 3.39 (1H, d. J = 9.2 Hz). 2.79 (2H, t. J = 7.6 Hz), 2.72 (2H, t, J = 7.6 Hz), 2.65 (1H, d, J = 9.2 Hz). 2.42 and 2.39 (1H, dd. J = 12.8 and 8.0 Hz), 2.11 (2H, quint, J = 7.6 Hz). 1.96 and 1.93 (1H. dd, J = 12.8, 3.6 Hz), 0.6-0.45 (4H. m) | 438 | 1.81 |

| | | | | | | |
|---|---|---|---|---|---|---|
| Ex: Example Number Cn: Compound Number MW: Molecular Weight | | | | | | |

Eleven compounds listed in Table 7-2 were synthesized by using various sulfonyl chlorides in place of isobutyl sulfonyl chloride under the same reaction conditions as in Example 25-3.

**Table 7-2**

| [Table 14] | | | |
|---|---|---|---|
| Compound Number | Compound Name | Sulfonyl Chlorides | Yield (%) |
| 5AI | (S)-5-(5-((4-methoxyphenyl)sulfonyl)-5-azabicyclo[2.4]heptan-6-yl)-3-(3-phenylpropyl)-1,2,4-oxadiazole | 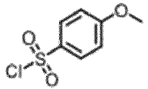 | 65 |
| 5AJ | (S)-5-(5-((4-fluorophenyl)sulfonyl)-5-azaspiro[2.4]heptan-6-yl)-3-(3-phenylpropyl)- 1,2,4-oxadiazole | 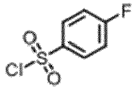 | 77 |
| 5AX | (S)-N,N-dimethyl-4-((6-(3-(3-phenylpropyl)-1,2,4-oxadiazole-5-yl)-5-azaspiro[2.4]heptan-5-yl)sulfonyl)aniline | 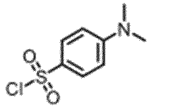 | 58 |
| 5AL | (S)-N,N-dimethyll-5-((6-(3-(3-phenylpropyl)- 1,2,4-oxadiazole-5-yl)-5-azaspiro[2.4] heptan-5-yl)sulfonyl)naphthalen-1-amine | 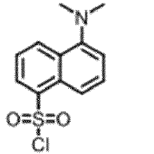 | 74 |
| 5AM | tert-buthyl (S)-3-(((6-(3-(3-phenylpropyl)-1,2,4-oxadiazole-5-yl)-5-azaspiro[2.4]hepcan-5-yl)sulfonyl)methyl)azetidin-1-carboxylate | 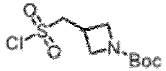 | 34 |
| 5AN | tert-buthyl (S)-4-(((6-(3-(3-phenylpropyl)-1,2,4-oxadiazole-5-yl)-5-azaspiro[2.4]heptan-5-yl)sulfonyl)methyl)piperidin-1-carboxylate | 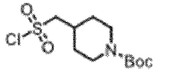 | 64 |
| 5AO | tert-buthyl (S)-4-((6-(3-(3-phenylpropyl)-1,2,4-oxadiazole-5-yl)-5-azaspiro[2.4]heptan-5-yl)sulfonyl)piperidin-1-carboxylate | 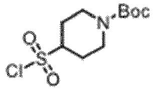 | 38 |
| 5AP | (S)-3-(3-phenylpropyl)-5-(5-((tetrahydro-2H-pyran-4-yl)sulfonyl)-5-azaspiro[2.4] heptan-6-yl)-1,2,4-oxadiazole | 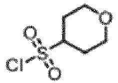 | 30 |

**Table 7-2 continued**

| | | | |
|---|---|---|---|
| SAC | ethyl (S)-1-((6-(3-(3-phenytpropyl)-1,2,4-ozadiazole-5-yl)-5-azaspiro[2.4]heptan-5-yl)sutfonyl)piperidin-4-carboxylate | 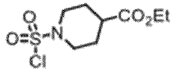 | 76 |
| SAD | (S)-3-(3-phenylpropyl)-5-(5-(piperidin-1-ylsulfonyl)-5-azaspiro[2.4]heptan-6-yl)-1,2,4-oxadiazole | 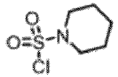 | 92 |
| SAB | (S)-4-((6-(3-(3-phenylpropyl)-1,2,4-oxadiazole-5-yl)-5-azaspiro[2.4]heptan-5-yl)sulfonyl)morpholine | 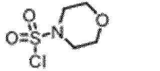 | 69 |

The chemical structure, molecular weight, 1H-NMR, [M+H], and RT of the above compounds are summarized in Table 7-3.

**Table 7-3**

| [Table 16] | | | | | |
|---|---|---|---|---|---|
| Ex | Chemical Structure | MW | ¹H-NMR δ ppm | [M+H] | RT :Min |
| 5AI | 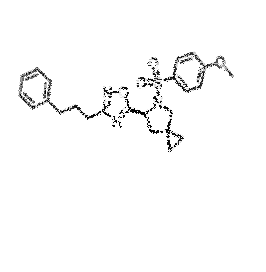 | 453.56 | (CDCl3) *δ*: 7.79(2H. d. J = 7.2 Hz), 7.30-7.15 (5H, m), 6.96 (2H, d. J = 7.2 Hz). 5.13 and 5.11 (1H, dd. J = 8.0, 4.4 Hz), 3.84 (3H, s), 3.39 and 3.37 (2H, ABq, J = 9.6 Hz), 2.73 (2H, t, J = 7.4 Hz), 2.69 (2H, t, J = 7.4 Hz), 2.21 and 2.18 (1H. dd, J = 12.8, 8.0 Hz). 2.06 (2H. quint, J = 7.4 Hz), 1.99 and 1.95 (1H, dd, J = 12.8, 4.4 Hz), 0.70-0.45 (4H, m) | 454 | 1.81 |
| 5AJ | 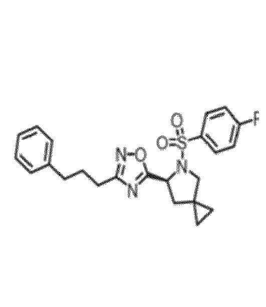 | 441.52 | (CDCI3) *δ*: 7.89-7.87 (2H, m), 7.35-7.15 (7H, m). 5.17 and 5.15 (1H, dd. J = 8.4, 4.4 Hz), 3.44 and 3.37 (2H, ABq, J = 9.6 Hz), 2.73 (2H, t, J = 7.2 Hz), 2.69 (2H, t, J = 7.2 Hz), 2.28 and 2.24 (1H, dd, J = 12.8, 8.4 Hz), 2.04 (2H, quint, J = 7.2 Hz), 2.00 and 1.98 (1H, dd, J = 12.8. 4.4 Hz), 0.52-0.48 (4H, m) | 442 | 1.82 |
| 5AK | 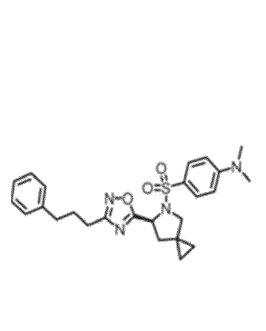 | 466.60 | (CDCI3) *δ*: 7.68 (2H, d, J = 6.8 Hz), 7.32-7.17 (5H, m), 6.65 (2H. d. J = 6.8 Hz), 5.11 and 5.08 (1H, dd, J = 8.4, 4.4 Hz), 3.65 and 3.38 (2H, ABq, J = 9.6 Hz). 3.01 (6H, s),2.74 (2H, t, J = 7.2 Hz), 2.68 (2H, t. J = 7.2 Hz), 2.20 and 2.17 (1H, dd, J - 12.8, 8.4 Hz), 2.06 (2H. quint, J = 7.2 Hz), 1.96 and 1.93 (1H, dd, J = | 467 | 1.82 |

**Table 7-3 continued**

| [Table 17] | | | | | |
|---|---|---|---|---|---|
| | | | 12.8, 4.4 Hz). 0.68-0.42 (4H, m) | | |
| 5AL | 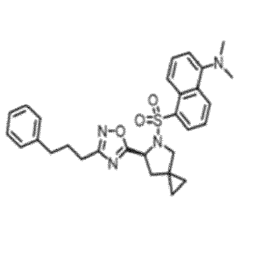 | 516.66 | (CDCI3) *δ*: 8.50 (1H. d, J = 8.4 Hz), 8.38 (1H, d, J - 8.8 Hz), 8.24 and 8.22 (1H, dd, J = 7.2. 1.2 Hz), 7.54-7.44 (2H, m). 7.32-7.12 (5H, m), 5.36 and 5.34 (1H, dd, J = 8.0, 4.4 Hz), 3.65 (1H, d, J = 9.6 Hz). 3.40 (1H, d, J - 9.6 Hz), 2.86 (6H, s), 2.61 (2H, t, J = 7.4 Hz), 2.58 (2H. t, J = 7.4 Hz), 2.35 and 2.31 (1H. dd, J = 12.8, 8.0 Hz). 2.20-1.93 (3H, m), 0.70-0.48 (4H, m) | 517 | 2.01 |
| 5AM | 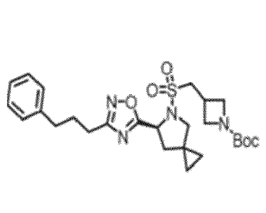 | 516.66 | (CDCI3) *δ*: 7.32-7.12 (5H, m), 5.38 and 5.36 (1H, dd, J = 8.0, 3.6 Hz), 4.12 and 4.08 (2H, ABq, J = 8.4 Hz), 3.80-3.65 (3H, m), 3.44-3.38 (2H, m), 3.25-3.20 <1H, m), 3.14-3.03 (1H. m), 2.75 (2H, t, J = 7.6 Hz). 2.70 (2H, t, J = 7.6 Hz), 2.53 and 2.49 (1H. dd, J - 12.8. 8.0 Hz), 2.13-2.02 (3H, m), 1.42 (9H. s) | 517 | 1.81 |
| 5AN | 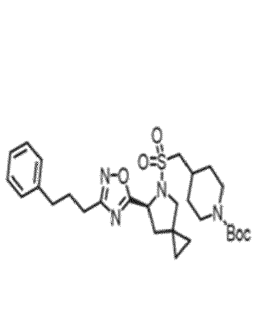 | 544.71 | (CDCI3) *δ*: 7.33-7.12 <5H, m), 5391 and 5.37 (1H, dd. J = 8.4, 4.0 Hz). 4.12-3.97 (2H, br s). 3.68 (1H, d. J = 9.2 Hz), 3.24 (1H, d, J = 9.2 Hz), 3.13-2.97 (2H, m). 2.80-2.62 (2H, m), 2.75 (2H, t, J = 7.2 Hz), 2.69 (2H, t, J - 7.2 Hz). 2.52 and. 2.49 (1H, dd. J - 12.8, 8.4 Hz), 2.18-2.01 (4H, m), 1.94-1.80 <2H, m), 1.45 (9H, s), 1.32-1.14 (3H. m), 0.78-0.49 (4H, m) | 545 | 1.87 |

**Table 7-3 continued 2**

| [Table 18] | | | | | |
|---|---|---|---|---|---|
| 5AO | 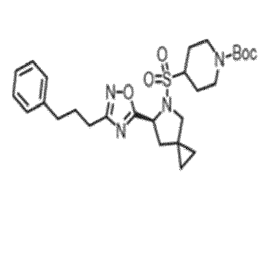 | 530.68 | (CDCI3) *δ*: 7.34-7.12 (5H. m). 5.43 and 5.41 (1H. dd. J = 8.0, 3.6 Hz), 4.32-4.08 (2H, br s), 3.76 (1H, d, J = 9.6 Hz). 3.23 (1H, d, J = 9.6 Hz), 3.22-3.08 (1H, m), 2.75 (2H, t, J = 7.2 Hz), 2.70 (2H, t, J = 7.2 Hz), 2.76-2.58 (2H. m), 2.52 and 2.49 (1H. dd, J = 12.8, 8.0 Hz), 2.18-2.02 (5H, m), 1.80-1.65 (2H, m), 1.45 (9H, s), 0.78-0.52 (4H, m) | 531 | 1.88 |
| 5AP | 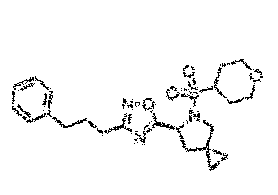 | 431.55 | (CDCI3) *δ*: 7.36-7.12 (5H. m), 5.45 and 5.42 (1H. dd, J = 8.0, 3.6 Hz), 4.10-4.00 (2H, m), 3.77 (1H, d, J = 9.6 Hz), 3.37-3.21 (3H, m), 3.24 (1H, d, J - 9.6 Hz), 2.76 (2H. t, J = 7.6 Hz), 2.70 (2H, t, J = 7.6 Hz), 2.52 and 2.49 (1H, dd, J = 12.8. 8.0 Hz), 2.18-1.77 (5H, m), 0.78-0.52 (4H, m) | 432 | 1.75 |
| 5AC | 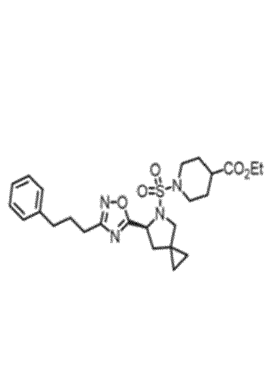 | 502.63 | (CDCl3) *δ*: 7.37-7.13 (5H, m), 5.21 and 5.19 (1H, dd, J = 8.4, 4.0 Hz), 4.11 (2H. q, J = 7.2 Hz), 3.75-3.59 (2H. m). 3.58 (1H, d. J - 9.6 Hz), 3.25 (1H, d, J = 9.6 Hz), 2.88-2.77 (2H, m), 2.76 (2H, t, J = 7.6 Hz), 2.70 (2H, t, J = 7.6 Hz), 2.47 and 2.44 (1H, dd, J - 12.8, 8.4 Hz), 2.31-2.22 (1H, m), 2.06 (2H, quint, J = 7.6 Hz), 2.01 and 1.99 (1H, dd, J = 12.8, 4.0 Hz), 1.95-1.84 (2H, m), 1.73-1.52 (2H. m). 1.23 (3H, t, J = 7.2 Hz). 0.82. 0.52 (4H, m) | 503 | 1.84 |

**Table 7-3 continued 3**

| [Table 19] | | | | | |
|---|---|---|---|---|---|
| 5AD | 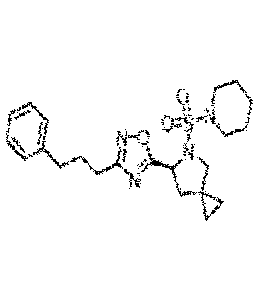 | 430.57 | (CDCl3) *δ*: 7.35-7.13 (5H. m). 5.21 and 5.19 (1H, dd, J - 8.4, 4.0 Hz), 3.59 (1H, d, J = 9.6 Hz), 3.25 (1H, d, J = 9.6 Hz). 3.23-3.08 (4H, m), 2.76 (2H, t, J - 7.6 Hz), 2.70 (2H, t, J = 7.6 Hz), 2.47 and 2.44 (1H. dd. J = 12.8, 8.4 Hz), 2.08 (2H. quint. J = 7.6 Hz), 1.98 and 1.95 (1H, dd, J = 12.8, 4.0 Hz), 1.58-1.23 (6H, m), 0.77-0.52 (4H. m) | 431 | 1.87 |
| 5AE | 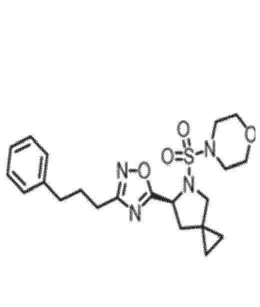 | 432.54 | (CDCl3) *δ*: 7.35-7.15 (5H, m), 5.24 and 5.22 (1H, dd, J = 8.4, 4.4 Hz),3.65-3.53 (4H, m), 3.62 (1H, d. J = 9.2 Hz), 3.27 (1H, d, J = 9.2 Hz), 3.26-3.14 (1H. m), 2.76 (2H, t, J = 7.6 Hz), 2.70 (2H, t, J = 7.6 Hz). 2.49 and 2.46 (1H, dd. J = 12.8, 8.4 Hz), 2.08 (2H, quint, J = 7.6 Hz), 2.01 and 1.98 (1H, dd, J = 12.8, 4.4 Hz), 0.78-0.53 (4H, m) | 433 | 1.74 |

| | | | | | |
|---|---|---|---|---|---|
| Ex: Example Number Cn: Compound Number MW: Molecular Weight | | | | | |

The following compounds can be prepared substantially according to the methods described in Examples 25-30.

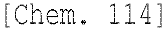

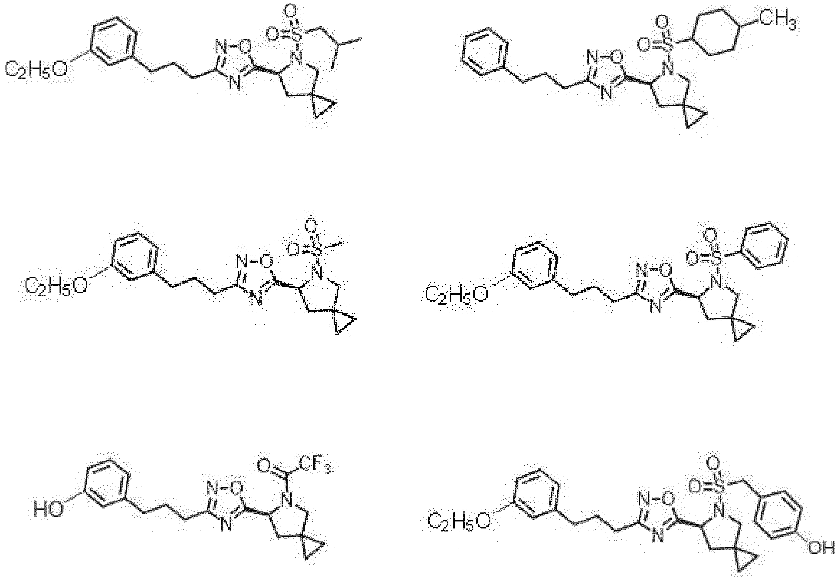

### Example 31

### 3-(3-phenylpropyl)-5-(1-isobutylsulfonyl-4-cyclobutylmethylpyrrolidin-2-yl)-1,2,4-oxadiazole 6A Example 31-1: 3-(3-phenylpropyl)-5-(1-tert-butoxycarbonyl-4-cyclobutylmethylpyrrolidin-2-yl)-1,2,4-oxadiazole 6-1

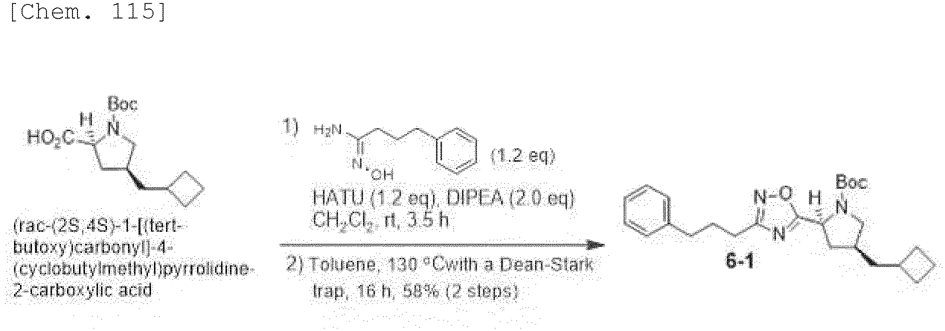

(rac-(2S,4S)-1-[(tert-butoxy)carbonyl]-4-(cyclobutyl methyl)pyrrolidine-2-carboxylic acid (283.4 mg, 1.0 mmol) was added to a 25 ml eggplant-shaped flask, and was dissolved in dichloromethane (7.0 ml). HATU (456.3 mg, 1.2 mmol) and diisopropylethylamine (0.34 ml, 2.0 mmol) were then added thereto and the mixture was stirred for 10 minutes at room temperature under a nitrogen atmosphere. N'-hydroxy-4-phenylbutanimidamide (213.9 mg, 1.2 mmol) was then added thereto while washing with dichloromethane (3.0 ml) and the mixture was stirred at room temperature for 3.5 hours.

The stirrer bar was removed and dichloromethane (20.0 ml) and 5% sodium bicarbonate aqueous solution (10.0 ml) were added to the reaction mixture. The separated organic layer was washed with distilled water (10.0 ml) and was dried over anhydrous magnesium sulfate. After filtration, the solvent was distilled off to afford a mixture of the imidamide intermediate and urea compound (740.0 mg). The mixture (740.0 mg) was dissolved in toluene (18.0 ml) in a 25 ml eggplant-shaped flask, which was placed in a Dean-Stark trap, and the mixture was heated to reflux at an oil bath temperature of 130°C for 16 hours. The solvent was distilled off under reduced pressure and the resulting residue was purified on silica gel column Q-Pac SI30 size 20 (hexane:ethyl acetate = 90:10 to 65:35). After distilling off the solvent, 247.5 mg (0.582 mmol, 58% (after 2 steps)) of the desired compound 6-1 (oil) was obtained.
Molecular Weight: 425.57;
1H-NMR δ ppm (CDCl3: a mixture of the rotational isomers) δ: 7.30-7.13 (5H, m), 5.02-4.85 (1H, m), 3.87-3.67 (1H, m), 3.15-3.06 (1H, m), 2.74 (2H, t, J = 7.6 Hz), 2.71 (2H, t, J = 7.6 Hz), 2.52-2.42 (1H, m), 2.35-2.22 (1H, m), 2-51-2.00 (1H, m), 2.07 (2H, quint, J = 7.6 Hz), 1.92-1.47 (9H, m), 1.42 and 1.25 (9H, s).
[M+H] ; 426
RT (minute); 2.26.

### Example 31-2: 3-(3-phenylpropyl)-5-(4-cyclobutylmethyl pyrrolidin-2-yl)-1,2,4-oxadiazole 6-2

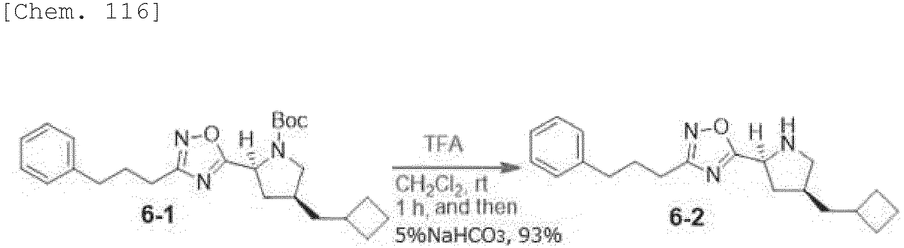

To a 25 ml eggplant-shaped flask, compound 6-1 (241.7 mg, 0.568 mmol) prepared in Example 31-1 was added, and dichloromethane (6.0 ml) and TFA (0.870 ml) were added thereto, followed by stirring the mixture at room temperature for 1 hour. After removing the stirrer bar and distilling off the solvent, the process of dissolving the residue in chloroform (10 ml) and conducting the distillation was repeated three times to remove the TFA. 5 % sodium bicarbonate aqueous solution (10 ml) was added to the residue to make it basic and the mixture was extracted with ethyl acetate (50 ml) . The organic layer was washed with distilled water (10 ml) and saturated brine (10 ml), then dried over anhydrous magnesium sulfate. After filtration, the solvent was distilled off under reduced pressure to afford 172.1 mg (0.529 mmol, 93%) of the desired compound 6-2 (oil).

### Example 31-3: 3-(3-phenylpropyl)-5-(1-isobutylsulfonyl-4-cyclobutylmethylpyrrolidin-2-yl)-1,2,4-oxadiazole 6A

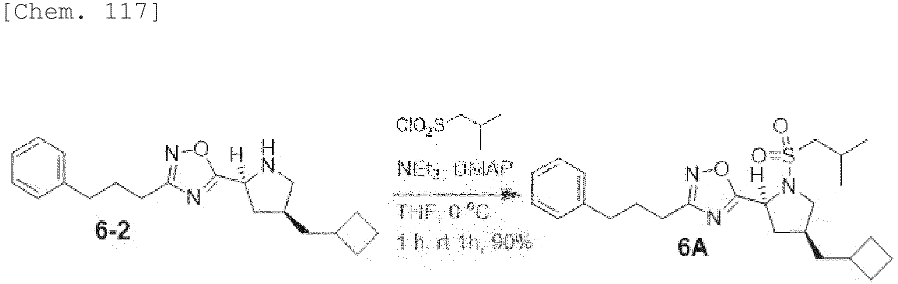

Ultra-dehydrated THF (1.5 ml), triethylamine (0.032 ml, 0.227 mmol) and DMAP (2.8 mg, 0.023 mmol) were added sequentially to a 4.0 ml screw-tube vial containing compound 6-2 (24.59 mg, 0.075 mmol) prepared in Example 31-2, and the mixture was cooled to 0°C, which was added with a solution of isobutylsulfonyl chloride (0.020 ml, 0.151 mmol) in ultra-dehydrated THF (0 .15 ml), followed by stirring the mixture at 0°C for 1 hour and at room temperature for 1 hour. The reaction was quenched by adding 0.2 M hydrochloric acid aqueous solution (1 ml) and the mixture was extracted with ethyl acetate (6 ml). After distilling off the solvent under reduced pressure, the residue was purified by Si02-PTLC (PLC Silica Gel 60 F254, 0.5 mm layer thickness, 20 x 20 cm; developing solvent: hexane:ethyl acetate = 3:1). The silica gel of the target portion was scraped off and eluted with chloroform containing 10% MeOH (20 ml). The solvent was distilled off under reduced pressure to afford 30.3 mg (0.068 mmol, 90%) of the desired compound 6A (oil).

### Example 32

### 3-(3-phenylpropyl)-5-(1-cyclohexylsulfonyl-4-cyclobutyl methylpyrrolidin-2-yl)-1,2,4-oxadiazole 6B

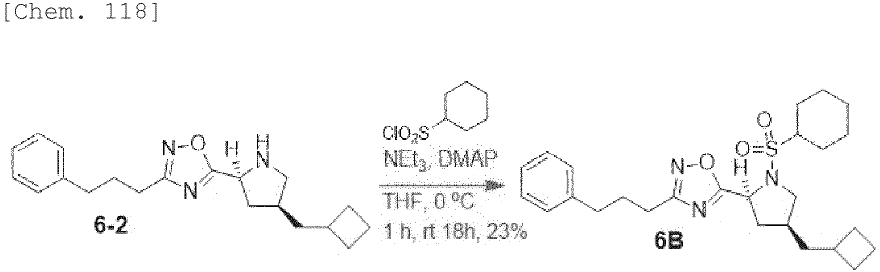

Ultra-dehydrated THF (1.5 ml), triethylamine (0.032 ml, 0.227 mmol) and DMAP (2.8 mg, 0.023 mmol) were added sequentially to a 4.0 ml screw-tube vial containing compound 6-2 (24.59 mg, 0.075 mmol) prepared in Example 31-2, and the mixture was cooled to 0°C, which was added with a solution of cyclohexanesulfonyl chloride (0.024 ml, 0.151 mmol) in ultra-dehydrated THF (0.15 ml), followed by stirring the mixture at 0°C for 1 hour and at room temperature for 18 hours. The reaction was quenched by adding 0.2 M hydrochloric acid aqueous solution (1 ml) and the mixture was extracted with ethyl acetate (6 ml). After distilling off the solvent under reduced pressure, the residue was purified by SiO2-PTLC (PLC Silica gel 60 F254, 0.5 mm layer thickness, 20 x 20 cm; developing solvent: hexane:ethyl acetate = 3:1) . The silica gel of the target portion was scraped off and eluted with chloroform containing 10% MeOH (20 ml). The solvent was distilled off under reduced pressure to afford 8.1 mg (0.017 mmol, 23%) of the desired compound 6B (oil).

### Example 33

### 3-(3-phenylpropyl)-5-(1-methanesulfonyl-4-cyclobutylmethyl pyrrolidin-2-yl)-1,2,4-oxadiazole 6C

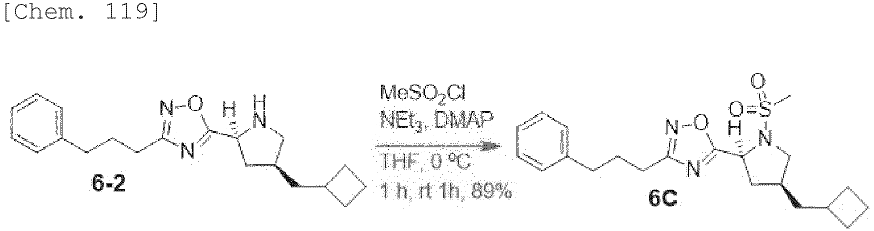

Ultra-dehydrated THF (1.5 ml), triethylamine (0.032 ml, 0.227 mmol) and DMAP (2.8 mg, 0.023 mmol) were added sequentially to a 4.0 ml screw-tube vial containing compound 6-2 (24.59 mg, 0.075 mmol) prepared in Example 31-2, and the mixture was cooled to 0° C, which was added with a solution of methanesulfonyl chloride (0.012 ml, 0.151 mmol) in ultra-dehydrated THF (0.15 ml), followed by stirring the mixture at 0°C for 1 hour and at room temperature for 1 hour. The reaction was quenched by adding 0.2 M hydrochloric acid aqueous solution (1 ml) and the mixture was extracted with ethyl acetate (6 ml). After distilling off the solvent under reduced pressure, the residue was purified by SiO2-PTLC (PLC Silica Gel 60 F254, 0.5 mm layer thickness, 20 x 20 cm; developing solvent: hexane:ethyl acetate = 3:1). The silica gel of the target portion was scraped off and eluted with chloroform containing 10% MeOH (20 ml). The solvent was distilled off under reduced pressure to afford 27.2 mg (0.067 mmol, 89%) of the desired compound 6C (oil).

### Example 34

### 3-(3-phenylpropyl)-5-(1-benzenesulfonyl-4-cyclobutylmethyl pyrrolidin-2-yl)-1,2,4-oxadiazole 6D

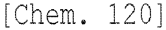

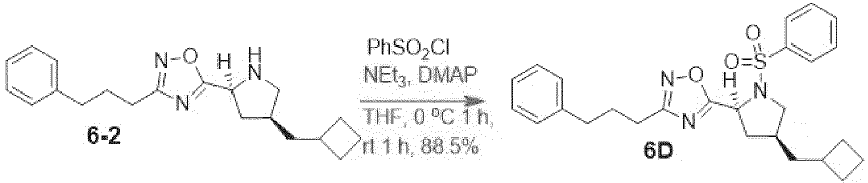

Ultra-dehydrated THF (1.5 ml), triethylamine (0.032 ml, 0.227 mmol) and DMAP (2.8 mg, 0.023 mmol) were added sequentially to a 4.0 ml screw-tube vial containing compound 6-2 (24.59 mg, 0.075 mmol) prepared in Example 31-2, and the mixture was cooled to 0°C, which was added with a solution of benzenesulfonyl chloride (0.027 ml, 0.128 mmol) in ultra-dehydrated THF (0.15 ml), followed by stirring the mixture at 0°C for 1 hour and at room temperature for 1 hour. The reaction was quenched by adding 0.2 M hydrochloric acid aqueous solution (1 ml) and the mixture was extracted with ethyl acetate (6 ml). After distilling off the solvent under reduced pressure, the residue was purified by SiO2-PTLC (PLC Silica Gel 60 F254, 0.5 mm layer thickness, 20 x 20 cm; developing solvent: hexane:ethyl acetate = 3:1). The silica gel of the target portion was scraped off and eluted with chloroform containing 10% MeOH (20 ml). The solvent was distilled off under reduced pressure to afford 31.1 mg (0.067 mmol, 88.5%) of the desired compound 6D (oil).

### Example 35

### 3-(3-phenylpropyl)-5-(1-benzylsulfonyl-4-cyclobutylmethyl pyrrolidin-2-yl)-1,2,4-oxadiazole 6E

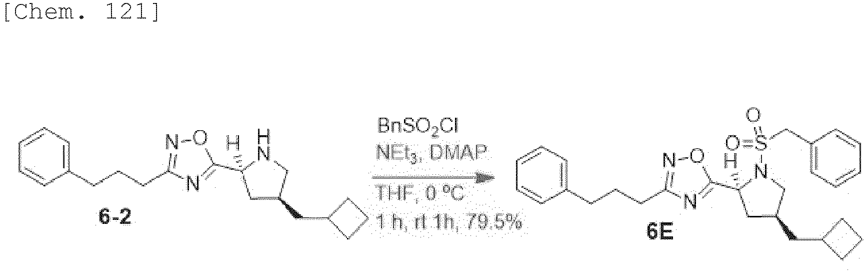

Ultra-dehydrated THF (1.5 ml), triethylamine (0.032 ml, 0.227 mmol) and DMAP (2.8 mg, 0.023 mmol) were added sequentially to a 4.0 ml screw-tube vial containing compound 6-2 (24.59 mg, 0.075 mmol) prepared in Example 31-2, and the mixture was cooled to 0°C, which was added with a solution of benzylsulfonyl chloride (28.8 mg, 0.151 mmol) in ultra-dehydrated THF (0.15 ml), followed by stirring the mixture at 0°C for 1 hour and at room temperature for 1 hour. The reaction was quenched by adding 0.2 M hydrochloric acid aqueous solution (1 ml) and the mixture was extracted with ethyl acetate (6 ml). After distilling off the solvent under reduced pressure, the residue was purified by SiO2-PTLC (PLC Silica Gel 60 F254, 0.5 mm layer thickness, 20 x 20 cm; developing solvent: hexane:ethyl acetate = 3:1). The silica gel of the target portion was scraped off and eluted with chloroform containing 10% MeOH (20 ml). The solvent was distilled off under reduced pressure to afford 28.8 mg (0.060 mmol, 79.5%) of the desired compound 6E (oil).

The compounds prepared in Examples 31-35 are described in Table 8-1 along with their physical property data.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acid-acetonitrile;
0-0.9 min linear gradient A:B (95:5)-A:B (10:90), 0.9-3 min A:B (10:90)

**Table 8-1**

| [Table 20] | | | | | | |
|---|---|---|---|---|---|---|
| **Ex** | **Cn** | **Chemical Structure** | **MW** | **¹H-NMR δ ppm** | **[M+H]** | **RT :Min** |
| **31** | **6A** | 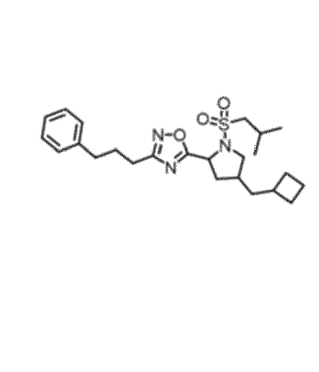 | **445.62** | **(COCl3) *δ*: 7.30-7.15 (5H, m), 5.20 and 5.18 (1H, dd, J = 8.4, 7.6 Hz), 3.88 and 3.86 (1H, dd, J = 10. 7.6 Hz), 2.93 (1H, t. J = 10 Hz), 2.93 (2H, d, J - 6.8 Hz), 2.73 (2H, t, J - 8.0 Hz), 2.70 (2H, t. J = 8.0 Hz), 2.65-2.55 (1H, m), 2.30-2.15 (3H, m), 2.08 (2H, quint, J = 8.0 Hz), 2.1-2.0 (1H, m), 1.90-1.73 (3H, m), 1.65-1.55 (3H, m), 1.50 (2H, t, J = 7.2 Hz). 1.0**5 **(3H, d, J = 6.8 Hz). 1.02 (3H, d. J - 6.8 Hz)** | **446** | **2.03** |
| **32** | **6B** | 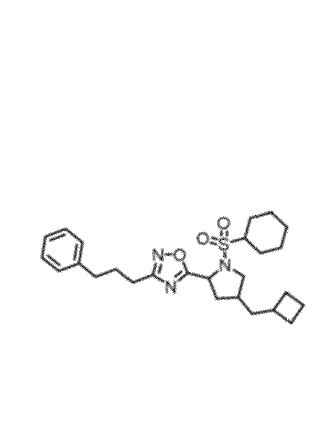 | **471.66** | **(CDCl3) *δ*: 7.30-7.15 (5H, m), 5.27 and 5.25 (1H. dd, J = 8.8, 8.0 Hz), 3.92 and 3.90 (1H, dd, J = 9.6, 7.2 Hz), 2.97 (1H. t. J = 9.6 Hz), 2.93-2.85 (1H, m), 2.74 (2H, t. J = 7.6 Hz), 2.70 (2H, t, J - 7.6 Hz), 2.64-2.57 (1H, m), 2.35-2.15 (2H, m), 2.07 (2H, quint, J = 7.6 Hz), 2.15-2.0 (3H, m), 1.90-1.68 (4H, m), 1.49 (2H, t, J = 7.2 Hz), 1.65-1.35 (6H, m), 1.35-1.06 (5H, m)** | **472** | **2.25** |

**Table 8-1 continued**

| [Table 21] | | | | | | |
|---|---|---|---|---|---|---|
| **33** | **6C** | 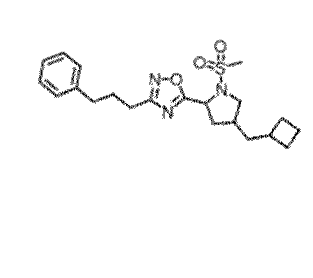 | **403.54** | **(CDCl3) *δ*: 7.35-7.15 (5H, m). 5.16 and 5.13 (1H, dd, J = 8.8, 8.0 Hz), 3.86 and 3.84 (1H, dd, J = 10, 7.6 Hz), 3.05 (1H, t. J = 10 Hz). 2.96 (3H, s), 2.76 t2H, t, J = 8.0 Hz), 2.70 (2H. t, J = 8.0 Hz). 2.65-2.55 (1H, m), 2.32-2.30 (2H, m), 2.06 (2H, quint, J = 8.0 Hz), 2.10-2.0 (1H, m), 1.90-1.74 (3H, m), 1.65-1.53 (3H, m), 1.51 (2H, t, J = 7.2 Hz)** | **404** | **1.85** |
| **34** | **6D** | 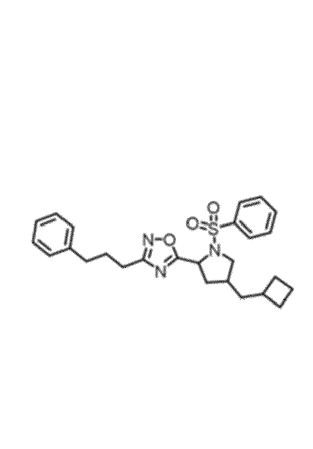 | **465.61** | **(CDCl3) *δ*: 7.85-7.80 (2H, m). 7.60-7.45 (3H. m), 7.30-7.15 (5H, m). 4.92 and 4.90 (1H, dd, J = 8.4. 7.6 Hz). 3.75 and 3.72 (1H, dd. J = 10.4, 7.2 Hz), 3.11 (1H, t, J 10.4 Hz), 2.72 (2H, t, J = 7.6 Hz), 2.69 (2H, t, J = 7.6 Hz), 2.46-2.38 (1H, m), 2.22-2.13 (1H, m), 2.06 (2H, quint, J = 7.6 Hz), 2.04-1.93 (2H, m), 1.90-1.70 (4H, m), 1.60-1.48 (2H, m), 1.45 (2H, t. J = 6.8 Hz)** | **466** | **2.01** |
| **35** | **6E** | 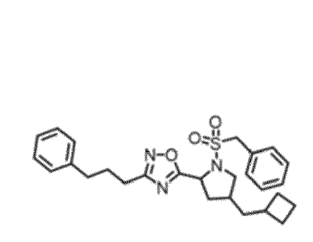 | **479.64** | **(CDCl3) *δ*: 7.50-7.15 (10H. m), 5.17 and 5.15 (1H, dd, J :, 8.4, 7.6 Hz). 4.41 (1H. d, J = 13.2 Hz), 4.28 (1H, d, J - 13.2 Hz), 3.46 and 3.44 (1H, dd, J = 10, 6.8 Hz), 2.79 (2H, t. J** | **480** | **2.01** |

**Table 8-1 continued 2**

| [Table 22] | | | | | | |
|---|---|---|---|---|---|---|
| | | | | **= 8.0 Hz), 2.72 (2H. t, J = 8.0 Hz). 2.58-2.52 (1H, m), 2.47 (1H, t, J = 10 Hz), 2.15-2.0 (1H, m), 2.0-1.9 (2H. m), 1.85-1.68 (3H, m), 1.58-1.42 (2H, m), 1.41-1.30 (2H, m)** | | |

| | | | | | | |
|---|---|---|---|---|---|---|
| Ex: Example Number Cn: Compound Number MW: Molecular Weight | | | | | | |

Eleven compounds listed in Table 8-2 were synthesized by using various sulfonyl chlorides in place of isobutyl sulfonyl chloride under the same reaction conditions as in Example 31-3.

**Table 8-2**

| [Table 23] | | | |
|---|---|---|---|
| **Compound Number** | **Compound Name** | **Sulfonyl Chlorides** | **Yield (%)** |
| **6AI** | **5-(4-(cydobuthyimethyl)-1-((4-inethoxy phenyl)sulfonyl)pyrrolidin-2-yl)-3-(3-phenylpropyl)-1,2,4-oxadiazole** | 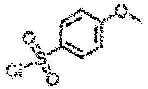 | **66** |
| **6AJ** | **5-(4-(cydobuthylmethyl)-1-((4-fluorophenyl) sulfonyl)pynoHdin-2-yl)-3-(3-phenylpropyl)-1,2,4-oxadiazole** | 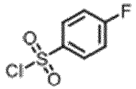 | **71** |
| **6AK** | **4-((4-(cydobuthylmethyl)-2-(3-(3-phenyl propyl)-1,2,4-oxadiazole-5-yl)pyrrolidin-1-yl)sutfonyl)-N,N-dimethylaniline** | 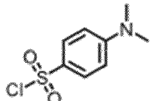 | **58** |
| **6AL** | **5-((4-(cyclobuthyimethyl)-2-(3-(3-phenyl propyl)-1,2,4-oxadiazole-5-yl)pyrrolidin-1-yl)sulfonyl)-N,N-dimethylnaphthalen-1-amine** | 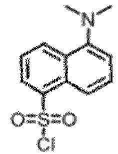 | **79** |
| **6AM** | **tert-buthyl 3-(((4-(cyclobuthylmethyl)-2-(3-(3-phenylpropyl)-1,2,4-oxadiazole-5-yl) pyrrolidin-1-yl)sulfonyl)methyl)azetidin-1-carboxylate** | 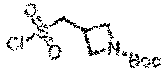 | **34** |
| **6AN** | **tert-buthyl 4-(((4-(cyclobuthylmethyl)-2-(3-(3-phenylpropyl)-1,2,4-oxadiazole-5-yl) pyrrolidin-1-yl)sulfonyl)methyl)piperidin-1-carboxylate** | 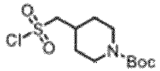 | **68** |
| **6AO** | **tert-buthyl 4-((4-(cydobuthylmethyl)-2-(3-(3-phenylpropyl)-1,2,4-oxadiazole-5-yl) pyrrolidin-1-yl)sulfonyl)piperidin-1-carboxylate** | 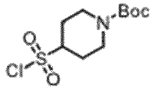 | **35** |
| **6AP** | **5-(4-(cydobuthylmethyl)-1-((tetrahydro-2H-pyran-4-yl)sulfonyl)pyrrolidin-2-yl)-3-(3-phenyfpropyl)-1,2,4-oxadiazole** | 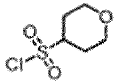 | **23** |

**Table 8-2 continued**

| [Table 24] | | | |
|---|---|---|---|
| **6AC** | **ethyl 1-((4-(cyclobuthylmethyl)-2-(3-(3-phenylpropyl)-1,2,4-oxadiazole-5-yl) pyrrolidin-1-yl)sulfonyl)piperidin-4-carboxylate** | 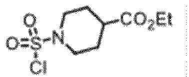 | **74** |
| **6AD** | **6-(4-(cyclobuthylmethyt)-1-(piperidin-1-ylsulfonyl)pyrrolidin-2-yl)-3-(3-phenylpropyl)- 1,2,4-oxadiazole** | 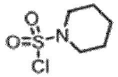 | **82** |
| **6AE** | **4-((4-(cyclobuthylmethyl)-2-(3-(3-phenylpropyl)-1,2,4-oxadiazole-5-yl) pyrrolidin-1-yyl)sulfonyl)morpholine** | 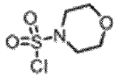 | **69** |

The structural formula, molecular weight, 1H-NMR, [M+H], and RT of the above compounds are summarized in Table 8-3.

**Table 8-3**

| [Table 25] | | | | | |
|---|---|---|---|---|---|
| **Ex** | **Chemical Structure** | **MW** | **1H-NMR δ ppm** | **[M +H]** | **RT :Min** |
| **6Al** | 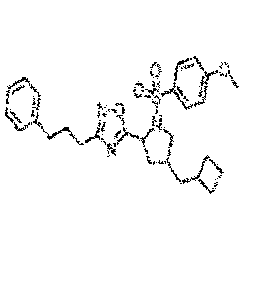 | **495.64** | **(COCl3) *δ*: 7.76 (2H, d, J = 6.8 Hz), 7.34-7.11 (5H. m), 6.95 (2H. d, J = 6.8 Hz), 4.88 and 4.86 (1H, dd, J = 8.6, 7.4 Hz), 3.83 (3H, s), 3.71 and 3.69 (1H, dd, J = 10,6. 7.0 Hz), 3.09 (1H, t. J = 10.6 Hz), 2.73 (2H, t. J - 7.2 Hz), 2.69 (2H, t. J = 7.2 Hz). 2.47-2.35 (1H, m), 2.25-2.13 (1H, m), 2.06 (2H, quint, J = 7.2 Hz), 2.04-1.92 (2H, m), 1.91-1.70 (4H, m), 1.60-1.48 (3H, m), 1.45 (2H, t, J = 7.2 Hz)** | **496** | **2.02** |
| **6AJ** | 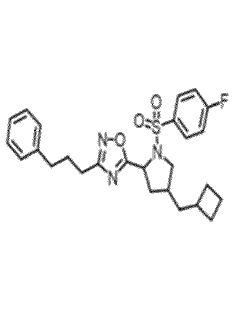 | **483.60** | **(CDCl3) *δ*: 7.90-7.78 (2H, m). 7.33-7.18 (7H, m). 4.94 and 4.92 (1H, dd, J = 8.8, 7.6 Hz), 3.73 and 3.71 (1H, dd, J 10.4. 7.2 Hz), 3.08 (1H, t. J = 10.4 Hz), 2.72 (2H, t, J = 7.2 Hz), 2.69 (2H, t. J = 7.2 Hz), 2.52-2.40 (1H, m). 2.27-2.14 (1H, m), 2.12-1.92 (2H, m). 2.04 (2H, quint, J = 7.2 Hz), 1.88-1.70-1.92 (4H. m), 1.60-1.50 (3H, m), 1.47 12H, t, J = 7.2 Hz)** | **484** | **2.01** |
| **6AK** | 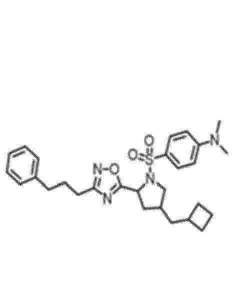 | **508.68** | **(CDCl3) *δ*: 7.65 (2H, d, J = 9.2 Hz), 7.48-7.12 (5H. m), 6.63 (2H, d, J = 9.2 Hz), 4.85 and 4.83 (1H. dd, J = 8.7, 7.6 Hz), 3.70 and 3.67 (1H, dd, J = 10,4, 7.2 Hz), 3.08 (1H, t. J = 10.4 Hz), 3.00 (6H, s), 2.72 t2H, t, J = 7.2 Hz), 2.68 (2H, t. J - 7.2 Hz), 2.43-2.34 (1H, m), 2.25-2.13 (1H. m). 2.06 (2H, quint, J = 7.2 Hz), 2.03-1.92 (2H, m), 1.88-1.70 (4H. m), 1.59-1.48 (3H, m), 1.44 (2H, t, J = 7.2 Hz)** | **509** | **2.06** |

**Table 8-3 continued**

| [Table 26] | | | | | |
|---|---|---|---|---|---|
| **6AL** | 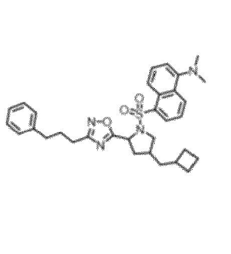 | **558.74** | **(CDCl3) *δ*: 8.48 (1H, d, J = 8.4 Hz), 8.30 (1H, d, J = 8.8 Hz), 8.19 and 8.17 (1H, dd, J = 7.2, 1.6 Hz), 7.54-7.38 (2H, m), 7.32-7.08 (6H, m), 5.13 and 5.10 (1H, dd, J = 8.8, 7.6 Hz), 4.02 and 3.99 (1H. dd. J - 10.4, 7.2 Hz), 3.09 (1H. t. J - 10.4 Hz), 2.83 (6H, s), 2.62 (2H, t, J = 7.2 Hz), 2.53 (2H, t. J = 7.2 Hz), 2.52-2.43 (1H, m), 2.27-2.14 (1H, m), 2.12-1.95 (3H, m), 1.93 (2H, quint, J = 7.2 Hz), 1.88-1.72 (3H. m), 1.63-1.50 (3H. m), 1.47 (2H, t, J - 7.2 Hz)** | **559** | **2.44** |
| **6AM** | 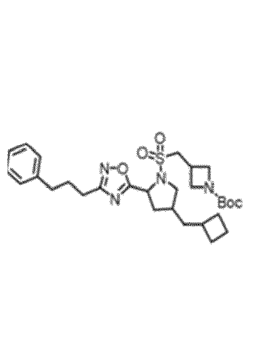 | **558.74** | **(CDCl3) *δ*: 7.34-7.12 (5H, m), 5.21 and 5.19 (1H, dd, J = 8.8, 7.6 Hz), 4.13-4.02 (2H, m), 3.89-3.81 (1H, m), 3.77-3.65 (2H, m), 3.38-3.30 (2H, m), 3.10-3.02 (1H, m), 2.92 (1H. t. J = 10.4 Hz), 2.75 (2H. t. J = 7.2 Hz), 2.70 (2H, t, J = 7.2 Hz), 2.67-2.56 (1H, m), 2.32-2.18 (2H, m), 2.12-1.98 (2H, m), 2.07 (2H, quint, J = 7.2 Hz), 1.92-1.72 (3H, m), 1.67-1.56 (3H, m), 1.50 (2H, t, J = 7.2 Hz), 1.42 (9H, s)** | **559** | **2.01** |
| **6AN** | 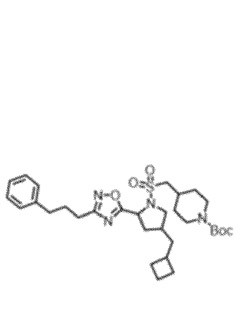 | **586.79** | **(CDCl3) *δ*: 7.33-7.12 (5H, m), 5.20 and 5.18 (1H, dd, J -= 8.8, 7.6 Hz). 4.13-3.97 (2H, br s). , 3.88 and 3.86 (1H, dd. J = 10.0, 7.6 Hz), 3.04-2.91 (3H, m), 2.78-2.66 (2H, m), 2.74 (2H, t, J = 7.2 Hz), 2.69 (2H, t, J = 7.2 Hz). 2.65-2.54 (1H, m), 2.32-2.19 (2H, m), 2.12-2.00 (3H, m), 2.07 (2H, quint, J = 7.2 Hz), 1.92-1.72 (5H, m), 1.66-1.55 (3H, m), 1.50 (2H, t, J - 7.2 Hz), 1.44 (9H, s)** | **587** | **2.12** |

**Table 8-3 continued 2**

| [Table 27] | | | | | |
|---|---|---|---|---|---|
| **6AO** | 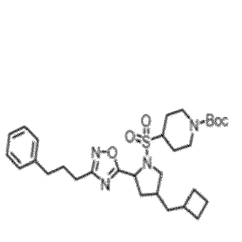 | **572.77** | **(CDCl3) *δ*: 7.35-7.12 (5H, m), 5.28 and 5.26 (1H. dd. J = 8.8, 8.0 Hz), 4.34-4.08 (2H, br s), 3.94 and 3.92 (1H, dd, J = 10,0, 7.6 Hz), 3.12-3.00 (1H. m), 2.96 (1H, t. J = 10.4 Hz), 2.74 (2H, t. J = 7.2 Hz), 2.70 (2H, t, J - 7.2 Hz), 2.69-2.52 (3H, m), 2.32-2.17 (2H. m), 2.12-1.98 (6H, m), 1.92-1.73 (3H, m). 1.72-1.53 (5H, m), 1.49 (2H, t, J = 7.2 Hz), 1.45 (9H, s)** | **573** | **2.15** |
| **6AP** | 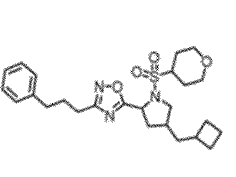 | **473.63** | **(CDCl3) *δ*: 7.35-7.12 (5H, m), 5.39 and 5.27 (1H, dd, J = 8.8, 8.0 Hz), 4.07-3.97 (2H. m), 4.01 and 3.97 (1H, dd, J = 10.0. 7.6 Hz), 3.34-3.22 (2H, m), 3.18-3.00 (1H. m), 2.96 (1H, t. J = 10.4 Hz), 2.75 (2H. t, J - 7.2 Hz), 2.70 (2H, t, J = 7.2 Hz), 2.66-2.57 (1H. m), 2.33-2.17 (2H, m), 2.13-2.01 (3H, m). 1,99-1.92 (2H, m), 1.89-1.71 (4H, m), 1.66-1.53 (4H, m), 1.49 (2H, t, J = 7.2 Hz)** | **474** | **1.93** |
| **6AC** | 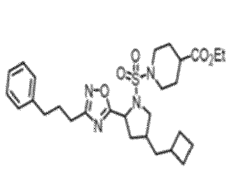 | **544.71** | **(CDCl3) *δ*: 7.34-7.12 (5H, m), 5.01 and 4.99 (1H, dd, J = 9.2, 7.2 Hz), 4.11 (2H, q, J = 7.2 Hz), 3.78 and 3.75 (1H, dd, J - 10.4. 7.6 Hz), 3.69-3.62 (1H. m), 3.58-3.52 (1H, m), 2.99 (1H, t. J = 10.4 Hz), 2.82-2.67 (1H. m), 2.75 (2H, t, J - 7.2 Hz), 2.70 (2H, t, J - 7.2 Hz), 2.58-2.47 (1H, m), 2.35-2.19 (3H, m), 2.12-1.99 (2H, m), 2.07 (2H, quint, J = 7.2 Hz), 1,92-1.83 (3H, m), 1.68-1.56 (4H, m), 1.51 (2H, t, J = 7.2 Hz). 1.25 (3H. t, J = 7.2 Hz)** | **545** | **2.08** |

**Table 8-3 continued 3**

| [Table 28] | | | | | |
|---|---|---|---|---|---|
| **6AD** | 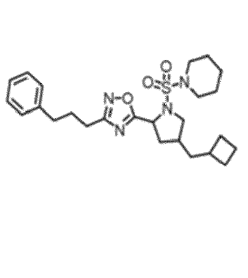 | **472.65** | **(CDCl3) *δ*: 7.35-7.13 (5H. m), 5.02 and 4.99 (1H. dd. J = 9.2, 7.6 Hz), 3.78 and 3.76 (1H. dd, J = 10,2, 7.8 Hz), 3.21-3.04 (4H, m), 2.97 (1H, t. J = 10.4 Hz). 2.75 (2H, t, J - 7.2 Hz), 2.70 (2H, t, J = 7.2 Hz), 2.57-2.47 (1N, m), 2.32-2.28 (2H, m), 2.12-1.99 (2H. m), 2.07 (2H, quint, J = 7.2 Hz), 1.93-1.69 (3H. m), 1.66-1.56 (3H, m), 1.56-1.39 (7H, m), 1.51 (2H, t, J = 7.2 Hz)** | **473** | **2.16** |
| **6AE** | 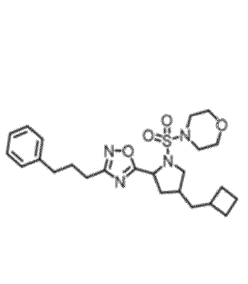 | **474.62** | **(CDCl3) *δ*: 7.33-7.11 (5H, m). 5.05 and 5.03 (1H, dd, J = 9.2, 7.6 Hz), 3.81 and 3.79 (1H. dd, J = 10,2. 7.8 Hz). 3.63-3.52 (4H, m), 3.23-3.15 (2H, m), 3.14-3.06 (2H, m), 3.01 (1H, t. J - 10.4 Hz). 3.01 (2H, t, J - 7.2 Hz), 2.75 (2H, t, J - 7.2 Hz). 2.71-2.50 (1H, m), 2.33-2.18 (2H, m), 2.13-1.99 (2H, m), 2.07 (2H, quint, J = 7.2 Hz), 1.92-1.72 (3H, m), 1.67-1.56 (4H, m), 1.51 (2H, t, J = 7.2 Hz)** | **475** | **1.94** |

| | | | | | |
|---|---|---|---|---|---|
| Ex: Example Number Cn: Compound Number MW: Molecular Weight | | | | | |

The following compounds can be prepared substantially according to the methods described in Examples 31-35.

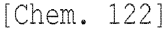

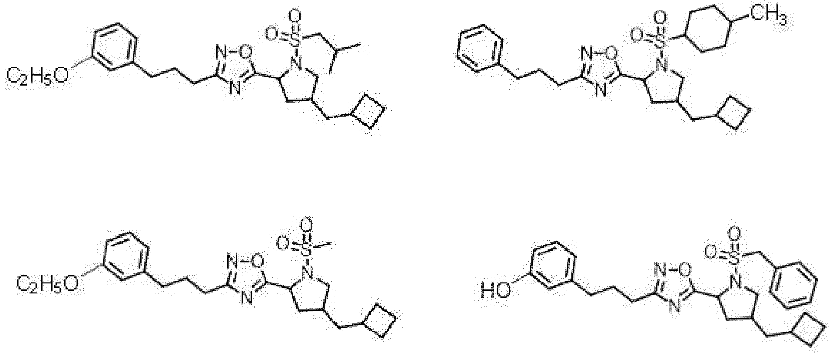

### Example 36

### 3-(3-Phenylpropyl)-5-[(2S,4S)-1-isobutylsulfonyl-4-phenyl pyrrolidin-2-yl]-1,2,4-oxadiazole 7A

### Example 36-1: 3-(3-phenylpropyl)-5-[(2S,4S)-1-tert-butoxy carbonyl-4-phenylpyrrolidin-2-yl]-1,2,4-oxadiazole 7-1

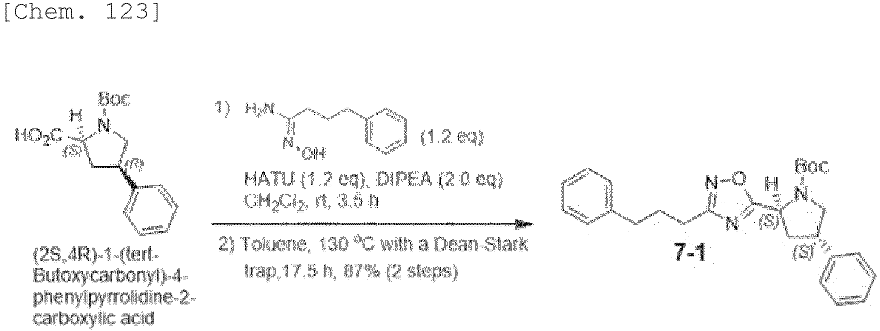

(2S,4S)-1-(tert-butoxycarbonyl)-4-phenylpyrrolidine-2-carboxylic acid (291.3 mg, 1.0 mmol) was added to a 25 ml eggplant-shaped flask and was dissolved in dichloromethane (7.0 ml) . HATU (456.3 mg, 1.2 mmol) and diisopropyl ethylamine (0.34 ml, 2.0 mmol) were then added thereto and the mixture was stirred for 10 minutes at room temperature under a nitrogen atmosphere. N'-hydroxy-4-phenyl butanimidamide (213.9 mg, 1.2 mmol) was then added while washing with dichloromethane (3.0 ml) and the mixture was stirred at room temperature for 3.5 hours.

The stirrer bar was removed and dichloromethane (20.0 ml) and 5% sodium bicarbonate aqueous solution (10.0 ml) were added to the reaction mixture. The separated organic layer was washed with distilled water (10.0 ml) and dried over anhydrous magnesium sulfate. After filtration, the solvent was distilled off to afford a mixture of the imidamide intermediate and urea compound (740.0 mg). The mixture (740.0 mg) was dissolved in toluene (18.0 ml) in a 25 ml eggplant-shaped flask, which was placed in a Dean-Stark trap, and the mixture was heated to reflux at an oil bath temperature of 130°C for 16 hours. The solvent was distilled off under reduced pressure and the resulting residue was purified on silica gel column Q-Pac SI30 size 20 (hexane:ethyl acetate = 90:10 to 40:60). After distilling off the solvent, 378.1 mg (0.872 mmol, 87% (after 2 steps)) of the desired compound 7-1 (oil) was obtained.
Molecular Weight: 433.55;
1H-NMR δ ppm (CDCl3: a mixture of the rotational isomers) δ: 7.38-7.15 (10H, m), 5.31 and 5.17 (1H, d, J=7.6 Hz), 4.18-4.02 (1H, m), 3.76-3.63 (1H, m), 3.56-3.38 (1H, m), 2.77 (2H, t, J = 7.6 Hz), 2.72 (2H, t, J = 7.6 Hz), 2.58-2.34 (2H, m), 2.10 (2H, quint, J = 7.6 Hz), 1.45 and 1.33 (9H, s).
[M+H]; 434
RT (minute); 2.00.

### Example 36-2: 3-(3-phenylpropyl)-5-[(2S,4S)-4-phenyl pyrrolidin-2-yl]-1,2,4-oxadiazole 7-2

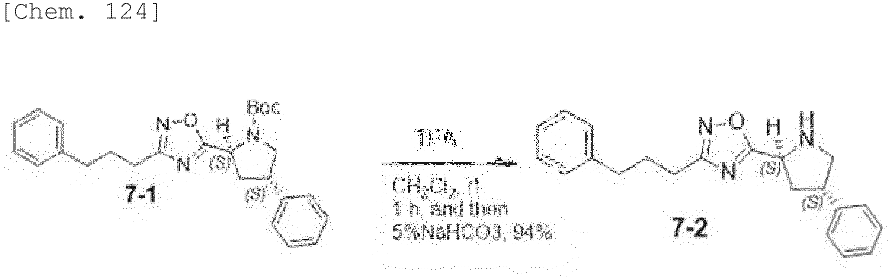

Compound 7-1 (370.1 mg, 0.854 mmol) prepared in Example 36-1 was added to a 25 ml eggplant-shaped flask, and dichloromethane (8.7 ml) and TFA (1.306 ml) were added thereto, followed by stirring the mixture at room temperature for 1 hour. After removing the stirrer bar and distilling off the solvent, the process of dissolving the residue in chloroform (10 ml) and conducting the distillation was repeated three times to remove the TFA. 5 % sodium bicarbonate aqueous solution (10 ml) was added to the residue to make it basic and the mixture was extracted with ethyl acetate (50 ml) . The organic layer was washed with distilled water (10 ml) and saturated brine (10 ml), and then the mixture was dried over anhydrous magnesium sulfate. After filtration, the solvent was distilled off under reduced pressure to afford 267.4 mg (0.802 mmol, 94%) of the desired compound 7-2 (oil).

### Example 36-3: 3-(3-phenylpropyl)-5-[(2S,4S)-1-isobutyl sulfonyl-4-phenylpyrrolidin-2-yl]-1,2,4-oxadiazole 7A

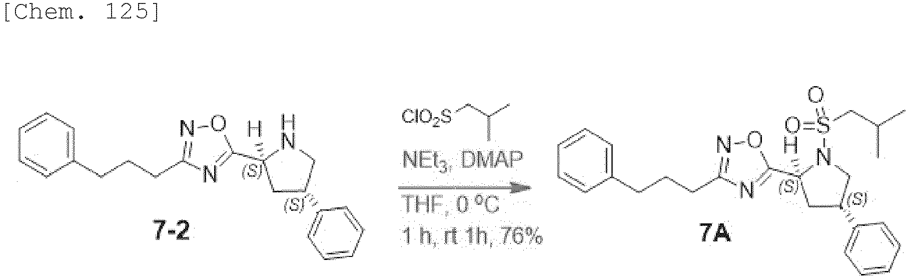

Ultra-dehydrated THF (1.75 ml), triethylamine (0.042 ml, 0.30 mmol) and DMAP (3.7 mg, 0.030 mmol) were added sequentially to a 4.0 ml screw-tube vial containing compound 7-2 (33.43 mg, 0.10 mmol) prepared in Example 36-2, and the mixture was cooled to 0°C, which was added with a solution of isobutylsulfonyl chloride (0.027 ml, 0.20 mmol) in ultra-dehydrated THF (0.25 ml), followed by stirring the mixture at 0°C for 1 hour and at room temperature for 1 hour. The reaction was quenched by adding 0.2 M hydrochloric acid aqueous solution (1 ml) and the mixture was extracted with ethyl acetate (6 ml). After distilling off the solvent under reduced pressure, the residue was purified by SiO₂-PTLC (PLC Silica Gel 60 F254, 0.5 mm layer thickness, 20 x 20 cm; developing solvent: hexane:ethyl acetate = 3:1). The silica gel of the target portion was scraped off and eluted with chloroform containing 10% MeOH (20 ml). The solvent was distilled off under reduced pressure to afford 34.6 mg (0.076 mmol, 76%) of the desired compound 7A (oil).

### Example 37

### 3-(3-Phenylpropyl)-5-[(2S,4S)-1-cyclohexylsulfonyl-4-phenyl pyrrolidin-2-yl]-1,2,4-oxadiazole 7B

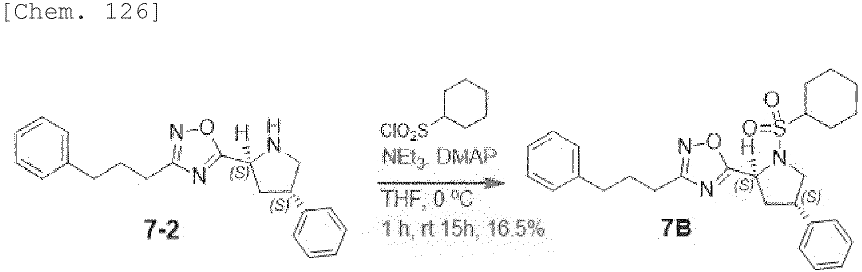

Ultra-dehydrated THF (1.75 ml), triethylamine (0.042 ml, 0.30 mmol) and DMAP (3.7 mg, 0.030 mmol) were added sequentially to a 4.0 ml screw-tube vial containing compound 7-2 (33.43 mg, 0.10 mmol) prepared in Example 36-2, and the mixture was cooled to 0°C, which was added with a solution of cyclohexanesulfonyl chloride (38.55 mg, 0.20 mmol) in THF (0.25 ml), followed by stirring the mixture at 0°C for 1 hour and at room temperature for 15 hours. The reaction was quenched by adding 0.2 M hydrochloric acid aqueous solution (1 ml) and the mixture was extracted with ethyl acetate (6 ml). After distilling off the solvent under reduced pressure, the residue was purified by SiO2-PTLC (PLC Silica gel 60 F254, 0.5 mm layer thickness, 20 x 20 cm; developing solvent: hexane:ethyl acetate = 3:1). The silica gel of the target portion was scraped off and eluted with chloroform containing 10% MeOH (20 ml). The solvent was distilled off under reduced pressure to afford 8.1 mg (0.017 mmol, 16.5%) of the desired compound 7B (oil).

### Example 38

### 3-(3-phenylpropyl)-5-[(2S,4S)-1-methanesulfonyl-4-phenyl pyrrolidin-2-yl]-1,2,4-oxadiazole 7C

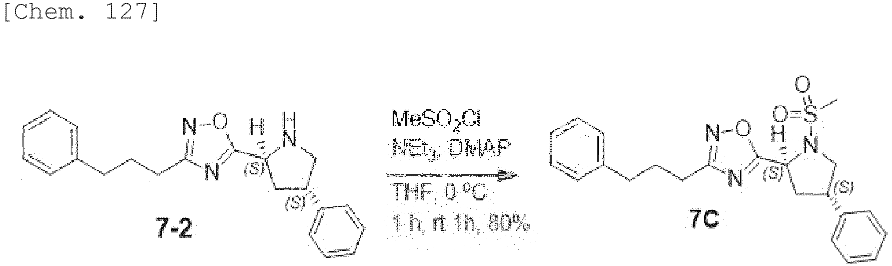

Ultra-dehydrated THF (1.75 ml), triethylamine (0.042 ml, 0.30 mmol) and DMAP (3.7 mg, 0.030 mmol) were added sequentially to a 4.0 ml screw-tube vial containing compound 7-2 (33.43 mg, 0.10 mmol) prepared in Example 36-2, and the mixture was cooled to 0° C, which was added with a solution of methanesulfonyl chloride (0.016 ml, 0.20 mmol) in ultra-dehydrated THF (0.25 ml), followed by stirring the mixture at 0°C for 1 hour and at room temperature for 1 hour. The reaction was quenched by adding 0.2 M hydrochloric acid aqueous solution (1 ml) and the mixture was extracted with ethyl acetate (6 ml). After distilling off the solvent under reduced pressure, the residue was purified by SiO2-PTLC (PLC Silica Gel 60 F254, 0.5 mm layer thickness, 20 x 20 cm; developing solvent: hexane:ethyl acetate = 2:1). The silica gel of the objective portion was scraped off and eluted with chloroform containing 10% MeOH (20 ml). The solvent was distilled off under reduced pressure to afford 33.1 mg (0.080 mmol, 80%) of the desired compound 7C (oil).

### Example 39

### 3-(3-phenylpropyl)-5-[(2S,4S)-1-benzenesulfonyl-4-phenyl pyrrolidin-2-yl]-1,2,4-oxadiazole 7D

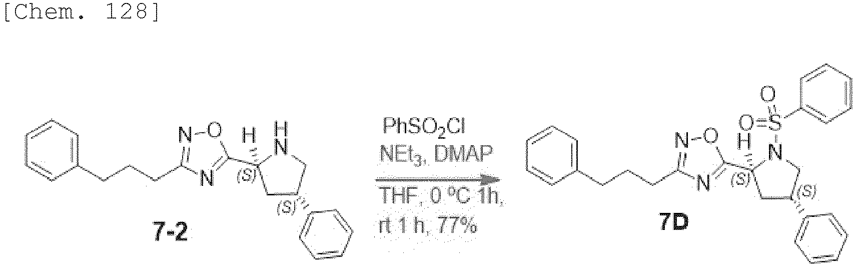

Ultra-dehydrated THF (1.75 ml), triethylamine (0.042 ml, 0.30 mmol) and DMAP (3.7 mg, 0.030 mmol) were added sequentially to a 4.0 ml screw-tube vial containing compound 7-2 (33.43 mg, 0.10 mmol) prepared in Example 36-2, and the mixture was cooled to 0°C, which was added with a solution of benzenesulfonyl chloride (0.026 ml, 0.20 mmol) in ultra-dehydrated THF (0 .25 ml), followed by stirring the mixture at 0°C for 1 hour and at room temperature for 1 hour. The reaction was quenched by adding 0.2 M hydrochloric acid aqueous solution (1 ml) and the mixture was extracted with ethyl acetate (6 ml). After distilling off the solvent under reduced pressure, the residue was purified by SiO2-PTLC (PLC Silica gel 60 F254, 0.5 mm layer thickness, 20 x 20 cm; developing solvent: hexane:ethyl acetate = 2:1). The silica gel of the target portion was scraped off and eluted with chloroform containing 10% MeOH (20 ml). The solvent was distilled off under reduced pressure to afford 36.6 mg (0.077 mmol, 77%) of the desired compound 7D (oil).

### Example 40

### 3-(3-Phenylpropyl)-5-[(2S,4S)-1-benzylsulfonyl-4-phenyl pyrrolidin-2-yl]-1,2,4-oxadiazole 7E

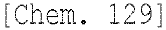

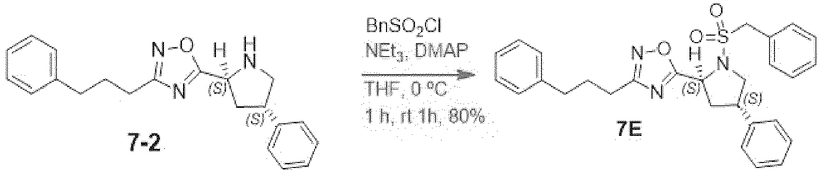

Ultra-dehydrated THF (1.75 ml), triethylamine (0.042 ml, 0.30 mmol) and DMAP (3.7 mg, 0.030 mmol) were added sequentially to a 4.0 ml screw-tube vial containing compound 7-2 (33.43 mg, 0.10 mmol) prepared in Example 36-2, and the mixture was cooled to 0° C, which was added with a solution of benzylsulfonyl chloride (38.2 mg, 0.20 mmol) in ultra-dehydrated THF (0 .25 ml), followed by stirring the mixture at 0°C for 1 hour and at room temperature for 1 hour. The reaction was quenched by adding 0.2 M hydrochloric acid aqueous solution (1 ml) and the mixture was extracted with ethyl acetate (6 ml). After distilling off the solvent under reduced pressure, the residue was purified by SiO2-PTLC (PLC Silica Gel 60 F254, 0.5 mm layer thickness, 20 x 20 cm; developing solvent: hexane:ethyl acetate = 2:1). The silica gel of the objective portion was scraped off and eluted with chloroform containing 10% MeOH (20 ml). The solvent was distilled off under reduced pressure to afford 38.5 mg (0.079 mmol, 80%) of the desired compound 7E (oil).

The compounds prepared in Examples 36-40 are described in Table 9-1 along with their physical property data.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acid-acetonitrile;
0-0.9 min linear gradient A:B (95:5)-A:B (10:90), 0.9-3 min A:B (10:90)

**Table 9-1**

| [Table 1] | | | | | | |
|---|---|---|---|---|---|---|
| Ex | Cn | Chemical Structure | MW | ¹H-NMR δ ppm | [M+H] | RT :Min |
| 36 | 7A | 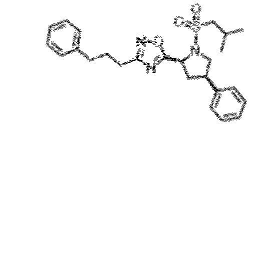 | 453.60 | (CDCl3) *δ*: 7.48-7.18 (10H, m), 5.45 and 5.44 (1H, dd, J = 6.4, 6.0 Hz), 3.89 and 3.87 (1H. dd, J = 8.4, 7.6 Hz), 3.78-3.67 (1H, m), 3.65 and 3.63 (1H. dd, J = 10, 8.4 Hz), 3.07-2.93 (2H, m), 2.78 (2H. t. J = 8.0 Hz). 2.72 (2H, t, J = 8.0 Hz), 2.58-2.52 (2H, m), 2.32-2.22 (1H, m), 2.10 (2H, quint, J = 8.0 Hz), 1.08 (3H, d, J - 6.8 Hz), 1.05 (3H, d. J = 6.8 Hz) | 454 | 1.89 |
| 37 | 7B | 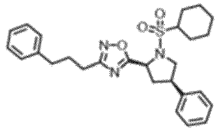 | 479.64 | (CDCl3) *δ*: 7.38-7.13 (10H, m), 5.48 and 5.46 (1H. dd, J = 7.6, 2.4 Hz), 3.87 and 3.85 (1H. dd, J = 8.0, 7.6 Hz), 3.82-3.65 (1H. m), 3.72 and 3.69 (1H, dd, J = 10, 7.6 Hz), 3.02-2.90 (1H, m), 2.77 (2H, t, J = 7.6 Hz), 2.72 (2H, t, J = 7.6 Hz), 2.60-2.44 (2H. m), 2.18-2.05 (2H. m), 2.11 (2H, quint, J = 7.6 Hz), 1.68-1.42 (2H. m), 1.38-1.10 (4H, m) | 480 | 2.00 |

**Table 9-1 continued**

| [Table 30] | | | | | | |
|---|---|---|---|---|---|---|
| | | | | | | |
| 38 | 7C | 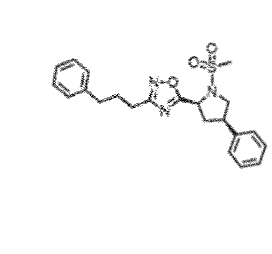 | 411.52 | (CDCl3) *δ*: 7.40-7.15 (10H, m), 5.42 and 5.41 (1H. dd, J =4.8, 2.8 Hz), 3.91 and 3.89 (1H, dd, J - 8.8, 7.6 Hz), 3.76-3.65 (1H, m), 3.63 and 3.59 (1H, dd, J = 10, 8.8 Hz), 3.02 (3H, s), 2.78 (2H, t, J - 8.0 Hz), 2.72 (1H, t, J = 8.0 Hz), 2.59-2.53 (2H, m), 2.10 (2H, quint, J = 8.0 Hz) | 412 | 1.77 |
| 39 | 7D | 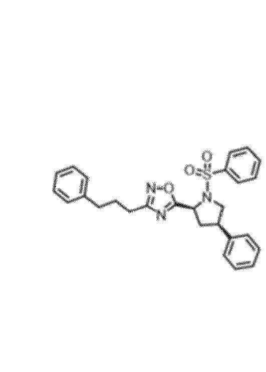 | 473.59 | (CDCl3) *δ*: 7.88-7.83 (2H, m), 7.63-7.45 (3H, m), 7.35-7.08 (10H, m), 5.28 and 5.26 (1H, dd, J - 8.8, 1.6 Hz), 3.99 and 3.97 (1H, dd, J = 9.2, 8.4 Hz), 3.85-3.75 (1H, m), 3.39 and 3.36 (1H, 10, 9.2 Hz), 2.73 (2H, t, J = 7.6 Hz), 2.71 (2H, t, J = 7.6 Hz), 2.43-2.24 (2H, m), 2.07 (2H, quint, J = 7.6 Hz) | 474 | 1.89 |
| 40 | 7E | 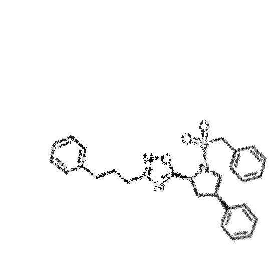 | 487.62 | (CDCl3) *δ*: 7.50-7.12 (10H, m), 5.27 and 5.25 (1H, dd, J = 8.4. 1.6 Hz). 4.46 (1H, d, J = 13.6 Hz), 4.36 (1H, d, J = 13.6 Hz). 3.65-3.53 (1H, m), 3.43-3.35 (2H, m), 2.80 (2H, t, J = 7.6 Hz), 2.74 (2H. t, J = 7.6 Hz), 2.48-2.34 (2H, m), 2.13 (2H, quint, J = 7.6 Hz) | 488 | 1.87 |

| | | | | | | |
|---|---|---|---|---|---|---|
| Ex: Example Number Cn: Compound Number MW: Molecular Weight | | | | | | |

Eleven compounds listed in Table 9-2 were synthesized by using various sulfonyl chlorides in place of isobutyl sulfonyl chloride under the same reaction conditions as in Example 36-3.

**Table 9-2**

| [Table 31] | | | |
|---|---|---|---|
| Compound Number | Compound Name | Sulfonyl Chlorides | Yield (%) |
| 7AI | 5-((2S,4S)-1-((4-methoxyphenyl)sulfonyl)-4-phenylpyrrolidin-2-yl)-3-(3-phenylpropyl)-1,2,4-oxadiazole | 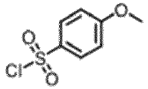 | 70 |
| 7AJ | 5-((2S,4S)-1-((4-fluorophenyl)sulfonyl)-4-phenylpyrrolidin-2-yl)-3-(3-phenylpropyl)-1,2,4-oxadiazole | 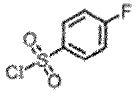 | 72 |
| 7AK | N,N-dimethyl-4-(((2S,4S)-4-phenyl2-(3-(3-phenylpropyl)-1,2,4-oxadiazole-5-yl)pyrrolidin -1-yl)sulfonyl)aniline | 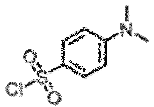 | 70 |
| 7AL | N,N-dimethyl-5-(((2S,4S)-4-phenyl2-(3-(3-phenylpropyl)-1,2,4-oxadiazole-5-yl)pyrrolidin -1-yl)sulfonyl)naphthalen-1-amine | 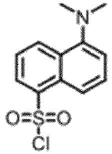 | 76 |
| TAM | tert-buthyl 3-((((2S,4S)-4-phenyl2-(3-(3-phenylpropyl)-1,2,4-oxadiazole-5-yl)pyrrolidin -1-yl)sulfonyl)methyl)azetidin-1-carboxylate | 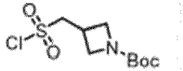 | 32 |
| 7AN | tert-buthyl 4-((((2S,4S)-4-phenyl2-(3-(3-phenylpropyl)-1,2,4-oxadiazole-5-yl)pyrrolidin -1-yl)sulfonyl)methyl)piperidin-1-carboxylate | 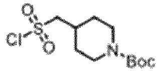 | 72 |
| 7AO | tert-buthyl 4-(((2S,4S)-4-phenyl2-(3-(3-phenylpropyl)-1,2,4-axadiazole-5-yl)pyrrolidin -1-yl)sulfonyl)piperidin-1-carboxylate | 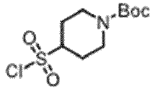 | 35 |
| 7AP | 5-((2S,4S)-4-phenyll-((tetrahydro-2H-pyran-4-yl)sulfonyl)pyrrolidin-2-yl)-3-(3-phenylpropyl)-1,2,4-oxadiazole | 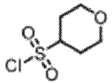 | 18 |

**Table 9-2 continued**

| | | | |
|---|---|---|---|
| 7AC | ethyl 1-(((2S,4S)-4-phenyl2-(3-(3-phenyl propyl)-1,2,4-ozadiazolee-5-yl)pyrrolidin-1-yl)sulfonyl)piperidin-4-caiboxylate | 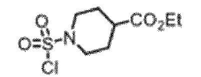 | 72 |
| 7AD | 5-((2S,4S)-4-phenyl1-(piperidin-1-ylsulfonyl) piperidin-2-yl)-3-(3-phenylpropyl)-1,2,4-oxadiazole | 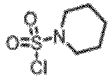 | 81 |
| 7AE | 4-(((2S,4S)-4-phenyl2-(3-(3-phenylpropyl)-1,2,4-oxadiazole-5-yl)pyrrolidin-1-yl)sulfonyl) morpholine | 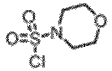 | 56 |

The structural formula, molecular weight, 1H-NMR, [M+H], and RT of the above compounds are summarized in Table 9-3.

**Table 9-3**

| [Table 32] | | | | | |
|---|---|---|---|---|---|
| Ex | Chemical Structure | MW | 1H-NMR δ ppm | [M+H] | RT :Min |
| 7AI | 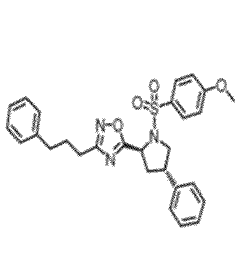 | 503.62 | (CDCl3); *δ*: 7.78 (2H, d, J = 6.8 Hz). 7.33-7.09 (5H. m), 6.95 (2H, t, J = 6.8 Hz), 5.24 and 5.22 (1H, d, J=8.8, 1.6 Hz). 3.95 (1H, t, J = 8.2 Hz), 3.83 (3H, s), 3.36 and 3.33 (1H, dd, J = 9.8, 9.0 Hz), 2.74 (2H, t, J - 7.2 Hz), 2.71 (2H, t. J = 7.2 Hz), 2.44-2.25 (2H, m), 2.07 (2H, quint, J = 7.2 Hz) | 504 | 1.91 |
| 7AJ | 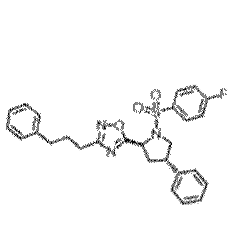 | 491.58 | (CDCl3; 6:7.90-7.82 (2H, m), 7.36-7.10 (12H, m), 5.29 and 5.27 (1H,dd. J=8.6, 1.8 Hz). 3.93 (1H, t. J = 8.2 Hz), 3.84-3.72 (1H. m). 3.40 (1H, t, J = 9.6 Hz), 2.73 (2H, t, J = 7.2 Hz), 2.71 (2H, t, J - 7.2 Hz), 2.47-2.31 (2H, m), 2.07 (2H, quint, J = 7.2 Hz) | 492 | 1.9 |
| 7AK | 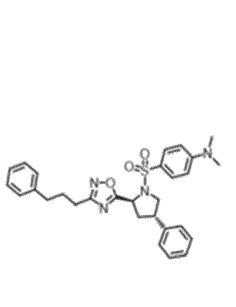 | 516.66 | (CDCl3); *δ*: 7.67 (2H, d, J = 7.2 Hz), 7.32-7.08 (10H, m), 6.64 (2H, d, J = 7.2 Hz), 5.20 and 5.18 (1H, dd, J=9.0. 1.4 Hz), 3.94 (1H. t. J = 7.8 Hz), 3.84-3.69 (1H. m), 3.33 and 3.31 (1H, dd, J = 10.2, 8.0 Hz), 3.29 (6H, s), 2.74 (2H, t, J = 7.2 Hz). 2.70 (2H, t. J = 7.2 Hz). 2.42-2.33 (1H. m), 2.32-2.21 (1H, m), 2.07 (2H. quint, J - 7.2 Hz) | 517 | 1.92 |

**Table 9-3 continued**

| [Table 33] | | | | | |
|---|---|---|---|---|---|
| 7AL | 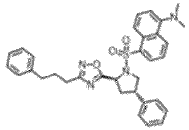 | 566.72 | (CDCl3); *δ*: 8.51 (1H, d, J = 8.4 Hz), 8.34 (1H, d. J = 8.8 Hz). 8.27 and 8.25 (1H, dd, J = 7.2, 1.2Hz), 7.54-7.44 (2H, m), 7.33-7.11 (11H, m), 5.44 and 5.42 (1H, dd, J=8.4, 1.6 Hz), 3.95 and 3.93 (1H. dd, J = 10.0, 8.4 Hz), 3.89-3.73 (1H, m). 3.59 and 3.57 (1H, dd, J - 10.0, 8.8 Hz). 2.85 (6H, s), 2647 (2H, t, J 7.2 Hz), 2.59 (2H, t, J = 7.2 Hz), 2.52-2.32 (2H, m), 1.95 (2H, quint, J = 7.2 Hz) | 567 | 2.13 |
| 7AM | 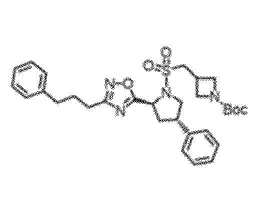 | 566.72 | (CDCl3); *δ*: 7.41-7.11 (10H, m), 5.45 and 5.43 (1H. dd. J=7.2, 4.8 Hz), 4.15-4.06 (2H, m), 3.88-3.82 (1H, m), 3.78-3.62 (4H, m), 3.44-3.36 (1H. m), 3.15-3.03 (1H, m), 2.76 (2H, t, J - 7.2 Hz), 2.72 (2H, t, J = 7.2 Hz), 2.61-2.52 (2H, m), 2.10 (2H, quint, J = 7.2 Hz), 1.42 (9H, s) | 567 | 1.89 |
| 7AN | 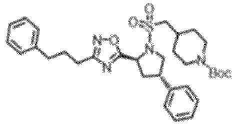 | 594.77 | (CDCl3); *δ*: 7.40-7.13 (10H, m), 5.45 and 5.43 (1H, dd, J=6.4, 4.8 Hz), 4.13-3.98 (2H, br s). 3.90-3.83 (1H, m), 3.77-3.59 (2H, m), 3.03 (2H, d, J = 6.4 Hz). 2.82-2.64 (2H, m), 2.77 (2H, t, J = 7.2 Hz), 2.70 (2H, t. J = 7.2 Hz), 2.61-2.52 (2H, m), 2.17-2.04 (1H. m), 2.09 (2H, quint, J - 7.2 Hz), 1.92-1.80 (2H, m), 1.45 (9H, s), 1.29-1.14 (2H, m) | 595 | 1.95 |
| 7AO | 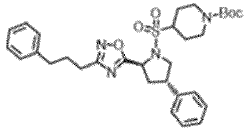 | 580.74 | (CDCl3); *δ*: 7.39-7.12 (IOH, m), 5.49 and 5.47 (1H, dd, J=6.0, 4.0 Hz), 4.33-4.08 (2H. br s), 3.90-3.80 (1H, m), 3.77-3.56 (2H, m), 3.18-3.04 (1H, m), 2.77 (2H, t, J = 7.2 Hz), 2.72 (2H, t. J = 7.2 Hz), 2.68-2.51 (4H, m). 2.14-2.02 (2H, m), 2.09 (2H, quint, J = 7.2 Hz), 1.78-1.65 (2H, m), 1.45 (9H, s) | 581 | 1.95 |

**Table 9-3 continued 2**

| [Table 34] | | | | | |
|---|---|---|---|---|---|
| 7AP | | 481.61 | (CDCl3); *δ*: 7.41-7.13 (10H, m). 5.50-5.48 (1H, m), 4.08-3.98 (2H, m), 3.90-3.82 <1H, m), 3.77-3.68 (2H, m), 3.34-3.17 (3H, m). 2.78 (2H. t, J = 7.2 Hz), 2.72 (2H, t, J - 7.2 Hz), 2.59-2.48 (2H, m), 2.09 (2H, quint, J = 7.2 Hz), 2.04-1.82 (4H, m) | 482 | 1.82 |
| 7AC | 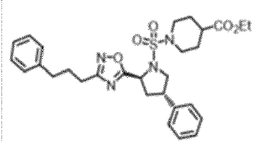 | 552.69 | (CDCl3); *δ*: 7.38-7.11 (10H, m), 5.28 and 5.26 (1H, d, J = 9.2, 1.6 Hz), 4.11 (2H, q, J = 7.2 Hz), 3.88-3.75 (2H. m), 3.73-3.58 (2H, m), 3.55 and 3.53 (1H, dd, J - 9.6, 8.4 Hz), 2.88-2.77 (1H, m), 2.78 (2H, t, J = 7.2 Hz), 2.72 (2H, t. J = 7.2 Hz), 2.62-2.50 (1H, m), 2.48-2.39 (1H, m), 2.37-2.22 (1H. m), 2.10 (2H, quint, J = 7.2 Hz), 1.94-1.84 (1H, m), 1.94-1.84 (2H, m), 1.74-1.63 (1H, m), 1.61-1.47 (2H, m), 1.23 (2H, t, J = 7.2 Hz) | 553 | 1.93 |
| 7AD | 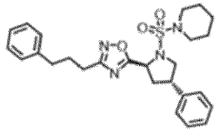 | 480.63 | (CDCl3); *δ*: 7.39-7.12 (10H, m), 5.28 and 5.26 (1H, d, J=9.2, 1.2 Hz), 3.88-3.73 (2H. m), 3.55 and 3.53 (1H, dd, J = 10.0, 8.4 Hz), 3.24-3.09 (4H, m). 2.78 (2H, t, J = 7.2 Hz). 2.72 (2H, t, J = 7.2 Hz), 2.61-2.49 (2H, m), 2.46-2.37 (1H, m), 2.10 (2H, quint, J = 7.2 Hz), 1.57-1.40 (6H, m) | 481 | 1.96 |
| 7AE | 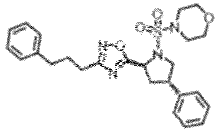 | 482.60 | (CDCl3); *δ*: 7.41-7.11 (10H, m), 5.32 and 5.30 (1H, d. J=8.8, 1.6 Hz), 3.87 (1H, t. J - 8.0 Hz), 3.84-3.72 (1H, m), 3.66-3.52 (5H, m), 3.27-3.13 (4H, m), 2.78 (2H, t. J = 7.2 Hz), 2.73 (2H, t. J = 7.2 Hz), 2.63-2.52 (1H, m), 2.48-2.40 (1H, m), 2.10 (2H, quint, J = 7.2 Hz) | 483 | 1.82 |

| | | | | | |
|---|---|---|---|---|---|
| Ex: Example Number Cn: Compound Number MW: Molecular Weight | | | | | |

The following compounds can be prepared substantially according to the methods described in Examples 36-40.

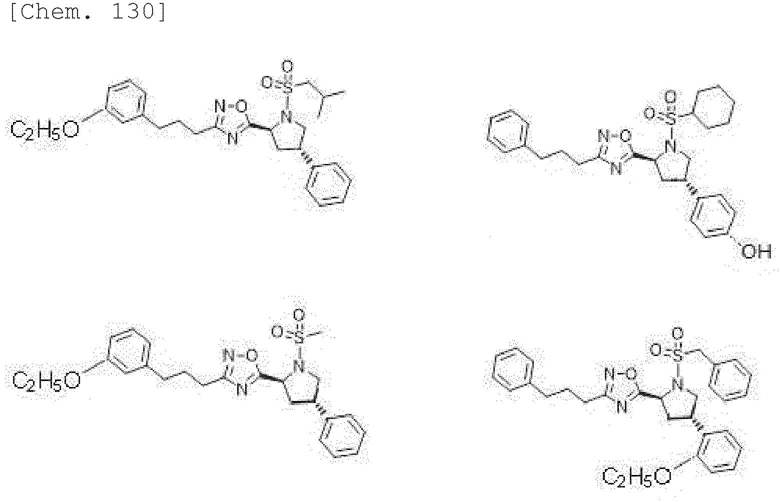

### Example 41

### 3-(3-phenylpropyl)-5-[(2S,4R)-1-isobutylsulfonyl-4-fluoro pyrrolidin-2-yl]-1,2,4-oxadiazole 8A

### Example 41-1: 3-(3-phenylpropyl)-5-[(2S,4R)-1-tert-butoxy carbonyl-4-fluoropyrrolidin-2-yl]-1,2,4-oxadiazole 8-1

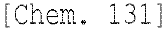

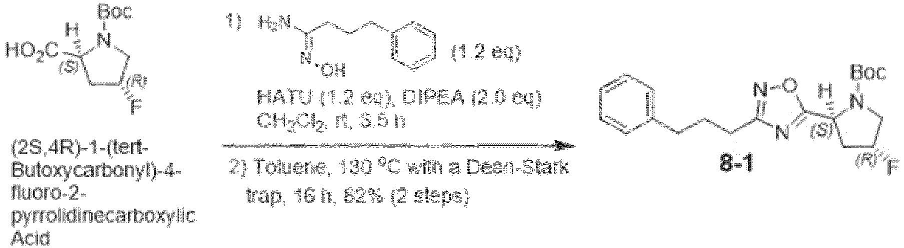

(2S,4R)-1-(tert-butoxycarbonyl)-4-fluoro-2-pyrrolidine carboxylic acid (233.2 mg, 1.0 mmol) was added to a 25 ml eggplant-shaped flask and dissolved in dichloromethane (7.0 ml). HATU (456.3 mg, 1.2 mmol) and diisopropylethylamine (0.34 ml, 2.0 mmol) were then added thereto and the mixture was stirred for 10 minutes at room temperature under a nitrogen atmosphere. N'-hydroxy-4-phenylbutanimidamide (213.9 mg, 1.2 mmol) was then added while washing with dichloromethane (3.0 ml), and the mixture was stirred at room temperature for 3.5 hours.

The stirrer bar was removed and dichloromethane (20.0 ml) and 5% sodium bicarbonate aqueous solution (10.0 ml) were added to the reaction mixture. The separated organic layer was washed with distilled water (10.0 ml) and dried over anhydrous magnesium sulfate. After filtration, the solvent was distilled off to afford a mixture of the imidamide intermediate and urea compound (740.0 mg). The mixture (740.0 mg) was dissolved in toluene (18.0 ml) in a 25 ml eggplant-shaped flask, which was placed in a Dean-Stark trap, and the mixture was heated to reflux at an oil bath temperature of 130°C for 16 hours. The solvent was distilled off under reduced pressure and the resulting residue was purified on silica gel column Q-Pac SI30 size 20 (hexane:ethyl acetate = 90:10 to 35:65). After distilling off the solvent, 308.6 mg (0.82 mmol, 82% (after 2 steps)) of the desired compound 8-1 (oil) was obtained.
Molecular Weight: 475.44;
1H-NMR δ ppm (CDCl3: a mixture of the rotational isomers) δ: 7.35-7.15 (5H, m), 5.36 and 5.23 (1H, m), 5.28-5.12 (1H, m), 4.08-3.87 (1H, m), 3.83-3.64 (1H, m), 2.78-2.66 (1H, m), 2.75 (2H, t, J = 7.6 Hz), 2.71 (2H, t, J = 7.6 Hz), 2.38-2.17 (1H, m), 2.08 (2H, quint, J = 7.6 Hz), 1.44 and 1.28 (9H, s).
[M+H]; 376
RT (minute); 1.80.

### Example 41-2: 3-(3-phenylpropyl)-5-[(2S,4R)-4-fluoro pyrrolidin-2-yl]-1,2,4-oxadiazole 8-2

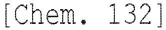

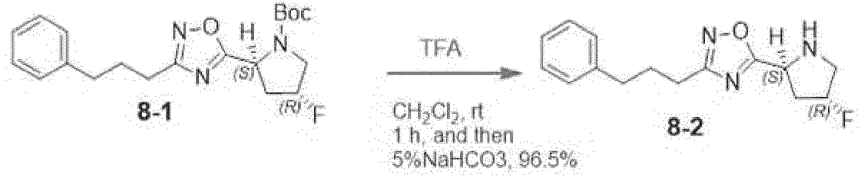

Compound 8-1 (304.3 mg, 0.81 mmol) prepared in Example 41-1 was added to a 25 ml eggplant-shaped flask, and dichloromethane (8.3 ml) and TFA (1.24 ml, 16.2 mmol) were added thereto, followed by stirring the mixture at room temperature for 1 hour. After removing the stirrer bar and distilling off the solvent, the process of dissolving the residue in chloroform (10 ml) and conducting the distillation was repeated three times to remove the TFA. 5 % sodium bicarbonate aqueous solution (10 ml) was added to the residue to make it basic and the mixture was extracted with ethyl acetate (50 ml) . The organic layer was washed with distilled water (10 ml) and saturated brine (10 ml), and then was dried over anhydrous magnesium sulfate. After filtration, the solvent was distilled off under reduced pressure to afford 215.7 mg (0.783 mmol, 96.5%) of the desired compound 8-2 (oil).

### Example 41-3: 3-(3-phenylpropyl)-5-[(2S,4R)-1-isobutyl sulfonyl-4-fluoropyrrolidin-2-yl]-1,2,4-oxadiazole 8A

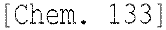

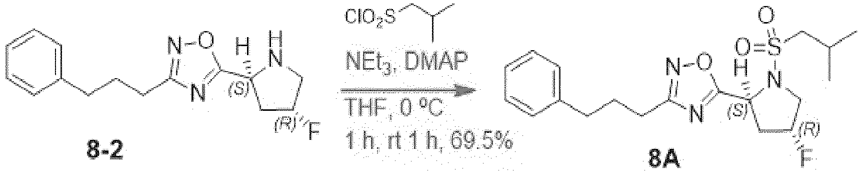

Ultra-dehydrated THF (1.75 ml), triethylamine (0.042 ml, 0.30 mmol) and DMAP (3.7 mg, 0.030 mmol) were added sequentially to a 4.0 ml screw-tube vial containing compound 8-2 (27.5 mg, 0.10 mmol) prepared in Example 41-2, and the mixture was cooled to 0°C, which was added with a solution of isobutylsulfonyl chloride (0.027 ml, 0.20 mmol) in ultra-dehydrated THF (0.25 ml), followed by stirring the mixture at 0°C for 1 hour and at room temperature for 1 hour. The reaction was quenched by adding 0.2 M hydrochloric acid aqueous solution (1 ml) and the mixture was extracted with ethyl acetate (6 ml). After distilling off the solvent under reduced pressure, the residue was purified by SiO2-PTLC (PLC Silica Gel 60 F254, 0.5 mm layer thickness, 20 x 20 cm; developing solvent: hexane:ethyl acetate = 1:1). The silica gel of the target portion was scraped off and eluted with chloroform containing 10% MeOH (20 ml). The solvent was distilled off under reduced pressure to afford 27.6 mg (0.069 mmol, 69.5%) of the desired compound 8A (oil).

### Example 42

### 3-(3-phenylpropyl)-5-[(2S,4R)-1-methanesulfonyl-4-fluoro pyrrolidin-2-yl]-1,2,4-oxadiazole 8C

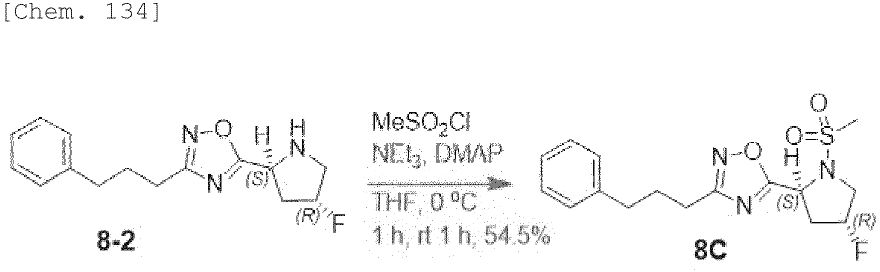

Ultra-dehydrated THF (1.75 ml), triethylamine (0.042 ml, 0.30 mmol) and DMAP (3.7 mg, 0.030 mmol) were added sequentially to a 4.0 ml screw-tube vial containing compound 8-2 (27.5 mg, 0.10 mmol) prepared in Example 41-2, and the mixture was cooled to 0°C, which was added with a solution of methanesulfonyl chloride (0.016 ml, 0.20 mmol) in ultra-dehydrated THF (0.(25 ml), followed by stirring the mixture at 0°C for 1 hour and at room temperature for 1 hour. The reaction was quenched by adding 0.2 M hydrochloric acid aqueous solution (1 ml) and the mixture was extracted with ethyl acetate (6 ml). After distilling off the solvent under reduced pressure, the residue was purified by SiO2-PTLC (PLC Silica gel 60 F254, 0.5 mm layer thickness, 20 x 20 cm; developing solvent: hexane:ethyl acetate = 1:1). The silica gel of the target portion was scraped off and eluted with chloroform containing 10% MeOH (20 ml). The solvent was distilled off under reduced pressure to afford 19.3 mg (0.054 mmol, 54.5%) of the desired compound 8C (oil).

### Example 43

### 3-(3-phenylpropyl)-5-[(2S,4R)-1-benzenesulfonyl-4-fluoro pyrrolidin-2-yl]-1,2,4-oxadiazole 8D

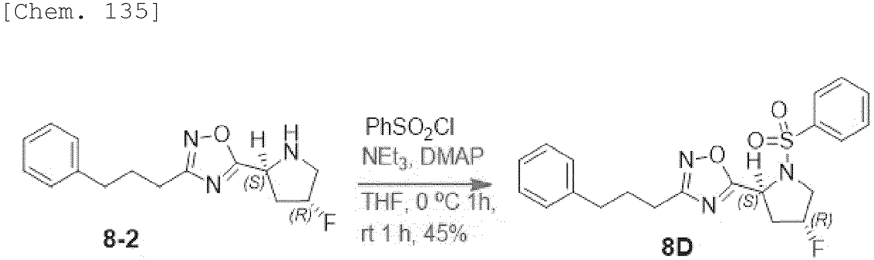

Ultra-dehydrated THF (1.75 ml), triethylamine (0.042 ml, 0.30 mmol) and DMAP (3.7 mg, 0.030 mmol) were added sequentially to a 4.0 ml screw-tube vial containing compound 8-2 (27.5 mg, 0.10 mmol) prepared in Example 41-2, and the mixture was cooled to 0°C, which was added with a solution of benzenesulfonyl chloride (0.026 ml, 0.20 mmol) in ultra-dehydrated THF (0 .25 ml), followed by stirring the mixture at 0°C for 1 hour and at room temperature for 1 hour. The reaction was quenched by adding 0.2 M hydrochloric acid aqueous solution (1 ml) and the mixture was extracted with ethyl acetate (6 ml). After distilling off the solvent under reduced pressure, the residue was purified by SiO2-PTLC (PLC Silica Gel 60 F254, 0.5 mm layer thickness, 20 x 20 cm; developing solvent: hexane:ethyl acetate = 1:1). The silica gel of the target portion was scraped off and eluted with chloroform containing 10% MeOH (20 ml). The solvent was distilled off under reduced pressure to afford 18.7 mg (0.045 mmol, 45%) of the desired compound 8D (oil).

### Example 44

### 3-(3-phenylpropyl)-5-[(2S,4R)-1-benzylsulfonyl-4-fluoro pyrrolidin-2-yl]-1,2,4-oxadiazole 8E

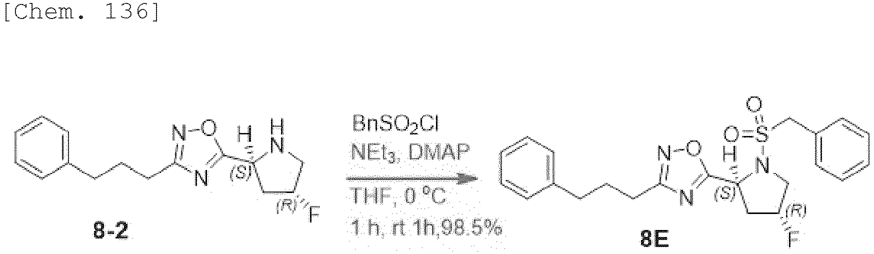

Ultra-dehydrated THF (1.75 ml), triethylamine (0.042 ml, 0.30 mmol) and DMAP (3.7 mg, 0.030 mmol) were added sequentially to a 4.0 ml screw-tube vial containing compound 8-2 (27.5 mg, 0.10 mmol) prepared in Example 41-2, and the mixture was cooled to 0°C, which was added with a solution of benzylsulfonyl chloride (38.2 mg, 0.20 mmol) in ultra-dehydrated THF (0 .25 ml), followed by stirring the mixture at 0°C for 1 hour and at room temperature for 1 hour. The reaction was quenched by adding 0.2 M hydrochloric acid aqueous solution (1 ml) and the mixture was extracted with ethyl acetate (6 ml). After distilling off the solvent under reduced pressure, the residue was purified by SiO2-PTLC (PLC Silica gel 60 F254, 0.5 mm layer thickness, 20 x 20 cm; developing solvent: hexane:ethyl acetate = 1:1). The silica gel of the objective portion was scraped off and eluted with chloroform containing 10% MeOH (20 ml). The solvent was distilled off under reduced pressure to afford 42.3 mg (0.098 mmol, 98.5%) of the desired compound 8E (oil).

The compounds prepared in Examples 41-44 are described in Table 10-1 along with their physical property data.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acid-acetonitrile;
0-0.9 min linear gradient A:B (95:5)-A:B (10:90), 0.9-3 min A:B (10:90)

**Table 10-1**

| [Table 35] | | | | | | |
|---|---|---|---|---|---|---|
| Ex | Cn | Chemical Structure | MW | ¹H-NMR δ ppm | [M+H] | RT :Min |
| 41 | 8A | 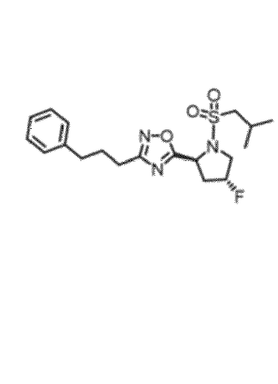 | 395.49 | (CDCl3) *δ*: 7.30-7.12 (5H, m), 5.43-5.37 and 5.30-5.24 (1H. m), 5.32 and 5.29 (1H, dd, J = 8.4 , 8.0 Hz), 4.06 and 4.01 (1H. dd, J = 12.8. 2.4 Hz), 3.75 and 3.65 (1H. dd, J= 12.8, 2.4 Hz), 2.93 and 2.91 (2H, d, J = 6.8 Hz). 2.76 (2H, t. J = 7.2 Hz). 2.70 (2H, t, J = 7.2 Hz), 2.52-2.43 (1H, m), 2.42-2.33 (1H, m), 2.32-2.20 (1H, m), 2.08 (2H, quint, J = 7.2 Hz), 1.08 (3H, d, J = 6.8 Hz), 1.05 (3H, d. J = 6.8 Hz) | 396 | 1.74 |
| 42 | 8C | 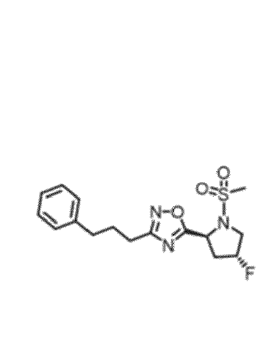 | 353.41 | (CDCl3) *δ*: 7.30-7.15 (5H. m), 5.40-5.39 (0.5H, m), 5.28-5.27 (0.5H. m), 5.23 and 5.21 (1.0H, dd, J =8.4, 7.6 Hz), 4.06 and 4.02 (1H, dd, J = 13.6, 2.4 Hz), 3.81 and 3.75 (O.5H, dd, J= 13.6, 2.4 Hz), 2.96 (1.5H, s), 2.95 (1.5H. s), 2.90-2.80 (1H, m), 2.77 (2H, t, J = 7.6 Hz), 2.70 (2H, t, J - 7.6 Hz), 2.52-2.43 (1H, m), 2.42-2.34 (1H, m), 2.08 (2H, quint, J = 7.6 Hz) | 354 | 1.63 |

**Table 10-1 continued**

| [Table 36] | | | | | | |
|---|---|---|---|---|---|---|
| 43 | 8D | 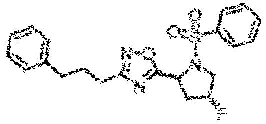 | 415.48 | (CDCl3) *δ*: 7.88-7.82 (2H. m), 7.62-7.47 (3H, m), 7.33-7.17 (5H, m), 5.30-5.26 and 5.18-5.12 (1H, m), 5.01 and 4.98 (1H. dd, J = 9.2, 7.2 Hz). 3.95 and 3.88 (1h, dd, J =13.5. 1.4 Hz), 3.87 and 3.81 (1H, dd, J = 13.5, 2.8 Hz), 2.74 (2H, t, J = 7.6 Hz), 2.70 (2H, t, J = 7.6 Hz), 2.68-2.56 (1H. m), 2.47-2.28 (1H. m), 2.07 (2H. quint, J - 7.6 Hz) | 416 | 1.73 |
| 44 | 8E | 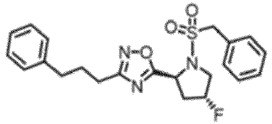 | 429.51 | (CDCl3) *δ*: 7.50-7.15 (10H, m), 5.32 and 5.29 (1H, dd, J - 8.4, 8.0 Hz), 5.25-5.20 and 5.15-5.07 (1H, m), 4.38 (1H, d. J = 13.5 Hz), 4.30 (1H, d, J = 13.5 Hz), 3.78 and 3.73 (1H, dd. J = 13.0, 2.9 Hz). 3.18 and 3.09 (1H. dd, J = 13.0, 2.8 Hz), 2.85-2.72 (1H. m), 2.79 (2H, t, J = 7.6 Hz). 2.72 (2H, t. J = 7.6 Hz), 2.40-2.23 (1H, m), 2.11 (2H, quint, J = 7.6 Hz) | 430 | 1.73 |

| | | | | | | |
|---|---|---|---|---|---|---|
| Ex: Example Number Cn: Compound Number MW: Molecular Weight | | | | | | |

Five compounds listed in Table 10-2 were synthesized by using various sulfonyl chlorides in place of isobutyl sulfonyl chloride under the same reaction conditions as in Example 41-3.

**Table 10-2**

| [Table 37] | | | |
|---|---|---|---|
| Compound Number | Compound Name | Sulfonyl Chlorides | Yield (%) |
| 8AI | 5-((2S,4R)-4-fluoro-1-((4-methoxyphenyl) sulfonyl)pyrrolidin-2-yl)-3-(3-phenylpropyl)-1,2,4-oxadiazole | 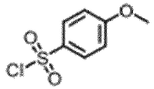 | 45 |
| 8AJ | 5-((2S,4R)-4-fluoro-1-((4-fluorophenyl) sulfonyl)pyrrolidin-2-yl)-3-(3-phenylpropyl)-1,2,4-oxadiazole | 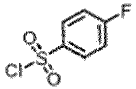 | 53 |
| 8AK | 4-(((2S,4R)-4-fluoro-2-(3-(3-phenylpropyl)-1,2,4-oxadiazole-5-yi)pyrrolidin-1-yl)sulfonyl)-N,N-dimethylaniline | 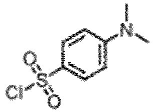 | 57 |
| RAL | 5-((2S,4R)-4-fluoro-2-(3-(3-phenylpropyl)-1,2,4-oxadiazole-5-yl)pyrrolidin-1-yl)sulfonyl)-N,N-dimethylnaphthalen-1-amine | 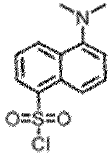 | 75 |
| 8AD | 5-((2S,4R)-4-fluoro-1-(piperidin-1-ylndfonyl) pyrrolidin-2-yl)-3-(3-phenylpropyl)-1,2,4-oxadiazole | 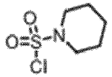 | 41 |

The structural formula, molecular weight, 1H-NMR, [M+H], and RT of the above compounds are summarized in Table 10-3.

**Table 10-3**

| [Table 38] | | | | | |
|---|---|---|---|---|---|
| Ex | Chemical Structure | MW | 1H-NMR δ ppm | [M+H] | RT :Min |
| 8AI | 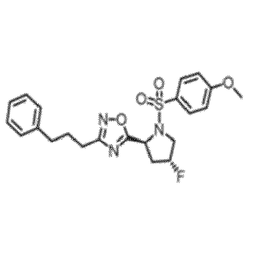 | 445.51 | (CDCl3) *δ*: 7.78 (2H, d, J = 6.8 Hz), 7.34-7.12 (5H, m), 6.97 (2H, d, J = 6.8 Hz). 5.30-5.26 and 5.16-5.13 (1H, m), 4.97 and 4.95 (1H, dd, J = 9.2, 7.2 Hz), 3.93-3.73 (2H, m), 3.84 (3H, s), 2.74 (2H, t, J = 7.2 Hz), 2.70 (2H, t. J = 7.2 Hz), 2.67-2.54 (1H, m), 2.47-2.28 (1H, m), 2.07 (2H, quint, J = 7.2 Hz) | 446 | 1.75 |
| 8AJ | 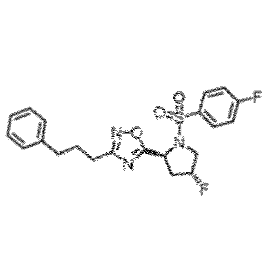 | 433.47 | (CDCl3) *δ*: 7.90-7.78 (2H. m), 7.33-7.10 (7H, m), 5.31-5.27 and 5.18-5.14 (1H, m). 4.99 and 4.97 (1H, dd, J = 9.2, 7.2 Hz), 3.97-3.73 (2H, m), 2.74 (2H, t, J = 7.2 Hz), 2.73-2.57 (1H, m), 2.70 (2H, t. J = 7.2 Hz), 2.47-2.28 (1H, m), 2.07 (2H, quint. J = 7.2 Hz) | 434 | 1.76 |
| 8AK | 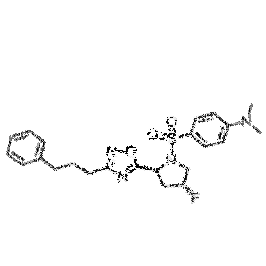 | 458.55 | (CDCl3) *δ*: 7.66 (2H, d, J = 7.2 Hz), 7.32-7.14 (5H, m), 6.65 (2H, d, J = 7.2 Hz), 5.30-5.27 and 5.15-5.12 (1H, m), 4.95 and 4.93 (1H, dd, J - 9.2, 6.8 Hz), 3.92-3.72 (2H, m), 3.02 (6H. s), 2.74 (2H. t, J = 7.2 Hz), 2.69 (2H, t. J = 7.2 Hz), 2.64-2.52 (1H, m), 2.46-2.27 (1H, m), 2.07 (2H, quint, J = 7.2 Hz) | 459 | 1.76 |

**Table 10-3 continued**

| [Table 39] | | | | | |
|---|---|---|---|---|---|
| 8AL | 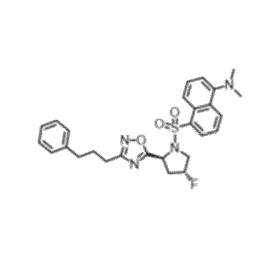 | 508.61 | (CDCl3) *δ*: 8.49 (1H, d, J = 8.4 Hz), 8.31 (1H, d, J = 8.4 Hz). 8.20 and 8.18 (1H, dd, J = 7.2, 1.2 Hz), 7.54-7.41 (2H, m), 7.32-7.08 (6H. m), 5.35-5.31 and 5.23-5.20 (1H, m), 5.29 and 5.27 (1H, dd, J = 8.8, 8.0 Hz), 4.28-4.12 (1H. m). 3.89-3.72 (1H, m), 2.84 (6H, s). 2.75-2.64 (1H, m), 2.61 (2H, t, J = 7.2 Hz), 2.53 (2H, t, J = 7.2 Hz), 2.48-2.30 (1H. m). 1.91 (2H, quint, J = 7.2 Hz) | 509 | 1.88 |
| 8AD | 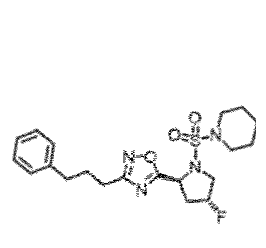 | 422.52 | (CDCl3) *δ:* 7.33-7.17 (5H. m). 5.41-5.37 and 5.27-5.24 (1H, m), 5.24 and 5.21 (1H, dd, J = 9.0, 7.4 Hz), 4.06-3.91 (1H, m), 3.79-3.62 (1H. m), 3.22-3.07 (4H, m), 2.82-2.67 (1H, m), 2.76 (2H. t. J = 7.2 Hz). 2.70 (2H, t, J = 7.2 Hz), 2.48-2.27 (1H, m), 2.06 (2H, quint, J = 7.2 Hz), 1.56-1.43 (6H, m) | 423 | 1.77 |

| | | | | | |
|---|---|---|---|---|---|
| Ex: Example Number Cn: Compound Number MW: Molecular Weight | | | | | |

The following compounds can be prepared substantially according to the method described in Examples 41-44.

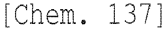

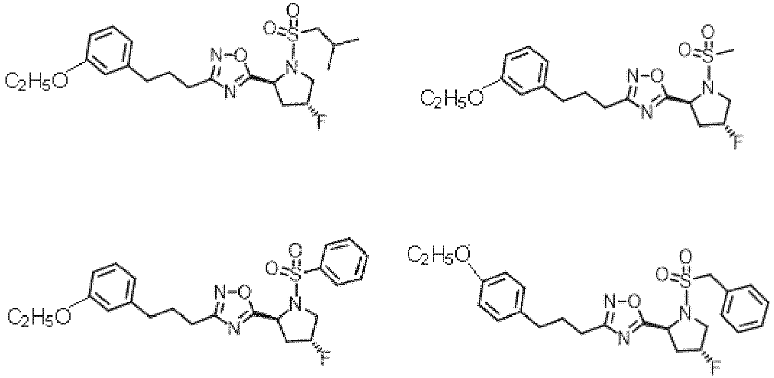

### Example 45

### 3-(2-phenylethyl)-5-[(2S)-1-tert-butoxycarbonylpyrrolidin-2-yl]-1,2,4-oxadiazole 9-1

### Example 45-1: N'-hydroxy-3-phenylpropanimidamide

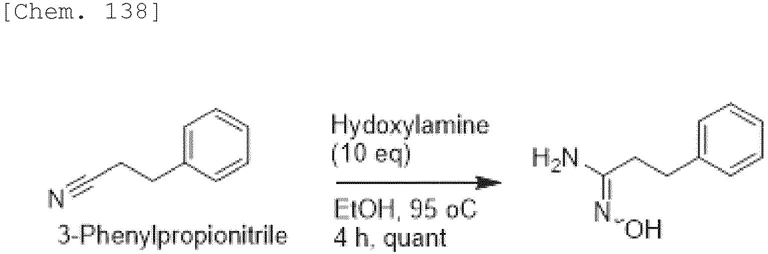

3-Phenylpropionitrile (526 mg, 4.01 mmol) and 50% hydroxylamine aqueous solution (2.36 ml, 40.1 mmol) were added to an eggplant-shaped flask, and were dissolved in anhydrous ethanol (16 ml), followed by heating the mixture to reflux at 95°C for 4 hours. After distilling off the solvent, the product was dried in vacuo to afford the title compound (oil, 657 mg, yield: 100%).

### Example 45-2: 3-(2-phenylethyl)-5-[(2S)-1-tert-butoxy carbonylpyrrolidin-2-yl]-1,2,4-oxadiazole 9-1

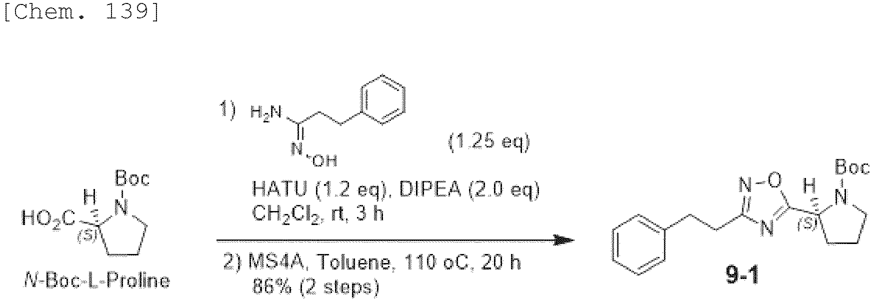

N-Boc-L-proline (100 mg, 0.465 mmol) was added to a 25 ml eggplant-shaped flask and was dissolved in dichloromethane (3.65 ml). HATU (212 mg, 0.558 mmol) and diisopropylethylamine (0.162 ml, 0.929 mmol) were then added thereto and the mixture was stirred for 10 minutes at room temperature under a nitrogen atmosphere. Then, N'-hydroxy-3-phenylpropanimidamide (95 mg, 0.581 mmol) prepared in Example 45-1 was added while washing with dichloromethane (1.0 ml), followed by stirring the mixture at room temperature for 3 hours.

After removing the stirrer bar and distilling off the solvent, the residue was purified on a silica gel column Q-Pac SI30 size 20 (hexane:ethyl acetate = 83:17 to 40:60). After distilling off the solvent, the imidamide intermediate was obtained as a mixture with a urea compound. The intermediate was added with pre-dried MS4Å (840 mg), and was then dissolved in ultra-dehydrated toluene (4.65 ml). The mixture was stirred at 110°C for 20 hours with a Dimroth condenser attached. After removing MS4Å by celite filtration, the solvent was distilled off and the resulting residue was purified on a silica gel column Q-Pac SI30 size 20 (hexane:ethyl acetate = 86:14 to 60:40). After distilling off the solvent, 138 mg (0.401 mmol, 86% (after 2 steps)) of the desired compound 9-1 (oil) was obtained.

### Example 46

### 3-(2-phenylethyl)-5-[(2S)-1-isobutylsulfonylpyrrolidin-2-yl]-1,2,4-oxadiazole 9A

### Example 46-1: 3-(2-phenylethyl)-5-[(2S)-pyrrolidin-2-yl]-1,2,4-oxadiazole TFA salt 9-2

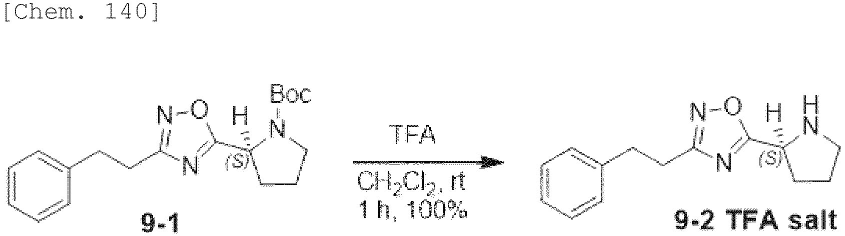

Compound 9-1 (20.6 mg, 0.060 mmol) prepared in Example 45 was added to a 10 ml eggplant-shaped flask, and dichloromethane (0.480 ml) and TFA (0.080 ml) were added thereto, followed by stirring the mixture at room temperature for 1 hour. After removing the stirrer bar and distilling off the solvent, the process of dissolving the residue in chloroform (1 ml) and conducting the distillation was repeated three times to remove the TFA. Then, vacuum drying was carried out for 2 hours to afford the desired compound 9-2 (amorphous solid) as a TFA salt (21 mg, 0.060 mmol, 100%).

### Example 46-2: 3-(2-phenylethyl)-5-[(2S)-1-isobutylsulfonyl pyrrolidin-2-yl]-1,2,4-oxadiazole 9A

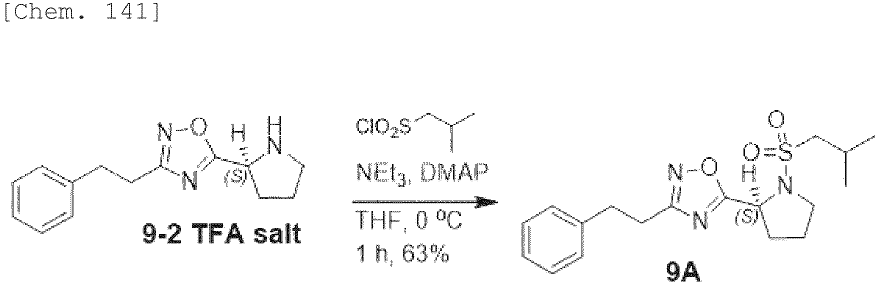

To a 10 ml eggplant-shaped flask, the TFA salt of compound 9-2 (21 mg, 0.060 mmol) was added, then ultra-dehydrated THF (0.60 ml), triethylamine (0.048 ml, 0.349 mmol) and DMAP (1.4 mg, 1.2 µmol) were added sequentially, and the mixture was cooled to 0°C, which was added with isobutylsulfonyl chloride (9.4 pl, 0.070 mmol), followed by stirring the mixture for 1 hour at 0°C. The reaction was quenched by adding 0.1 M hydrochloric acid aqueous solution (1 ml) and the mixture was extracted with ethyl acetate (1 ml x 2). After the extract was dried over magnesium sulfate, which was then filtered out, followed by distilling off the solvent, the residue was purified on a silica gel column Q-Pac SI30 size 10 (chloroform:methanol = 100:0 to 95:5) . After distilling off the solvent, 13.7 mg (0.038 mmol, 63%) of the desired compound 9A (oil) was obtained.

The compounds prepared in Examples 45-46 are described in Table 11 along with their physical property data.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acidacetonitrile;
0-0.9 min linear gradient A:B (95:5)-A:B (10:90), 0.9-3 min A:B (10:90)

**Table 11**

| [Table 40] | | | | | | |
|---|---|---|---|---|---|---|
| Ex | Cn | Chemical Structure | MW | ¹H-NMR δ ppm | [M+H] | RT :Min |
| 45 | 9-1 | 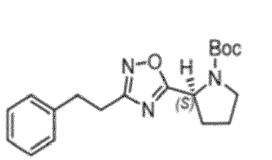 | 343.43 | 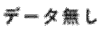 | 344 | 1.80 |
| 46 | 9A | 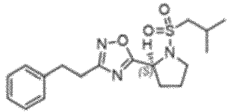 | 363.48 | (CDCl3) δ: 7.34-7.19 (5H, m), 5.27 (1H, dd, J = 8.4, 3.2 Hz), 3.72-3.63 (1H, m). 3.55-3.47 (1H, m), 3.11-3.01 (4H, m), 3.00-2.90 (2H, m), 2.48-2.33 (1H, m), 2.29-2.10 (4H, m) 1.09 (3H, d, J = 6.8 Hz). 1.04 (3H, d, J = 6.8 Hz) | 364 | 1.73 |

| | | | | | | |
|---|---|---|---|---|---|---|
| Ex: Example Number Cn: Compound Number MW: Molecular Weight | | | | | | |

The following compounds can be prepared substantially according to the method described in Examples 45-46.

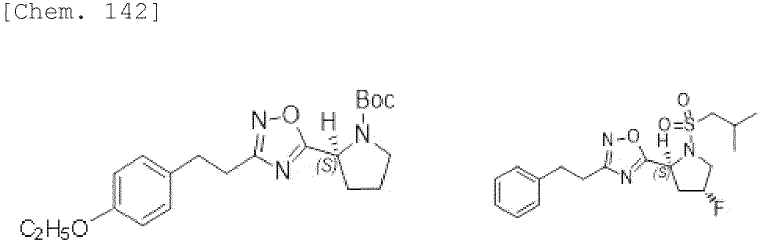

### Example 47

### 3-(3-phenylethyl)-5-[(2S)-1-tert-butoxycarbonylpiperidin-2-yl]-1,2,4-oxadiazole 10-1

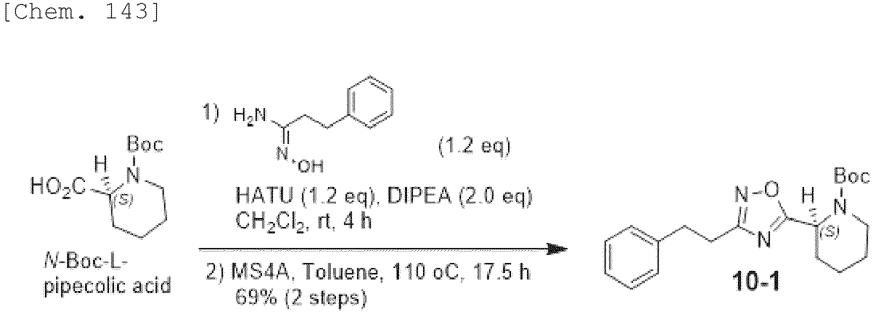

N-Boc-L-pipecolinic acid (100 mg, 0.436 mmol) was added to a 25 ml eggplant-shaped flask and was dissolved in dichloromethane (2.86 ml). HATU (199 mg, 0.523 mmol) and diisopropylethylamine (0.113 ml, 0.872 mmol) were then added thereto and the mixture was stirred for 10 minutes at room temperature under a nitrogen atmosphere. N'-hydroxy-4-phenylbutanimidamide (93.3 mg, 0.523 mmol) was then added while washing with dichloromethane (1.5 ml), and the mixture was stirred at room temperature for 4 hours. After removing the stirrer bar and distilling off the solvent, the residue was purified on a silica gel column Q-Pac SI30 size 20 (hexane:ethyl acetate = 84:16 to 50:50). After distilling off the solvent, the imidamide intermediate was obtained. The intermediate was added with pre-dried MS4Å (875 mg), and was then dissolved in ultra-dehydrated toluene (4.66 ml). The mixture was stirred at 110°C for 17.5 hours with a Dimroth condenser attached. After removing MS4Å by celite filtration, the solvent was distilled off and the resulting residue was purified on silica gel column Q-Pac SI30 size 20 (hexane:ethyl acetate = 91:9 to 75:25). After distilling off the solvent, 115 mg (0.320 mmol, 69% (after 2 steps)) of the desired compound 10-1 (oil) was obtained.

### Example 48

### 3-(2-phenylethyl)-5-[(2S)-1-isobutylsulfonylpiperidin-2-yl]-1,2,4-oxadiazole 10A

### Example 48-1: 3-(2-phenylethyl)-5-[(2S)-piperidin-2-yl]-1,2,4-oxadiazole TFA salt 10-2

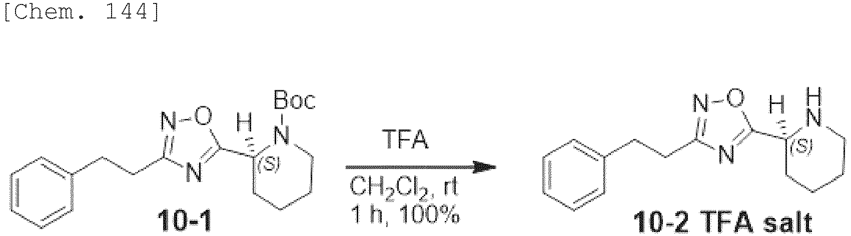

Compound 10-1 (20.4 mg, 0.057 mmol) prepared in Example 47 was added to a 10 ml eggplant-shaped flask, and dichloromethane (0.480 ml) and TFA (0.080 ml) were added thereto, followed by stirring the mixture at room temperature for 1 hour. After removing the stirrer bar and distilling off the solvent, the process of dissolving the residue in chloroform (1 ml) and conducting the distillation was repeated three times to remove the TFA. Then, vacuum drying was carried out for 2 hours to afford the desired compound 10-2 (amorphous solid) as a TFA salt (21 mg, 0.057 mmol, 100%).

### Example 48-2: 3-(2-phenylethyl)-5-[(2S)-1-isobutyl sulfonyl piperidin-2-yl]-1,2,4-oxadiazole 10A

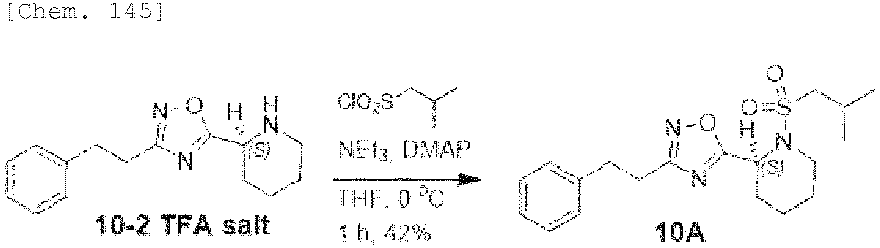

To a 10 ml eggplant-shaped flask, the TFA salt (21 mg, 0.057 mmol) of 10-2 prepared in Example 48-1 was added, then ultra-dehydrated THF (0.885 ml), triethylamine (0.049 ml, 0.354 mmol) and DMAP (2.2 mg, 1.8 µmol) were sequentially added, and the mixture was cooled to 0°C, which was added with isobutylsulfonyl chloride (17.9 ul, 0.133 mmol), followed by stirring the mixture at 0°C for 1 hour. The reaction was quenched by adding 0.1 M hydrochloric acid aqueous solution (1 ml) and the mixture was extracted with ethyl acetate (1 ml x 2). After the extract was dried over magnesium sulfate, which was then filtered out, followed by distilling off the solvent, the residue was purified by silica gel column Q-pack SI30 size 10 (chloroform:methanol = 100:0 to 95:5) and preparative TLC (hexane:ethyl acetate = 5:1). After distilling off the solvent, 9.0 mg (0.024 mmol, 42%) of the desired compound 10A (oil) was obtained.

The compounds prepared in Examples 47-48 are described in Table 12 along with their physical property data.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acidacetonitrile;
0-0.9 min linear gradient A:B (95:5)-A:B (10:90), 0.9-3 min A:B (10:90)

**Table 12**

| [Table 41] | | | | | | |
|---|---|---|---|---|---|---|
| Ex | Cn | Chemical Structure | MW | ¹H-NMR δ ppm | [M+H] | RT :Min |
| 47 | 10-1 | 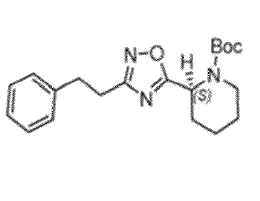 | 357.45 | 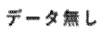 | 358 | 1.93 |
| 48 | 10A | 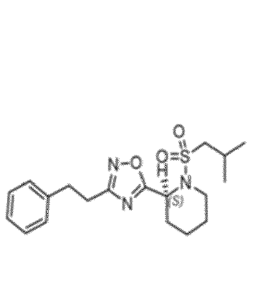 | 377.50 | (CDCl3) *δ*: 7.32-7.17 (5H. m), 5.42 (1H, d, J = 4.4 Hz), 3.79 (1H, dt, J = 12.8, 1.2 Hz). 3.15 (1H. td, J = 12.2, 4.0 Hz), 3.07 (4H, s), 2.99-2.87 (2H, m), 2.34-2.21 (2H, m), 2.08-1.96 (1H, m), 1.83-1.60 (3H, m), 1.44-1.31 (1H, m), 1.10 (3H, d, J = 6.8 Hz), 1.08 (3H. d, J = 6.8 Hz) | 378 | 1.81 |

| | | | | | | |
|---|---|---|---|---|---|---|
| Ex: Example Number Cn: Compound Number MW: Molecular Weight | | | | | | |

The following compounds can be prepared substantially according to the method described in Examples 47-48.

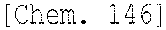

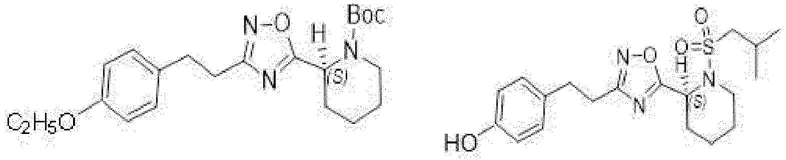

### Example 49

### 3-[(1-naphthoylamino)methyl]-5-[(2S)-1-isobutylsulfonyl pyrrolidin-2-yl]-1,2,4-oxadiazole 11A

### Example 49-1: 3-[(tert-butoxycarbonylamino)methyl]-5-{(2S)-1-[(9h-Fluorene-9-yl)methoxycarbonyl]pyrrolidin-2-yl}-1,2,4-oxadiazole 11-1

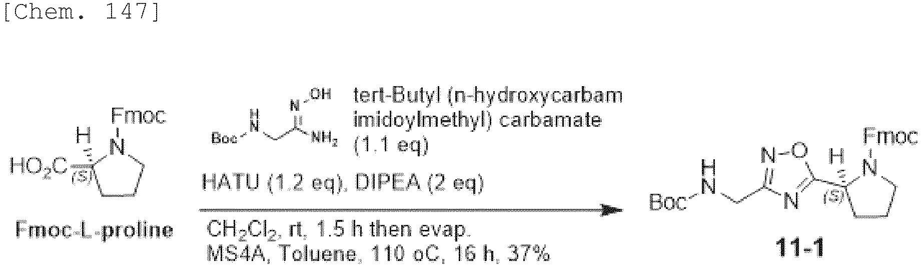

N-Fmoc-L-proline (200 mg, 0.593 mmol) was added to a 25 ml eggplant-shaped flask and was dissolved in dichloromethane (1.964 ml). HATU (271 mg, 0.711 mmol) and diisopropylethylamine (0.207 ml, 1.19 mmol) were then added thereto and the mixture was stirred for 10 minutes at room temperature under a nitrogen atmosphere. Then, tert-butyl (n-hydroxycarbamidoylmethyl) carbamate (129 mg, 0.652 mmol) was added while washing with dichloromethane (1 ml) and the mixture was stirred at room temperature for 1.5 hours. After distilling off the solvent and drying in vacuo, Molecular Sieves 4Å (1.5 g) and super-dehydrated toluene (5.93 ml) were added thereto and the mixture was stirred at 110°C for 16 hours. After filtering off the solids using celite and distilling off the solvent, the product was purified on a silica gel column Q-Pac SI30 size 20 (hexane:ethyl acetate = 83:17 to 57:43). After distilling off the solvent, 107 mg (0.219 mmol, 37%) of the desired compound 11-1 (white amorphous solid) was obtained.

### Example 49-2: 3-[(1-naphthoylamino)methyl]-5-{(2S)-1-[(9H-fluoren-9-yl)methoxycarbonyl]pyrrolidin-2-yl}-1,2,4-oxadiazole 11-2

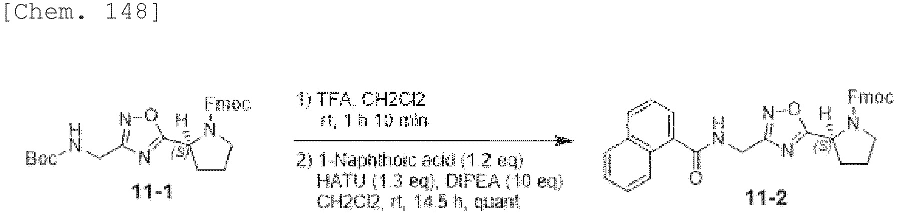

Compound 11-1 (39.7 mg, 0.081 mmol) prepared in Example 49-1 was added to a 10 ml eggplant-shaped flask, and then dichloromethane (0.81 ml) and TFA (0.12 ml) were added thereto, followed by stirring the mixture for 1 hour and 10 minutes at room temperature. After removing the stirrer bar and distilling off the solvent, the process of dissolving the residue in chloroform (1 ml) and conducting the distillation was repeated three times to remove the TFA. After drying in vacuo for 3 hours, the mixture was then added with dichloromethane (1.22 ml), HATU (40 mg, 0.105 mmol), and 1-naphthalenecarboxylic acid (16.7 mg, 0.097 mmol) sequentially, and finally with diisopropylethylamine (0.141 ml, 0.809 mmol), followed by stirring the mixture at room temperature for 14.5 h. After distilling off the solvent, saturated sodium bicarbonate aqueous solution (0.5 ml) was added thereto and the mixture was extracted with ethyl acetate (1 ml x 2). The organic layer was washed with saturated brine (1 ml), the solvent was distilled off, and the residue was purified on a silica gel column Q-Pac SI20 size 10 (hexane:ethyl acetate = 83:17 to 40:60). After distilling off the solvent, 44 mg (0.081 mmol, 100%) of the desired compound 11-2 (white amorphous solid) was obtained.

### Example 49-3: 3-[(1-naphthoylamino)methyl]-5-[(2S)-1-(pyrrolidin-2-yl)]-1,2,4-oxadiazole 11-3

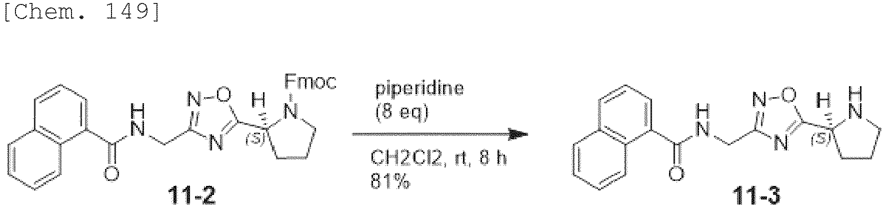

Compound 11-2 (23.7 mg, 0.044 mmol) prepared in Example 49-2 was added to a 10 ml eggplant-shaped flask, and dichloromethane (0.58 ml) and piperidine (35.5 pl, 0.348 mmol) were added thereto, followed by stirring the mixture at room temperature for 8 hours. After removing the stirrer bar and distilling off the solvent, the residue was purified on a silica gel column Q-Pac SI20 size 10 (hexane:ethyl acetate = 50:50, and chloroform: methanol = 100:0 to 95:5). After distilling off the solvent, 11.4 mg (0.035 mmol, 81%) of the desired compound 11-3 (colorless, amorphous solid) was obtained.

### Example 49-4: 3-[(1-naphthoylamino)methyl]-5-[(2S)-1-isobutylsulfonylpyrrolidin-2-yl]-1,2,4-oxadiazole 11A

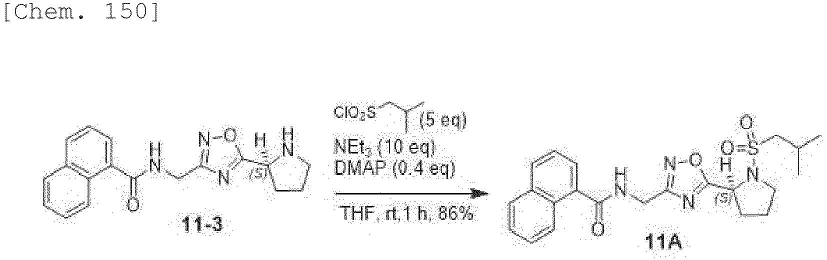

Compound 11-3 (6.2 mg, 0.019 mmol) prepared in Example 49-3 was added to a 10-ml eggplant-shaped flask, then ultra-dehydrated THF (0.641 ml), triethylamine (26.7 pl, 0.192 mmol) and DMAP (0.94 mg, 0.008 mmol) were added sequentially, and finally isobutylsulfonyl chloride (12.9 pl, 0.096 mmol) was added thereto, followed by stirring the mixture at room temperature for 1 hour. The reaction was quenched by adding 0.4 M hydrochloric acid aqueous solution (2 ml) and the mixture was extracted with ethyl acetate (1 ml x 2) . The organic layer was dried over magnesium sulfate, which was then filtered out, the solvent was distilled off, and the residue was purified on a silica gel column Q-Pac SI20 size 10 (hexane:ethyl acetate = 80:20 to 34:66). After distilling off the solvent, 7.3 mg (16.5 µmol, 86%) of the desired compound 11A (white amorphous solid) was obtained.

### Example 50

### 3-[(1-naphthoylamino)methyl]-5-[(2S)-1-cyclohexylsulfonyl pyrrolidin-2-yl]-1,2,4-oxadiazole 11B

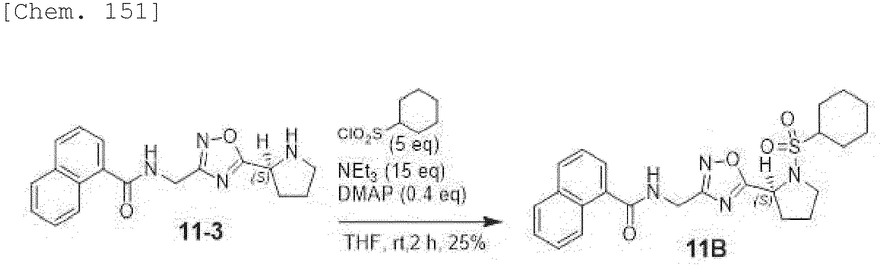

Compound 11-3 (5.7 mg, 0.018 mmol) prepared in Example 49-3 was added to a 10 ml eggplant-shaped flask, then ultra-dehydrated THF (0.589 ml), triethylamine (36.8 pl, 0.265 mmol) and DMAP (0.87 mg, 0.007 mmol) were added sequentially, and finally cyclohexanesulfonyl chloride (14.3 µl, 0.088 mmol) was added thereto, followed by stirring the mixture for 2 hours. The reaction was quenched by adding 0.5 M hydrochloric acid aqueous solution (1 ml) and the mixture was extracted with ethyl acetate (1 ml x 2) . The organic layer was dried over magnesium sulfate, which was then filtered out, the solvent was distilled off, and the residue was purified on a silica gel column Q-Pac SI20 size 10 (hexane:ethyl acetate = 80:20 to 34:66. After distilling off the solvent, 2.1 mg (4.48 µmol, 25%) of the desired compound 11B (white amorphous solid) was obtained.

The compounds prepared in Examples 49-50, as well as compounds prepared substantially according to the methods described in Examples 49-50, are described in Table 13 along with their physical property data.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acidacetonitrile;
0-0.9 min linear gradient A:B (95:5)-A:B (10:90), 0.9-3 min A:B (10:90)

**Table 13**

| [Table 42] | | | | | | |
|---|---|---|---|---|---|---|
| Ex | Cn | Chemical Structure | MW | ¹H-NMR δ ppm | [M+H] | **RT** :Min |
| 49 | 11A | 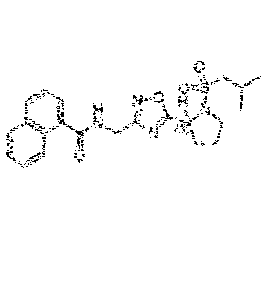 | 442.53 | (CDCl3) δ: 8.39 (1H, d, J = 8.0 Hz), 7.96 (1H. d, J = 8.4 Hz), 7.89 (1H, d. J = 8.0 Hz), 7.71 (1H. dd, J = 7.2, 1.2 Hz), 7.61-7.45 (3H. m), 5.30 (tH, dd. J = 8.4, 3.6 Hz), 4.90 (2H, d, J = 5.6 Hz), 3.70-3.62 (1H, m), 3.57-3.50 (1H, m), 3.00-2.90 (2H, m), 2.49-2.37 (1H, m), 2.32. 2.10 (4H, m), 1.06 (3H, d, J = 6.8 Hz), 1.03 (3H, d, J = 6.8 Hz) | 443 | 1.60 |
| 50 | 11B | 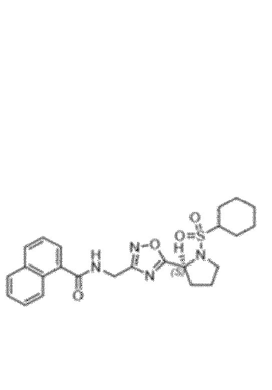 | 469.57 | (CDCl3) d: 8.38 (1H, d, J = 8.0 Hz), 7.96 (1H, d, J = 8.4 Hz), 7.89 (1H, d, J = 7.6 Hz), 7.71 (1H, dd, J = 7.2, 1.2 Hz), 7.61-7.45 (3H, m), 6.55 (1H, br-s), 5.34 (1H, dd, J = 8.4, 3.6 Hz), 4.90 (2H. d. J = 6.0 Hz), 3.79-3.69 (1H, m), 3.55-3.47 (1H, m), 2.98-2.88 (1H, m), 2.49-2.37 (1H, m). 2.27-2.06 (4H. m), 1.86-1.76 (2H. m), 1.64-1.41 (3H, m), 1.30-1.05 (3H, m) | 469 | 1.66 |

| | | | | | | |
|---|---|---|---|---|---|---|
| Ex: Example Number Cn: Compound Number MW: Molecular Weight | | | | | | |

The following compounds can be prepared substantially according to the method described in Examples 49-50.

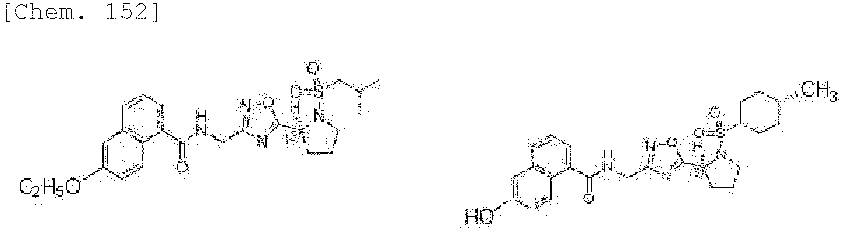

### Example 51

### (S) -N-Benzyl-2-(3-(3-phenylpropyl)-1,2,4-oxadiazol-5-yl) pyrrolidine-1-carboxamide 1F

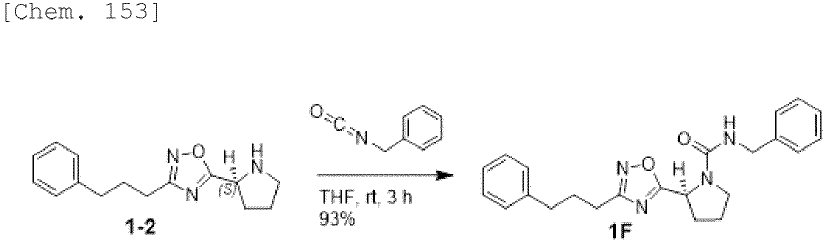

Compound 1-2 (5.3 mg, 21 µmol) prepared in Example 2-1 was added to a 4 ml vial, and THF (0.275 ml) and benzyl isocyanate (3.1 µl, 25 µmol) were added sequentially, followed by stirring the mixture at room temperature for 3 hours. The reaction was quenched by adding water and the mixture was extracted with ethyl acetate. After drying over magnesium sulfate, which was then filtered off, the solvent was distilled off and the residue was purified on a silica gel column Q-pack SI20 size 10 (hexane:ethyl acetate = 90:10 to 50:50. After distilling off the solvent, 7.4 mg (19 µmol, 93%) of the desired compound 1F (amorphous solid) was obtained.

### Example 52

### (S) -N-isopropyl-2-(3-(3-phenylpropyl)-1,2,4-oxadiazol-5-yl)pyrrolidine-1-carboxamide 1G

### Example 52-1: 4-Nitrophenyl (S)-2-(3-(3-phenylpropyl)-1,2,4-oxadiazol-5-yl)pyrrolidine-1-carboxylate 1-3

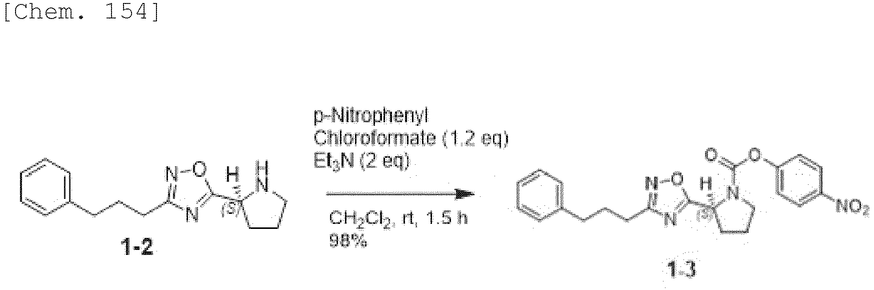

Compound 1-2 (32.8 mg, 0.127 mmol) prepared in Example 2-1 was added to a dried 10 ml eggplant-shaped flask while washing with dichloromethane (1.28 ml), and triethylamine (35.3 pl, 0.255 mmol) was added thereto. After cooling to 0°C, the mixture was added with p-nitrophenyl chloroformate (31.5 mg, 0.153 mmol) and the resultant mixture was stirred at room temperature for 1.5 hours. After removing the stirrer bar and distilling off the solvent, water was added thereto and the mixture was extracted with ethyl acetate. After drying over magnesium sulfate, which was then filtered off, the solvent was distilled off, and the residue was purified on a silica gel column Q-pack SI20 size 10 (hexane:ethyl acetate = 90:10 to 50:50). After distilling off the solvent, 52.9 mg (19 mmol, 98%) of the desired compound 1-3 (oil) was obtained.

### Example 52-2: (S)-N-isopropyl-2-(3-(3-phenylpropyl)-1,2,4-oxadiazol-5-yl)pyrrolidine-1-carboxamide 1G

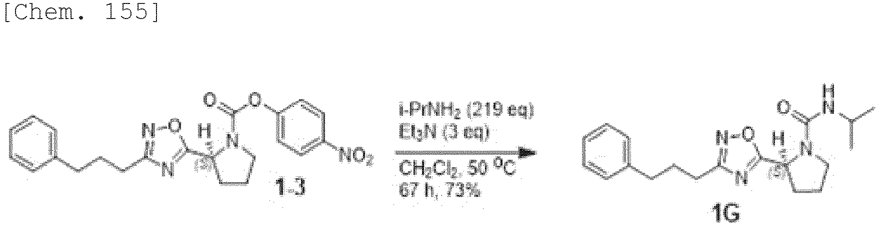

To a 4 ml vial, compound 1-3 (7.6 mg, 18 µmol) prepared in Example 52-1 was added, and dichloromethane (0.486 ml), triethylamine (7.5 µl, 54 µmol) and isopropylamine (0.344 ml, 3.94 mmol) were added sequentially, followed by stirring the mixture at 50°C for 67 hours. After distilling off the solvent and the excess reagents, the residue was purified on a silica gel column Q-pack SI20 size 10 (hexane:ethyl acetate = 80:20 to 20:80). After distilling off the solvent, 4.5 mg (13 µmol, 73%) of the desired compound 1G (oil) was obtained.

### Example 53

### (S) -N-isopropyl-2-(3-phenethyl-1,2,4-oxadiazol-5-yl) piperidine-1-carboxamide 10G

### Example 53-1: (S)-3-phenethyl-5-(piperidin-2-yl)-1,2,4-oxadiazole 10-2

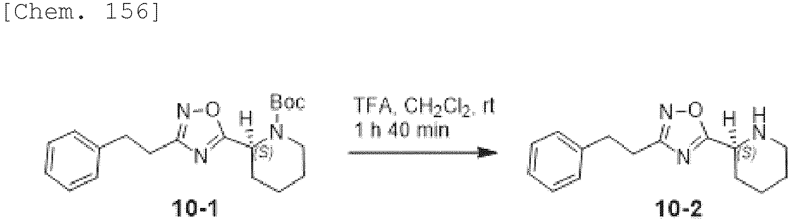

To a 4 ml vial, compound 10-1 (92.4 mg, 0.258 mmol) prepared in Example 47 was added, and dichloromethane (1.0 ml), TFA (0.333 ml) and water (16.7 pl) were added thereto, followed by stirring the mixture at room temperature for 1 hour and 40 minutes. After removing the stirrer bar and distilling off the solvent, saturated sodium bicarbonate aqueous solution (5 ml) was added thereto to make it basic, and then the mixture was extracted with ethyl acetate. The solvent was distilled off and the resulting residue was purified on a silica gel column Q-pack SI20 size 10 (hexane:ethyl acetate = 75:25 to 0:100). After distilling off the solvent, 62 mg (0.241 mmol, 93%) of the desired compound 10-2 (oil) was obtained.

### Example 53-2: 4-Nitrophenyl (S)-2-(3-phenethyl-1,2,4-oxadiazol-5-yl)piperidine-1-carboxylate 10-3

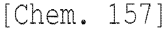

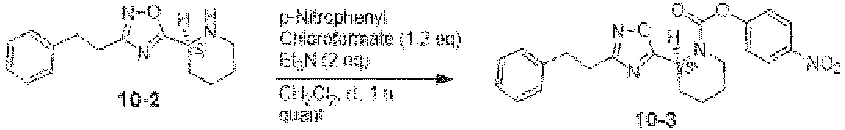

Compound 10-2 (31.5 mg, 0.122 mmol) prepared in Example 53-1 was added to a 10 ml eggplant-shaped flask while washing with dichloromethane (1.22 ml), and triethylamine (33.9 pl, 0.245 mmol) and p-nitrophenyl chloroformate (30.2 mg, 0.147 mmol) were added sequentially, followed by stirring the mixture at room temperature for 1 hour. After removing the stirrer bar and distilling off the solvent, water was then added thereto and the mixture was extracted with ethyl acetate. After drying over magnesium sulfate, which was then filtered off, the solvent was distilled off, and the residue was purified on a silica gel column Q-pack SI20 size 10 (hexane:ethyl acetate = 95:5 to 75:25). After distilling off the solvent, 52 mg (0.122 mmol, 100%) of the desired compound 10-3 (oil) was obtained.

### Example 53-3: (S)-N-isopropyl-2-(3-phenethyl-1,2,4-oxadiazol-5-yl)piperidine-1-carboxamide 10G

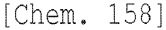

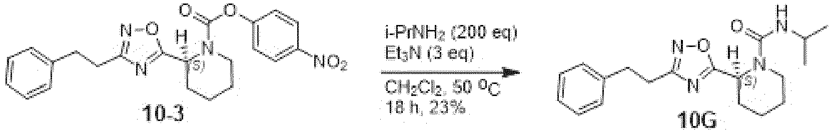

Compound 10-3 (21.5 mg, 51 µmol) prepared in Example 53-2 was added to a 10 ml eggplant-shaped flask, and dichloromethane (1.454 ml), triethylamine (21.2 pl, 0.153 mmol) and isopropylamine (0.890 ml, 10.17 mmol) were added sequentially, followed by stirring the mixture at 50°C for 18 hours. After distilling off the solvent and excess reagent, the residue was purified on a silica gel column Q-pack SI20 size 10 (hexane:ethyl acetate = 95:5 to 60:40). After distilling off the solvent, 4.0 mg (12 µmol, 23%) of the desired compound 10G (oil) was obtained.

### Example 54

### (S) -N-(tert-butyl)-2-(3-(3-phenylpropyl)-1,2,4-oxadiazol-5-yl)pyrrolidine-1-carboxamide 1H

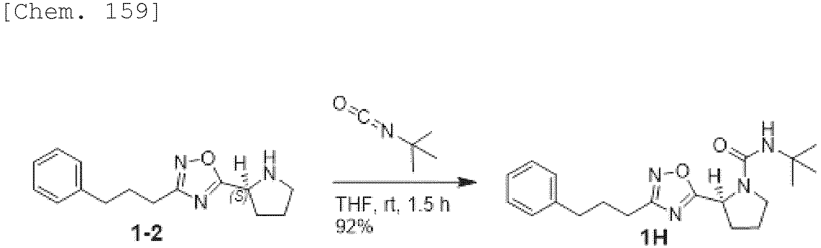

Compound 1-2 (6.3 mg, 24 µmol) prepared in Example 2-1 was added to a 4 ml vial, and THF (0.326 ml) and t-butyl isocyanate (14.7 pl, 0.122 mmol) were added sequentially, followed by stirring the mixture at room temperature for 1 hour 30 min. After the solvent and excess reagent were removed, water was added and the mixture was extracted with ethyl acetate. After distilling off the solvent, the residue was purified on a silica gel column Q-pack SI20 size 10 (hexane:ethyl acetate = 90:10 to 34:66). After distilling off the solvent, 8.0 mg (22 µmol, 92%) of the desired compound 1H (oil) was obtained.

### Example 55

### Tert-butyl (S)-2-(3-(2-(pyridin-4-yl)ethyl)-1,2,4-oxadiazol -5-yl)piperidine-1-carboxylate 12-1

### Example 55-1: N'-hydroxy-3-(pyridin-4-yl)propanimidamide

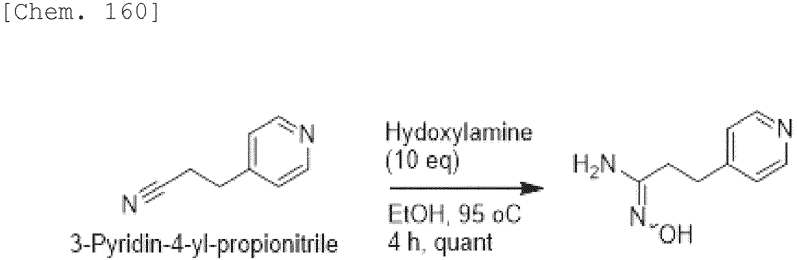

3-Pyridi-4-yl-propionitrile (497 mg, 3.76 mmol) and 50% hydroxylamine aqueous solution (2.22 ml, 37.6 mmol) were added to an eggplant-shaped flask, and anhydrous ethanol (12.5 ml) was added thereto, followed by heating the mixture to reflux at 95°C for 4 hours. After distilling off the solvent, the residue was dried in vacuo to afford the title compound (oil, 621 mg, yield: 100%).

### Example 55-2: tert-butyl (S)-2-(3-(2-(pyridin-4-yl)ethyl)-1,2,4-oxadiazol-5-yl)piperidine-1-carboxylate 12-1

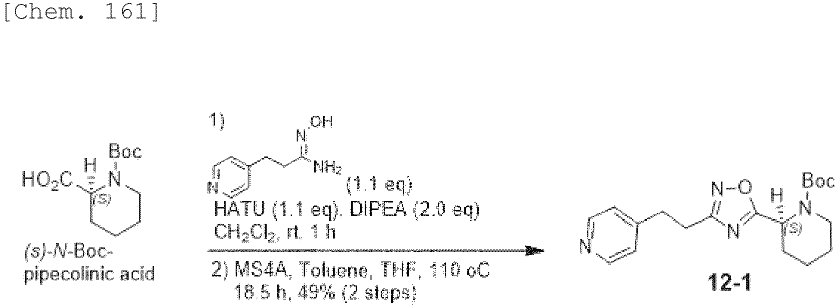

N-Boc-L-pipecolinic acid (300 mg, 1.31 mmol) was added to a 25 ml eggplant-shaped flask and was dissolved in dichloromethane (3.54 ml). HATU (547 mg, 1.44 mmol) and diisopropylethylamine (0.456 ml, 2.62 mmol) were then added thereto and the mixture was stirred for 6 minutes at room temperature under a nitrogen atmosphere. Then, N'-hydroxy-3-pyridi-4-yl-propanimidamide (238 mg, 1.44 mmol) prepared in Example 55-1 was added thereto while washing with dichloromethane (3.0 ml) and the mixture was stirred at room temperature for 1 hour.

After removing the stirrer bar and removing the solvent, water was added thereto, the mixture was extracted with ethyl acetate, and the extract was washed with saturated brine, followed by distilling off the solvent. The residue was purified on silica gel column Q-pack SI30 size 20 (chloroform:methanol = 100:0 to 95:5). After distilling off the solvent, the imidamide intermediate was obtained as a mixture with a urea compound. The intermediate was added with pre-dried MS4Å (2.46 g), and was then dissolved in ultra-dehydrated toluene (6.54 ml) and ultra-dehydrated THF (0.654 ml). The mixture was stirred at 110°C for 18.5 hours with a Dimroth condenser attached. After removing MS4Å by celite filtration and distilling off the solvent, the resulting residue was purified on silica gel column Q-pack SI30 size 20 (chloroform:methanol = 100:0 to 95:5). After distilling off the solvent, 231 mg (0.644 mmol, 49%, (after 2 steps)) of the desired compound 12-1 (oil) was obtained.

### Example 56

### (S) -N-(tert-butyl)-2-(3-(2-(pyridin-4-yl)ethyl)-1,2,4-oxadiazol-5-yl)piperidine-1-carboxamide 12H

### Example 56-1: (S)-5-(piperidin-2-yl)-3-(2-(pyridin-4-yl) ethyl)-1,2,4-oxadiazole 120-2

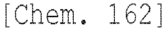

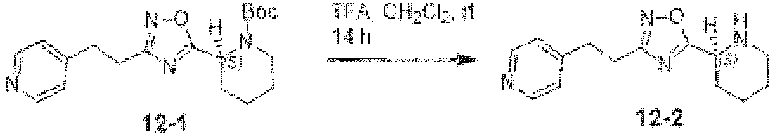

Compound 12-1 (226 mg, 0.631 mmol) was added to a 25 ml eggplant-shaped flask, and dichloromethane (1.58 ml) and TFA (0.525 ml) were added thereto, followed by stirring the mixture at room temperature for 14 hours. After removing the stirrer bar and distilling off the solvent, saturated sodium bicarbonate aqueous solution (5 ml) was then added thereto to make it basic, and then the mixture was extracted with ethyl acetate. After drying over magnesium sulfate, which was then filtered off and the solvent was distilled off to afford 131 mg (0.509 mmol, 81%) of the desired compound 12-2 (oil).

### Example 56-2: (S)-N-(tert-butyl)-2-(3-(2-(pyridin-4-yl) ethyl)-1,2,4-oxadiazol-5-yl)piperidine-1-carboxamide 12H

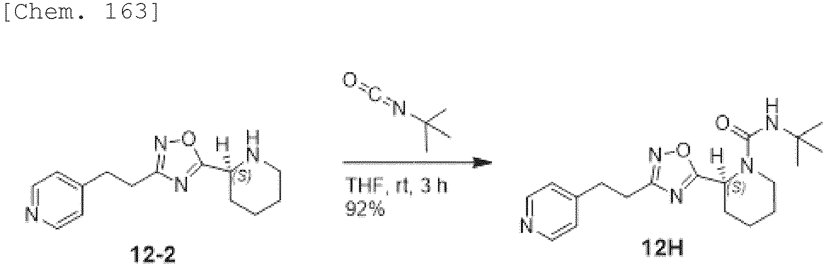

Compound 12-2 (11.5 mg, 45 µmol) prepared in Example 56-1 was added to a 4 ml vial, and THF (0.594 ml) and t-butyl isocyanate (26.8 pl, 0.223 mmol) were added sequentially, followed by stirring the mixture at room temperature for 3 hours. After adding water and extracting the mixture with ethyl acetate, the solvent was distilled off, and the residue was purified on a silica gel column Q-pack SI20 size 10 (hexane:ethyl acetate = 80:20 to 20:80). After distilling off the solvent, 14.7 mg (41 µmol, 92%) of the desired compound 12H (oil) was obtained.

### Example 57

### (S) -5-(1-(Isobutylsulfonyl)piperidin-2-yl)-3-(2-(pyridin-4-yl)ethyl)-1,2,4-oxadiazole 12A

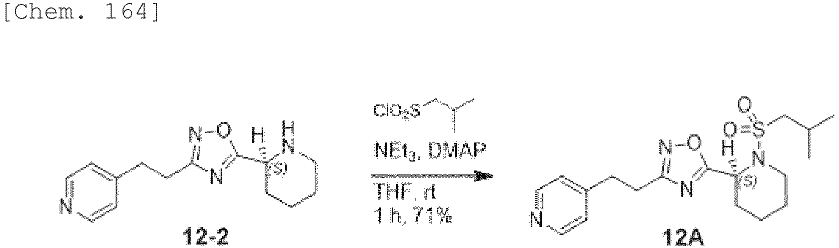

Compound 12-2 (22.3 mg, 86 µmol) prepared in Example 56-1 was added to a 10 ml eggplant-shaped flask, and THF (0.863 ml), triethylamine (108.µl, 0.777 mmol) and DMAP (2.1 mg, 17 µmol) were added sequentially, then isobutylsulfonyl chloride (34.9 pl, 0.259 mmol) was added thereto, followed by stirring the mixture at room temperature for 1 hour. The reaction was quenched by adding methanol (50 pl) and stirring for 10 minutes. After distilling off the solvent, saturated sodium bicarbonate aqueous solution was added thereto and the mixture was extracted with ethyl acetate. After distilling off the solvent, the residue was purified on a silica gel column Q-pack SI20 size 10 (chloroform:methanol = 100:0 to 95:5). After distilling off the solvent, 23.3 mg (62 µmol, 71%) of the desired compound 12A (oil) was obtained.

The physical property data for the compounds prepared in Examples 51-57 are shown in Table 14.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acidacetonitrile;
0-0.9 min linear gradient A:B (95:5)-A:B (10:90), 0.9-3 min A:B (10:90)

**Table 14**

| [Table 43] | | | | | | |
|---|---|---|---|---|---|---|
| Ex | Cn | Chemical Structure | MW | ¹H-NMR δ ppm | [M+H] | RT :Min |
| 51 | 1F | 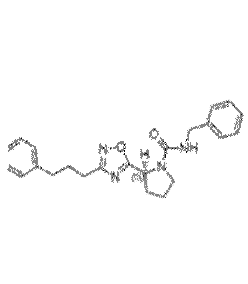 | 390.49 | CDCl3) d: 7.31-7.16 (10H, m), 5.24 (1H. dd, J = 8.0, 2.4 Hz), 4.90 (1H. br-s). 4.46 (1H, dd. J = 14.4, 6.0 Hz), 4.37 (1H, dd, J = 14.4, 5.6 Hz). 3.58 (1H, td, J = 8.4. 3.2 Hz), 3.44 (1H, q, J = 7.2 Hz), 2.75-2.67 (4H, m), 2.36-2.01 (6H. m) | 391 | 1.69 |
| 52 | 1G | 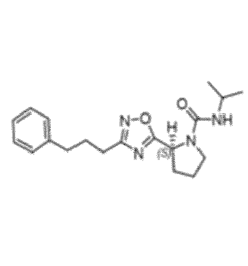 | 342.44 | CDCl3) d: 7.31-7.16 (5H, m). 5.19 (1H. dd, J = 8.0. 2.4 Hz), 4.37 (1H, br.d. J = 7.2 Hz), 3.94 (1H, sep, J = 5.8 Hz), 3.53 (1H. m), 3.41 (1H, m), 2.76-2.68 (4H, m), 2.31-2.03 (6H, m), 1.14 (6H, t, J = 6.7 Hz) | 343 | 1.64 |
| 53 | 10G | 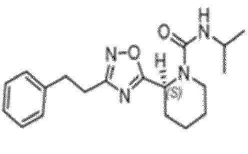 | 342.44 | (CDCl3) d: 7.33-7.15 (5H. m), 5.79 (1H, m), 4.41 (1H, d, J = 7.2 Hz), 3.99 (1H, m), 3.55 (1H, br-d, J = 12.8 Hz). 3.19-3.10 (1H, m). 3.10-2.99 (4H, m), 2.31 (1H, br-d, J = 13.6 Hz), 1.91 (1H, m), 1.77-1.66 (2H, m), 1.62-1.50 (1H, m), 1.38-1.26 (1H. m), 1.18 (6H, d, J = 6.4 Hz) | 343 | 1.67 |
| 54 | 1H | 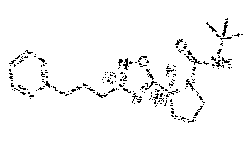 | 356.47 | (CDCl3) d: 7.30-7.14 (5H. m), 5.17 (1H, dd. J = 8.0, 2.8 Hz), 4.48 (1H, s), 3.56-3.48 (1H, m), 3.43- | 357 | 1.71 |

**Table 14 continued**

| [Table 44] | | | | | | |
|---|---|---|---|---|---|---|
| | | | | 3.36 (1H, m), 2.76-2.67 (4H, m), 2.32-2.03 (6H, m), 1.33 (9H. s) | | |
| 55 | 12-1 | 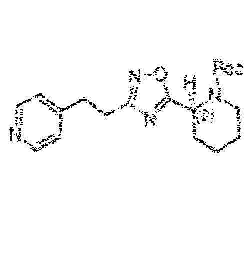 | 358.44 | (CDCl3) d: 8.51 (2H, dd, J = 4.4, 1.6 Hz), 7.16 (2H, dd, J = 4.4, 1.6 Hz), 5.52. 5.38 (1H, m), 4.20-3.90 (1H, br-s), 3.13-3.01 (4H, m), 3.00-2.80 (1H, br-s), 2.32-2.20 (1H, m), 1.98-1.83 (1H, m), 1.78-1.20 (13H, m) | 359 | 1.29 |
| 56 | 12H | 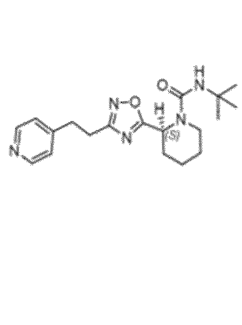 | 357.46 | (CDCl3) d: 8.51 (2H, dd, J = 4.4, 1.6 Hz). 7.14 (2H, dd, J = 4.4, 1.6 Hz), 5.77 (1H, dd, J = 5.6, 2.0 Hz), 4.51 (1H, br-s), 3.49 (1H, br-d, J = 12.4 Hz), 3.15-3.02 (5H, m), 2.29 (1H, br-d, J = 13.6 Hz), 1.91 (1H, m), 1.76-1.49 (3H, m), 1.36 (9H, s). 1.32. 1.19 (1H, m) | 358 | 0.63 |
| 57 | 12A | 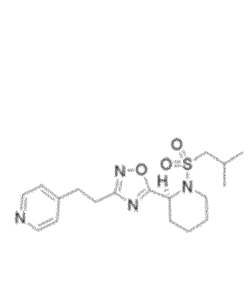 | 378.49 | (CDCl3) d: 8.53 (2H, br-d, J = 4.4 Hz), 7.16 (2H, br-d, J = 6.0 Hz), 5.41 (1H, d, J = 4.8 Hz), 3.79 (1H, br-d, J = 11.6 Hz), 3.18-3.09 (1H, m), 3.10 (4H, s), 2.97-2.86 (2H, m), 2.33-2.21 (2H. m), 2.07-1.96 (1H, m), 1.83-1.58 (2H, m), 1.42-1.28 (1H, m), 1.09 (3H, d, J = 7.6 Hz), 1.08 (3H, d, J = 7.6 Hz) | 379 | 1.24 |

| | | | | | | |
|---|---|---|---|---|---|---|
| Ex: Example Number Cn: Compound Number MW: Molecular Weight | | | | | | |

### Example 58

### (1s,4s)-N-(tert-butyl)-1-(3-(2-(pyridin-4-yl)ethyl)-1,2,4-oxadiazol-5-yl)-2-azabicyclo[2.2.2]octane-2-carboxamide 13H Example 58-1: 5-((1s,4s)-2-azabicyclo[2.2.2]octan-1-yl)-3-(2-(pyridin-4-yl)ethyl)-1,2,4-oxadiazole 13-2

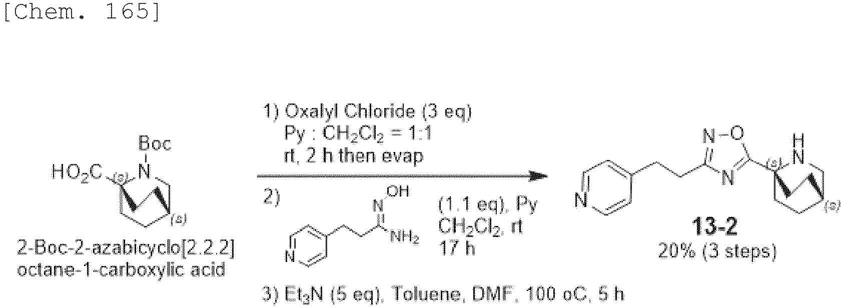

N-Boc-2-azabicyclo[2.2.2]octane-1-carboxylic acid (24.5 mg, 96 µmol) was added to a 10 ml eggplant-shaped flask and was dissolved in dichloromethane (0.480 ml) and pyridine (0.480 ml). Oxalyl chloride (25.2 µl, 0.288 mmol) was slowly added dropwise thereto and the mixture was stirred for 2 hours at room temperature under a nitrogen atmosphere. After distilling off the solvent and excess reagents, azeotropic distillation with toluene was conducted once, dichloromethane (0.960 ml) and pyridine (387 ul, 4.80 mmol) were added and N'-hydroxy-3-pyridi-4-yl-propanimidamide (17.4 mg, 0.106 mmol) was added thereto, followed by stirring the mixture at room temperature for 17 hours. After removing the stirrer bar and distilling off the solvent, water was added thereto, the mixture was washed with ethyl acetate, and the aqueous layer was concentrated to afford the intermediate as a hydrochloride salt. This was subjected to azeotropic distillation with toluene three times, and ultra-dehydrated toluene (0.480 ml), ultra-dehydrated DMF (0.960 ml) and triethylamine (66.5 pl, 0.480 mmol) were added thereto, followed by stirring the mixture at 100°C for 5 hours with a Dimroth condenser attached. The solvent was distilled off, and water and saturated sodium bicarbonate aqueous solution were added thereto, followed by extracting the mixture extracted with ethyl acetate. After distilling off the solvent, the resulting residue was purified on a silica gel column Q-pack SI30 size 20 (chloroform:methanol = 100:0 to 90:10). After distilling off the solvent, 5.7 mg (20 µmol, 20% (after 3 steps)) of the desired compound 13-2 (oil) was obtained.

### Example 58-2: (1s,4s)-N-(tert-butyl)-1-(3-(2-(pyridin-4-yl) ethyl)-1,2,4-oxadiazol-5-yl)-2-azabicyclo[2.2.2]octane-2-carboxamide 13H

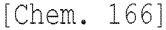

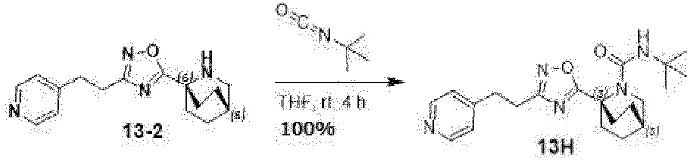

Compound 13-2 (2.7 mg, 9 µmol) prepared in Example 58-1 was added to a 10 ml eggplant-shaped flask, and THF (0.317 ml) and t-butyl isocyanate (11.4 ul, 95 µmol) were added sequentially, followed by stirring the mixture at room temperature for 4 hours. After distilling off the solvent as it was, 3.6 mg (9 µmol, 100%) of the desired compound 13H (oil) was obtained.

### Example 59

### Tert-butyl (1s,4s)-1-(3-(2-(pyridin-4-yl)ethyl)-1,2,4-oxadiazol-5-yl)-2-azabicyclo[2.2.2]octane-2-carboxylate 13-1

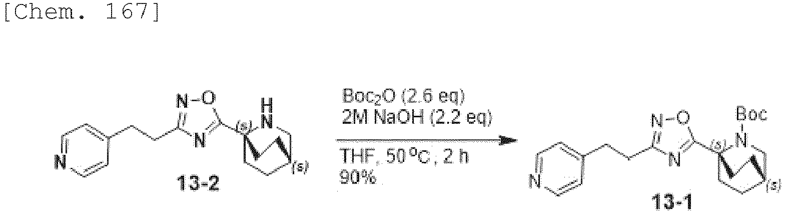

Compound 13-2 (3.0 mg, 11 µmol) prepared in Example 58-1 was added to a 10 ml eggplant-shaped flask, THF (0.422 ml), di-t-butyl bicarbonate (6.1 mg, 27 µmol), and 2M sodium hydroxide solution (11.6 µl, 23 µmol) were added sequentially, followed by stirring the mixture at 50°C for 2 hours. Water was added and the mixture was extracted with ethyl acetate, the solvent was distilled off, and the resulting residue was purified on a silica gel column Silica HC D 5 g (chloroform:methanol = 100:0 to 95:5). After distilling off the solvent, 3.7 mg (9.6 µmol, 90%) of the desired compound 13-1 (oil) was obtained.

Physical property data for the compounds prepared in Examples 58-59 are shown in Table 15.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acidacetonitrile;
0-0.9 min linear gradient A:B (95:5)-A:B (10:90), 0.9-3 min A:B (10:90)

**Table 15**

| [Table 45] | | | | | | |
|---|---|---|---|---|---|---|
| Ex | Cn | Chemical Structure | MW | ¹H-NMR δ ppm | [M+H] | RT :Min |
| 58 | 13H | 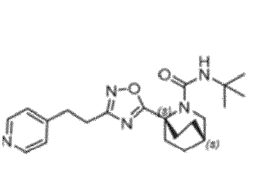 | 383.5 | (CDCl3) δ: 8.49 (2H, br-d, J = 5.2 Hz), 7.13 (2H, br-d, J = 4.8 Hz). 4.09 (1H, s). 3.49 (2H, d, J = 2.4 Hz), 3.11-2.98 (4H, m), 2.61-2.50 (2H, m), 2.05-1.73 (7H, m), 1.28 <9H, s) | 384 | 0.63 |
| 59 | 13-1 | 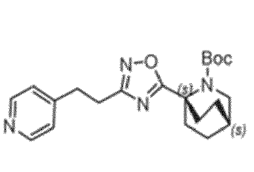 | 384.48 | (CDCl3) δ: 8.52 (2H, br-d, J = 3.6 Hz). 7.19 (2H, br-d, J - 4.8 Hz), 3.61 (2H, br-s), 3.16-3.01 (4H, m), 2.48 (2H, br-s). 2.20-1.70 (7H, m), 1.50-1.10 (9H, m) | 385 | 1.28 |

| | | | | | | |
|---|---|---|---|---|---|---|
| Ex: Example Number Cn: Compound Number MW: Molecular Weight | | | | | | |

### Example 60

### Isobutyl (S)-2-(3-(3-phenylpropyl)-1,2,4-oxadiazol-5-yl) pyrrolidine-1-carboxylate 1I

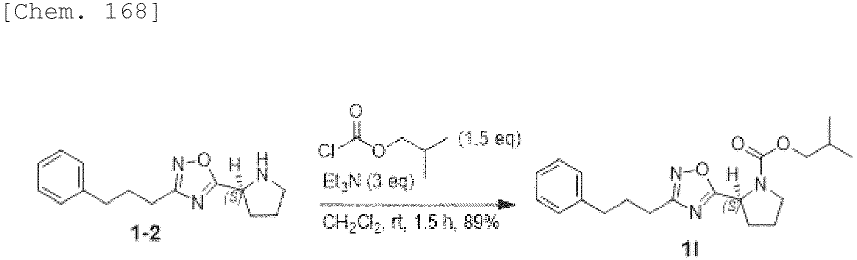

Compound 1-2 (6.4 mg, 25 µmol) prepared in Example 2-2 was added to a 4 ml vial, and dichloromethane (0.332 ml), triethylamine (10.3 ul, 75 µmol) and isobutyl chloroformate (5.0 µl, 37 µmol) were added sequentially, followed by stirring the mixture at room temperature for 1 hour 30 minutes. The reaction was quenched by adding water and the mixture was extracted with ethyl acetate. After drying over magnesium sulfate, which was then filtered off, the solvent was distilled off, and the residue was purified on a silica gel column Silica HC 5 g (hexane: ethyl acetate = 95:5 to 66:34). After distilling off the solvent, 7.9 mg (22 µmol, 89%) of the desired compound 1I (amorphous solid) was obtained.

### Example 61

### Benzyl (S)-2-(3-(3-phenylpropyl)-1,2,4-oxadiazol-5-yl) pyrrolidine-1-carboxylate 1J

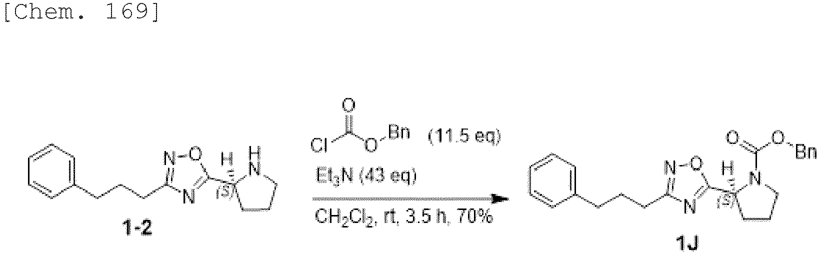

Compound 1-2 (5.7 mg, 25 µmol) prepared in Example 2-2 was added to a 4 ml vial, and dichloromethane (0.295 ml), triethylamine (132 pl, 0.952 mmol) and benzyl chloroformate (37.4 pl, 0.255 mmol) were added sequentially, followed by stirring the mixture at room temperature for 3 hours 30 minutes. The reaction was quenched by adding water and the mixture was extracted with ethyl acetate. After drying over magnesium sulfate, which was then filtered off, the solvent was distilled off, and the residue was purified on a silica gel column Silica HC 5 g (hexane:ethyl acetate = 100:0 to 66:34). After distilling off the solvent, 6.1 mg (15.6 µmol, 70%) of the desired compound 1J (oil) was obtained.

### Example 62

### Methyl (S)-2-(3-(3-phenylpropyl)-1,2,4-oxadiazol-5-yl) pyrrolidine-1-carboxylate 1K

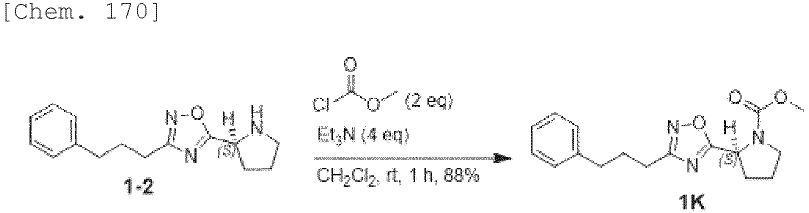

Compound 1-2 (5.9 mg, 23 µmol) prepared in Example 2-2 was added to a 4 ml vial, and dichloromethane (0.306 ml), triethylamine (12.7 pl, 92 µmol) and methyl chloroformate (3.7 pl, 46 µmol) were added sequentially, followed by stirring at room temperature for 1 hour. The reaction was quenched by adding water and the mixture was extracted with ethyl acetate. After drying over magnesium sulfate, which was then filtered off, the solvent was distilled off, and the residue was purified on a silica gel column Silica HC 5 g (hexane:ethyl acetate = 90:10 to 60:40). After distilling off the solvent, 6.1 mg (16 µmol, 70%) of the desired compound 1K (oil) was obtained.

### Example 63

### Isopropyl (S)-2-(3-(3-phenylpropyl)-1,2,4-oxadiazol-5-yl) pyrrolidine-1-carboxylate 1L

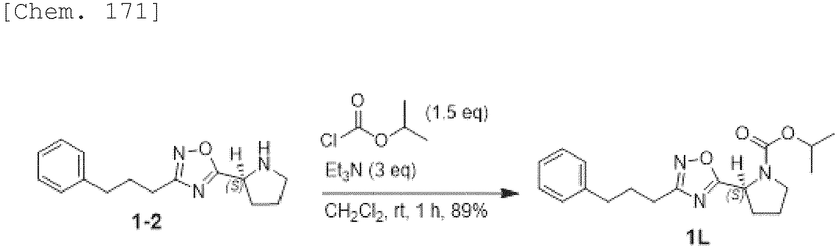

Compound 1-2 (6.5 mg, 25 µmol) prepared in Example 2-2 was added to a 4 ml vial, and dichloromethane (0.337 ml), triethylamine (10.5 ul, 76 µmol) and isopropyl chloroformate (4.5 pl, 38 µmol) were added sequentially, followed by stirring the mixture at room temperature for 1 hour. The reaction was quenched by adding water and the mixture was extracted with ethyl acetate. After drying over magnesium sulfate, which was then filtered off, the solvent was distilled off, and the residue was purified on a silica gel column Silica HC 5 g (hexane:ethyl acetate = 95:5 to 66:34). After distilling off the solvent, 7.7 mg (22 µmol, 89%) of the desired compound 1L (oil) was obtained.

### Example 64

### Phenyl (S)-2-(3-(3-phenylpropyl)-1,2,4-oxadiazol-5-yl)pyrrolidine-1-carboxylate 1M

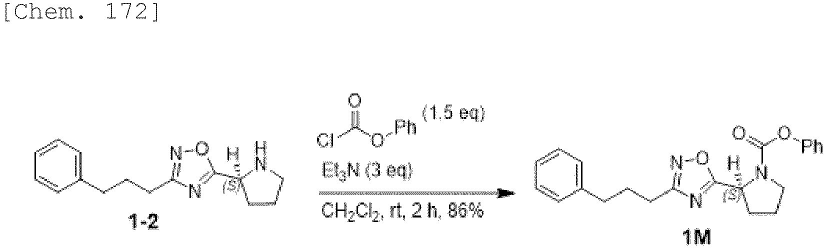

Compound 1-2 (6.5 mg, 25 µmol) prepared in Example 2-2 was added to a 4 ml vial, and dichloromethane (0.337 ml), triethylamine (10.5 ul, 76 µmol) and phenyl chloroformate (4.9 µl, 38 µmol) were added sequentially, followed by stirring the mixture at room temperature for 2 hours. The reaction was quenched by adding water and the mixture was extracted with ethyl acetate. After drying over magnesium sulfate, which was then filtered off, the solvent was distilled off, and the residue was purified on a silica gel column Silica HC 5 g (hexane:ethyl acetate = 95:5 to 66:34). After distilling off the solvent, 8.2 mg (22 µmol, 86%) of the desired compound 1M (oil) was obtained.

### Example 65

### (-)-menthyl (S)-2-(3-(3-phenylpropyl)-1,2,4-oxadiazol-5-yl) pyrrolidine-1-carboxylate 1N

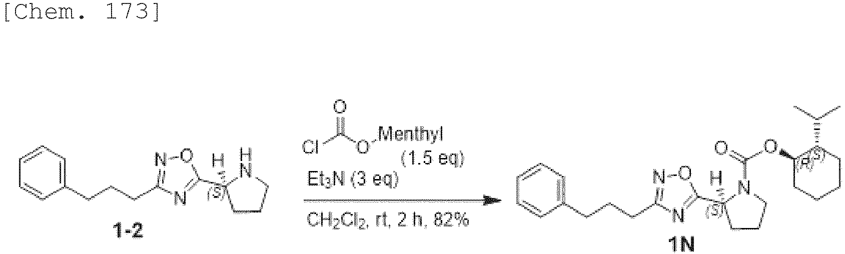

Compound 1-2 (6.7 mg, 26 µmol) prepared in Example 2-2 was added to a 4 ml vial, and dichloromethane (0.347 ml), triethylamine (10.8 µl, 78 µmol) and menthyl chloroformate (8.5 µl, 39 µmol) were added sequentially, followed by stirring the mixture at room temperature for 2 hours. The reaction was quenched by adding water and the mixture was extracted with ethyl acetate. After drying over magnesium sulfate, which was then filtered off, the solvent was distilled off, and the residue was purified on a silica gel column Silica HC 5 g (hexane:ethyl acetate = 95:5 to 75:25). After distilling off the solvent, 9.4 mg (21 µmol, 82%) of the desired compound 1N (oil) was obtained.

### Example 66

### Cyclopentyl (S)-2-(3-(3-phenylpropyl)-1,2,4-oxadiazol-5-yl) pyrrolidine-1-carboxylate 1O

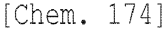

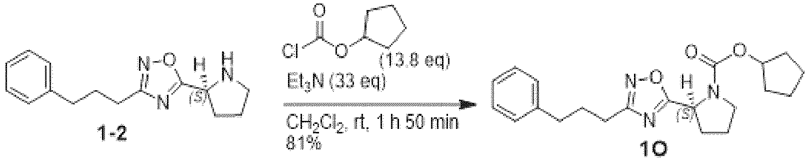

Compound 1-2 (6.9 mg, 27 µmol) prepared in Example 2-2 was added to a 4 ml vial, and dichloromethane (0.358 ml), triethylamine (123 pl, 0.885 mmol) and cyclopentyl chloroformate (48.5 pl, 37 µmol) were added sequentially, followed by stirring the mixture at room temperature for 1 hour 50 minutes. The reaction was quenched by adding water and the mixture was extracted with ethyl acetate. After drying over magnesium sulfate, which was then filtered off, the solvent was distilled off, and the residue was purified on a silica gel column Silica HC 5 g (hexane:ethyl acetate = 95:5 to 66:34). After distilling off the solvent, 8.0 mg (21 µmol, 81%) of the desired compound 1O (oil) was obtained.

Physical property data for the compounds prepared in Examples 60-66 are shown in Table 16.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acidacetonitrile;
0-0.9 min linear gradient A:B (95:5)-A:B (10:90), 0.9-3 min A:B (10:90)

**Table 16**

| [Table 46] | | | | | | |
|---|---|---|---|---|---|---|
| Ex | Cn | Chemical Structure | MW | ¹H-NMR δ ppm | [M+H] | RT :Min |
| 60 | 1I | 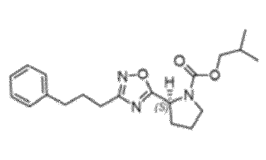 | 357.45 | (CDCl3) δ: 7.31-7.16 (5H, m), 5.16 (0.4H, br-d, J = 8.0 Hz), 5.08 (0.6H, dd, J = 8.4, 2.8 Hz), 3.89-3.48 (4H. m), 2.77-2.66 (4H, m), 2.43-1.70 (8H, m), 0.93 (2.4H, d, J = 6.8 Hz), 0.75 (3.6H, d, J = 6.4 Hz) | 358 | 1.84 |
| 61 | 1J | 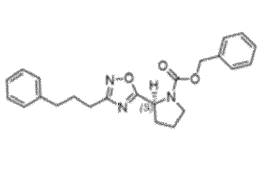 | 391.47 | (CDCl3) δ: 7.39-7.13 (10H, m), 5.21-4.97 (3H, m), 3.78-3.68 (1H, m), 3.65-3.51 (1H, m), 2.77-2.64 (4H. m), 2.42-2.27 (1H, m), 2.16-1.96 (5H, m) | 392 | 1.82 |
| 62 | 1K | 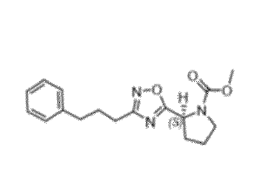 | 315.37 | (CDCl3) δ: 7.31-7.15 (5H, m), 5.16 (0.5H, br-d, J = 8.0 Hz). 5.07 (0.5H, br-d, J - 8.0 Hz), 3.71 (1.SH, s), 3.62 (1.5H. s), 3.73-3.45 (2H. m), 2.78-2.65 (4H, m), 2.42-2.25 (1H, m), 2.17-1.95 (5H, m) | 316 | 1.67 |
| 63 | 1L | 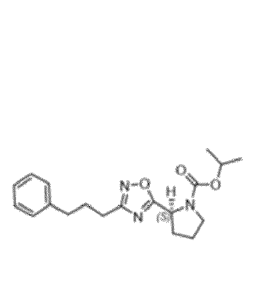 | 343.43 | (CDCl3) δ: 7.32-7.16 (5H, m), 5.14 (0.5H, br-d, J = 7.6 Hz), 5.04 (O.SH, dd, J = 8.0, 3.2 Hz), 4.93-4.79 (1H, m), 3.15-3.43 (2H, m), 2.77-2.67 (4H, m), 2.43-2.25 (1H, m), 2.16-1.93 (5H, m), 1.24 (3H, br-d, J = 5.6 Hz), 1.16 (1.5H, d, J = 6.4Hz). 0.96 (1.5H, d, J = 6.0 Hz) | 344 | 1.79 |

**Table 16 continued**

| [Table 47] | | | | | | |
|---|---|---|---|---|---|---|
| 64 | 1M | 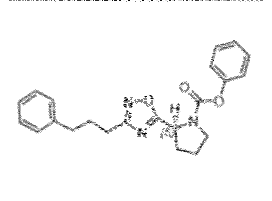 | 377.44 | (CDCl3) 7.37-6.94 (10H, m), 5.31 (0.5H, dd, J = 8.0. 3.6 Hz). 5.23 (0.5H. dd, J = 8.0. 2.8 Hz), 3.96-3.62 (2H, m), 2.19-2.65 (4H, m), 2.53-2.36 (1H, m), 2.28-2.03 (5H. m) | 378 | 1.8 |
| 65 | 1N | 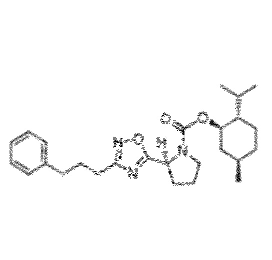 | 439.60 | (CDCl3) δ: 7.31-7.13 (5H, m), 5.15 (0.4H, br-d, J = 8.0 Hz), 5.02 (0.6H, dd. J = 8.0. 2.8 Hz), 4.57-4.45 (1H, m), 3.78-3.45 (2H, m), 2.76-2.67 (4H. m), 2.43-2.26 (1H, m), 2.14. 0.34 (12H, m), 0.88 (3H, d, J = 6.8 Hz), 0.78 (3H, d, J = 6.8 hz). 0.73 (3H, d, J = 6.4 Hz) | 440 | 2.32 |
| 66 | 1O | 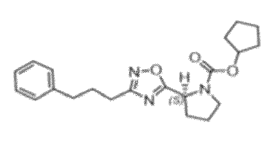 | **369.47** | (CDCl3) δ: 7.31-7.15 (5H. m), 5.17-4.98 (2H, m), 3.75-3.42 (2H, m), 2.76-2.67 (4H, m), 2.42-2.25 (1H, m), 2.16-1.30 (13H, m) | 370 | 1.85 |

| | | | | | | |
|---|---|---|---|---|---|---|
| Ex: Example Number Cn: Compound Number MW: Molecular Weight | | | | | | |

### Example 67

### Tert-butyl (1R,3S,4S)-3-(3-phenethyl-1,2,4-oxadiazol-5-yl)-2-azabicyclo[2.2.1]heptane-2-carboxylate 14-1

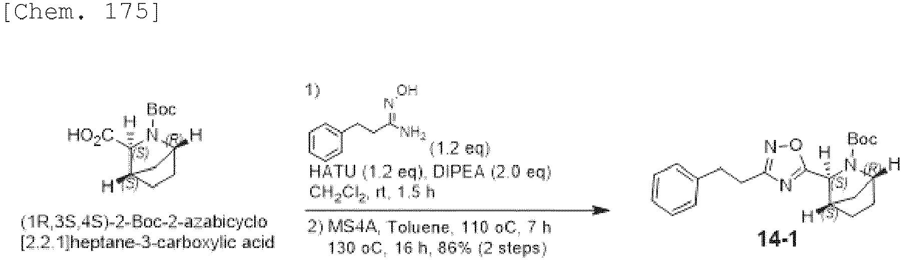

2-Boc-2-azabicyclo[2.2.1]heptane-3-carboxylic acid (200 mg, 0.829 mmol) was added to a 25 ml eggplant-shaped flask and was dissolved in dichloromethane (4.03 ml). HATU (378 mg, 0.995 mmol) and diisopropylethylamine (0.289 ml, 1.658 mmol) were then added thereto, and the mixture was stirred for 10 minutes at room temperature under a nitrogen atmosphere. Then, N'-hydroxy-3-phenylpropanimidamide (163 mg, 0.995 mmol) was added while washing with dichloromethane (1.5 ml), and the mixture was stirred at room temperature for 1 hour 30 minutes.

After removing the stirrer bar and distilling off the solvent, the residue was purified on a silica gel column Q-pack SI30 size 20 (hexane:ethyl acetate = 90:10 to 50:50). After distilling off the solvent, the imidamide intermediate was obtained. Pre-dried MS4Å (1.61 g) was added to the intermediate, which was then dissolved in ultra-dehydrated toluene (4.15 ml). The mixture was stirred at 110°C for 7 hours and at 130°C for 16 hours with a Dimroth condenser attached. Removing MS4Å by celite filtration and distilling off the solvent, the resulting residue was purified on silica gel column Q-pack SI30 size 20 (hexane:ethyl acetate = 100:0 to 66:34). After distilling off the solvent, 264 mg (0.716 mmol, 86% (after 2 steps)) of the desired compound 14-1 (oil) was obtained.

### Example 68

### 5-((1R,3S,4S)-2-(isobutylsulfonyl)-2-azabicyclo[2.2.1] heptan-3-yl)-3-phenethyl-1,2,4-oxadiazole 14A

### Example 68-1: 5-((1R,3S,4S)-2-azabicyclo[2.2.1]heptan-3-yl) -3-phenethyl-1,2,4-oxadiazole 14-2

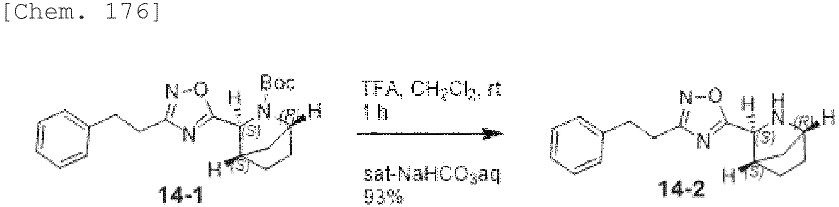

Compound 14-1 (252 mg, 0.681 mmol) prepared in Example 67 was added to a 10 ml eggplant-shaped flask, and dichloromethane (1.70 ml) and TFA (0.567 ml) were added thereto, followed by stirring the mixture at room temperature for 1 hour. After removing the stirrer bar and distilling off the solvent, saturated sodium bicarbonate aqueous solution (5 ml) was then added thereto to make it basic, and then the mixture was extracted with ethyl acetate. After drying over magnesium sulfate, filtered, and removing the solvent, 171 mg (0.636 mmol, 93%) of the desired compound 14-2 (oil) was obtained.

### Example 68-2: 5-((1R,3S,4S)-2-(isobutylsulfonyl)-2-azabicyclo[2.2.1]heptan-3-yl)-3-phenethyl-1,2,4-oxadiazole 14A

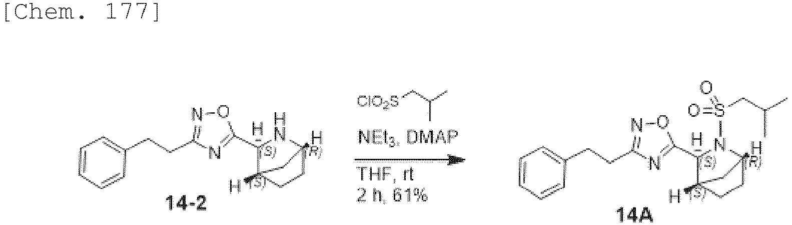

Compound 14-2 (12 mg, 45 µmol) prepared in Example 68-1 was added to a 10 ml eggplant-shaped flask, and THF (0.446 ml), triethylamine (55.6 µl, 0.401 mmol) and DMAP (1.1 mg, 9 µmol) were added sequentially, then isobutylsulfonyl chloride (18.0 pl, 0.134 mmol) was added thereto, followed by stirring the mixture at room temperature for 2 hours. The reaction was quenched by adding methanol (50 pl) and stirring for 10 minutes. After distilling off the solvent, saturated sodium bicarbonate aqueous solution was added and the mixture was extracted with ethyl acetate. After distilling off the solvent, the residue was purified on a silica gel column Q-pack SI20 size 10 (hexane:ethyl acetate = 100:0 to 75:25). After distilling off the solvent, 10.7 mg (27 µmol, 61%) of the desired compound 14A (oil) was obtained.

Physical property data for the compounds prepared in Examples 67-68 are shown in Table 17.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acid-acetonitrile;
0-0.9 min linear gradient A:B (95:5)-A:B (10:90), 0.9-3 min A:B (10:90)

**Table 17**

| [Table 48] | | | | | | |
|---|---|---|---|---|---|---|
| Ex | Cn | Chemical Structure | MW | ¹H-NMR δ ppm | [M + H] | RT :Min |
| 67 | 14-1 | 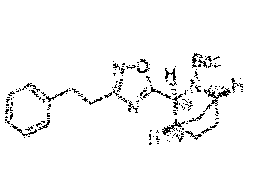 | 369.47 | (CDCl3) δ: 7.31-7.16 (5H. m), 4.61 (0.5H, s), 4.48 (0.5H, s), 4.45 (0.5H, br-s), 4.32 (0.5H, br-s), 3.10-2.98 (4H, m), 2.71 (1H, br-s), 2.07 (1H, m), 1.89-1.35 (5H, m), 1.47 (4.5H, s), 1.28 (4.5H, s) | 370 | 1.85 |
| 68 | 14A | 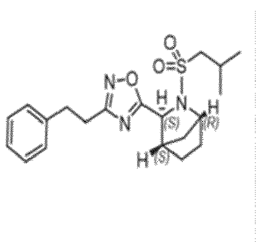 | 389.51 | (CDCl3) δ: 7.32-7.19 (5H, m), 4.78 (1H, s), 4.27 (1H, s), 3.01-2.90 (6H, m). 2.85 (1H, d, J - 4.0 Hz), 2.33-2.20 (2H, m), 2.09-2.03 (1H, m), 1.92-1.71 (3H, m), 1.52 (tH, d, J = 10.4 Hz), 1.08 (3H, d, J = 6.8 Hz), 1.02 (3H. d, 6.8 Hz) | 390 | 1.81 |

| | | | | | | |
|---|---|---|---|---|---|---|
| Ex: Example Number Cn: Compound Number MW: Molecular Weight | | | | | | |

### Example 69

### (S) -2-Phenyl-1-(2-(3-(3-phenylpropyl)-1,2,4-oxadiazol-5-yl) pyrrolidin-1-yl)ethan-1-one 1P

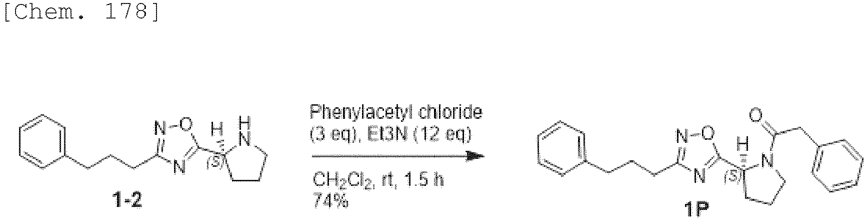

Compound 1-2 (8.5 mg, 33 µmol) prepared in Example 2-2 was added to a 4 ml vial, and dichloromethane (0.330 ml), triethylamine (54.9 pl, 0.396 mmol) and phenylacetyl chloride (13.8 µl, 99 µmol) were added sequentially, followed by stirring the mixture at room temperature for 1 hour 30 minutes. The reaction was quenched by adding methanol (20 µl), saturated sodium bicarbonate aqueous solution was added and the mixture was extracted with ethyl acetate. After distilling off the solvent, the residue was purified on a silica gel column Q-pack SI20 size 10 (hexane:ethyl acetate = 100:0 to 50:50). After distilling off the solvent, 9.2 mg (24.5 µmol, 74%) of the desired compound 1P (oil) was obtained.

### Example 70

### (S) -3-Methyl-1-(2-(3-(3-phenylpropyl)-1,2,4-oxadiazol-5-yl)pyrrolidin-1-yl)butan-1-one 1Q

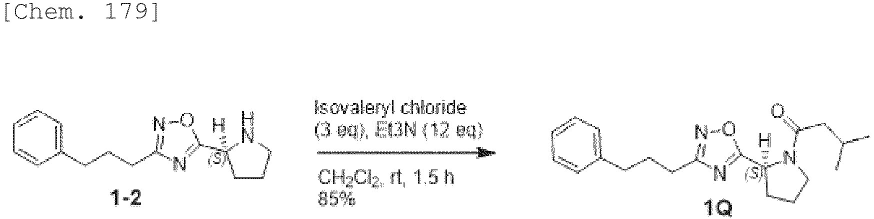

Compound 1-2 (8.8 mg, 34 µmol) prepared in Example 2-2 was added to a 4 ml vial, and dichloromethane (0.342 ml), triethylamine (56.9 µl, 0.410 mmol) and isovaleryl chloride (12.6 pl, 0.103 mmol) were added sequentially, followed by stirring the mixture at room temperature for 1 hour 30 minutes. The reaction was quenched by adding methanol (20 pl), saturated sodium bicarbonate aqueous solution was added and the mixture was extracted with ethyl acetate. After distilling off the solvent, the residue was purified on a silica gel column Q-pack SI20 size 10 (hexane:ethyl acetate = 100:0 to 50:50). After distilling off the solvent, 9.9 mg (29 µmol, 85%) of the desired compound 1Q (oil) was obtained.

### Example 71

### (S) -1-(2-(3-(3-(3-phenylpropyl)-1,2,4-oxadiazol-5-yl) pyrrolidin-1-yl)ethan-1-one 1R

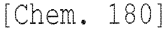

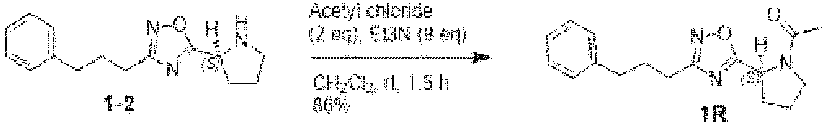

Compound 1-2 (11.4 mg, 44 µmol) prepared in Example 2-2 was added to a 4 ml vial, and dichloromethane (0.443 ml), triethylamine (49.1 pl, 0.354 mmol) and acetyl chloride (6.4 pl, 88.6 µmol) were added sequentially, followed by stirring the mixture at room temperature for 1 hour 30 minutes. The reaction was quenched by adding methanol (20 pl), saturated sodium bicarbonate aqueous solution was added and the mixture was extracted with ethyl acetate. After distilling off the solvent, the residue was purified on a silica gel column Q-pack SI20 size 10 (hexane:ethyl acetate = 90:10 to 0:100). After distilling off the solvent, 11.4 mg (38 µmol, 86%) of the desired compound 1R (oil) was obtained.

### Example 72

### (S) -Phenyl(2-(3-(3-phenylpropyl)-1,2,4-oxadiazol-5-yl) pyrrolidin-1-yl)methanone 1S

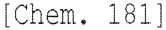

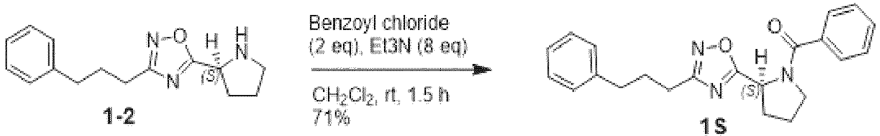

Compound 1-2 (18.8 mg, 34 µmol) prepared in Example 2-2 was added to a 4 ml vial, and dichloromethane (0.342 ml), triethylamine (37.9 pl, 0.274 mmol) and benzoyl chloride (8.1 pl, 68 µmol) were added sequentially, followed by stirring the mixture at room temperature for 1 hour 30 minutes. The reaction was quenched by adding methanol (20 µl), saturated sodium bicarbonate aqueous solution was added and the mixture was extracted with ethyl acetate. After distilling off the solvent, the residue was purified on a silica gel column Q-pack SI20 size 10 (hexane:ethyl acetate = 100:0 to 50:50). After distilling off the solvent, 8.8 mg (24 µmol, 71%) of the desired compound 1S (oil) was obtained.

### Example 73

### (S) -5-(1-isopentylpyrrolidin-2-yl)-3-(3-phenylpropyl)-1,2,4-oxadiazole 1T

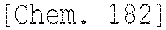

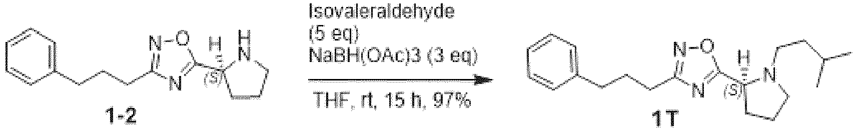

To compound 1-2 (7.7 mg, 30 µmol) prepared in Example 2-2 in a 4 ml vial, isovalerylaldehyde (16.4 pl, 0.150 mmol) was added while washing with THF (0.4 ml). After stirring at room temperature for 10 minutes, the mixture was added with sodium triacetoxyborohydride (19 mg, 90 µmol), and the mixture was stirred at room temperature for 15 hours. The reaction was quenched by adding saturated sodium bicarbonate aqueous solution and the mixture was extracted with ethyl acetate. After distilling off the solvent, the residue was purified on a silica gel column Q-pack SI20 size 10 (hexane:ethyl acetate = 100:0 to 80:20). After distilling off the solvent, 9.5 mg (29 µmol, 97%) of the desired compound 1T (oil) was obtained.

### Example 74

### (S) -5-(1-(cyclohexylmethyl)pyrrolidin-2-yl)-3-(3-phenyl propyl)-1,2,4-oxadiazole 1U

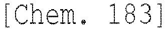

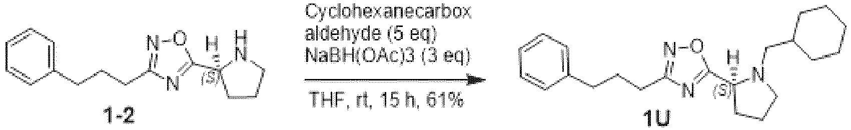

To compound 1-2 (7.9 mg, 31 µmol) prepared in Example 2-2 in a 4 ml vial, cyclohexanecarboxaldehyde (18.9 µl, 0.153 mmol) was added while washing with THF (0.41 ml). After stirring at room temperature for 10 minutes, sodium triacetoxyborohydride (19.5 mg, 92 µmol) was added thereto and the mixture was stirred at room temperature for 15 hours. The reaction was quenched by adding saturated sodium bicarbonate aqueous solution and the mixture was extracted with ethyl acetate. After distilling off the solvent, the residue was purified on a silica gel column Q-pack SI20 size 10 (hexane:ethyl acetate = 100:0 to 90:10). After distilling off the solvent, 6.7 mg (19 µmol, 61%) of the desired compound 1U (oil) was obtained.

### Example 75

### (S) -5-(1-(4-fluorobenzyl)pyrrolidin-2-yl)-3-(3-phenyl propyl)-1,2,4-oxadiazole 1V

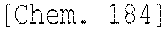

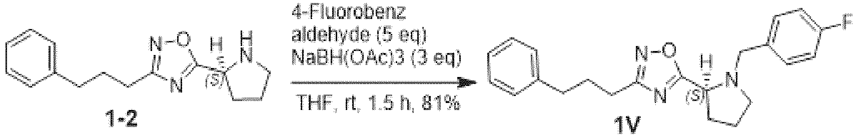

To compound 1-2 (7.1 mg, 28 µmol) prepared in Example 2-2 in a 4 ml vial, 4-fluorobenzaldehyde (14.8 pl, 0.138 mmol) was added while washing with THF (0.37 ml). After stirring at room temperature for 10 min, sodium triacetoxyborohydride (17.5 mg, 83 µmol) was added thereto, and the mixture was stirred at room temperature for 1 hour 30 minutes. The reaction was quenched by adding saturated sodium bicarbonate aqueous solution and the mixture was extracted with ethyl acetate. After distilling off the solvent, the residue was purified on a silica gel column Q-pack SI20 size 10 (hexane:ethyl acetate = 100:0 to 86:14). After distilling off the solvent, 8.2 mg (22 µmol, 81%) of the desired compound 1V (oil) was obtained.

Physical property data for the compounds prepared in Examples 69-75 are shown in Table 18.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acid-acetonitrile;
0-0.9 min linear gradient A:B (95:5)-A:B (10:90), 0.9-3 min A:B (10:90)

**Table 18**

| [Table 49] | | | | | | |
|---|---|---|---|---|---|---|
| Ex | Cn | Chemical Structure | MW | ¹H-NMR δ ppm | [M+H] | RT :Min |
| 69 | 1P | 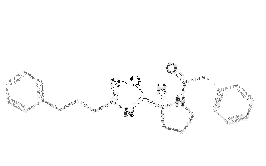 | 375.47 | (CDCl3) δ: 7.31-7.15 (10H, m), 5.33 (0.8H, dd, J = 8.4, 3.2 Hz), 5.15 (0.2H, dd, J = 8.0, 1.6 Hz), 3.82-3.53 (2H, m), 3.72 (2H, s), 2.75-2.67 (4H, m), 2.32-2.00 (6H, m) | 376 | 1.74 |
| 70 | 1Q | 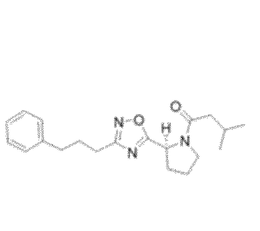 | 341.46 | (CDCl3) δ: 7.31-7.13 (5H, m), 5.32 (0.8H, m), 5.16 (0.2H. dd, J = 8.0. 1.6 Hz), 3.81-3.55 (2H, m), 2.78-2.66 (4H, m), 2.34-2.02 (9H, m), 0.96 (4.8H, d. J = 6.4 Hz), 0.92 (0.6H, d. J = 6.4 Hz), 0.81 (0.6H, d, J = 6.4 Hz) | 342 | 1.7 5 |
| 71 | 1R | 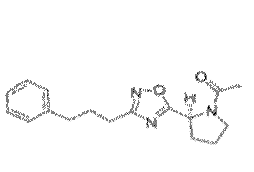 | 299.37 | (CDCl3) δ: 7.35-7.13 (5H, m), 5.31 (0.8H, dd, J - 8.0, 3.2 Hz), 5.11 (0.2H, dd, J = 8.0, 2.0 Hz). 3.82-3.55 (2H, m), 2.78-2.66 (4H, m), 2.45-2.01 (6H, m), 2.12 (3H, s) | 300 | 1.58 |
| 72 | 1S | 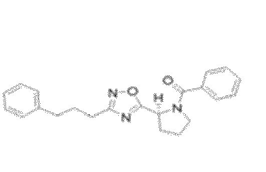 | 361.45 | (CDCl3) δ: 7.63-7.16 (10H, m), 5.50 (0.7H, dd, J = 8.0, 4.8 Hz), 5.07 (0.3H, br-s), 3.95-3.58 (2H, m), 2.80-2.65 (4H, m), 2.45 (1H. m), 2.17-1.94 (5H, m) | 362 | 1.72 |
| 73 | 1T | 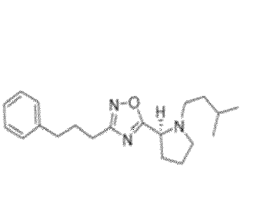 | 327.47 | (CDCl3) δ: 7.30-7.15 (5H, m), 3.86 (1H, m), 3.21 (1H, m), 2.78-2.66 (4H, m), 2.66-2.58 (1H, m), 2.50-2.18 (3H, m), 2.13-2.01 (4H, m), 1.96-1.85 (1H, m), 1.56 (1H, sep. J = 6.8 Hz), 1.41-1.25 (2H, m), 0.85 (3H, d, J = 6.8 Hz), 0.82 (3H, d, J = 6.4 Hz) | 328 | 1.39 |

**Table 18 continued**

| [Table 50] | | | | | | |
|---|---|---|---|---|---|---|
| 74 | 1U | 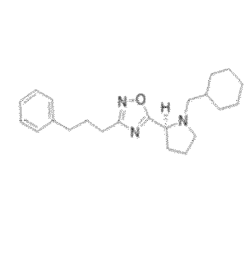 | 353.51 | (CDCl3) δ: 7.31-7.15 (5H, m), 3.85 (1H, dd. J = 8.4, 5.2 Hz), 3.20-3.14 (1H, m), 2.78-2.67 (4H, m), 2.45 (1H, q. J = 8.8 Hz), 2.35 (1H, dd, J.. 12.0, 8.4 Hz), 2.27-2.19 (2H, m), 2.13-2.01 (4H, m), 1.95-1.78 (2H, m). 1.68-1.58 (4H. m), 1.43-1.33 (1H, m), 1.23-1.04 (3H. m), 0.86-0.71 (2H. m) | 354 | 1.59 |
| 75 | 1V | 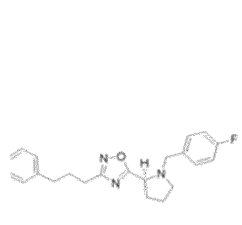 | 365.45 | (CDCl3) δ: 7.31-7.17 (8H, m), 6.98-6.91 (2H, m), 3.93 (1H, dd, J = 8.4, 5.6 Hz), 3.78 (1H, d. J - 13.2 Hz), 3.55 (1H, d, J = 13.2 Hz), 3.11-3.05 (1H, m), 2.76-2.67 (4H, m), 2.53-2.44 (1H, m), 2.33-2.23 (1H, m), 2.16-1.97 (4H. m), 1.93-1.83 (1H. m) | 366 | 1.77 |

| | | | | | | |
|---|---|---|---|---|---|---|
| Ex: Example Number Cn: Compound Number MW: Molecular Weight | | | | | | |

### Example 76

### Tert-butyl (1s,4s)-1-(3-phenethyl-1,2,4-oxadiazol-5-yl)-2-azabicyclo[2.2.2]octane-2-carboxylate 15-1

### Example 76-1: 5-((1s,4s)-2-azabicyclo[2.2.2]octan-1-yl)-3-(2-(pyridin-4-yl)ethyl)-1,2,4-oxadiazole 15-2

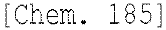

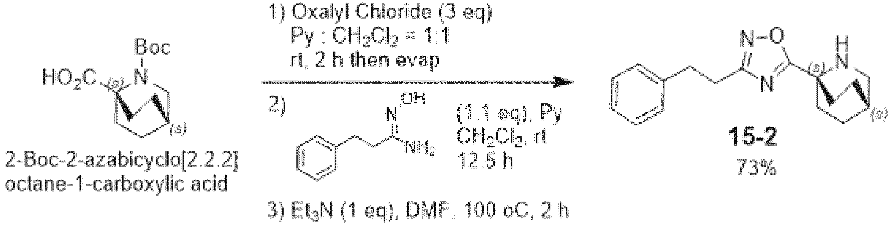

N-Boc-2-azabicyclo[2.2.2]octane-1-carboxylic acid (24.5 mg, 96 µmol) was added to a 10 ml eggplant-shaped flask and was dissolved in dichloromethane (0.480 ml) and pyridine (0.480 ml). Oxalyl chloride (25.2 pl, 0.288 mmol) was slowly added dropwise, and the mixture was then stirred for 2 hours at room temperature under a nitrogen atmosphere. After distilling off the solvent and excess reagents, azeotropic distillation with toluene was conducted once, then, dichloromethane (0.360 ml) and pyridine (387 ul, 4.80 mmol) were added thereto, and further N'-hydroxy-3-phenylpropanimidamide (17.3 mg, 0.106 mmol) was added while washing with dichloromethane (0.6 ml), followed by stirring the mixture at room temperature for 12 hours 30 minutes. After removing the stirrer bar and distilling off the solvent, water was added and washed with ethyl acetate, and the aqueous layer was concentrated to give the intermediate as hydrochloride salt. The intermediate was subjected to azeotropic distillation with toluene twice, and ultra-dehydrated DMF (1.20 ml) and triethylamine (13.3 µl, 96 µmol) were added thereto, followed by stirring the mixture at 100°C for 2 hours with a Dimroth condenser attached. The solvent was distilled off, water and saturated sodium bicarbonate aqueous solution were added, and the mixture was extracted with ethyl acetate. After distilling off the solvent, the resulting residue was purified on a silica gel column Q-pack SI30 size 10 (chloroform:methanol = 100:0 to 95:5). After distilling off the solvent, 19.8 mg (70 µmol, 73% (after 3 steps)) of the desired compound 15-2 (oil) was obtained.

### Example 76-2: tert-butyl (1s,4s)-1-(3-phenethyl-1,2,4-oxadiazol-5-yl)-2-azabicyclo[2.2.2]octane-2-carboxylate 15-1

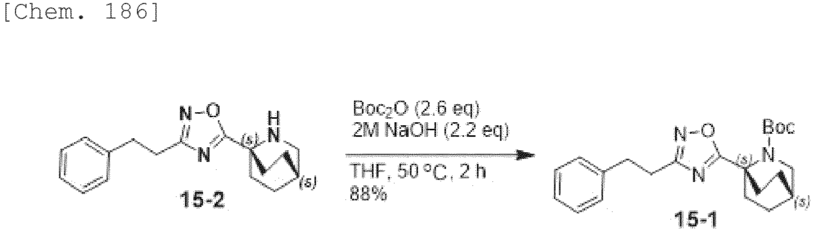

Compound 15-2 (3.2 mg, 11 µmol) prepared in Example 76-1 was added to a 10 ml eggplant-shaped flask, and THF (0.452 ml), di-t-butyl bicarbonate (6.5 mg, 29 µmol) and 2M sodium hydroxide aqueous solution (12.4 pl, 25 µmol) were added sequentially, followed by stirring the mixture at 50°C for 2 hours. Water was added thereto, the mixture was extracted with ethyl acetate, then the solvent was distilled off, and the resulting residue was purified on a silica gel column Silica HC D 5 g (hexane:ethyl acetate = 100:0 to 75:25). After distilling off the solvent, 3.7 mg (9.6 µmol, 86%) of the desired compound 15-1 (oil) was obtained.

### Example 77

### 5-((1s,4s)-2-(methylsulfonyl)-2-azabicyclo[2.2.2]octan-1-yl)-3-phenethyl-1,2,4-oxadiazole 15C

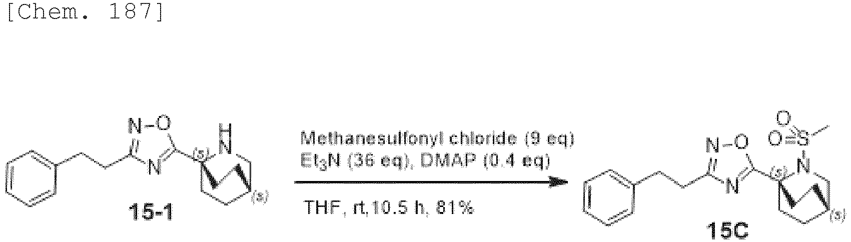

Compound 15-1 (3.8 mg, 13 µmol) prepared in Example 76-2 was added to a 10 ml eggplant-shaped flask, and ultra-dehydrated THF (0.67 ml), triethylamine (66.9 µl, 0.483 mmol) and DMAP (0.7 mg, 5 µmol) were added sequentially, and finally methanesulfonyl chloride (11.9 pl, 0.121 mmol) was added thereto, followed by stirring the mixture at room temperature for 10 hours 30 minutes. The reaction was quenched by adding methanol (20 µl), and saturated sodium bicarbonate aqueous solution was added thereto, followed by extracting the mixture with ethyl acetate. After distilling off the solvent, the residue was purified by silica gel column Silica HC D 5 g (hexane:ethyl acetate = 100:0 to 50:50). After distilling off the solvent, 3.9 mg (11 µmol, 81%) of the desired compound 15C (oil) was obtained.

Physical property data for the compounds prepared in Examples 76-77 are shown in Table 19.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acid-acetonitrile;
0-0.9 min linear gradient A:B (95:5)-A:B (10:90), 0.9-3 min A:B (10:90)

**Table 19**

| [Table 51] | | | | | | |
|---|---|---|---|---|---|---|
| Ex | Cn | Chemical Structure | MW | ¹H-NMR δ ppm | [M+H] | RT :Min |
| 76 | 15.1 | 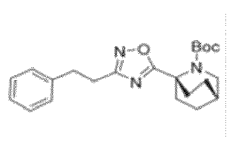 | 383.49 | (CDCl3) δ: 7.31-7.18 (5H, m), 3.62 (2H, br-s), 3.10-2.98 (4H, m), 2.49 (2H, br-s), 2.04-1.69 (7H, m), 1.50-1.12 (9H, m) | 384 | 1.89 |
| 77 | 15C | 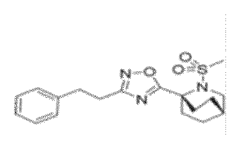 | 361.46 | (CDCl3) δ: 7.31-7.17 (5H, m), 3.65 (2H, m), 3.11-3.02 (4H, m), 2.97 (3H, s), 2.66-2.55 (2H, m), 2.16-2.10 (1H, m), 2.02-1.74 (6H, m) | 362 | 1.68 |

| | | | | | | |
|---|---|---|---|---|---|---|
| Ex: Example Number Cn: Compound Number MW: Molecular Weight | | | | | | |

### Example 78

### Tert-butyl (S)-2-(5-phenethyl-1,2,4-oxadiazol-3-yl) piperidine-1-carboxylate C2-16-1

### Example 78-1: tert-butyl (S)-2-(N'-hydroxy carbamidoyl)piperidine-1-carboxylate 16-0

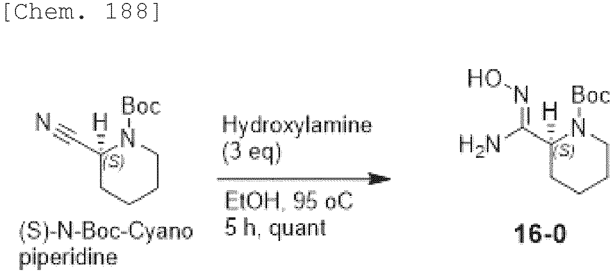

(S)-1-N-Boc-cyanopiperidine (300 mg, 1.43 mmol) and 50% hydroxylamine aqueous solution (0.252 ml, 4.28 mmol) were added to a 10 ml eggplant-shaped flask and were dissolved in anhydrous ethanol (1.9 ml), followed by heating the solution to reflux at 95°C for 5 hours. After distilling off the solvent, the product was dried in vacuo to afford the desired compound 16-0 (white solid, 347 mg, yield: 100%).

### Example 78-2: tert-butyl (S)-2-(5-phenethyl-1,2,4-oxadiazol-3-yl)piperidine-1-carboxylate C2-16-1

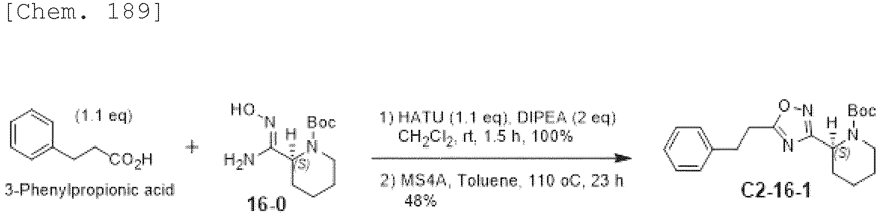

3-Phenylpropionic acid (38.8 mg, 0.253 mmol) was added to a 10 ml eggplant-shaped flask and was dissolved in dichloromethane (0.545 ml). HATU (96.3 mg, 0.253 mmol) and diisopropylethylamine (80.2 ul, 0.460 mmol) were then added thereto, and the mixture was stirred for 5 minutes at room temperature under a nitrogen atmosphere. Then, compound 16-0 (56 mg, 0.230 mmol) was added while washing with dichloromethane (1.0 ml), and the mixture was stirred at room temperature for 1 hour 30 minutes.

After removing the stirrer bar and distilling off the solvent, water was added thereto and the mixture was extracted with ethyl acetate. After distilling off the solvent, the residue was purified on a silica gel column Q-pack SI20 size 10 (hexane:ethyl acetate = 100:0 to 60:40). After distilling off the solvent, 86.4 mg (0.230 mmol, 100%) of the imidamide intermediate was obtained. The imidamide (24 mg, 64 µmol) was added with pre-dried MS4Å (120 mg), and was dissolved in ultra-dehydrated toluene (1.28 ml). The mixture was stirred at 110°C for 23 hours with a Dimroth condenser attached. After removing MS4Å by cotton plug filtration and distilling off the solvent, the resulting residue was purified on a silica gel column Q-pack SI20 size 10 (hexane:ethyl acetate = 100:0 to 80:20). After distilling off the solvent, 10.9 mg (30.5 µmol, 48%) of the desired compound C2-16-1 (oil) was obtained.

### Example 79

### Tert-butyl (S)-2-(5-(3-phenylpropyl)-1,2,4-oxadiazol-3-yl) piperidine-1-carboxylate C3-16-1

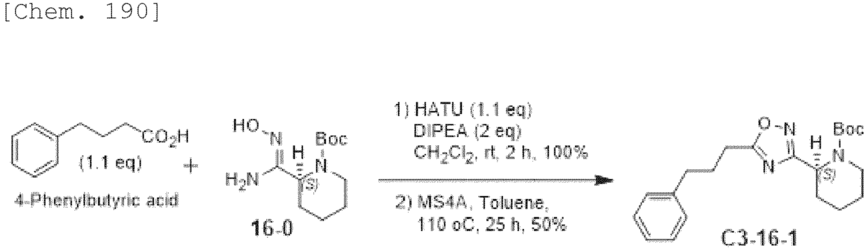

4-Phenylbutanoic acid (34.1 mg, 0.203 mmol) was added to a 10 ml eggplant-shaped flask and was dissolved in dichloromethane (0.50 ml). HATU (77.4 mg, 0.203 mmol) and diisopropylethylamine (64.4 ul, 0.370 mmol) were then added thereto, and the mixture was stirred for 5 minutes at room temperature under a nitrogen atmosphere. Compound 16-0 (45 mg, 0.185 mmol) was then added while washing with dichloromethane (0.733 ml), and the mixture was stirred at room temperature for 2 hours.

After removing the stirrer bar and distilling off the solvent, water was added and the mixture was extracted with ethyl acetate. After distilling off the solvent, the residue was purified on a silica gel column Q-pack SI20 size 10 (hexane:ethyl acetate = 100:0 to 60:40). After distilling off the solvent, 72 mg (0.185 mmol, 100%) of the imidamide intermediate was obtained. The imidamide (38.4 mg, 99 µmol) was added with with pre-dried MS4Å (192 mg), and was dissolved in ultra-dehydrated toluene (1.32 ml). The mixture was stirred at 110°C for 25 hours with a Dimroth condenser attached. After removing MS4Å by cotton plug filtration and distilling off the solvent, the resulting residue was purified on a silica gel column Q-pack SI20 size 10 (hexane:ethyl acetate = 100:0 to 80:20). After distilling off the solvent, 18.2 mg (49 µmol, 50%) of the desired compound C3-16-1 (oil) was obtained.

### Example 80

### Tert-butyl (2S,4S)-2-(3-benzyl-1,2,4-oxadiazol-5-yl)-4-phenylpyrrolidine-1-carboxylate C1-7-1

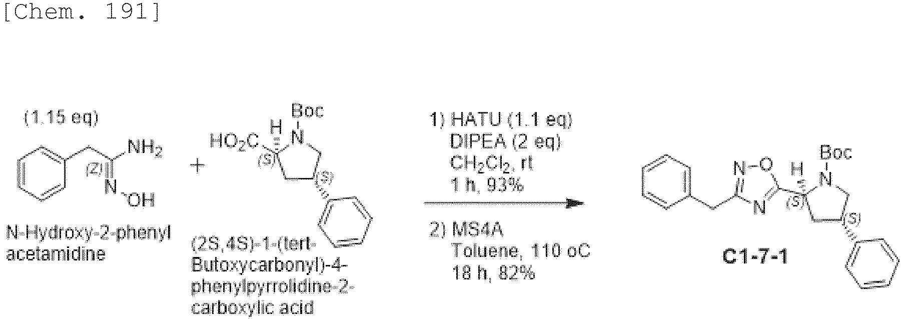

(2S,4S)-1-(tert-butoxycarbonyl)-4-phenylpyrrolidine-2-carboxylic acid (50 mg, 0.172 mmol) was added to a 10 ml eggplant-shaped flask and was dissolved in dichloromethane (0.716 ml). HATU (71.8 mg, 0.189 mmol) and diisopropyl ethylamine (59.8 pl, 0.343 mmol) were then added thereto, and the mixture was stirred for 5 minutes at room temperature under a nitrogen atmosphere. N-hydroxy-2-phenylacetamidine (29.6 mg, 0.197 mmol) was then added while washing with dichloromethane (1.0 ml), and the mixture was stirred at room temperature for 1 hour.

After removing the stirrer bar and distilling off the solvent, the residue was purified on a silica gel column Q-pack SI20 size 10 (hexane:ethyl acetate = 100:0 to 50:50). After distilling off the solvent, 67.4 mg (0.159 mmol, 93%) of the imidamide intermediate was obtained. Pre-dried MS4Å (337 mg) was added to the intermediate, which was then dissolved in ultra-dehydrated toluene (1.99 ml). The mixture was stirred at 110°C for 18 hours with a Dimroth condenser attached. After removing MS4Å by celite filtration and distilling off the solvent, the resulting residue was purified on silica gel column Q-pack SI30 size 20 (hexane:ethyl acetate = 100:0 to 80:20). After distilling off the solvent, 53 mg (0.131 mmol, 82%) of the desired compound C1-7-1 (oil) was obtained.

### Example 81

### Tert-butyl (2S,4S)-2-(3-phenethyl-1,2,4-oxadiazol-5-yl)-4-phenylpyrrolidine-1-carboxylate C2-7-1

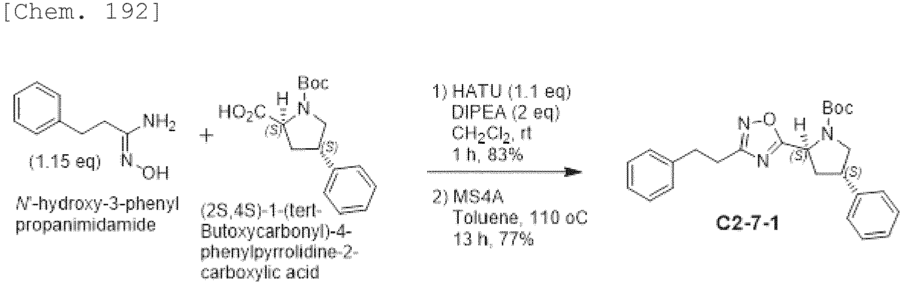

(2S,4S)-1-(tert-butoxycarbonyl)-4-phenylpyrrolidine-2-carboxylic acid (50 mg, 0.172 mmol) was added to a 10 ml eggplant-shaped flask and was dissolved in dichloromethane (0.716 ml). HATU (71.8 mg, 0.189 mmol) and diisopropyl ethylamine (59.8 pl, 0.343 mmol) were then added thereto, and the mixture was stirred for 5 minutes at room temperature under a nitrogen atmosphere. N-hydroxy-3-phenyl propanimidamide (32.4 mg, 0.197 mmol) was then added while washing with dichloromethane (1.0 ml), and the mixture was stirred at room temperature for 1 hour.

After removing the stirrer bar and distilling off the solvent, the residue was purified on a silica gel column Q-pack SI20 size 10 (hexane:ethyl acetate = 100:0 to 50:50). After distilling off the solvent, 62.6 mg (0.143 mmol, 83%) of the imidamide intermediate was obtained. Pre-dried MS4Å (312 mg) was added to the intermediate, which was then dissolved in ultra-dehydrated toluene (1.43 ml). The mixture was stirred at 110°C for 13 hours with a Dimroth condenser attached. After removing MS4Å by celite filtration and distilling off the solvent, the resulting residue was purified on silica gel column Q-pack SI30 size 20 (hexane:ethyl acetate = 100:0 to 75:25). After distilling off the solvent, 46 mg (0.110 mmol, 77%) of the desired compound C2-7-1 (oil) was obtained.

### Example 82

### Tert-butyl (2S,4S)-4-phenyl-2-(3-(4-phenylbutyl)-1,2,4-oxadiazol-5-yl)pyrrolidine-1-carboxylate C4-7-1

### Example 82-1: N'-hydroxy-5-phenylpentanimidamide

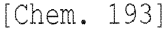

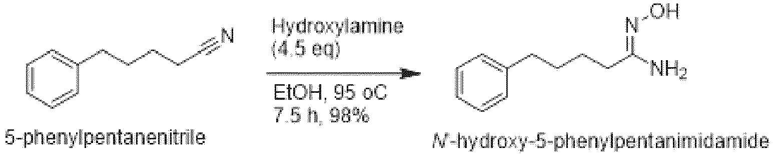

5-Phenylpentanenitrile (269 mg, 1.69 mmol) and 50% hydroxylamine aqueous solution (0.449 ml, 7.61 mmol) were added to a 10 ml eggplant-shaped flask and were dissolved in anhydrous ethanol (2.26 ml), followed by heating the solution to reflux at 95°C for 7 hours 30 minutes. After distilling off the solvent, the product was dried in vacuo to afford the desired compound, N'-hydroxy-5-phenylpentanimidamide (light black-white solid, 318 mg, yield: 98%).

### Example 82-2:

### Tert-butyl (2S,4S)-4-phenyl-2-(3-(4-phenylbutyl)-1,2,4-oxadiazol-5-yl)pyrrolidine-1-carboxylate C4-7-1

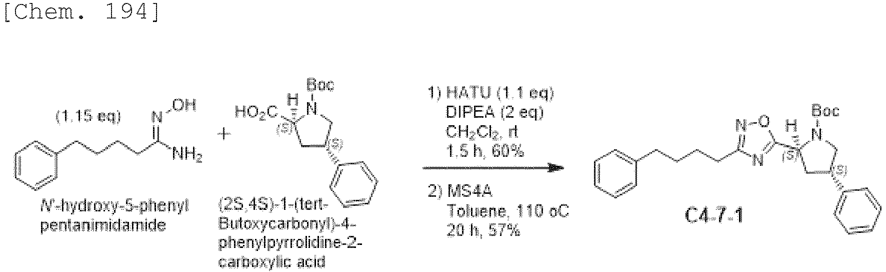

(2S,4S)-1-(tert-butoxycarbonyl)-4-phenylpyrrolidine-2-carboxylic acid (50 mg, 0.172 mmol) was added to a 10 ml eggplant-shaped flask and was dissolved in dichloromethane (0.716 ml). HATU (71.8 mg, 0.189 mmol) and diisopropyl ethylamine (59.8 pl, 0.343 mmol) were then added thereto, and the mixture was stirred for 5 minutes at room temperature under a nitrogen atmosphere. Then, N-hydroxy-5-phenylpentanimidamide (37.9 mg, 0.197 mmol) was added while washing with dichloromethane (1.0 ml), and the mixture was stirred at room temperature for 1 hour 30 minutes.

After removing the stirrer bar and distilling off the solvent, the residue was purified on a silica gel column Q-pack SI20 size 10 (hexane:ethyl acetate = 100:0 to 60:40). After distilling off the solvent, 47.9 mg (0.103 mmol, 60%) of the imidamide intermediate was obtained. Pre-dried MS4Å (240 mg) was added to the intermediate, which was then dissolved in ultra-dehydrated toluene (1.37 ml). The mixture was stirred at 110°C for 20 hours with a Dimroth condenser attached. After removing MS4Å by celite filtration and distilling off the solvent, the resulting residue was purified on silica gel column Q-pack SI30 size 20 (hexane:ethyl acetate = 100:0 to 75:25). After distilling off the solvent, 26 mg (58 µmol, 57%) of the desired compound C4-7-1 (oil) was obtained.

### Example 83

### Tert-butyl (2S,4S)-4-phenyl-2-(3-(5-phenylpentyl)-1,2,4-oxadiazol-5-yl)pyrrolidine-1-carboxylate C5-7-1

### Example 83-1: N'-hydroxy-6-phenylhexaneimidamide

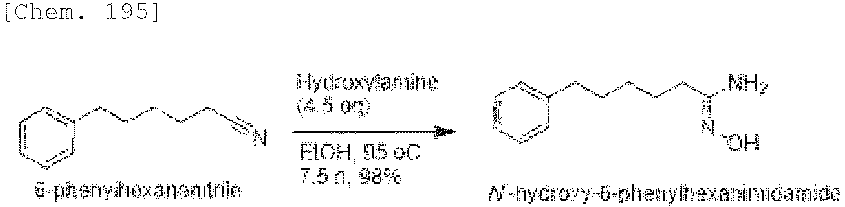

6-Phenylhexanenonitrile (783 mg, 4.52 mmol) and 50% hydroxylamine aqueous solution (1.20 ml, 20.3 mmol) were added to a 10 ml eggplant-shaped flask, and were dissolved in anhydrous ethanol (5.65 ml), followed by heating to reflux at 95°C for 7 hours 30 minutes. After distilling off the solvent, the product was dried in vacuo to afford the desired compound, N'-hydroxy-6-phenylhexaneimidamide (light black-white solid, 918 mg, yield: 98%).

### Example 83-2: tert-butyl (2S,4S)-4-phenyl-2-(3-(5-phenyl pentyl)-1,2,4-oxadiazol-5-yl)pyrrolidine-1-carboxylate C5-7-1

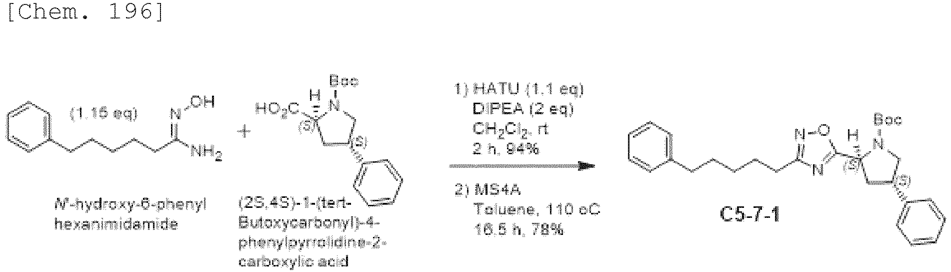

(2S,4S)-1-(tert-butoxycarbonyl)-4-phenylpyrrolidine-2-carboxylic acid (50 mg, 0.172 mmol) was added to a 10 ml eggplant-shaped flask and was dissolved in dichloromethane (0.716 ml). HATU (71.8 mg, 0.189 mmol) and diisopropyl ethylamine (59.8 pl, 0.343 mmol) were then added thereto, and the mixture was stirred for 5 minutes at room temperature under a nitrogen atmosphere. Then, N-hydroxy-6-phenylhexaneimidamide (40.7 mg, 0.197 mmol) was added while washing with dichloromethane (1.0 ml), and the mixture was stirred at room temperature for 2 hours.

After removing the stirrer bar and distilling off the solvent, the residue was purified on a silica gel column Q-pack SI20 size 10 (hexane:ethyl acetate = 100:0 to 60:40). After distilling off the solvent, 77.4 mg (0.161 mmol, 94%) of the imidamide intermediate was obtained. Pre-dried MS4Å (387 mg) was added to the intermediate, which was then dissolved in ultra-dehydrated toluene (1.61 ml). The mixture was stirred at 110°C for 16 hours and 30 minutes with a Dimroth condenser attached. After removing MS4Å by celite filtration and distilling off the solvent, the resulting residue was purified on silica gel column Q-pack SI20 size 10 (hexane:ethyl acetate = 100:0 to 80:20). After distilling off the solvent, 58.3 mg (0.126 mmol, 78%) of the desired compound C5-7-1 (oil) was obtained.

The physical property data for the compounds prepared in Examples 78-83 are shown in Table 20.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acid-acetonitrile;
0-0.9 min linear gradient A:B (95:5)-A:B (10:90), 0.9-3 min A:B (10:90)

**Table 20**

| [Table 52] | | | | | | |
|---|---|---|---|---|---|---|
| Ex | Cn | Chemical Structure | MW | ¹H-NMR δ ppm | [M+H] | RT :Min |
| 78 | C2-16-1 | 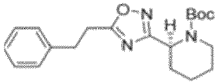 | 357.45 | (CDCl3) δ: 7.32-7.18 (5H, m), 5.46 (1H, br.s), 4.03 (1H, br-d, J = 12.8 Hz), 3.20-3.00 (4H, m), 2.98-2.90 (1H. m), 2.24 (1H, br-d, J = 13.2 Hz), 1.90-1.81 (1H, m), 1.70-1.26 (4H, m), 1.46 (9H, s) | 358 | 1.89 |
| 79 | C3-16-1 | 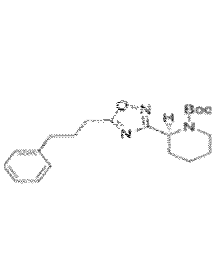 | 371.48 | (CDCl3) δ: 7.31-7.16 (5H, m). 5.47 (1H, br-s), 4.04 (1H, br-d, J = 12.0 Hz), 2.98 (1H, m), 2.87 C2H, t. J = 7.6 Hz), 2.72 (2H, t, J = 7.6 Hz). 2.28-2.22 (1H, m). 2.14 (2H, quin, J = 7.6 Hz), 1.90-1.80 (1H, m), 1.70-1.27 (4H, m), 1.46 (9H, s) | 372 | 1.97 |
| 80 | C1-7-1 | 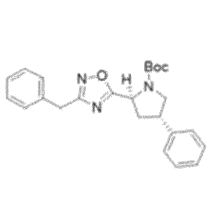 | 405.50 | (CDCl3) δ: 7.36-7.21 (10H, m), 5.30 (0.4H. d. J = 8.4 Hz). 5.14 (0.6H. d, J = 6.8 Hz), 4.15-4.00 (3H, m), 3.67 (1H, m), 3.53-3.38 (1H, m), 2.53-2.36 (2H. m), 1.45 (3.6H, s), 1.21 (5.4H. s) | 406 | 1.88 |
| 81 | C2-7-1 | 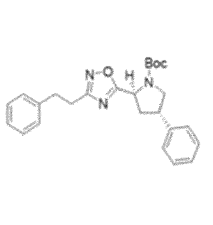 | 419.53 | (CDCl3) δ: 7.38-7.19 (10H, m), 5.32 (0.4H. d. J = 8.0 Hz), 5.18 (0.6H, d, J = 8.0 Hz), 4.17-4.03 (1H, m), 3.69 (1H, m), 3.56-3.40 (1H. m), 3.12-3.02 (4H, m), 2.56-2.35 (2H, m), 1.47 (3.6H. s), 1.32 (5.4H, s) | 420 | 1.95 |
| 82 | C4-7-1 | 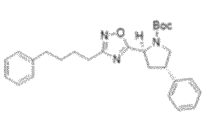 | 447.58 | (CDCl3) δ: 7.35-7.12 (10H. m), 5.31 (0.4H, d, J = 8.4 Hz), 5.16 (0.6H, d, J = 6.8 Hz), 4.16-4.02 (1H, m), 3.69 (1H, | 448 | 2.08 |

**Table 20 continued**

| [Table 53] | | | | | | |
|---|---|---|---|---|---|---|
| | | | | m), 3.54-3.39 (1H, m), 2.77 (2H, t, J = 7.2 Hz), 2.66 (2H. t, J = 7.2 Hz), 2.55-2.35 (2H, m), 1.86-1.67 (4H, m), 1.46 (3.6H, s), 1.32 (5.4H, s) | | |
| 83 | C5-7-1 | 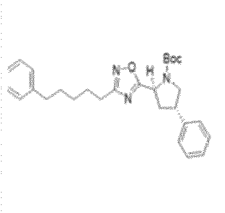 | 461.61 | (CDCl3) δ: 7.36-7.11 (10H, m), 5.31 (0.45H, d, J = 8.0 Hz), 5.16 (0.55H, d. J = 7.2 Hz), 4.16-4.02 (1H, m), 3.69 (1H, m), 3.55-3.40 (1H, m), 2.74 (2H, t, J = 7.2 Hz). 2.62 (2H, t, J = 7.6 Hz), 2.55-2.35 (2H, m), 1.79 (2H, quin, J = 7.6 Hz). 1.67 (2H. m), 1.46 (4H, s), 1.48-1.39 (2H, m), 1.33 (5H. s) | 462 | 2.19 |

| | | | | | | |
|---|---|---|---|---|---|---|
| Ex: Example Number Cn: Compound Number MW: Molecular Weight | | | | | | |

### Example 84

### Tert-butyl (S)-2-(5-phenethyl-1,3,4-oxadiazol-2-yl) piperidine-1-carboxylate 17-1

### Example 84-1: tert-butyl (S)-2-(hydrazinecarbonyl) piperidine-1-carboxylate 17-0

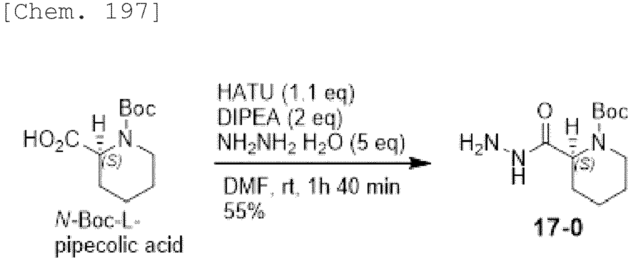

N-Boc-L-pipecolinic acid (100 mg, 0.436 mmol) was added to a 10 ml eggplant-shaped flask and was dissolved in DMF (0.5 ml). HATU (182 mg, 0.480 mmol) and diisopropyl ethylamine (0.152 ml, 0.872 mmol) were then added thereto, and the mixture was stirred for 10 minutes at room temperature under a nitrogen atmosphere. This solution was then added dropwise to a 10 ml eggplant-shaped flask containing hydrazine-mono-hydrate (108 ul, 2.18 mmol) dissolved in DMF (0.5 ml), while washing with DMF (0.744 ml), and the mixture was stirred at room temperature for 1 hour 40 minutes. The reaction was stopped with saturated sodium bicarbonate aqueous solution and the mixture was extracted with ethyl acetate. After distilling off the solvent, the residue was purified on a silica gel column Q-pack SI30 size 10 (chloroform:methanol = 100:0 to 95:5). After distilling off the solvent, 58.7 mg (0.241 mmol, 55%) of the desired compound 17-0 (white solid) was obtained.

### Example 84-2: tert-butyl (S)-2-(5-phenethyl-1,3,4-oxadiazol-2-yl)piperidine-1-carboxylate 17-1

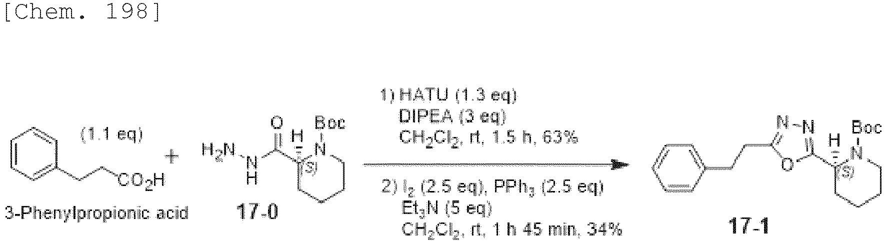

Compound 17-0 (18.7 mg, 77 µmol) prepared in Example 84-1 and 3-phenylpropanoic acid (13 mg, 85 µmol) were added to a 10 ml eggplant-shaped flask and were dissolved in dichloromethane (0.77 ml). HATU (38 mg, 0.100 mmol) and diisopropylethylamine (40.2 pl, 0.231 mmol) were then added thereto, and the mixture was stirred at room temperature under a nitrogen atmosphere for 1 hour 30 minutes.

After removing the stirrer bar and distilling off the solvent, saturated sodium bicarbonate aqueous solution was added thereto and the mixture was extracted with ethyl acetate. After distilling off the solvent, the residue was purified on a silica gel column Q-pack SI20 size 10 (chloroform:methanol = 100:0 to 95:5). After distilling off the solvent, 19.9 mg (53 µmol, 63%) of the intermediate was obtained, which was a condensed product (amorphous solid). Triphenylphosphine (21.7 mg, 79 µmol) and iodine (20.3 mg, 79 µmol) were then added to a pre-dried 10 ml eggplant-shaped flask and were dissolved in dichloromethane (0.448 ml). Triethylamine (21.8 pl, 0.157 mmol) was then slowly added dropwise, and then the condensed product (11.8 mg, 31 µmol) was added slowly while washing with dichloromethane (0.60 ml), followed by stirring the mixture at room temperature for 1 hour 45 minutes. The solvent was distilled off and the resulting residue was purified on a silica gel column Q-pack SI20 size 10 (hexane:ethyl acetate = 100:0 to 66:34). After distilling off the solvent, 3.8 mg (10.6 µmol, 34%) of the desired compound 17-1 (oil) was obtained.

### Example 85

### Tert-butyl (S)-2-(5-(3-phenylpropyl)-1,3,4-oxadiazol-2-yl) piperidine-1-carboxylate 18-1

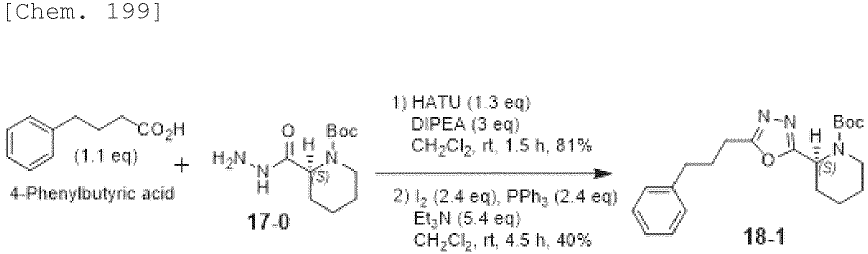

Compound 17-0 (26 mg, 0.107 mmol) prepared in Example 84-1 and 4-phenylbutanoic acid (19.7 mg, 0.118 mmol) were added to a 10 ml eggplant-shaped flask and were dissolved in dichloromethane (1.07 ml). HATU (52.8 mg, 0.139 mmol) and diisopropylethylamine (55.8 ul, 0.321 mmol) were then added thereto, and the mixture was stirred at room temperature under a nitrogen atmosphere for 1 hour 30 minutes.

After removing the stirrer bar and distilling off the solvent, saturated sodium bicarbonate aqueous solution was added thereto and the mixture was extracted with ethyl acetate. After distilling off the solvent, the residue was purified on a silica gel column Q-pack SI20 size 10 (hexane:ethyl acetate = 100:0 to 50:50). After distilling off the solvent, 36.8 mg (94.5 µmol, 81%) of the intermediate was obtained, which was a condensed product (amorphous solid). Triphenylphosphine (48.1 mg, 0.174 mmol) and iodine (45.2 mg, 0.174 mmol) were then added to a pre-dried 10 ml eggplant-shaped flask and were dissolved in dichloromethane (0.65 ml). Triethylamine (54.4 ul, 0.392 mmol) was then slowly added dropwise, and the condensed product (28.3 mg, 73 µmol) was added while washing with dichloromethane (0.80 ml), followed by stirring the mixture at room temperature for 4 hours 30 minutes. The solvent was distilled off and the resulting residue was purified on a silica gel column Q-pack SI20 size 10 (hexane:ethyl acetate = 100:0 to 66:34). After distilling off the solvent, 10.7 mg (28.86 µmol, 40%) of the desired compound 18-1 (oil) was obtained.

### Example 86

### Tert-butyl (2S,4S)-4-phenyl-2-(5-(3-phenylpropyl)-4H-1,2,4-triazol-3-yl)pyrrolidine-1-carboxylate 19-1

### Example 86-1: tert-Butyl (2S,4S)-2-(hydrazinecarbonyl)-4-phenylpyrrolidine-1-carboxylate 19-0

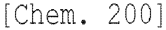

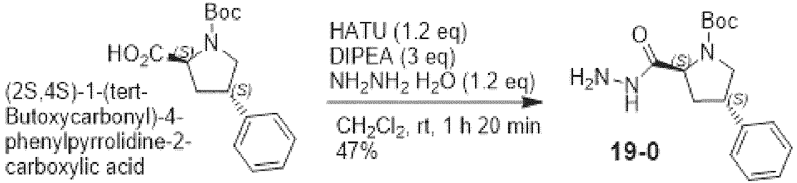

(2S,4S)-1-(tert-butoxycarbonyl)-4-phenylpyrrolidine-2-carboxylic acid (50 mg, 0.172 mmol) was added to a 10 ml eggplant-shaped flask and was dissolved in dichloromethane (1.216 ml). HATU (78.3 mg, 0.206 mmol) and diisopropyl ethylamine (89.7 ul, 0.515 mmol) were then added thereto, and the mixture was stirred for 10 minutes at room temperature under a nitrogen atmosphere. A solution of hydrazine-mono-hydrate (10.2 pl, 0.206 mmol) in dichloromethane (0.5 ml) was then added thereto, and the mixture was stirred at room temperature for 1 hour 20 minutes. After removing the stirrer bar and distilling off the solvent, the reaction was stopped with saturated sodium bicarbonate aqueous solution and the mixture was extracted with ethyl acetate. After distilling off the solvent, the residue was purified on a silica gel column Q-pack SI20 size 10 (chloroform:methanol = 100:0 to 95:5). After distilling off the solvent, 24.8 mg (81 µmol, 47%) of the desired compound 19-0 (amorphous solid) was obtained.

### Example 86-2: Ethyl 4-phenylbutanimidate

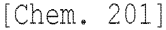

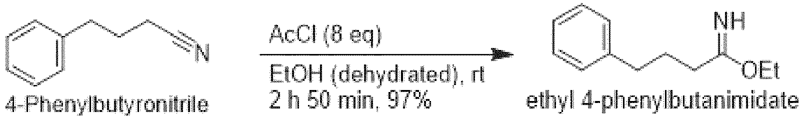

4-Phenylbutyronitrile (0.740 ml, 5.0 mmol) was added to an eggplant-shaped flask and was dissolved in anhydrous ethanol (3.47 ml), to which acetyl chloride (2.82 ml, 40 mmol) was added slowly dropwise while cooling with cold water. The mixture was then stirred at room temperature for 2 hours 50 minutes, the solvent was distilled off, and the mixture was dried in vacuo to afford the title compound (oil, 928 mg, yield: 97%).

### Example 86-3: tert-butyl (2S,4S)-4-phenyl-2-(5-(3-phenyl propyl)-4H-1,2,4-triazol-3-yl)pyrrolidine-1-carboxylate 19-1

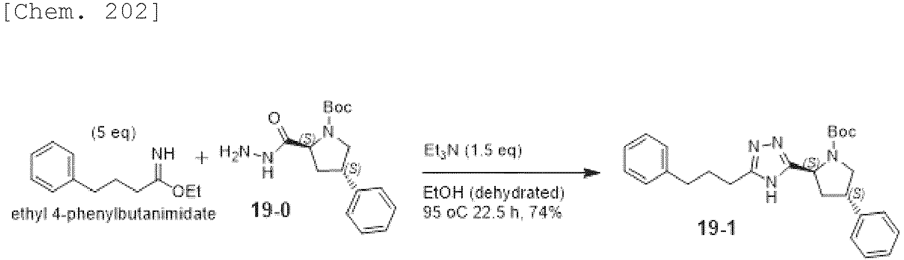

Compound 19-0 (6.6 mg, 22 µmol) prepared in Example 86-1, ethyl-4-phenylbutanimidate (21.1 mg, 0.108 mmol) prepared in Example 86-2 and triethylamine (4.5 pl, 32.4 µmol) were added to a 10 ml eggplant-shaped flask, and were dissolved in ultra-dehydrated ethanol (1.08 ml), followed by stirring the mixture at 95°C for 22 hours 30 minutes under a nitrogen atmosphere.

After removing the stirrer bar and distilling off the solvent, the residue was purified on a silica gel column Q-pack SI20 size 10 (chloroform:methanol = 98:2) . After distilling off the solvent, 6.9 mg (16 µmol, 74%) of the desired compound 19-1 (amorphous solid) was obtained.

### Example 87

### Tert-butyl (S)-2-(5-(3-phenylpropyl)-4H-1,2,4-triazol-3-yl) piperidine-1-carboxylate 20-1

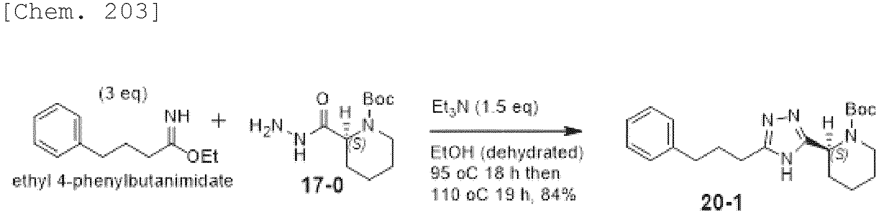

Compound 17-0 (25.3 mg, 0.104 mmol) prepared in Example 84-1, ethyl-4-phenylbutanimidate (60.9 mg, 0.312 mmol) prepared in Example 86-2 and triethylamine (21.6 pl, 0.156 mmol) were added to a 10 ml eggplant-shaped flask, and were dissolved in ultra-dehydrated ethanol (1.30 ml), followed by stirring the mixture at 95°C for 18 hours and at 110°C for 19 hours under a nitrogen atmosphere.

After removing the stirrer bar and distilling off the solvent, the residue was purified on a silica gel column Q-pack SI30 size 10 (chloroform:methanol = 95:5). After distilling off the solvent, 32.3 mg (87 µmol, 84%) of the desired compound 20-1 (amorphous solid) was obtained.

### Example 88

### 5-((2S)-1-(tert-butylsulfinyl)piperidin-2-yl)-3-(3-phenyl propyl)-1,2,4-oxadiazole 2W

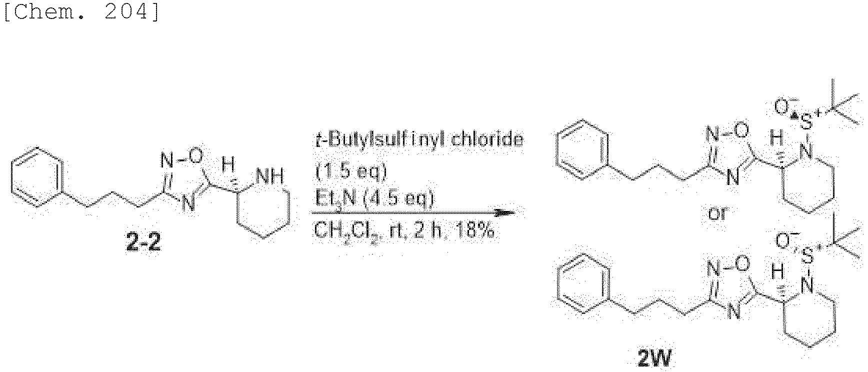

Compound 2-2 (19.6 mg, 72 µmol) prepared in Example 9-2, CH₂Cl₂ (0.722 ml) and triethylamine (45.1 µl, 0.325 mmol) were added sequentially to a 10 ml eggplant-shaped flask, and finally t-butylsulfinyl chloride (18.8 µl, 0.108 mmol) was added slowly, followed by stirring the mixture at room temperature for 2 hours. The reaction was quenched by adding saturated sodium bicarbonate aqueous solution and the mixture was extracted with ethyl acetate. After distilling off the solvent, the residue was purified on a silica gel column Q-pack SI30 size 10 (hexane:ethyl acetate = 100:0 to 66:34). The more polar diastereomer was harvested and the solvent was distilled off to afford 4.9 mg (13 µmol, 18%) of the desired compound 2W (oil). It is noted that the stereochemistry of the sulfoxide in compound 2W has not been determined.

### Example 89

### (S) -5-(1-(tert-butylsulfonyl)piperidin-2-yl)-3-(3-phenyl propyl)-1,2,4-oxadiazole 2X

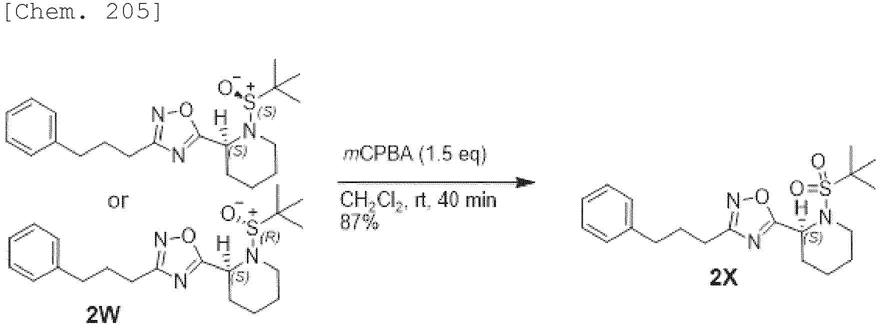

To a 10 ml eggplant-shaped flask, compound 2W (11.8 mg, 31 µmol) prepared in Example 88 was added, which was dissolved in CH₂Cl₂ (1.05 ml), and 65% m-chloroperbenzoic acid (10.8 mg, 41 µmol) was added slowly, followed by stirring the mixture at room temperature for 40 minutes. The reaction was quenched by adding saturated sodium bicarbonate aqueous solution and saturated sodium sulfite solution and the mixture was extracted with ethyl acetate. After distilling off the solvent, the residue was purified on a silica gel column Q-pack SI20 size 10 (hexane:ethyl acetate = 100:0 to 80:20). After distilling off the solvent, 12.9 mg (33 µmol, 87%) of the desired compound 2X (oil) was obtained.

### Example 90

### (S) -N-((5-(1-(cyclohexylsulfonyl)piperidin-2-yl)-1,2,4-oxadiazol-3-yl)methyl)benzo[d][1,3]dioxol-5-carboxamide 21B Example 90-1: tert-butyl (S)-((5-(piperidin-2-yl)-1,2,4-oxadiazol-3-yl)methyl)carbamate 21-2

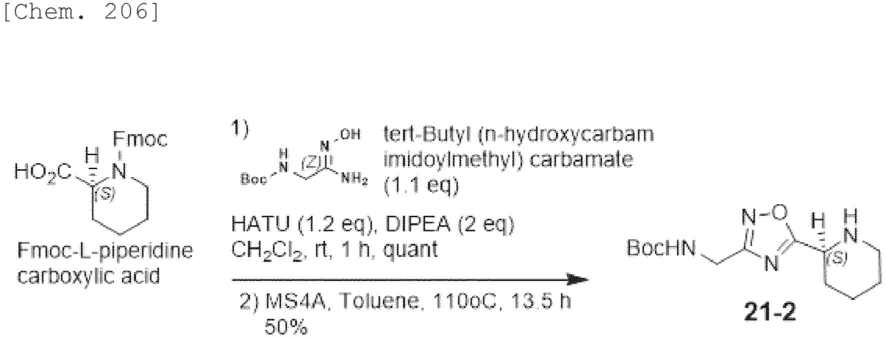

N-Fmoc-L-piperidine carboxylic acid (200 mg, 0.569 mmol) was added to a 25 ml eggplant-shaped flask and was dissolved in dichloromethane (2.29 ml). HATU (260 mg, 0.683 mmol) and diisopropylethylamine (0.198 ml, 1.14 mmol) were then added thereto, and the mixture was stirred for 7 minutes at room temperature under a nitrogen atmosphere. Then, tert-butyl (n-hydroxycarbamidoylmethyl) carbamate (123 mg, 0.626 mmol) was added while washing with dichloromethane (1.5 ml), and the mixture was stirred at room temperature for 1 hour. The solvent was distilled off and dried in vacuo, and after distilling off the solvent, the residue was purified on a silica gel column Q-pack SI30 size 20 (hexane:ethyl acetate = 100:0 to 50:50). After distilling off the solvent, 340 mg of the intermediate, which was a condensed product, was obtained as a mixture with tetramethylurea. To the intermediate, molecular sieves 4Å (1.49 g) and ultra-dehydrated toluene (3.79 ml) were added, and the mixture was stirred at 110°C for 13 hours 30 minutes. After removing the solids by celite filtration and distilling off the solvent, the residue was purified on a silica gel column Q-pack SI30 size 20 (chloroform:methanol = 100:0 to 95:5). After distilling off the solvent, 79.9 mg (0.283 mmol, 50%) of the desired compound 21-2 (oil) was obtained.

### Example 90-2: tert-butyl (S)-((5-(1-(cyclohexylsulfonyl) piperidin-2-yl)-1,2,4-oxadiazol-3-yl)methyl)carbamate 21-3

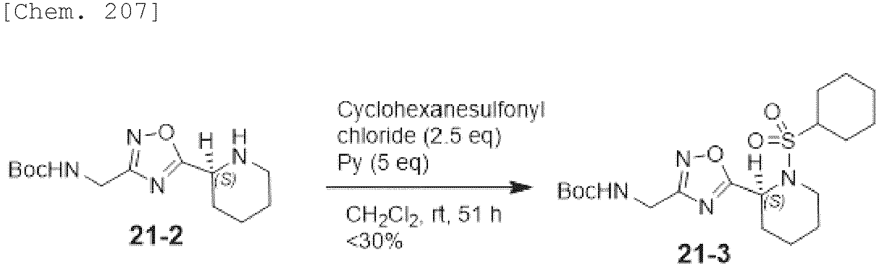

Compound 21-2 (40.3 mg, 0.143 mmol) prepared in Example 90-1, CH₂Cl₂ (0.827 ml) and pyridine (57.6 ul, 0.714 mmol) were added sequentially to a 10 ml eggplant-shaped flask, and the mixture was ice-cooled, which was added finally with cyclohexanesulfonyl chloride (54.5 mg, 0.357 mmol) while washing with CH₂Cl₂ (0.6 ml), followed by stirring the mixture at room temperature for 51 hours. After distilling off the solvent, the residue was purified on a silica gel column Q-pack SI20 size 10 (hexane:ethyl acetate = 100:0 to 60:40). After distilling off the solvent, 18.4 mg (43 µmol, 30%) of the desired compound 21-3 (oil) was obtained. Example 90-3: (S)-N-((5-(1-(cyclohexylsulfonyl)piperidin-2-yl)-1,2,4-oxadiazol-3-yl)methyl)benzo[d] [1,3]dioxol-5-carboxamide 21B

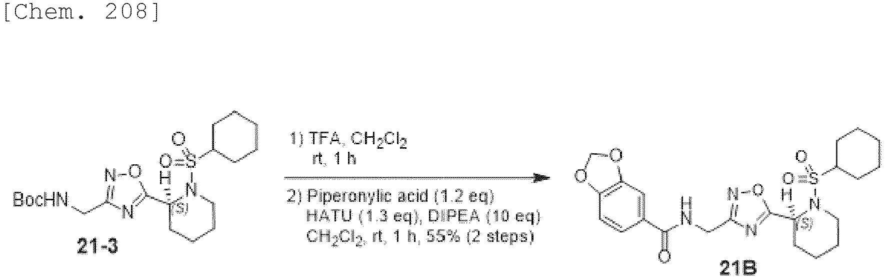

Compound 21-3 (9.0 mg, 21 µmol) prepared in Example 90-2 was added to a 10 ml eggplant-shaped flask, and dichloromethane (0.42 ml) and TFA (84 µl) were added thereto, followed by stirring the mixture at room temperature for 1 hour. After removing the stirrer bar and distilling off the solvent, saturated sodium bicarbonate aqueous solution was then added thereto and the mixture was extracted with ethyl acetate. The solution was dried over magnesium sulfate, which was then filtered out, and the solvent was distilled off to afford 5.7 mg (17.4 µmol) of the amine-free intermediate. Then, dichloromethane (0.70 ml), HATU (10.4 mg, 27 µmol) and piperonyl acid (4.3 mg, 26 µmol) were added sequentially, and finally diisopropylethylamine (36.6 pl, 0.21 mmol) was added thereto, followed by stirring the mixture at room temperature for 1 hour. After the solvent was distilled off, saturated sodium bicarbonate aqueous solution was added and the mixture was extracted with ethyl acetate. The organic layer was washed with saturated brine, the solvent was distilled off, and the residue was purified on a silica gel column Q-pack SI20 size 10 (hexane:ethyl acetate = 100:0 to 60:40). After distilling off the solvent, 5.5 mg (11.5 µmol, 55% (after 2 steps)) of the desired compound 21B (white amorphous solid) was obtained.

### Example 91

### (S) -3-Methyl-1-(2-(3-(3-phenylpropyl)-1,2,4-oxadiazol-5-yl) piperidin-1-yl)butan-1-one 2Q

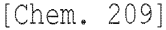

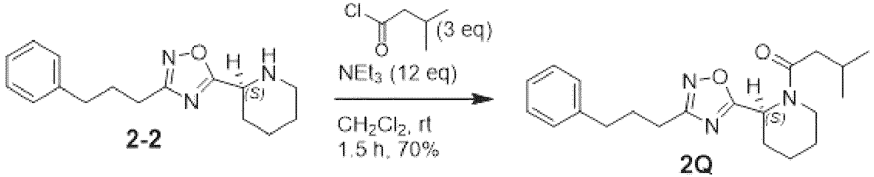

To a 4 ml vial, compound 2-2 (5.4 mg, 19.9 µmol) prepared in Example 9-2, CH₂Cl₂ (0.398 ml) and triethylamine (33.1 pl, 0.239 mmol) were added sequentially, and finally isovaleryl chloride (7.34 ul, 59.7 µmol) was added thereto, followed by stirring the mixture at room temperature for 1 hour 30 minutes. The reaction was quenched by adding methanol (20 pl), and saturated sodium bicarbonate aqueous solution was added thereto, followed by extracting the mixture with ethyl acetate. After distilling off the solvent, the residue was purified on a silica gel column Q-pack SI20 size 10 (hexane:ethyl acetate = 100:0 to 50:50). After distilling off the solvent, 5.0 mg (14 µmol, 70%) of the desired compound 2Q (oil) was obtained.

### Example 92

### (S) -1-(2-(3-(3-(3-phenylpropyl)-1,2,4-oxadiazol-5-yl) piperidin-1-yl)ethan-1-one 2R

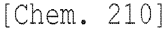

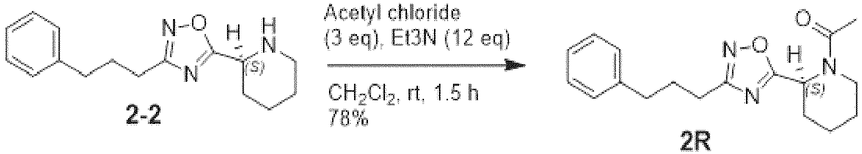

Compound 2-2 (5.2 mg, 19 µmol) prepared in Example 9-2 was added to a 4 ml vial, and dichloromethane (0.383 ml), triethylamine (31.9 µl, 0.230 mmol) and acetyl chloride (4.15 pl, 57.5 µmol) were added sequentially, followed by stirring the mixture at room temperature for 1 hour 30 minutes. The reaction was quenched by adding methanol (20 pl), and saturated sodium bicarbonate aqueous solution was added thereto, followed by extracting the mixture with ethyl acetate. After distilling off the solvent, the residue was purified on a silica gel column Q-pack SI20 size 10 (hexane:ethyl acetate = 100:0 to 40:60). After distilling off the solvent, 4.7 mg (15 µmol, 78%) of the desired compound 2R (oil) was obtained.

### Example 93

### (S) -Phenyl(2-(3-(3-phenylpropyl)-1,2,4-oxadiazol-5-yl) piperidin-1-yl)methanone 2S

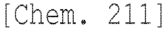

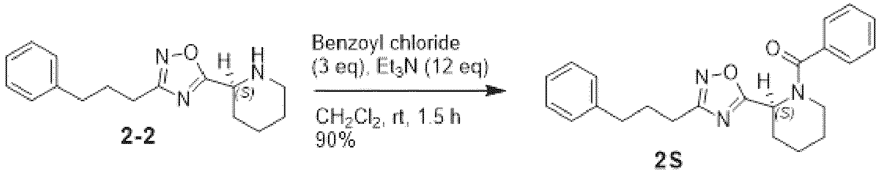

Compound 2-2 (5.2 mg, 19 µmol) prepared in Example 9-2 was added to a 4 ml vial, and dichloromethane (0.383 ml), triethylamine (31.9 pl, 0.230 mmol) and benzoyl chloride (6.8 ul, 57.5 µmol) were added sequentially, followed by stirring the mixture at room temperature for 1 hour 30 minutes. The reaction was quenched by adding methanol (20 pl), and saturated sodium bicarbonate aqueous solution was added thereto, followed by extracting the mixture with ethyl acetate. After distilling off the solvent, the residue was purified on a silica gel column Q-pack SI20 size 10 (hexane:ethyl acetate = 100:0 to 66:34). After distilling off the solvent, 6.5 mg (17.3 µmol, 71%) of the desired compound 2S (oil) was obtained.

### Example 94

### (S) -2-Phenyl-1-(2-(3-(3-phenylpropyl)-1,2,4-oxadiazol-5-yl)piperidin-1-yl)ethan-1-one 2P

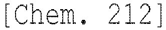

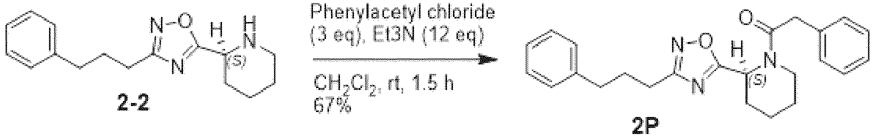

Compound 2-2 (5.0 mg, 18.4 µmol) prepared in Example 9-2 was added to a 4 ml vial, and dichloromethane (0.369 ml), triethylamine (30.6 pl, 0.221 mmol) and phenylacetyl chloride (7.7 pl, 55 µmol) were added sequentially, followd by stirring the mixture at room temperature for 1 hour 30 minutes. The reaction was quenched by adding methanol (20 pl), and saturated sodium bicarbonate aqueous solution was added thereto, followed by extracting the mixture with ethyl acetate. After distilling off the solvent, the residue was purified on a silica gel column Q-pack SI20 size 10 (hexane:ethyl acetate = 100:0 to 60:40). After distilling off the solvent, 4.8 mg (12.3 µmol, 67%) of the desired compound 2P (oil) was obtained.

### Example 95

### (S) -cyclohexyl(2-(3-(3-phenylpropyl)-1,2,4-oxadiazol-5-yl) piperidin-1-yl)methanone 2Y

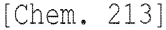

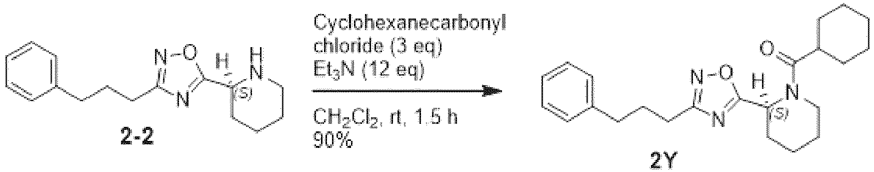

Compound 2-2 (7.0 mg, 25.8 µmol) prepared in Example 9-2 was added to a 4 ml vial, and dichloromethane (0.516 ml), triethylamine (42.9 pl, 0.310 mmol) and cyclohexanecarbonyl chloride (10.7 µl, 77.4 µmol) were added sequentially, followed by stirring the mixture at room temperature for 1 hour 30 minutes. The reaction was quenched by adding methanol (20 µl), and saturated sodium bicarbonate aqueous solution was added thereto, followed by extracting the mixture with ethyl acetate. After distilling off the solvent, the residue was purified on a silica gel column Q-pack SI20 size 10 (hexane:ethyl acetate = 100:0 to 75:25). After distilling off the solvent, 9.1 mg (23.9 µmol, 93%) of the desired compound 2Y (oil) was obtained.

### Example 96

### (S) -5-(1-isopentylpiperidin-2-yl)-3-(3-phenylpropyl)-1,2,4-oxadiazole 2T

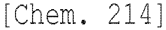

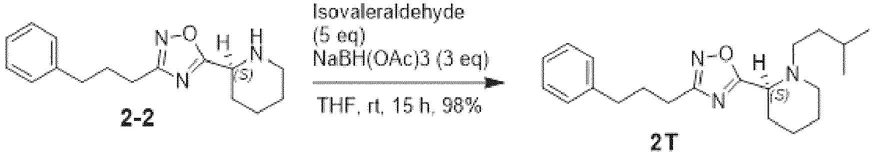

To compound 2-2 (6.8 mg, 25 µmol) prepared in Example 9-2 in a 4 ml vial, isovalerylaldehyde (13.8 pl, 0.125 mmol) was added while washing with THF (0.33 ml). After stirring at room temperature for 10 minutes, the mixture was added with sodium triacetoxybolohydride (15.9 mg, 75 µmol) and the mixture was stirred at room temperature for 15 hours. The reaction was quenched by adding saturated sodium bicarbonate aqueous solution and the mixture was extracted with ethyl acetate. After distilling off the solvent, the residue was purified on a silica gel column Q-pack SI30 size 10 (hexane:ethyl acetate = 100:0 to 90:10). After distilling off the solvent, 8.4 mg (24.6 µmol, 98%) of the desired compound 2T (oil) was obtained.

### Example 97

### (S) -5-(1-ethylpiperidin-2-yl)-3-(3-phenylpropyl)-1,2,4-oxadiazole 2Z

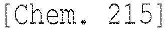

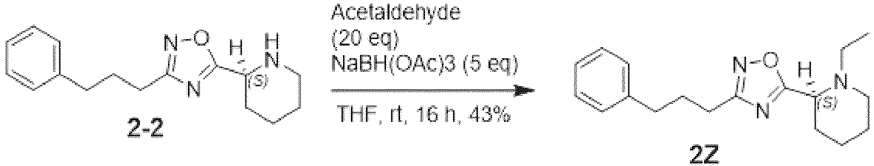

To compound 2-2 (6.7 mg, 25 µmol) prepared in Example 9-2 in a 4 ml vial, acetaldehyde (28 pl, 0.494 mmol) and THF (0.33 ml) were added, and the mixture was stirred at room temperature for 10 minutes. Then, sodium triacetoxybolohydride (26.2 mg, 0.123 mmol) was added thereto, and the mixture was stirred at room temperature for 16 hours. The reaction was quenched by adding saturated sodium bicarbonate aqueous solution and the mixture was extracted with ethyl acetate. After distilling off the solvent, the residue was purified on a silica gel column Q-pack SI30 size 10 (hexane:ethyl acetate = 100:0 to 60:40). After distilling off the solvent, 3.2 mg (24 µmol, 43%) of the desired compound 2Z (oil) was obtained.

### Example 98

### (S) -5-(1-benzylpiperidin-2-yl)-3-(3-phenylpropyl)-1,2,4-oxadiazole 2AA

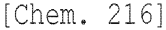

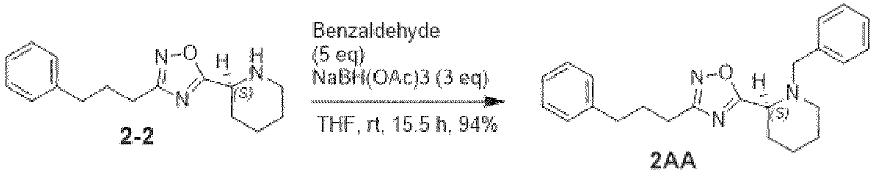

To compound 2-2 (6.8 mg, 25 µmol) prepared in Example 9-2 in a 4 ml vial, benzaldehyde (12.9 pl, 0.125 mmol) and THF (0.33 ml) were added, and the mixture was stirred at room temperature for 10 minutes. Then, sodium triacetoxybolohydride (15.9 mg, 75 µmol) was added thereto, and the mixture was stirred at room temperature for 15 hours 30 minutes. The reaction was quenched by adding saturated sodium bicarbonate aqueous solution and the mixture was extracted with ethyl acetate. After distilling off the solvent, the residue was purified by NH column DNH-30 size 10 (hexane:ethyl acetate = 100:0 to 90:10). After distilling off the solvent, 8.6 mg (23.8 µmol, 94%) of the desired compound 2AA (oil) was obtained.

### Example 99

### (S) -5-(1-phenethylpiperidin-2-yl)-3-(3-phenylpropyl)-1,2,4-oxadiazole 2AB

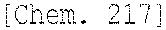

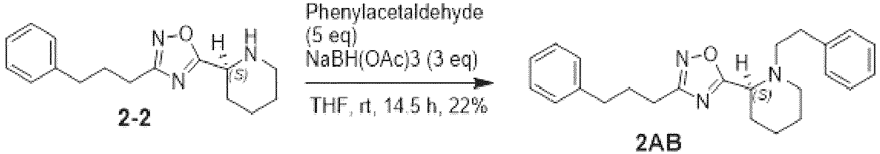

To compound 2-2 (6.4 mg, 24 pmol) prepared in Example 9-2 in a 4 ml vial, phenylacetaldehyde (26.5 µl, 0.118 mmol) and THF (0.31 ml) were added, and the mixture was stirred at room temperature for 10 minutes. Then, sodium triacetoxybolohydride (15 mg, 71 µmol) was added thereto, and the mixture was stirred at room temperature for 14 hours 30 minutes. The reaction was quenched by adding saturated sodium bicarbonate aqueous solution and the mixture was extracted with ethyl acetate. After distilling off the solvent, the residue was purified by NH column DNH-30 size 10 (hexane:ethyl acetate = 100:0 to 87:13). After distilling off the solvent, 2.0 mg (5.33 µmol, 22%) of the desired compound 2AB (film form) was obtained.

### Example 100

### (S) -5-(1-(cyclohexylmethyl)piperidin-2-yl)-3-(3-phenyl propyl)-1,2,4-oxadiazole 2U

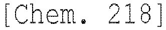

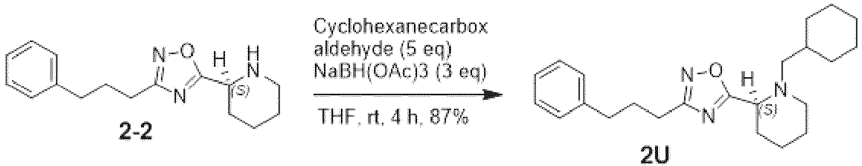

To compound 2-2 (6.8 mg, 25 pmol) prepared in Example 9-2 in a 4 ml vial, cyclohexanecarboxyaldehyde (15.4 µl, 0.125 mmol) was added while washing with THF (0.33 ml). After stirring at room temperature for 10 minutes, sodium triacetoxybolohydride (15.9 mg, 75 µmol) was added thereto, and the mixture was stirred at room temperature for 4 hours. The reaction was quenched by adding saturated sodium bicarbonate aqueous solution and the mixture was extracted with ethyl acetate. After distilling off the solvent, the residue was purified on NH column DNH-30 size 10 (hexane:ethyl acetate = 100:0 to 95:5). After distilling off the solvent, 8.0 mg (21.8 µmol, 87%) of the desired compound 2U (oil) was obtained.

### Example 101

### Ethyl (S)-1-((2-(3-(3-phenylpropyl)-1,2,4-oxadiazol-5-yl) piperidin-1-yl)sulfonyl)piperidine-4-carboxylate 2AC

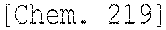

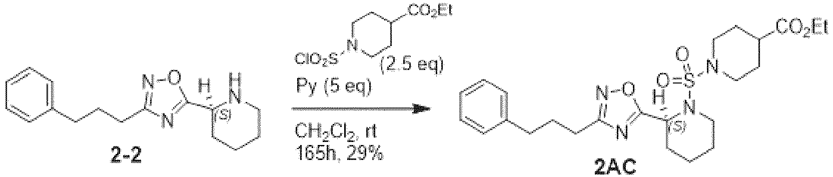

Compound 2-2 (37.3 mg, 0.138 mmol) prepared in Example 9-2 was added to a 10 ml eggplant-shaped flask, then CH₂Cl₂ (0.775 ml) and pyridine (55.5 µl, 0.687 mmol) were added sequentially, and the mixture was ice-cooled, which was added finally with ethyl-1-chlorosulfonylpiperidine-4-carboxylate (92.5 mg, 0.344 mmol) while washing with CH₂Cl₂ (0.6 ml), followed by stirring the mixture at room temperature for 165 hours. After the solvent was distilled off, saturated sodium bicarbonate aqueous solution was added and the mixture was extracted with ethyl acetate. After distilling off the solvent, the residue was purified on a silica gel column Q-pack SI20 size 10 (hexane:ethyl acetate = 100:0 to 75:25). After distilling off the solvent, 19.5 mg (39.7 µmol, 29%) of the desired compound 2AC (oil) was obtained.

### Example 102

### (S) -3-(3-phenylpropyl)-5-(1-(piperidine-1-ylsulfonyl) piperidin-2-yl)-1,2,4-oxadiazole 2AD

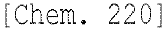

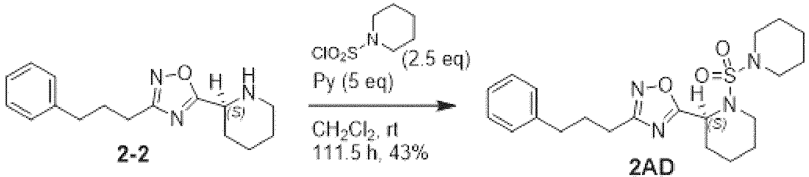

Compound 2-2 (31.2 mg, 0.115 mmol) prepared in Example 9-2 was added to a 10 ml eggplant-shaped flask, then CH₂Cl₂ (0.70 ml) and pyridine (46.4 µl, 0.575 mmol) were added sequentially, and the mixture was ice-cooled, which was finally added with piperidine-1-sulfonyl chloride (54.5 mg, 0.297 mmol) while washing with CH₂Cl₂ (0.45 ml), followed by stirring the mixture at room temperature for 111 hours 30 minutes. After the solvent was distilled off, saturated sodium bicarbonate aqueous solution was added and the mixture was extracted with ethyl acetate. After distilling off the solvent, the residue was purified on a silica gel column Q-pack SI20 size 10 (hexane:ethyl acetate = 100:0 to 0:100). After distilling off the solvent, 20.3 mg (48.5 µmol, 43%) of the desired compound 2AD (oil) was obtained.

### Example 103

### (S) -4-((2-(3-(3-phenylpropyl)-1,2,4-oxadiazol-5-yl) piperidin-1-yl)sulfonyl)morpholine 2AE

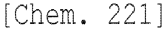

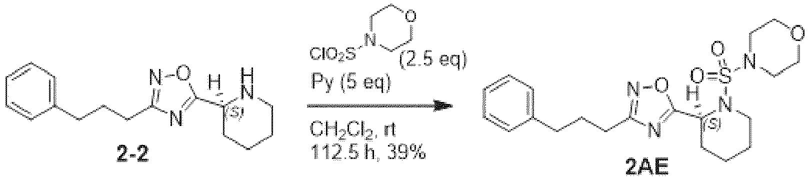

Compound 2-2 (33.7 mg, 0.124 mmol) prepared in Example 9-2 was added to a 10 ml eggplant-shaped flask, then CH₂Cl₂ (0.792 ml) and pyridine (50.1 µl, 0.621 mmol) were added sequentially, and the mixture was ice-cooled, which was added finally with morpholine-4-sulfonyl chloride (61.6 mg, 0.315 mmol) while washing with CH₂Cl₂ (0.45 ml), followed by stirring the mixture at room temperature for 112 hours 30 minutes. After the solvent was distilled off, saturated sodium bicarbonate aqueous solution was added and the mixture was extracted with ethyl acetate. After distilling off the solvent, the residue was purified on a silica gel column Q-pack SI20 size 10 (hexane:ethyl acetate = 100:0 to 0:100). After distilling off the solvent, 20.2 mg (48 µmol, 39%) of the desired compound 2AE (oil) was obtained.

### Example 104

### Tert-butyl (S)-2-(3-(3-(3,4-dimethoxyphenyl)propyl)-1,2,4-oxadiazol-5-yl)piperidine-1-carboxylate 22-1

### Example 104-1: 4-(3,4-Dimethoxyphenyl-N'-hydroxybutane imidamide

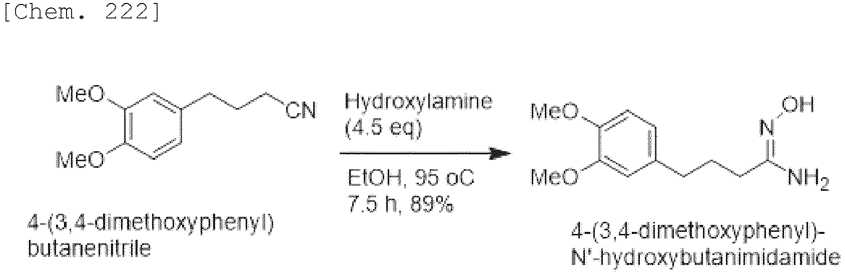

4-(3,4-Dimethoxyphenylbutanenitrile (603 mg, 2.94 mmol) and 50% hydroxylamine aqueous solution (0.780 ml, 13.22 mmol) were added to an eggplant-shaped flask and were dissolved in anhydrous ethanol (3.67 ml), followed by heating the mixture to reflux at 95°C for 7 hours 30 minutes. After distilling off the solvent, the product was dried in vacuo to afford the title compound (white solid, 625 mg, yield: 89%).

### Example 104-2: tert-butyl (S)-2-(3-(3-(3,4-dimethoxyphenyl) propyl)-1,2,4-oxadiazol-5-yl)piperidine-1-carboxylate 22-1

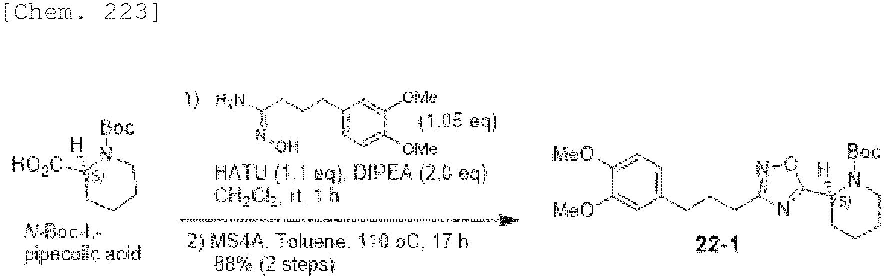

N-Boc-L-pipecolinic acid (200 mg, 0.872 mmol) was added to a 25 ml eggplant-shaped flask and was dissolved in dichloromethane (2.36 ml). HATU (365 mg, 0.960 mmol) and diisopropylethylamine (0.304 ml, 1.745 mmol) were then added thereto, and the mixture was stirred for 10 minutes at room temperature under a nitrogen atmosphere. Then, 4-(3,4-dimethoxyphenyl-N'-hydroxybutaneimidamide (218 mg, 0.916 mmol) was added while washing with dichloromethane (6 ml), and the mixture was stirred at room temperature for 1 hour. After removing the stirrer bar and distilling off the solvent, the residue was purified on a silica gel column Q-pack SI30 size 20 (hexane:ethyl acetate = 90:10 to 50:50). After distilling off the solvent, the imidamide intermediate was obtained. Pre-dried MS4Å (1.96 g) was added to the intermediate, which was then dissolved in ultra-dehydrated toluene (4.36 ml). The mixture was stirred at 110°C for 17 hours with a Dimroth condenser attached. After removing MS4Å by celite filtration and distilling off the solvent, the resulting residue was purified on silica gel column Q-pack SI30 size 20 (hexane:ethyl acetate = 100:0 to 75:25). After distilling off the solvent, 331 mg (0.767 mmol, 88% (after 2 steps)) of the desired compound 22-1 (amorphous solid) was obtained.

### Example 105

### (S) -5-(1-(cyclohexylsulfonyl)piperidin-2-yl)-3-(3-(3,4-dimethoxyphenyl)propyl)-1,2,4-oxadiazole 22B and byproduct 22B'

### Example 105-1: (S)-3-(3-(3,4-dimethoxyphenyl)propyl)-5-(piperidin-2-yl)-1,2,4-oxadiazole 22-2

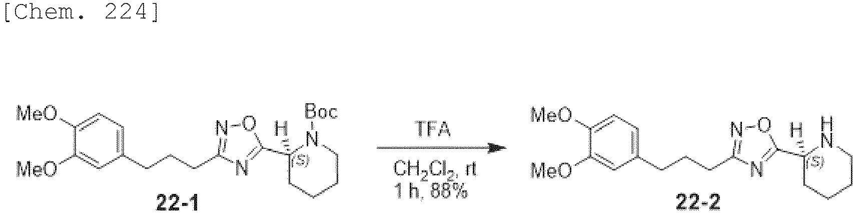

Compound 22-1 (304 mg, 0.704 mmol) prepared in Example 104-2 was added to a 10 ml eggplant-shaped flask, and dichloromethane (1.76 ml) and TFA (0.587 ml) were added thereto, followed by stirring the mixture at room temperature for 1 hour. After removing the stirrer bar and distilling off the solvent, then saturated sodium bicarbonate aqueous solution was added and the mixture was extracted with ethyl acetate. The solution was dried over magnesium sulfate, which was then filtered out, and the solvent was distilled off to afford 204 mg (0.616 mmol, 88%) of the desired compound 22-2 (amorphous solid).

### Example 105-2: (S)-5-(1-(cyclohexylsulfonyl)piperidin-2-yl)-3-(3-(3,4-dimethoxyphenyl)propyl)-1,2,4-oxadiazole 22B, and byproduct 22B'

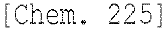

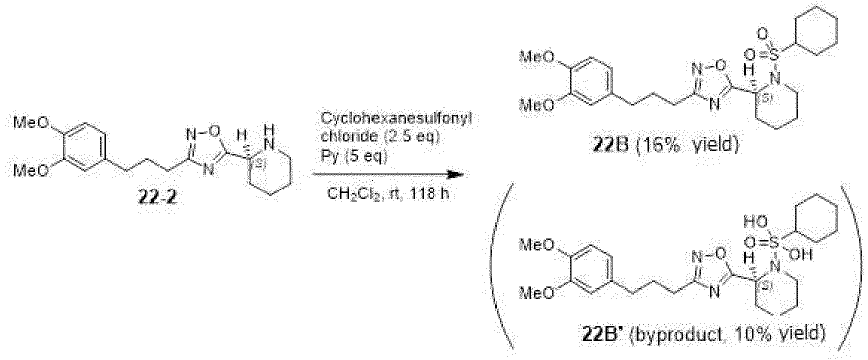

To a 10 ml eggplant-shaped flask, compound 22-2 (39.4 mg, 0.119 mmol) prepared in Example 104-2, CH₂Cl₂ (0.589 ml) and pyridine (48 µl, 0.594 mmol) were added sequentially, and the mixture was ice-cooled, which was added finally with cyclohexanesulfonyl chloride (58.3 mg, 0.303 mmol) while washing with CH₂Cl₂ (0.6 ml), followed by stirring the mixture at room temperature for 118 hours. After the solvent was distilled off, saturated sodium bicarbonate aqueous solution was added thereto and the mixture was extracted with ethyl acetate. After distilling off the solvent, the residue was purified on a silica gel column Q-pack SI20 size 10 (hexane:ethyl acetate = 100:0 to 0:100). After distilling off the solvent, 9.0 mg (18.8 µmol, 16%) of the desired compound 22B (amorphous solid) and 5.8 mg (11.7 µmol, 10%) of the byproduct 22B' (amorphous solid) were obtained.

### Example 106

### Tert-butyl (S)-2-(3-(2-oxo-2-(phenylamino)ethyl)-1,2,4-oxadiazol-5-yl)piperidine-1-carboxylate 23-1

### Example 106-1: tert-butyl (S)-2-(3-(2-ethoxy-2-oxoethyl)-1,2,4-oxadiazol-5-yl)piperidine-1-carboxylate 23-0

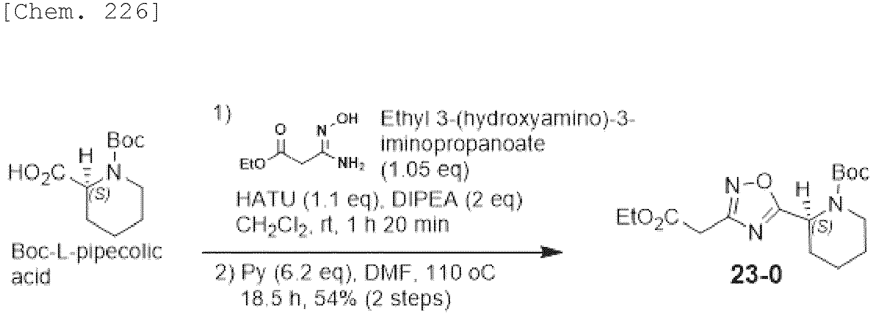

N-Boc-L-pipecolinic acid (150 mg, 0.654 mmol) was added to a 25 ml eggplant-shaped flask and was dissolved in dichloromethane (3.27 ml). HATU (274 mg, 0.720 mmol) and diisopropylethylamine (0.228 ml, 1.308 mmol) were then added thereto, and the mixture was stirred for 10 minutes at room temperature under a nitrogen atmosphere. Ethyl 3-hydroxyamino-3-iminopropanoate (100 mg, 0.687 mmol) was then added while washing with dichloromethane (0.8 ml), and the mixture was stirred at room temperature for 1 hour 20 minutes. After removing the stirrer bar and distilling off the solvent, the residue was purified on a silica gel column Q-pack SI30 size 20 (hexane:ethyl acetate = 84:16 to 50:50). After distilling off the solvent, the imidamide intermediate was obtained. The intermediate was added with pyridine (0.327 ml, 4.05 mmol) and was dissolved in ultra-dehydrated DMF (3.27 ml). The mixture was stirred at 110°C for 18 hours and 30 minutes with a Dimroth condenser attached. The solvent was distilled off, water was added and the mixture was extracted with diethyl ether. The solvent was distilled off and the resulting residue was purified on silica gel column Q-pack SI30 size 20 (hexane:ethyl acetate = 100:0 to 75:25). After distilling off the solvent, 120 mg (0.352 mmol, 54% (after 2 steps)) of the desired compound 23-0 (oil) was obtained.

### Example 106-2: tert-butyl (S)-2-(3-(2-oxo-2-(phenylamino) ethyl)-1,2,4-oxadiazol-5-yl)piperidine-1-carboxylate 23-1

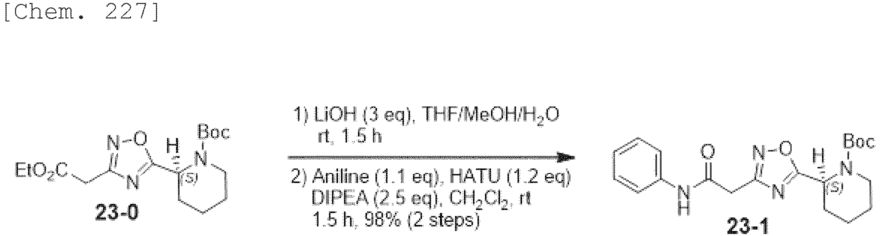

Compound 23-0 (59.4 mg, 0.175 mmol) prepared in Example 106-1 was added to a 10 ml eggplant-shaped flask, then was dissolved in tetrahydrofuran (0.467 ml), methanol (0.70 ml) and water (0.233 ml), and lithium hydroxide monohydrate (22 mg, 0.530 mmol) was added thereto, followed by stirring the mixture at room temperature for 1 hour 30 minutes. Then, 1M hydrochloric acid aqueous solution (0.6 ml) was added to the mixture to make it acidic, and the organic solvent was distilled off, followed by extracting the residue with ethyl acetate. The solution was dried over magnesium sulfate, which was then filtered out, and the solvent was distilled off to afford 54.5 mg (0.175 mmol) of carboxylic acid. Then, dichloromethane (1.75 ml), HATU (79.9 mg, 0.210 mmol) and diisopropylethylamine (76.2 µl, 0.438 mmol) were added, and finally aniline (17.8 µl, 0.193 mmol) was added thereto, followed by stirring the mixture at room temperature for 1 hour 30 minutes. After distilling off the solvent, the residue was purified on a silica gel column Q-pack SI30 size 10 (hexane:ethyl acetate = 100:0 to 50:50). After distilling off the solvent, 66 mg (0.170 mmol, 98% (after 2 steps)) of the desired compound 23-1 (white amorphous solid) was obtained.

### Example 107

### 5-((2S)-1-(tert-butylsulfinyl)piperidin-2-yl)-3-(3-phenyl propyl)-1,2,4-oxadiazole 2W'

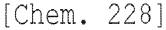

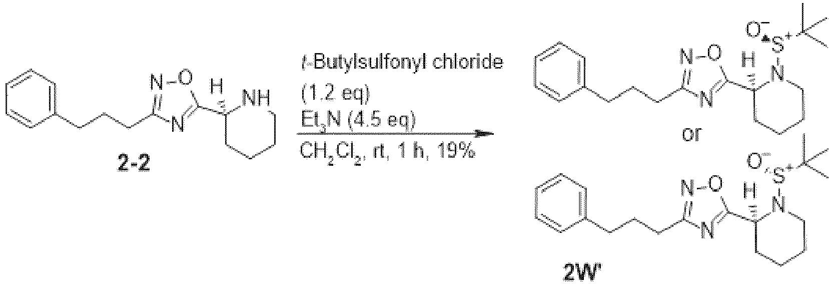

Compound 2-2 (23.4 mg, 86 pmol) prepared in Example 9-2, CH₂Cl₂ (0.862 ml) and triethylamine (53.8 ul, 0.388 mmol) were added sequentially to a 10 ml eggplant-shaped flask, and finally t-butyl sulfinyl chloride (17.9 µl, 0.103 mmol) was added slowly thereto, followed by stirring the mixture at room temperature for 1 hour. The reaction was quenched by adding saturated sodium bicarbonate aqueous solution and the mixture was extracted with ethyl acetate. After distilling off the solvent, the residue was purified on a silica gel column Q-pack SI30 size 10 x 2 consolidated (hexane:ethyl acetate = 100:0 to 75:25). After distilling off the solvent, the less-polar diastereomer was harvested to afford 6.1 mg (16 µmol, 19%) of the desired compound 2W' (oil) . It is noted that the stereochemistry of the sulfoxide in compound 2W' has not been determined.

Physical property data for the compounds prepared in Examples 84-107 are shown in Table 21.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acid-acetonitrile;
0-0.9 min linear gradient A:B (95:5)-A:B (10:90), 0.9-3 min A:B (10:90)

**Table 21**

| [Table 54] | | | | | | |
|---|---|---|---|---|---|---|
| **Ex** | **Cn** | **Chemical Structure** | **MW** | **¹H-NMR δ ppm** | **[M+H]** | **RT :Min** |
| 84 | 17-1 | 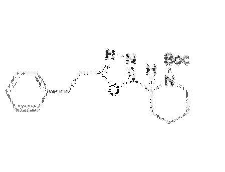 | 357.45 | (CDCl3) δ: 7.31-7.16 (5H, m). 5.53 (1H, br-s), 4.07. 3.95 (1H, m), 3.18-3.06 (4H, m), 2.79 (1H, br-s), 2.25 (1H, br-d, J - 13.6 Hz), 1.90-1.43 (5H, m), 1.48 (9H, s) | 358 | 1.78 |
| 85 | 18-1 | 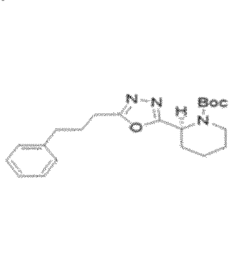 | 371.48 | (CDCl3) ð: 7.32-7.15 (5H, m). 5.53 (1H, br-s), 4.04 (1H, br-d, J = 10.0 Hz), 2.84 (2H, t, J = 7.6 Hz). 2.72 (2H, t, J = 7.6 Hz), 2.29 (1H. br-d. J = 13.6 Hz), 2.11 (2H, quin, J = 7.6 Hz), 1.93-1.82 (1H, m), 1.76-1.40 (5H, m), 1.47 (9H, s) | 372 | 1.84 |
| 86 | 19-1 | 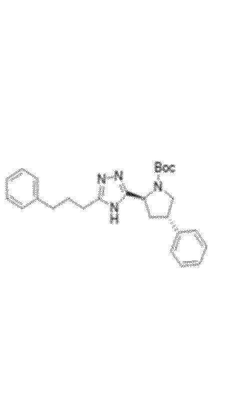 | 432.57 | (CDCl3) δ: 11.8-10.7 (1H, br-s), 7.35-7.15 (10H, m), 5.14 (1H. d, J = 8.4 Hz), 4.08-3.99 (0.2H, m), 3.85 (0.8H, t, J = 9.2 Hz), 3.68 (0.8H, m), 3.56-3.45 (0.2H, m), 3.37 (1H, t, J = 10.4 Hz), 3.01 (0.8H, dd, J = 12.0, 5.6 Hz), 2.77 (2H, t. J = 8.0 Hz), 2.71 (2H. t, J = 7.6 Hz). 2.59-2.49 (0.2H, m), 2.38 (1H, m), 2.15-2.04 (2H, m), 1.48 (7.2H. s). 1.37 (1.8H. s) | 433 | 1.74 |
| 87 | 20-1 | 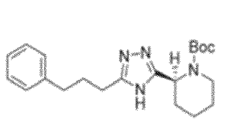 | 370.50 | (CDCl3) δ: 7.30-7.14 (5H, m), 5.42 (1H, d, J = 4.4 Hz), 4.00 (1H, br-d, J = 12.0 Hz). 2.89.2.77 (1H, m), 2.77 (2H, t, J = 8.0 Hz), 2.70 (2H, t, J | 371 | 1.7 |

**Table 21 continued**

| [Table 55] | | | | | | |
|---|---|---|---|---|---|---|
| | | | | = 8.0 Hz), 2.38 (1H, br-d, J = 13.6 Hz), 2.08 (2H, quin, J - 7.6 Hz), 1.89-1.53 (5H, m), 1.47 (9H, s) | | |
| 88 | 2W | 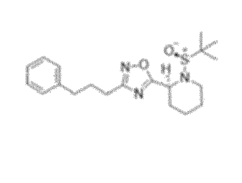 | 375.53 | (CDCl3) δ: 7.31-7.15 (5H. m), 4.79 (1H, dd. J = 4.8, 3.6 Hz), 3.44 (1H, dt, J = 13.2, 4.0 Hz), 3.32-3.25 (1H. m), 2.77 (2H, t, J = 8.0 Hz), 2.70 (2H. t, J = 8.0 Hz). 2.18-1.98 (4H, m). 1.75-1.52 (4H. m), 1.16 (9H, s) | 376 | 1.82 |
| | | 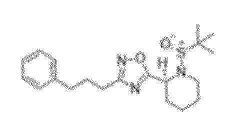 | | | | |
| 89 | 2X | 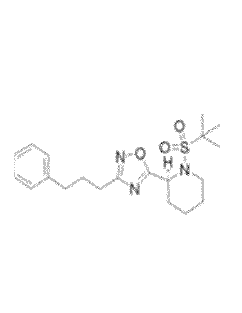 | 391.53 | (CDCl3) δ: 7.30-7.15 (5H, m). 5.30 (1H. br-s), 3.78 (1H, br-d, J = 14.0 Hz). 3.36 (1H, m). 2.78 (2H, t, J = 7.6 Hz), 2.71 (2H, t, J - 7.6 Hz), 2.27 (1H, dq, J = 13.6, 2.4 Hz). 2.10 (2H, quin. J = 7.6 Hz). 2.09-1.98 (1H, m), 1.78 (1H. br-d, J = 13.6 Hz), 1.72-1.62 (2H, m). 1.47-1.35 (1H, m), 1.38 (9H, s) | 392 | 1.87 |
| 90 | 218 | 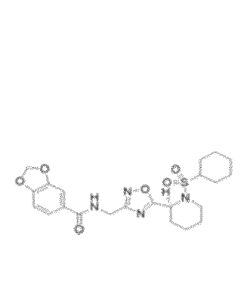 | 476.55 | (CDCl3) δ: 7.35 (1H, dd, J = 8.0, 2.0 Hz), 7.31 (1H, d, J r, 1.2 Hz), 6.85 (1H, d, J - 8.0 Hz). 6.57 (1H, br-s), 6.04 (2H, s), 5.35 (1H, d, J - 4.8 Hz). 4.80 (2H, d, J = 5.6 Hz), 3.80 (1H, br.d, J = 14.4 Hz). 3.27-3.19 (1H, m), 2.94 (1H, tt, J = 12.0, 3.2 Hz), 2.31 (1H, dd. J = 13.2, 3.2 Hz), 2.16 (1H, br-d, J = 11.2 Hz). 2.10-1.98 (1H. m), 1.90-1.13 (13H, m) | 477 | 1.63 |

**Table 21 continued 2**

| [Table 56] | | | | | | |
|---|---|---|---|---|---|---|
| 91 | 2Q | 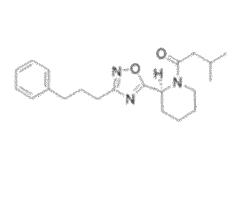 | 355.48 | (CDCl3) δ: 7.30-7.15 (5H, m). 6.16 (1H, br-d, J = 4.8 Hz), 5.31.5.25 (0.5H, m), 4.70-4.60 (0.5H, m), 3.86 (1H. br-d, J = 14.4 Hz), 3.27 (1H. td, J - 13.2, 2.4 Hz), 2.78-2.66 (4H, m), 2.41-2.02 (5H, m), 1.90-1.68 (3H, m), 1.58-1.31 (2H, m), 1.00 (6H, d, J 6.4 Hz) | 356 | 1.86 |
| 92 | 2R | 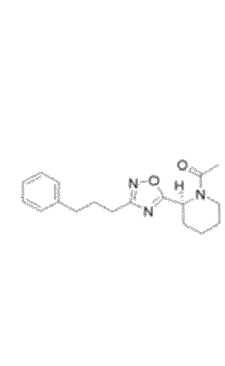 | 313.4 | (CDCl3) δ: 7.31-7.16 (5H, m), 6.12 (1H, d, J = 5.2 Hz), 5.21 (0.45H. br-d, J = 3.2 Hz), 4.61 (0.55H. br-d. J = 11.6 Hz), 3.78 (1H, br-d, J = 14.0 Hz), 3.30 (1H, td, J = 13.2, 2.8 Hz). 2.75 (2H. t, J = 8.0 Hz), 2.70 (2H, t, J = 8.0 Hz), 2.36 (1H, br-d, J = 13.6 Hz). 2.19 (3H, s), 2.08 (2H. quin, J = 7.6 Hz), 1.90-1.67 (2H, m), 1.58-1.33 (2H, m) | 314 | 1.68 |
| 93 | 2S | 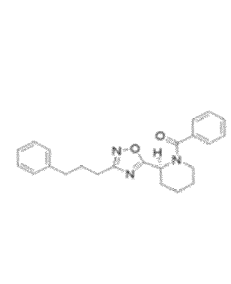 | 375.47 | (CDCl3) δ: 7.49-7.17 (10H, m). 6.23 (1H, br-s), 5.15 (0.5H. br-s), 4.71 (0.5H, br-s), 3.73 (1H. br-s),3.24 (1H. br-s), 2.78 (2H, t, J = 7.6 Hz), 2.72 (2H, t. J = 7.6 Hz), 2.44 (1H. br-s), 2.11 (2H. quin, J = 7.6 Hz), 1.99 (1H, br-s), 1.81 (1H. br-d, J = 13.2 Hz). 1.69-1.39 (2H, m) | 376 | 1.84 |
| 94 | 2P | 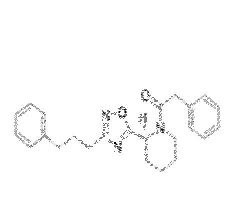 | 389.5 | (CDCl3) δ: 7.34-7.17 (10H. m), 6.16 (1H, br-d, J = 5.2 Hz), 5.26 (0.5H, br-d, J = 3.2 Hz), 4.66 (0.5H, br-d, J = 11.6 Hz), 3.84 (2H, s), 3.80 (1H, s). 3.21-3.12 (1H. m), 2.78-2.66 (4H, m), 2.36 (1H, br-d. J = 13.6 Hz), 2.07 (2H, quin, J = 7.6 Hz), 1.89-1.76 | 390 | 1.83 |

**Table 21 continued 3**

| [Table 57] | | | | | | |
|---|---|---|---|---|---|---|
| | | | | (1H, m), 1.75-1.66 (1H. m), 1.43-1.18 (2H, m) | | |
| 95 | 2Y | 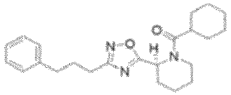 | 381.52 | (CDCl3) δ: 7.30-7.15 (5H, m), 6.15 (1H. d, J = 5.2 Hz), 5.33 (0.5H, br-s), 4.63 (0.5H. br-d. J = 13.6 Hz), 3.90 (1H, br-d, J = 13.6 Hz), 3.23 (1H. td. J = 13.6, 2.8 Hz), 2.76-2.67 (4H, m). 2.62-2.52 (1H, m), 2.37 (1H, br-d, J = 14.8 Hz), 2.07 (2H, quin, J = 7.6 Hz), 1.88-1.22 (14H, m) | 382 | 1.95 |
| 96 | 2T | 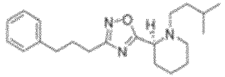 | 341.50 | (CDCl3) δ: 7.30-7.13 (5H. m), 3.85 (1H, dd, J - 7.2, 4.8 Hz), 3.02 (1H. dt, J = 12.4, 4.8 Hz), 2.77 (2H. t, J = 7.6 Hz), 2.69 (2H, t, J = 7.6 Hz), 2.37-2.17 (3H, m), 2.09 (2H, quin, J = 7.6 Hz), 1.92-1.84 (2H, m), 1.84-1.64 (3H. m). 1.55-1.39 (2H, m),1.39-1.31 (2H, m), 0.82 (3H, d. J = 6.8 Hz). 0.79 (3H. d. J = 6.8 Hz) | 342 | 1.4 |
| 97 | 2Z | 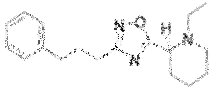 | 299.42 | (CDCl3) δ: 7.30-7.13 (5H, m), 3.85 (1H, dd, J = 7.6, 4.4 Hz), 3.04 (1H, dt, J = 12.0. 6.8 Hz), 2.77 (2H, t, J - 7.2 Hz). 2.69 (2H, t. J = 7.6 Hz), 2.44 (1H, dq, J = 12.8. 7.2 Hz), 2.34-2.23 (2H, m), 2.14-2.05 (2H. m), 1.93-1.53 (5H, m). 1.50-1.37 (1H, m), 1.03 (3H, t, J = 7.2 Hz) | 300 | 1.23 |
| 98 | 2AA | 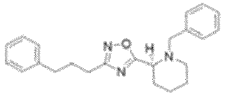 | 361.49 | (CDCl3) δ: 7.30-7.15 (10H, m), 3.91 (1H, t, J = 6.0 Hz), 3.59 (1H, d, J = 13.6 Hz), 3.38 (1H, d, J = 13.6 Hz), 2.93 (1H, dt, J - 10.8, 5.2 Hz). 2.78 (2H, t, J = 7.6 Hz), | 362 | 1.88 |

**Table 21 continued 4**

| [Table 58] | | | | | | |
|---|---|---|---|---|---|---|
| | | | | 2.71 (2H, t, J = 7.6 Hz), 2.32-2.24 (1H, m), 2.11 (2H, quin, J - 7.6 Hz), 1.93 (2H, m), 1.75 (1H, m), 1.66-1.53 (4H. m), 1.45 (1H, m) | | |
| 99 | 2AB | 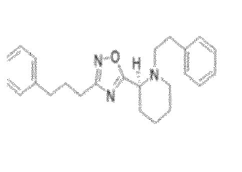 | 375.52 | (CDCl3) δ: 7.30-7.08 (10H, m), 3.97 (1H, dd. J = 7.2, 4.8 Hz), 3.07 (1H, m), 2.87-2.57 (7H, m), 2.55.2.46 (2H. m), 2.09 (2H, quin, J = 8.0 Hz), 1.91 (2H, m), 1.84-1.42 (6H, m) | 376 | 1.6 |
| 100 | 2U | 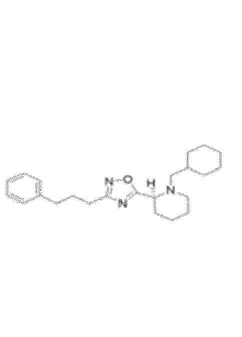 | 367.54 | (CDCl3) δ: 7.30-7.15 (5H. m), 3.82 (1H. t, J - 6.0 Hz), 2.98 (2H, ddd, J = 11.2, 6.0, 4.4 Hz), 2.77 (2H, t, J = 8.0 Hz). 2.70 (2H, t, J - 7.6 Hz), 2.25 (1H, ddd, J - 11.6, 7.2, 4.4 Hz), 2.13-2.02 (4H. m). 1.88 (2H, q, J = 6.0 Hz), 1.84 (1H, br-d, J = 14.4 Hz), 1.77-1.56 (7H, m),1.50-1.35 (2H, m), 1.28-1.02 (3H, m), 0.81-0.66 (2H, m) | 368 | 1.72 |
| 101 | 2AC | 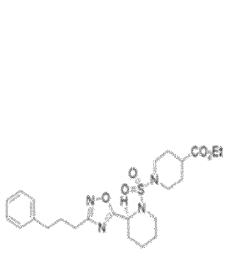 | 490.62 | (CDCl3) δ; 7.31-7.15 (5H. m), 5.25 (1H, br-d, J = 5.2 Hz), 4.13 (2H, q, J = 7.2 Hz), 3.76 (1H. br-d, J = 13.2 Hz), 3.69-3.56 (2H, m),3.32-3.23 (1H, m), 2.88 (2H, m), 2.78 (2H, t, J = 7.6 Hz), 2.72 (2H, t, J = 7.6 Hz), 2.39-2.32 (1H, m), 2.21 (1H, dd, J = 11.2, 2.4 Hz), 2.09 (2H, quin, J = 7.6 Hz), 2.08-1.91 (3H, m), 1.80-1.38 (6H, m), 1.25 (3H, t, J - 7.2 Hz) | 491 | 1.87 |

**Table 21 continued 5**

| [Table 59] | | | | | | |
|---|---|---|---|---|---|---|
| 102 | 2AD | 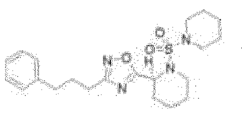 | 418.56 | (CDCl3) δ; 7.31-7.15 (5H, m). 5.26 (1H, br-d, J = 5.2 Hz), 3.71 (1H, br-d, J - 12.8 Hz). 3.30-3.25 (1H. m), 3.17-3.13 (3H, m), 2.78 (2H, **t,** J = 7.6 Hz), 2.72 (2H, t, J = 7.6 Hz), 2.23-1.97 (4H, m), 1,80-1.42 (11H, m) | 419 | 1.92 |
| 103 | 2AE | 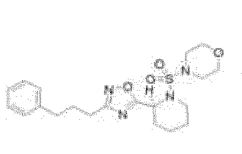 | **420.53** | (CDCl3) δ: 7.31-7.16 (5H, m). 5.27 (1H, br-d, J = 5.2 Hz), 3.80 (1H. br-d, J = 13.2 Hz), 3.72-3.62 (3H, m), 3.34-3.25 (1H, m), 3.19 (4H, t, J = 4.8 Hz), 2.78 (2H, t, J = 7.6 Hz), Z.74 (2H, t, J - 7.6 Hz), 2.23 (1H, dd, J = 13.6, 2.8 Hz), 2.14-1.98 (3H. m), 1.82-1.40 (5H, m) | 421 | 1.77 |
| 104 | 22-1 | 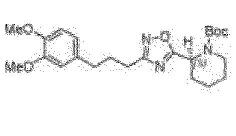 | 431.5.3 | (CDCl3) δ: 6.82 - 6.71 (3H, *m*), 5.54 (1H. m), 4.04 (1H. br-s), 3.88 (3H, s), 3.86 (3H, s), 2.29 (1H, br-d, J = 12.0 Hz), 2.05 (2H, quin. J = 7.2 Hz), 1.95-1.84 (1H, m), 1.76-1.22 (4H. m), 1.46 (9H, br-s) | 432 | 1.84 |
| 105 | 228 | 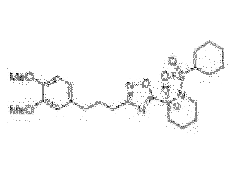 | 477.62 | (CDCl3) δ: 6.82-6.70 (3H, m), 5.33 (1H, br-d, J = 5.2 Hz), 3.88 (3H. s), 3.86 (3H, s). 3.81 (1H, m), 3.27-3.19 (1H, m), 2.95 (1H, tt, J = 12.0, 3.2 Hz). 2.77 (2H, t, J = 7,6 Hz), 2.66 (2H, t, J = 7.6 Hz), 2.29 (1H, br-d, J = 14.0 Hz), 2.17 (2H, br-d, J = 12.4 Hz), 2.12-1.96 (3H, m). 1.90-1.11 (12H. m) | 418 | 1.82 |
| | 22B' | | 495,64 | (CDCl3) δ: 6.82-6.70 (3H, m), 5.25 (1H, br-d, J = 4.0 Hz). 5.04 (1H, dd. J = 5.2, 2.4 Hz), 3.88 (3H, s), 3.86 (3^{,}H, s), 3.68 (1H, br-d, J = | 496 | 1.89 |

**Table 21 continued 6**

| [Table 60] | | | | | | |
|---|---|---|---|---|---|---|
| | | | | 14.0 Hz), 3.53-3.27 (2H, m), 2.77 (2H, td, J = 7.6, 4.4 Hz), 2.65 (2H, t, J = 7.6 Hz), 2.22-1.13 (18H, m) | | |
| 106 | 23-1 | 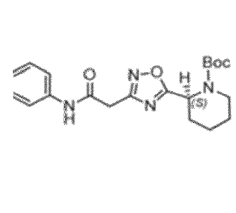 | 386.45 | (CDCl3) δ: 7.53 (2H, dd, J = 8.4, 1.2 Hz), 7.33 (2H, t, J = 8.0 Hz), 7.12 (1H, t, J = 7.2 Hz), 5.80-5.43 (1H, m), 4.10 (1H. br-s). 3.91(2H, s), 2.97 (1H. br-s), 2.35 (1H, br-d, J = 12.8 Hz). 1.96 (1H, tdd, J 13.6, 5.6, 3.6 Hz), 1.82-1.18 (4H, m), 1.46 (9H, s) | 387 | 1.69 |
| 107 | 2W' | 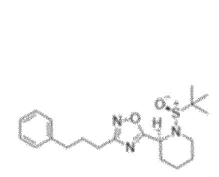 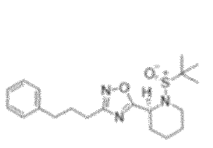 | 375.53 | (CDCl3) δ: 7.32-7.14 (5H, m), 4.97 (1H, m). 3.31-3.26 (1.6H, m), 3.16-3.07 (0.4H, m), 2.78 (2H, t, J = 7.6 Hz), 2.71 (2H, t. J = 7.6 Hz), 2.20-2.13 (1H, m), 2.10 (2H, quin, J - 7.6 Hz), 1.97 (1H. m), 1.85-1.25 (4H, m), 1.15 (9H, s) | 376 | 1.83 |

| | | | | | | |
|---|---|---|---|---|---|---|
| Ex: Example Number Cn: Compound Number MW: Molecular Weight | | | | | | |

### Example 108

### Tert-butyl (1R,3S,4S)-3-(5-((1H-indol-3-yl)methyl)-4H-1,2,4-triazol-3-yl)-2-azabicyclo[2.2.1]heptane-2-carboxylate 24-1

### Example 108-1: tert-butyl (1R,3S,4S)-3-(hydrazinecarbonyl)-2-azabicyclo[2.2.1]heptane-2-carboxylate 24-0

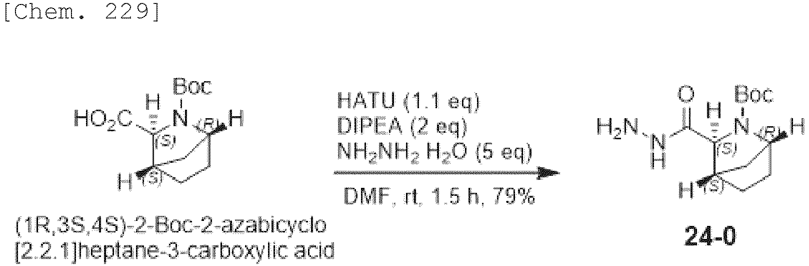

2-Boc-2-azabicyclo[2.2.1]heptane-3-carboxylic acid (606 mg, 2.512 mmol) was added to a 10 ml eggplant-shaped flask and was dissolved in DMF (3.0 ml). HATU (1.05 g, 2.76 mmol) and diisopropylethylamine (0.875 ml, 5.02 mmol) were then added thereto, and the mixture was stirred for 10 minutes at room temperature under a nitrogen atmosphere. This solution was then added dropwise to a 10 ml eggplant-shaped flask containing hydrazine-mono-hydrate (623 µl, 12.56 mmol) dissolved in DMF (1.5 ml), washed with DMF (1.779 ml), and the mixture was stirred at room temperature for 1 hour 30 minutes. After the solvent was distilled off, the reaction was quenched with saturated sodium bicarbonate aqueous solution and the mixture was extracted with ethyl acetate. After distilling off the solvent, the residue was purified on a silica gel column Q-pack SI30 size 60 (chloroform:methanol = 100:0 to 95:5). After distilling off the solvent, 508 mg (1.99 mmol, 79%) of the desired compound 24-0 (amorphous solid) was obtained.

### Example 108-2: Ethyl 2-(1H-indol-3-yl)acetimidate

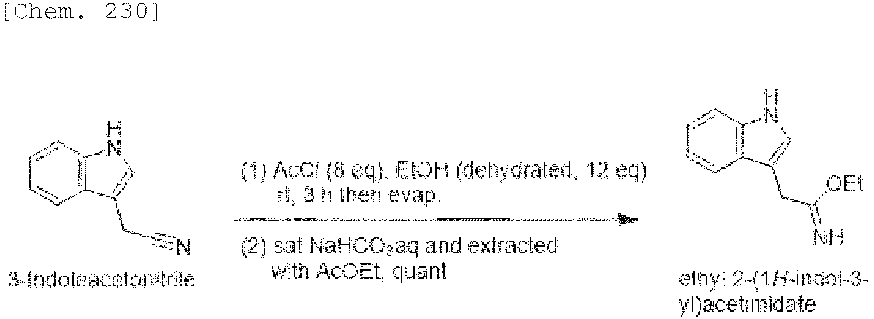

3-Indole acetonitrile (200 mg, 1.28 mmol) was added to an eggplant-shaped flask and was dissolved in anhydrous ethanol (0.89 ml), to which acetyl chloride (0.724 ml, 10.2 mmol) was added slowly dropwise while cooling with cold water. The mixture was then stirred at room temperature for 3 hours, the solvent was distilled off, and the mixture was dried in vacuo for 15 minutes. The residue was dissolved in ethyl acetate and saturated sodium bicarbonate aqueous solution was slowly added thereto, followed by extracting the solution with ethyl acetate. After distilling off the solvent and drying in vacuo, the title compound (light brown solid, 258 mg, yield: 100%) was obtained.

### Example 108-3: tert-butyl (1R,3S,4S)-3-(5-((1H-indol-3-yl)methyl)-4H-1,2,4-triazol-3-yl)-2-azabicyclo[2.2.1] heptane-2-carboxylate 24-1

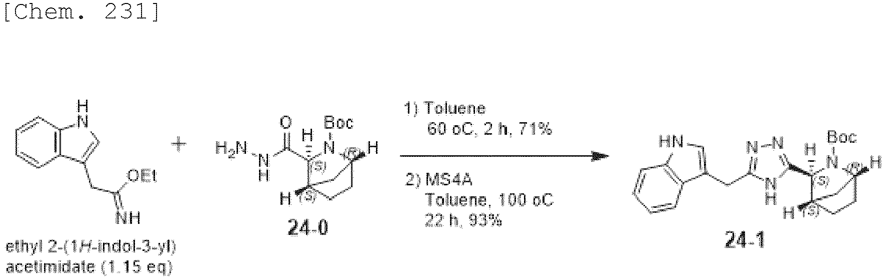

Ethyl 2-(1h-Indole-3-yl)acetoimidate (99.4 mg, 0.481 mmol) prepared in Example 108-3 was added to a 25 ml eggplant-shaped flask, to which compound 24-0 (108.5 mg, 0.425 mmol) prepared in Example 108-1 was added with dissolving in ultra-dehydrated toluene (8.50 ml) and the mixture was stirred at 60°C for 2 hours under a nitrogen atmosphere. After removing the stirrer bar and distilling off the solvent, the residue was purified on a silica gel column Q-pack SI30 size 20 (chloroform:methanol = 95:5). After distilling off the solvent, 124 mg (0.301 mmol, 71%) of the iminohydrazine intermediate (white solid) was obtained. Pre-dried MS4Å (620 mg) was added to the intermediate, which was then dissolved in ultra-dehydrated toluene (6.03 ml). The mixture was stirred at 100°C for 22 hours with a Dimroth condenser attached. After removing MS4Å by celite filtration and distilling off the solvent, the resulting residue was purified on silica gel column Q-pack SI30 size 20 (chloroform:methanol = 100:0 to 93:7). After distilling off the solvent, 110 mg (0.280 mmol, 93%) of the desired compound 24-1 (light brown solid) was obtained.

### Example 109

### (1R,3S,4S)-3-(5-((1H-indol-3-yl)methyl)-4H-1,2,4-triazol-3-yl)-N-benzyl-2-azabicyclo[2.2.1]heptane-2-carboxamide 24F and 24F'

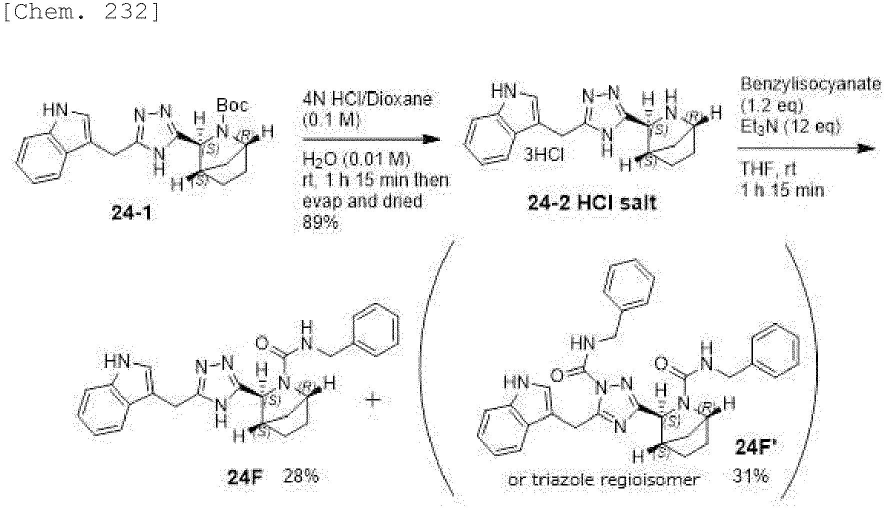

Compound 24-1 (46.5 mg, 0.118 mmol) prepared in Example 108-3 was added to a 10 ml eggplant-shaped flask, to which were added 4 N HCl/dioxane (1.18 ml) and water (0.118 ml), and the mixture was stirred at room temperature for 1 hour 15 minutes. After removing the stirrer bar and distilling off the solvent, the residue was dried in vacuo to afford 42.4 mg (0.105 mmol, 89%) of the tri-hydrochloride salt. 8.0 mg (20 µmol) of 24-2 tri-hydrochloride salt was dissolved in ultra-dehydrated tetrahydrofuran (0.397 ml), to which triethylamine (33 µl, 0.238 mmol) and benzylisocyanate (3.0 µl, 24 pmol) were added, and the mixture stirred at room temperature for 1 hour 15 minutes. The reaction was quenched by adding water and the mixture was extracted with ethyl acetate. The solvent was distilled off and the resulting residue was purified on a silica gel column Q-pack SI30 size 10 (chloroform:methanol = 100:0 to 93:7). After distilling off the solvent, 2.4 mg (5.6 µmol, 28%) of the desired compound 24F (film form) and 3.4 mg (6.1 µmol, 31%) of byproduct 24F' (amorphous solid) were obtained.

### Example 110

### (1R,3S,4S)-3-(5-((1H-indol-3-yl)methyl)-4H-1,2,4-triazol-3-yl)-N-(tert-butyl)-2-azabicyclo[2.2.1]heptane-2-carboxamide 24H

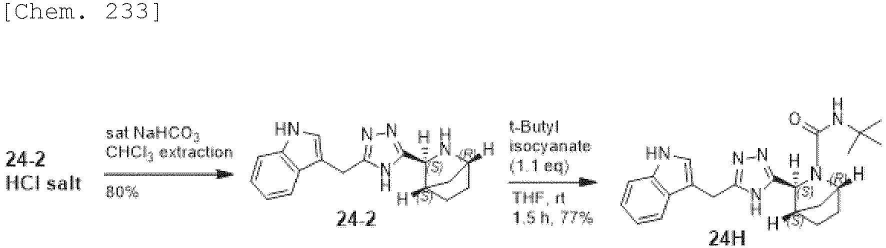

24-2 tri-hydrochloride salt (7.6 mg, 18.9 umol) prepared in Example 109 was added to a test tube, to which saturated sodium bicarbonate aqueous solution was added to made it basic, and the mixture was extracted with chloroform. After filtration of the organic layer with a phase separator, the solvent was distilled off to afford 4.4 mg (15.0 µmol, 80%) of compound 24-2.
This was dissolved in ultra-dehydrated tetrahydrofuran (0.25 ml) and t-butyl isocyanate (2.0 µl, 16 µmol) was added while washing with ultra-dehydrated tetrahydrofuran (0.25 ml), followed by stirring the mixture for 1 hour 30 min at room temperature. The solvent was distilled off and the resulting residue was purified on a silica gel column Q-pack SI20 size 10 (chloroform:methanol = 100:0 to 93:7). After distilling off the solvent, 4.4 mg (11.2 µmol, 77%) of the desired compound 24H (film form) was obtained.

### Example 111

### Tert-butyl (1R,3S,4S)-3-(5-(2-(pyridin-4-yl)ethyl)-4H-1,2,4-triazol-3-yl)-2-azabicyclo[2.2.1]heptane-2-carboxylate 25-1

### Example 111-1: Ethyl 3-(pyridin-4-yl)propanimidate hydrochloride

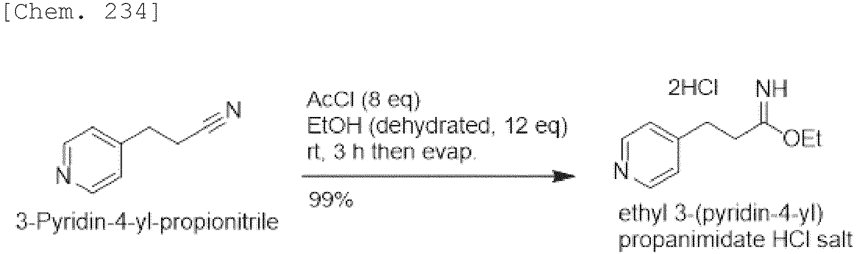

3-Pyridin-4-yl-propionitrile (200 mg, 1.513 mmol) was added to an eggplant-shaped flask and was dissolved in anhydrous ethanol (1.05 ml), to which acetyl chloride (0.856 ml, 12.1 mmol) was added slowly dropwise while cooling with cold water. The mixture was then stirred at room temperature for 3 hours. The solvent was distilled off and the residue was dried in vacuo for 15 min to afford the title compound (white solid, 250 mg, yield: 99%).

### Example 111-2: tert-butyl (lR,3S,4S)-3-(5-(2-(pyridin-4-yl)ethyl)-4H-1,2,4-triazol-3-yl)-2-azabicyclo[2.2.1] heptane-2-carboxylate 25-1

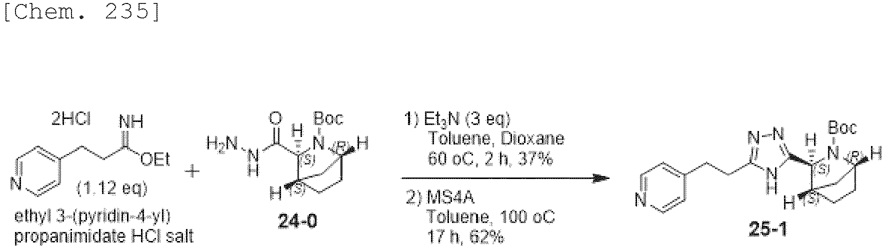

Compound 24-0 (19 mg, 74 pmol) prepared in Example 108-1, ethyl 3-(pyridin-4-yl)propanimidate hydrochloride (21 mg, 83 µmol) and triethylamine (30.9 µl, 0.223 mmol) were added to a 10 ml eggplant-shaped flask, and were dissolved in ultra-dehydrated toluene (0.992 ml) and dioxane (0.496 ml), followed by stirring the mixture at 60°C for 2 h under argon atmosphere. After removing the stirrer bar and distilling off the solvent, the residue was purified on a silica gel column Q-pack SI30 size 20 (chloroform:methanol = 100:0 to 80:20). After distilling off the solvent, 10.6 mg (27.4 µmol, 37%) of the iminohydrazine intermediate (white solid) was obtained. Pre-dried MS4Å (53 mg) was added to the intermediate, which was then dissolved in ultra-dehydrated toluene (0.912 ml). The mixture was stirred at 100°C for 17 hours with a Dimroth condenser attached. After removing MS4Å by cotton plug filtration and distilling off the solvent, the resulting residue was purified on a silica gel column Q-pack SI30 size 10 (chloroform:methanol = 100:0 to 90:10). After distilling off the solvent, 6.3 mg (17.7 µmol, 62%) of the desired compound 25-1 (oil) was obtained.

### Example 112

### Compound 25AD'

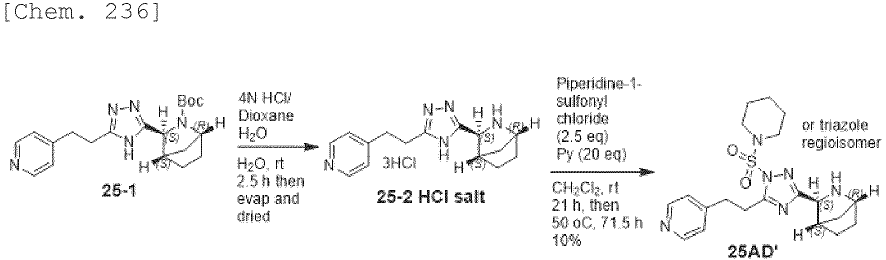

Compound 25-1 (22 mg, 60 pmol) prepared in Example 111-2 was added to a 10 ml eggplant-shaped flask, to which were added 4 N hydrochloric acid/dioxane (0.595 ml) and water (60 µl), and the mixture was stirred at room temperature for 2 hours 30 minutes. By removing the stirrer bar, distilling off the solvent, and drying in vacuo, 22.5 mg (60 µmol, 100%) of the trihydrochloride salt was obtained. This was dissolved in dichloromethane (0.444 ml), to which was added pyridine (95.9 ul, 1.188 mmol), and the mixture was finally ice-cooled, which was then added with piperidine-1-sulfonyl chloride (26.2 mg, 0.143 mmol) with washing with CH₂Cl₂ (0.150 ml), followed by stirring the mixture for 21 hours at room temperature and for 71 hours 30 minutes at 50°C. After distilling off the solvent, adding water thereto, and washing the same with chloroform, saturated sodium bicarbonate aqueous solution was added to the aqueous layer to make it basic and the mixture was extracted with chloroform. After distilling off the solvent, the residue was purified on a silica gel column Q-pack SI30 size 10 (chloroform: methanol: water = 100:0:0 to 65:25:4). After distilling off the solvent, 2.4 mg (5.76 µmol, 10%) of the desired compound 25AD' (film form) was obtained.

### Example 113

### (1R,3S,4S)-2-(isobutylsulfonyl)-3-(5-(2-(pyridin-4-yl)ethyl)-4H-1,2,4-triazol-3-yl)-2-azabicyclo[2.2.1]heptane 25A

### Example 113-1: (1R,3S,4S)-3-(5-(2-(pyridin-4-yl)ethyl)-4H-1,2,4-triazol-3-yl)-2-azabicyclo[2.2.1]heptane 25-2

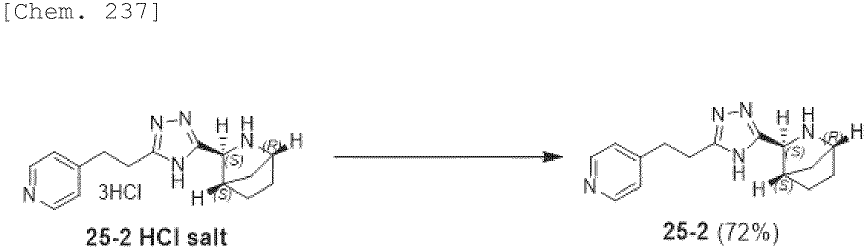

To a test tube, 25-2 tri-hydrochloride salt (28 mg, 74 µmol) prepared in Example 112 and water were added, and the mixture was washed with chloroform. The mixture was added with saturated sodium hydrogen carbonate solution to make it basic, and the mixture was washed again with chloroform, followed by filtering off the organic layer using a phase separator. After distilling off the remaining aqueous layer, the residue was washed with a solution of chloroform/methanol = 3/1 using an ultrasonic cleaner, the solid was filtered off, and the filtrate obtained was concentrated to afford 15 mg (55.7 µmol, 72%) of compound 25-2.

### Example 113-2: (1R,3S,4S)-2-(isobutylsulfonyl)-3-(5-(2-(pyridin-4-yl)ethyl)-4H-1,2,4-triazol-3-yl)-2-azabicyclo [2.2.1]heptane 25A

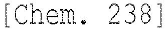

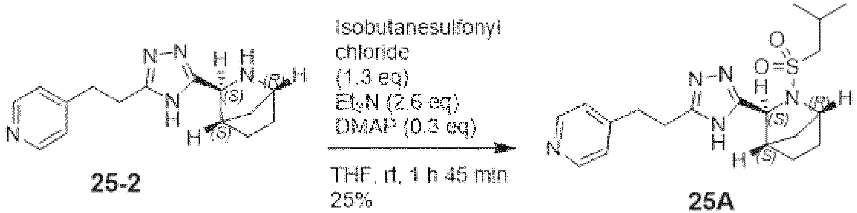

Compound 25-2 (5.7 mg, 21 pmol) prepared in Example 111-2 was added to a 10 ml eggplant-shaped flask, then ultra-dehydrated THF (0.423 ml), triethylamine (7.6 µl, 55 µmol) and DMAP (0.78 mg, 6 pmol) were added sequentially, and isobutylsulfonyl chloride (3.7 µl, 28 µmol) was added thereto, followed by stirring the mixture at room temperature for 1 hour 45 minutes. Methanol (30 µl) was added thereto and the mixture was stirred for 5 minutes. Then, reaction was quenched by adding saturated sodium bicarbonate aqueous solution and the mixture was extracted with ethyl acetate. After distilling off the solvent, the residue was purified on a silica gel column Q-pack SI30 size 10 (chloroform:methanol = 100:0 to 70:30). After distilling off the solvent, 2.0 mg (5.1 µmol, 25%) of the desired compound 25A (amorphous solid) was obtained.

### Example 114

### (1R,3S,4S)-2-(piperidine-1-ylsulfonyl)-3-(5-(2-(pyridin-4-yl)ethyl)-4H-1,2,4-triazol-3-yl)-2-azabicyclo[2.2.1]heptane 25AD and 25AD"

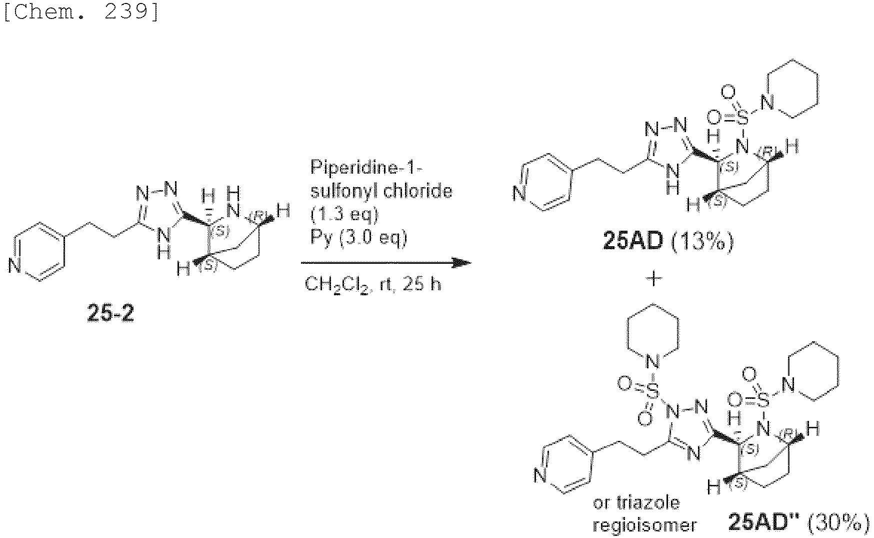

Compound 25-2 (15 mg, 55.7 pmol) prepared in Example 111-2 was added to a 10 ml eggplant-shaped flask and was dissolved in dichloromethane (0.496 ml), to which was added pyridine (13.5 µl, 0.167 mmol), and the mixture was finally ice-cooled. Piperidine-1-sulfonyl chloride (13.3 mg, 72 pmol) was added to the mixture while washing with CH₂Cl₂ (0.30 ml), and the mixture was stirred at room temperature for 25 hours. After distilling off the solvent, saturated sodium bicarbonate aqueous solution was added to make it basic and the mixture was extracted with ethyl acetate. After distilling off the solvent, the residue was purified on a silica gel column Q-pack SI30 size 10 (chloroform: methanol: water = 100:0 to 90:10). After distilling off the solvent, 3.1 mg (7.4 µmol, 13%) of the desired compound 25AD (film form) and 7.0 mg (12.4 µmol, 30%) of byproduct 25AD'' (film form) were obtained.

### Example 115

### 5-((2S)-1-((adamantan-1-yl)sulfinyl)piperidin-2-yl)-3-(3-phenylpropyl)-1,2,4-oxadiazole 2AF and 2AF'

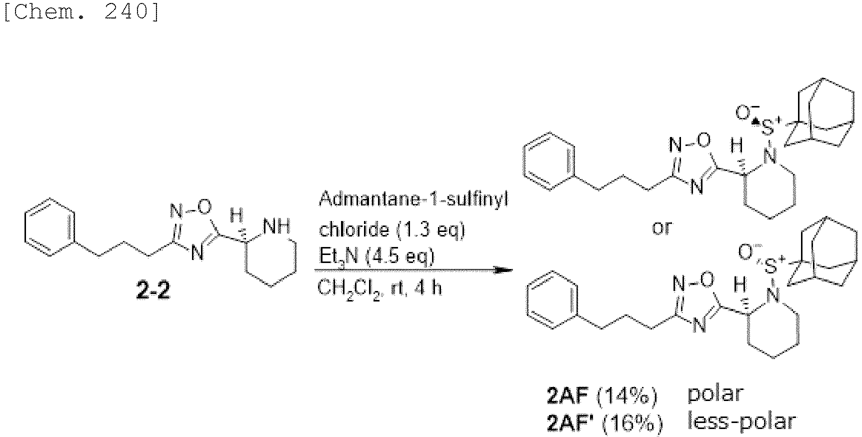

Compound 2-2 (41 mg, 0.151 mmol) prepared in Example 9-2, dichloromethane (1 ml) and triethylamine (94.2 µl, 0.680 mmol) were added sequentially to a 10 ml eggplant-shaped flask, and finally adamantane-1-sulfinyl chloride (44.5 mg, 0.193 mmol) was added slowly while washing with dichloromethane (0.51 ml), followed by stirring the mixture at room temperature for 4 hours. The reaction was quenched by adding saturated sodium bicarbonate aqueous solution and the mixture was extracted with ethyl acetate. After distilling off the solvent, the residue was purified on a silica gel column Q-pack SI30 size 10 (hexane:ethyl acetate = 100:0 to 75:25). After distilling off the solvent, 9.9 mg (21.8 µmol, 14%) of the desired compound 2AF (polar diastereomer, oil) and 11.2 mg (24.7 µmol, 16%) of 2AF' (less-polar diastereomer, oil) were obtained. It should be noted that the stereochemistry of the sulfoxide in compound 2AF and compound 2AF' has not been determined.

### Example 116

### 5-((S)-1-((adamantan-1-yl)sulfonyl)piperidin-2-yl)-3-(3-phenylpropyl)-1,2,4-oxadiazole 2AG

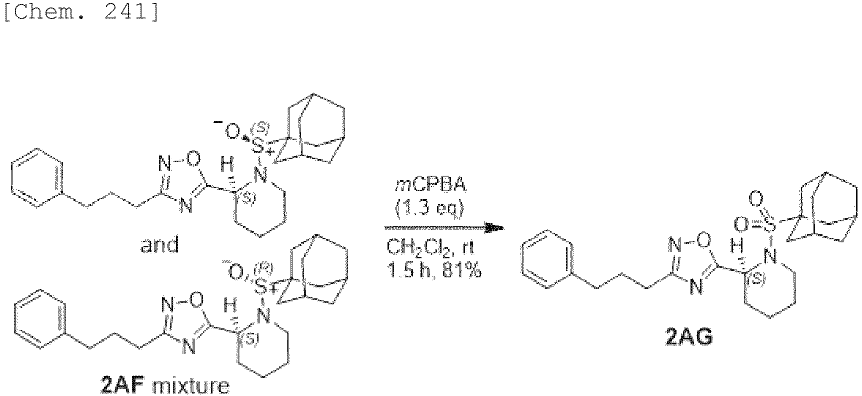

Compound 2AF (13.5 mg, 30 pmol) prepared in Example 115 was added to a 10 ml eggplant-shaped flask and was dissolved in CH₂Cl₂ (0.992 ml), to which was added 65% m-chloroperbenzoic acid (10.3 mg, 39 µmol) slowly, followed by stirring the mixture for 1 hour 30 minutes at room temperature. The reaction was quenched by adding saturated sodium bicarbonate aqueous solution and saturated sodium sulfite aqueous solution and the mixture was extracted with ethyl acetate. After distilling off the solvent, the residue was purified on a silica gel column Q-pack SI30 size 10 (hexane:ethyl acetate = 100:0 to 83:17). After distilling off the solvent, 11.4 mg (24.3 µmol, 81%) of the desired compound 2AG (oil) was obtained.

### Example 117

### (S) -N-((5-(1-(cyclohexylsulfonyl)piperidin-2-yl)-1,2,4-oxadiazol-3-yl)methyl)-1-naphthamide 26B

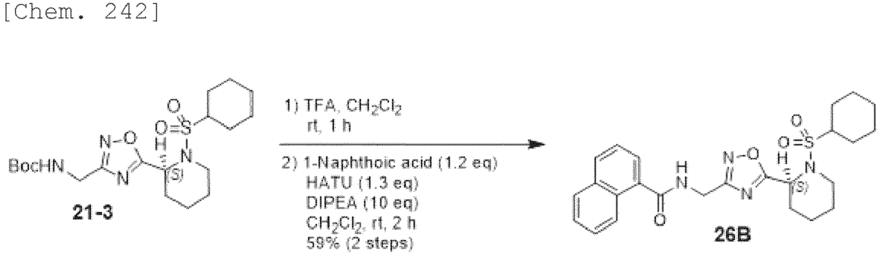

Compound 21-3 (9.4 mg, 21.9 pmol) prepared in Example 90-2 was added to a 10 ml eggplant-shaped flask, and dichloromethane (0.439 ml) and TFA (88 ul) were added thereto, followed by stirring the mixture at room temperature for 1 hour. After removing the stirrer bar and distilling off the solvent, saturated sodium bicarbonate aqueous solution was then added thereto and the mixture was extracted with ethyl acetate. The solution was dried over magnesium sulfate, which was then filtered out, and the solvent was distilled off to afford 7.2 mg (21.9 pmol) of the amine-free intermediate. Dichloromethane (0.406 ml), HATU (6.0 mg, 15.8 µmol) and 1-naphthoic acid (2.57 mg, 14.6 µmol) were then added sequentially to the amine-free intermediate (4.0 mg, 12.2 µmol), and then diisopropylethylamine (21.2 µl, 0.122 mmol) was finally added thereto, followed by stirring the mixture at room temperature for 2 hours. After the solvent was distilled off, saturated sodium bicarbonate aqueous solution was added and the mixture was extracted with ethyl acetate. The organic layer was washed with saturated brine, the solvent was distilled off, and the residue was purified on a silica gel column Q-pack SI20 size 10 (hexane:ethyl acetate = 100:0 to 50:50. After distilling off the solvent, 3.5 mg (7.25 µmol, 59% (after 2 steps)) of the desired compound 26B (white amorphous solid) was obtained.

### Example 118

### (S) -N-((5-(1-(cyclohexylsulfonyl)piperidin-2-yl)-1,2,4-oxadiazol-3-yl)methyl)-3,4-dimethoxybenzamide 27B

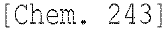

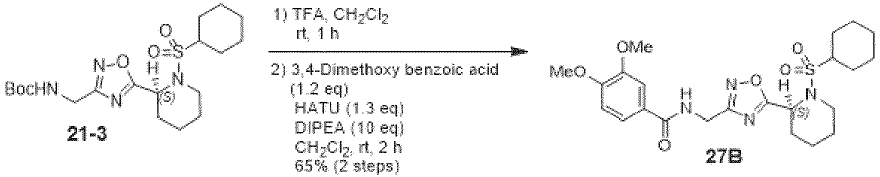

Compound 21-3 (9.4 mg, 21.9 pmol) prepared in Example 90-2 was added to a 10 ml eggplant-shaped flask, and dichloromethane (0.439 ml) and TFA (88 µl) were added thereto, followed by stirring the mixture at room temperature for 1 hour. After removing the stirrer bar and distilling off the solvent, saturated sodium bicarbonate aqueous solution was then added thereto and the mixture was extracted with ethyl acetate. The solution was dried over magnesium sulfate, which was then filtered out, and the solvent was distilled off to afford 7.2 mg (21.9 µmol) of the amine-free intermediate. Dichloromethane (0.322 ml), HATU (4.8 mg, 12.5 pmol) and 3,4-dimethoxybenzoic acid (2.15 mg, 11.6 µmol) were then added sequentially to the amine-free intermediate (3.2 mg, 9.7 pmol), and diisopropylethylamine (16.8 ul, 97 µmol) was finally added, followed by stirring the mixture at room temperature for 2 hours. After the solvent was distilled off, saturated sodium bicarbonate aqueous solution was added thereto and the mixture was extracted with ethyl acetate. The organic layer was washed with saturated brine, the solvent was distilled off, and the residue was purified on a silica gel column Q-pack SI20 size 10 (hexane:ethyl acetate = 100:0 to 25:75). After distilling off the solvent, 3.1 mg (6.29 µmol, 65% (after 2 steps)) of the desired compound 27B (white amorphous solid) was obtained.

### Example 119

### Tert-butyl (1R,3S,4S)-3-(5-((1H-indol-2-yl)methyl)-4H-1,2,4-triazol-3-yl)-2-azabicyclo[2.2.1]heptane-2-carboxylate 28-1

### Example 119-1: Ethyl 2-(1H-indol-2-yl)acetimidate

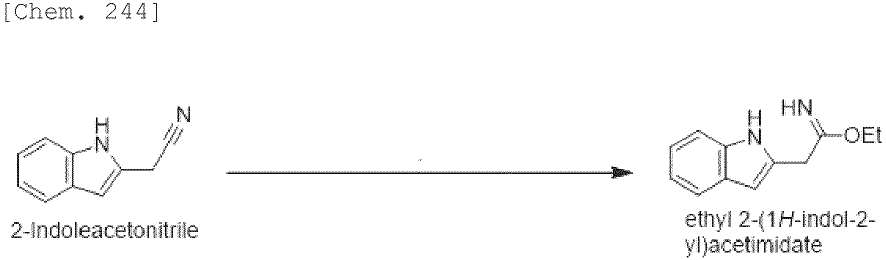

2-Indole acetonitrile (50 mg, 0.32 mmol) was added to an eggplant-shaped flask and was dissolved in anhydrous ethanol (0.796 ml), to which acetyl chloride (0.181 ml, 2.56 mmol) was added slowly dropwise while cooling with cold water. The mixture was then stirred at room temperature for 3 hours, the solvent was distilled off, and the mixture was dried in vacuo for 20 minutes. The residue was dissolved in ethyl acetate and saturated sodium bicarbonate aqueous solution was slowly added. The solvent was distilled off and the residue was dried in vacuo, to afford the title compound (black-brown solid, 59.4 mg, yield: 92%).

### Example 119-2: tert-butyl (1R,3S,4S)-3-(5-((1H-indol-2-yl)methyl)-4H-1,2,4-triazol-3-yl)-2-azabicyclo[2.2.1] heptane-2-carboxylate 28-1

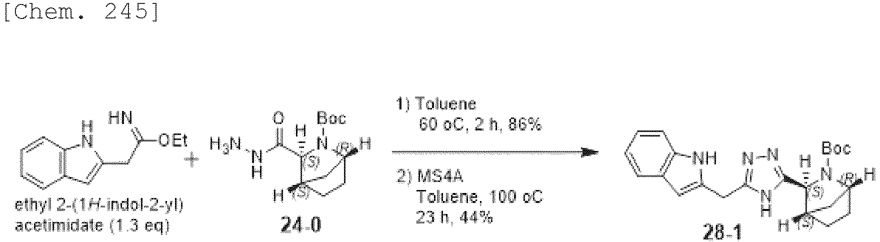

Ethyl 2-(1h-Indole-2-yl)acetoimidate (59.4 mg, 0.294 mmol) prepared in Example 119-1 was added to a 25 ml eggplant-shaped flask, to which compound 24-0 (57.7 mg, 0.226 mmol) prepared in Example 108 was added while dissolving in ultra-dehydrated toluene (4.52 ml), and the mixture was stirred at 60°C under a nitrogen atmosphere for 2 hours. After removing the stirrer bar and distilling off the solvent, the residue was purified on a silica gel column Q-pack SI30 size 20 (chloroform:methanol = 100:0 to 93:7). After distilling off the solvent, 79.8 mg (0.194 mmol, 86%) of the iminohydrazine intermediate (black solid) was obtained. Pre-dried MS4Å (399 mg) was added to the intermediate, which was then dissolved in ultra-dehydrated toluene (3.88 ml). The mixture was stirred at 100°C for 23 hours with a Dimroth condenser attached. After removing MS4Å by celite filtration and distilling off the solvent, the resulting residue was purified on silica gel column Q-pack SI30 size 10 (chloroform:methanol = 100:0 to 95:5). After distilling off the solvent, 33.4 mg (84.9 µmol, 44%) of the desired compound 28-1 (light brown solid) was obtained.

Physical property data for the compounds prepared in Examples 108-119 are shown in Table 22.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acid-acetonitrile;
0-0.9 min linear gradient A:B (95:5)-A:B (10:90), 0.9-3 min A:B (10:90)

**Table 22**

| [Table 61] | | | | | | |
|---|---|---|---|---|---|---|
| Ex | Cn | Chemical Structure | MW | ¹H-NMR δ ppm | [M+H] | RT:Min |
| 108 | 24-1 | 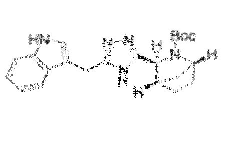 | 393.49 | (CDCl3) δ: 8.31-8.15 (1H, m), 7.60 (0.7H, d. J = 8.0 Hz), 7.56-7.50 (O.SH. m), 7.38-7.30 (1H, m), 7.22-6.99 (3H, m), 4.42-4.05 (4H. m). 3.05 (0.7H. s), 2.75 (0.3H, s), 1.98-1.23 (6H, m), 1.48 (9H, s) | 394 | 1.49 |
| 109 | 24F | 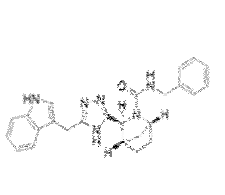 | 426.52 | (CDCl3) δ: 8.17 (1H, s). 7.53 (1H, m), 7.36-7.02 (8H, m), 4.49-4.33 (3H, m), 4.17 (2H, s), 4.08 (1H, m). 3.07 (1H, br-s), 2.05 (1H, d, J = 9.2 Hz), 1.90-1.36 (5H, m) | 427 | 1.45 |
| | 24F' | | 559.67 | (CDCl3) δ: 7.90 (1H, s), 7.63 (1H, d, J = 7.6 Hz), 7.39-7.04 (13H, m), 6.32 (1H, br-s), 4.67 (1H, d, J = 16.0 Hz), 4.60 (1H, d, J = 16.0 Hz), 4.59-4.48 (2H, m), 4.35-4.19 (4H, m), 2.88 (1H. s), 2.04 (1H, d, J = 9.6 Hz). 1.98-1.88 (1H, m), 1.77 (1H, tt, J = 12.4, 4.0 Hz), 1.65 (1H, in), 1.52 (1H, m), 1.37 (1H, d, J = 10.0 Hz) | 560 | 1.72 |
| 110 | 24H | 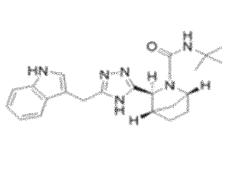 | 392.51 | (CDCl3) δ: 8.08 (1H, br-s), 7.61 (1H. d, J = 8.0 Hz), 7.36 (1H, d, J = 8.0 Hz), 7.22-7.07 (3H, m), 4.38 (1H. s), 4.22 (2H, s), 3.93 (1H, br-s), 3.14 (1H, br-s), 2.02 (1H. br-d, J = 10.4 | 393 | 1.46 |

**Table 22 continued**

| [Table 62] | | | | | | |
|---|---|---|---|---|---|---|
| | | | | Hz), 1.87-1.22 (5H, m), 1.34 (9H. s) | | |
| 111 | 25-1 | 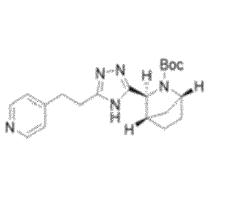 | 369.47 | (CDCl3) δ: 8.48 (2H, d, J = 5.2 Hz), 7.16 (2H, d, J = 5.2 Hz), 4.43 (0.2 H, s), 4.39 (0.8H, s), 4.30 (0.2H. s), 4.15 (0.8H, s), 3.19 (1H. s), 3.13-3.02 (4H. m), 1.90-1.25 (4H. m), 1.49 (9H, s) | 370 | 0.58 |
| 112 | 25AD' | | 416.54 | (CDCl3) δ: 8.50 (2H, dd, J - 4.4, 1.6 Hz), 7.16 (2H, dd, J = 4.4, 2.0 Hz), 3.96 (1H, s), 3.65 (1H, s), 3.36-3.28 (6H, m), 3.11 (2H, t, J = 8.0 Hz), 2.61 (1H, s), 2.12-1.52 (10H. m), 1.30 (1H, d, J - 10.0 Hz), 1.25 (1H, s) | 417 | 1.09 |
| 113 | 25A | 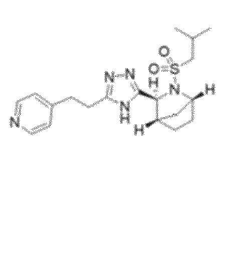 | 389.52 | (CDCl3) δ: 8.49 (2H, dd. J = 4.4, 1.2 Hz), 7.14 (2H, dd. J = 4.4, 1.6 Hz), 4.52 (1H, s), 4.25 (1H, s), 3.11-3.00 (5H. m), 2.94 (1H, dd, J = 13.6, 5.6 Hz), 2.78 (1H. dd. J = 14.0, 7.2 Hz), 2.38-2.13 (2H, m), 2.02 (1H, br-d, J = 10.4 Hz), 1.90 (1H, m), 1.76-1.60 (2H, m), 1.51 (1H. d, J = 10.4 Hz), 1.08 (3H, d, J = 6.4 Hz), 1.02 (3H. d, J = 6.8 Hz) | 390 | 0.58 |
| 114 | 25AD | 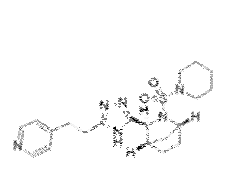 | 416.54 | (CDCl3) δ: 8.53 (2H, br-s), 7.19 (2H, br-s), 4.44 (1H, s). 4.09 (1H, s), 3.16-3.07 (8H. m), 3.02 (1H, d, J = 3.2 Hz), 2.36 (1H. m), 1.96 (1H, d, J :_:: 10.4 Hz), 1.92. 0.78 (IOH, m) | 417 | 0.57 |

**Table 22 continued 2**

| [Table 63] | | | | | | |
|---|---|---|---|---|---|---|
| | 25AD" | | 563.74 | (CDCl3) δ: 8.51 (1.1H, d, J = 6.0 Hz), 8.48 (0.9H. d. J = 6.0 Hz), 7.18 (1.1H, d, J = 6.0 Hz), 7.12 (0.9H, d, J = 6.0 Hz), 4.98 (0.45H, s), 4.39 (0.55H, s), 4.18 (1H, s), 3.44-2.98 (13H, m), 2.59-2.26 (4H. m), 1.88-1.21 (14H, m) | = 564 | 1.41 |
| 115 | 2AF' | 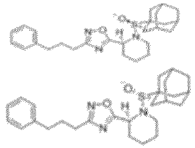 | 453.65 | (CDCl3) δ: 7.31-7.15 (5H, m), 4.95 (1H, br.d, J = 4.0 Hz), 3.30-3.24 (2H, m), 2.79 (2H, t. J = 7.6 Hz), 2.72 (2H, t, J = 7.6 Hz), 2.21-1.50 (21H, m) | 454 | 2.16 |
| | 2AF | 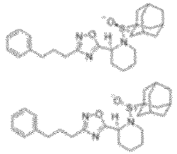 | 453.65 | (CDCl3) δ: 7.31-7.15 (5H, m). 4.73 (1H, t, J = 4.4 Hz), 3.40-3.28 (2H, m), 2.77 (2H, t, J = 7.6 Hz), 2.71 (2H. t, J = 7.6 Hz), 2.19-1.57 (21H, m) | 454 | 2.1 |
| 116 | 2AG | 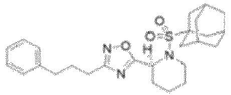 | 469.64 | (CDCl3) δ: 7.32-7.14 (5H, m), 5.26 (1H. br-s), 3.73 (1H, br-s), 3.33 (1H, br-s), 2.78 (2H, t, J = 7.6 Hz), 2.71 (2H, t. J = 7.6 Hz), 2.27 (1H, dq, J = 13.6, 2.4 Hz), 2.13-1.29 (20H, m) | 470 | 2.16 |
| 117 | 268 | 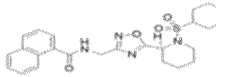 | 482.6 | (CDCl3) δ: 8.39 (1H, d, J = 8.4 Hz), 7.96 (1H, d, J = 8.4 Hz), 7.89 (1H, dd, J = 7.6, 2.4 Hz), 7.71 (1H, d. J - 6.8 Hz), 7.60-7.45 (3H, m), 6.57 (1H. br-s). 5.37 (1H, d, J = 5.2 Hz), 4.93 (2H, d, J = 5.6 Hz), 3.80 (1H, br-d, J = 13.6 Hz), 3.30-3.22 (1H, m), 2.94 (1H, tt, J = 12.0. 3.2 Hz), 2.33 (1H, dd, J - 13.2, 2.4 Hz), 2.16 (1H, br-d. J = 13.2 Hz), 2.10- | 483 | 1.7 |

**Table 22 continued 3**

| [Table 64] | | | | | | |
|---|---|---|---|---|---|---|
| | | | | 1.99 (1H, m), 1.88-1.10 (12H. m) | | |
| 118 | 278 | 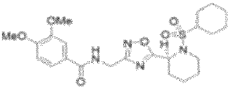 | 492.59 | (CDCl3) δ: 7.46 (1H, d, J = 2.0 Hz), 7.35 (1H, dd. J = 8.4, 2.0 Hz), 6.89 (1H, d, J = 8.4 Hz), 6.65 (1H, br-s), 5.35 (1H, d, J = 5.2 Hz), 4.82 (2H, d, J = 5.6 Hz), 3.79 (1H, d, J = 13.6 Hz), 3.28-3.20 (1H. m), 2.94 (1H, tt, J = 12.0, 3.2 Hz), 2.32 (1H, d, J - 12.8 Hz), 2.16 (1H. br-d, J - 12.4 Hz), 2.09-1.97 (1H. m), 1.90-1.13 (13H, m) | 493 | 1.59 |
| 119 | 28-1 | 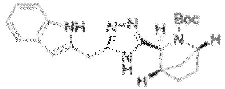 | 393.49 | (CDCl3) δ: 11.54 (1H. br-s), 8.98 (1H, br-s), 7.53 (1H, d, J = 8.0 Hz), 7.31 (1H, dd, J = 8.0. 0.8 Hz), 7.14-6.99 (2H, m), 6.38 (1H, s), 4.47-4.15 (4H, m), 3.21 (0.8H, br-s), 2.85 (0.2H. br-s), 1.89-1.24 (6H, m), 1.49 (7.2H. s). 1.32 (1.8H, s) | 394 | 1.58 |

| | | | | | | |
|---|---|---|---|---|---|---|
| Ex: Example Number Cn: Compound Number MW: Molecular Weight | | | | | | |

### Example 120

### (1R,3S,4S)-2-(cyclohexylsulfonyl)-3-(5-(2-(pyridin-4-yl) ethyl)-4H-1,2,4-triazol-3-yl)-2-azabicyclo[2.2.1]heptane 29B

### Example 120-1: (1R,3S,4S)-2-(cyclohexylsulfonyl)-2-azabicyclo[2.2.1]heptane-3-carbohydrazide 29-0

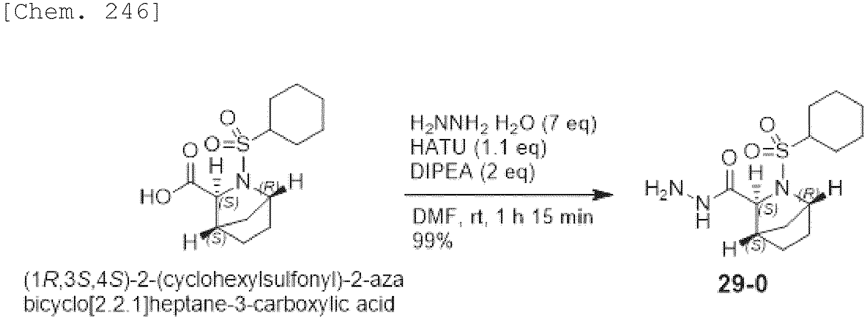

2-Cyclohexylsulfonyl-2-azabicyclo[2.2.1]heptane-3-carboxylic acid (18.5 mg, 64 µmol) was added to a 10 ml eggplant-shaped flask and was dissolved in DMF (0.3 ml). HATU (26.9 mg, 71 µmol) and diisopropylethylamine (22.4 ul, 0.129 mmol) were then added thereto, and the mixture was stirred for 10 minutes at room temperature under a nitrogen atmosphere. This solution was then added dropwise to a 10 ml eggplant-shaped flask containing hydrazine-mono-hydrate (22.3 ul, 0.451 mmol) dissolved in DMF (0.205 ml) while washing with DMF (0.3 ml), and the mixture was stirred at room temperature for 1 hour 15 minutes. The reaction was quenched with saturated sodium bicarbonate aqueous solution and the mixture was extracted with ethyl acetate. After distilling off the solvent, the residue was purified on a silica gel column Q-pack SI30 size 10 (chloroform:methanol = 100:0 to 95:5). After distilling off the solvent, 19.2 mg (63.7 µmol, 99%) of the desired compound 29-0 (amorphous solid) was obtained.

### Example 120-2: (1R,3S,4S)-2-(cyclohexylsulfonyl)-3-(5-(2-(pyridin-4-yl)ethyl)-4H-1,2,4-triazol-3-yl)-2-azabicyclo [2.2.1]heptane 29B

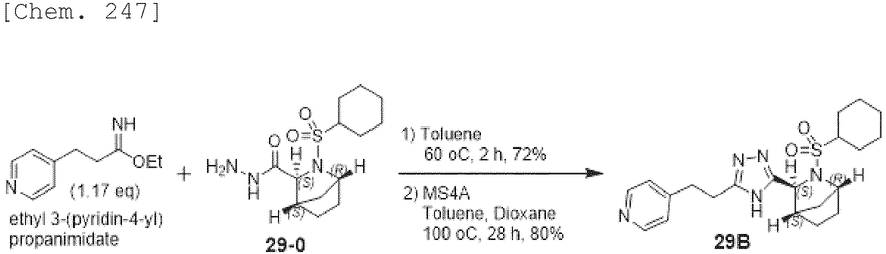

Compound 29-0 (19.2 mg, 64 µmol) prepared in Example 120-1 and ethyl 3-(pyridin-4-yl)propanimidate (19.5 mg, 0.108 mmol) were added to a 10 ml eggplant-shaped flask, and were dissolved in ultra-dehydrated toluene (1.5 ml), followed by stirring the mixture at 60°C for 2 h under argon atmosphere. After removing the stirrer bar and distilling off the solvent, the residue was purified on a silica gel column Q-pack SI30 size 10 (chloroform:methanol = 100:0 to 87:13). After distilling off the solvent, 20 mg (46 µmol, 72%) of the iminohydrazine intermediate (white solid) was obtained. Pre-dried MS4Å (100 mg) was added to the intermediate, which was then dissolved in ultra-dehydrated toluene (1.53 ml) and dioxane (0.51 ml). The mixture was stirred at 100°C for 28 hours with a Dimroth condenser attached. After removing MS4Å by cotton plug filtration and distilling off the solvent, the resulting residue was purified on silica gel column Q-pack SI30 size 10 (chloroform:methanol = 100:0 to 87:13). After distilling off the solvent, 15.3 mg (36.8 µmol, 80%) of the desired compound 29B (white solid) was obtained.

### Example 121

### Tert-butyl -(3-(5-(5-(1-(cyclohexylsulfonyl) piperidin-2-yl)-1,2,4-oxadiazol-3-yl) propyl)benzyl)carbamate 30B

### Example 121-1: tert-butyl-(4-(4-amino-4-(hydroxyimino) butyl)benzyl)carbamate

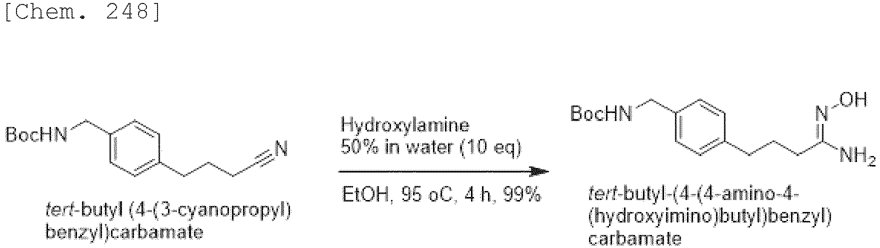

T-butyl-4-(3-cyanopropyl)benzylcarbamate (38.4 mg, 0.140 mmol) and 50% hydroxylamine aqueous solution (83µl, 1.4 mmol) were added to an eggplant-shaped flask, and were dissolved in anhydrous ethanol (1.0 ml), followed by heating to reflux at 95°C for 4 hours. After distilling off the solvent, the product was dried in vacuo to afford the title compound (amorphous solid, 42.6 mg, yield: 99%).

### Example 121-2: tert-butyl (S)-(4-(3-(3-(5-(1-(cyclohexyl sulfonyl)piperidin-2-yl)-1,2,4-oxadiazol-3-yl)propyl) benzyl)carbamate 30B

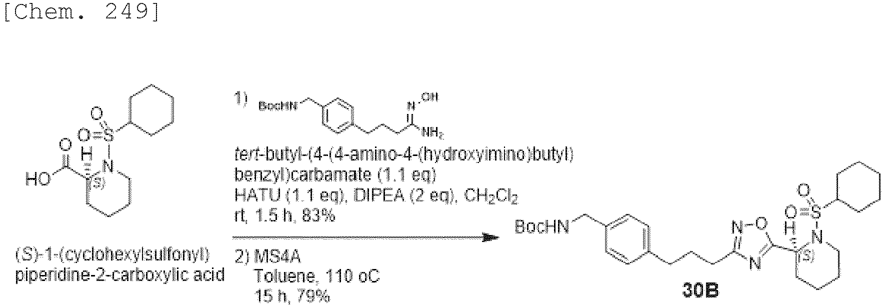

(S)-1-(cyclohexylsulfonyl)piperidine-2-carboxylic acid (35 mg, 0.127 mmol) was added to a 10 ml eggplant-shaped flask and was dissolved in dichloromethane (0.471 ml). HATU (53.2 mg, 0.140 mmol) and diisopropylethylamine (44.3 ul, 0.254 mmol) were then added thereto, and the mixture was stirred for 5 minutes at room temperature under a nitrogen atmosphere. Then, tert-butyl-(4-(4-amino-4-(hydroxyimino) butyl)benzyl)carbamate (43 mg, 0.140 mmol) prepared in Example 121-1 was added dropwise while washing with dichloromethane (0.8 ml), and the mixture was stirred at room temperature for 1 hour 30 minutes. After distilling off the solvent, the residue was purified on silica gel column Q-pack SI30 size 20 (hexane:ethyl acetate = 100:0 to 40:60). After distilling off the solvent, 59.3 mg (0.105 mmol, 83%) of the imidamide intermediate (amorphous solid) was obtained. Pre-dried MS4Å (297 mg) was added to the intermediate, which was then dissolved in ultra-dehydrated toluene (1.05 ml). The mixture was stirred at 110°C for 15 hours with a Dimroth condenser attached. After removing MS4Å by celite filtration and distilling off the solvent, the resulting residue was purified on silica gel column Q-pack SI30 size 10 (hexane:ethyl acetate = 100:0 to 66:34). After distilling off the solvent, 45.5 mg (83.2 µmol, 79%) of the desired compound 30B (amorphous solid) was obtained.

### Example 122

### (1R,3S,4S)-3-(5-(1h-Indole-3-yl)methyl)-4H-1,2,4-triazol-3-yl)-N-(quinolin-3-ylmethyl)-2-azabicyclo[2.2.1]heptane-2-carboxamide 24AH

### Example 122-1: (1R,3S,4S)-3-(hydrazinecarbonyl)-N-(quinolin -3-ylmethyl)-2-azabicyclo[2.2.1]heptane-2-carboxamide 31-0

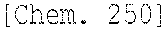

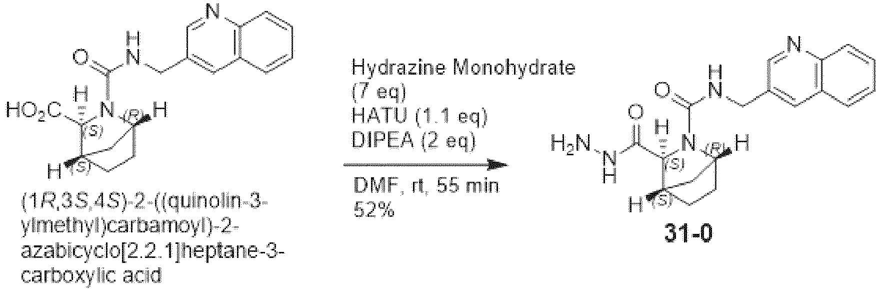

2-(quinolin-3-ylmethyl)carbamoyl)-2-azabicyclo[2.2.1] heptane -3-carboxylic acid (22 mg, 68 µmol) was added to a 10 ml eggplant-shaped flask and was dissolved in DMF (0.345 ml). HATU (28.3 mg, 74.4 µmol) and diisopropylethylamine (23.6 ul, 0.135 mmol) were then added thereto, and the mixture was stirred for 10 minutes at room temperature under a nitrogen atmosphere. This solution was then added dropwise to a 10 ml eggplant-shaped flask containing hydrazine-monohydrate (23.5 µl, 0.473 mmol) dissolved in DMF (0.20 ml), washed with DMF (0.3 ml), and the mixture was stirred at room temperature for 55 minutes. The reaction was quenched with saturated sodium bicarbonate aqueous solution and the mixture was extracted with ethyl acetate. After distilling off the solvent, the residue was purified on a silica gel column Q-pack SI30 size 10 (chloroform:methanol = 100:0 to 80:20). After distilling off the solvent, 11.9 mg (35 µmol, 52%) of the desired compound 31-0 (amorphous solid) was obtained.

### Example 122-2: (1R,3S,4S)-3-(5-(1h-Indole-3-yl)methyl)-4H-1,2,4-triazol-3-yl)-N-(quinolin-3-ylmethyl)-2-azabicyclo [2.2.1]heptane-2-carboxamide 24AH

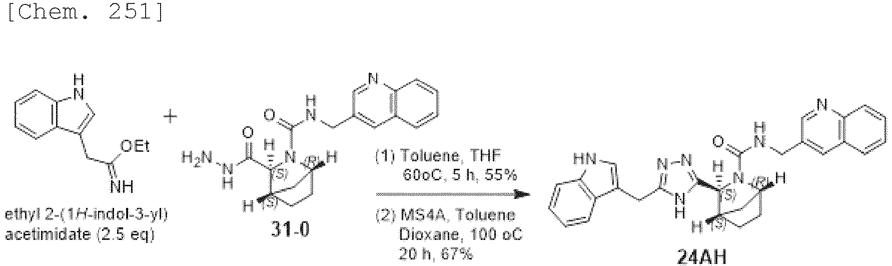

Ethyl 2-(1h-Indole-3-yl) acetimidate (8.4 mg, 41 µmol) was added to a 10 ml eggplant-shaped flask, to which were added compound 31-0 (5.5 mg, 16 µmol) prepared in Example 122-1, ultra-dehydrated toluene (0.81 ml) and ultra-dehydrated THF (0.405 ml), and the mixture was stirred at 60°C for 5 hours under a nitrogen atmosphere. After removing the stirrer bar and distilling off the solvent, the residue was purified on a silica gel column Q-pack SI30 size 10 (chloroform:methanol = 100:0 to 80:20). After distilling off the solvent, 4.4 mg (8.88 µmol, 55%) of the iminohydrazine intermediate (white solid) was obtained. Pre-dried MS4Å (44 mg) was added to the intermediate, which was then dissolved in ultra-dehydrated toluene (0.888 ml) and dioxane (0.444 ml). The mixture was stirred at 100°C for 20 hours with a Dimroth condenser attached. After removing MS4Å by cotton plug filtration and distilling off the solvent, the resulting residue was purified on a silica gel column Q-pack SI30 size 10 (chloroform:methanol = 100:0 to 90:10). After distilling off the solvent, 2.8 mg (5.86 µmol, 67%) of the desired compound 24AH (white solid) was obtained.

### Example 123

### (1R,3S,4S)-3-(5-(1h-Indole-2-yl)methyl)-4H-1,2,4-triazol-3-yl)-N-(quinolin-3-ylmethyl)-2-azabicyclo[2.2.1]heptane-2-carboxamide 28AH

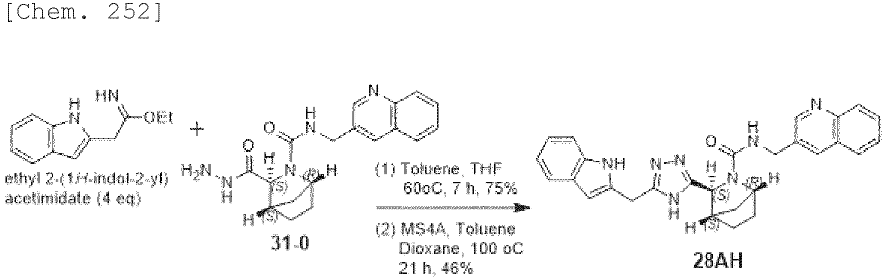

Ethyl 2-(1h-Indole-3-yl)acetimidate (15.6 mg, 77 µmol) was added to a 10 ml eggplant-shaped flask, to which were added compound 31-0 (6.4 mg, 19 µmol), ultra-dehydrated toluene (0.943 ml) and ultra-dehydrated THF (0.471 ml), and the mixture was stirred at 60°C for 7 h under nitrogen atmosphere. After removing the stirrer bar and distilling off the solvent, the residue was purified on a silica gel column Q-pack SI30 size 10 (chloroform:methanol = 100:0 to 80:20). After distilling off the solvent, 7.0 mg (14.1 µmol, 75%) of the iminohydrazine intermediate (yellowish black-brown solid) was obtained. Pre-dried MS4Å (70 mg) was added to the intermediate, which was then dissolved in ultra-dehydrated toluene (1.41 ml) and dioxane (0.71 ml). The mixture was stirred at 100°C for 22 hours with a Dimroth condenser attached. After removing MS4Å by cotton plug filtration and distilling off the solvent, the resulting residue was purified on a silica gel column Q-pack SI30 size 10 (chloroform:methanol = 100:0 to 90:10). After distilling off the solvent, 3.1 mg (6.49 µmol, 46%) of the desired compound 28AH (light brown solid) was obtained.

### Example 124

### Methyl (S)-2-(1-(cyclohexylsulfonyl)piperidin-2-yl)-5-methyloxazole-4-carboxylate 32B

### Example 124-1: Methyl (S)-2-((S)-1-(cyclohexylsulfonyl) piperidine-2-carboxamido)-3-oxobutanoate 32-1

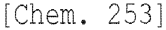

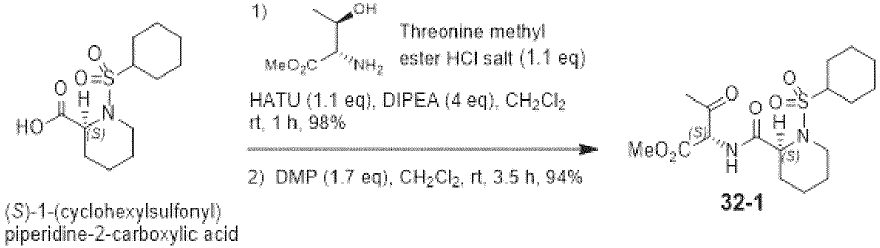

(S)-1-(cyclohexylsulfonyl)piperidine-2-carboxylic acid (50 mg, 0.182 mmol) and threonine methyl ester hydrochloride (34.9 mg, 0.200 mmol) were added to a 10 ml eggplant-shaped flask and were dissolved in dichloromethane (0.471 ml). Diisopropylethylamine (0.127 ml, 0.726 mmol) and HATU (76.0 mg, 0.200 mmol) were then added thereto, and the mixture was stirred for 1 hour at room temperature under a nitrogen atmosphere. After distilling off the solvent, the residue was purified on a silica gel column Q-pack SI30 size 10 (hexane:ethyl acetate = 80:20 to 0:100). After distilling off the solvent, 69.5 mg (0.178 mmol, 98%) of the amide intermediate (amorphous solid) was obtained. The intermediate (68 mg, 0.174 mmol) was dissolved in dichloromethane (2.47 ml), Dess-Martin reagent (132.2 mg, 0.312 mmol) was added thereto, and the mixture was stirred at room temperature for 3 h 30 minutes. The reaction was quenched by adding saturated sodium bicarbonate aqueous solution and the mixture was extracted with ethyl acetate. The solvent was distilled off and the resulting residue was purified on a silica gel column Q-pack SI30 size 10 (hexane:ethyl acetate = 100:0 to 50:50). After distilling off the solvent, 63.7 mg (0.164 mmol, 94%) of the desired compound 32-1 (amorphous solid) was obtained.

### Example 124-2: Methyl (S)-2-(1-(cyclohexylsulfonyl) piperidin-2-yl)-5-methyloxazole-4-carboxylate 32B

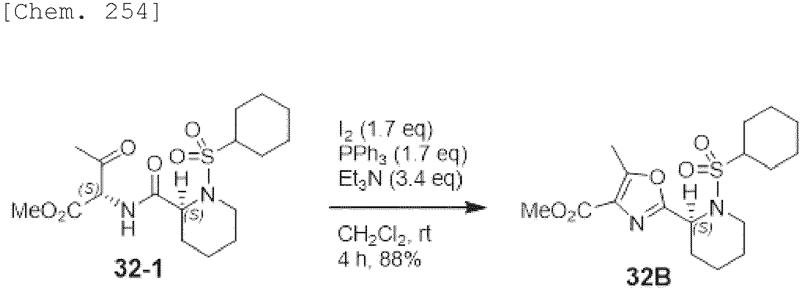

Triphenylphosphine (16.4 mg, 60 µmol) and iodine (15.4 mg, 60 µmol) were added to a 10 ml eggplant-shaped flask and were dissolved in dichloromethane (0.567 ml). Triethylamine (16.5 µl, 0.119 mmol) was then slowly added thereto, and the amide 32-1 (13.6 mg, 0.35 mmol) prepared in Example 124-1 was added while washing with dichloromethane (0.60 ml), followed by stirring the mixture at room temperature under an argon atmosphere for 4 hours. Saturated sodium thiosulfate aqueous solution and saturated sodium bicarbonate aqueous solution were added thereto and the mixture was extracted with ethyl acetate. After distilling off the solvent, the residue was purified on a silica gel column Q-pack SI30 size 10 (hexane:ethyl acetate = 100:0 to 66:34). After distilling off the solvent, 11.5 mg (31 µmol, 88%) of the desired compound 32B (amorphous solid) was obtained.

### Example 125

### (S) -2-(1-(Cyclohexylsulfonyl)piperidin-2-yl)-5-methyl oxazole-4-carboxylic acid 33B

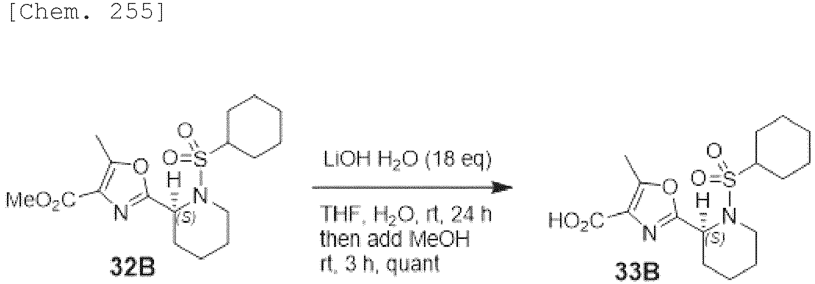

Compound 32B (8.5 mg, 23 µmol) prepared in Example 124 was added to a 10 ml eggplant-shaped flask and was dissolved in tetrahydrofuran (0.671 ml) and water (63 µl). Lithium hydroxide monohydrate (17.4 mg, 0.414 mmol) was added thereto, and the mixture was stirred at room temperature for 24 hours. Methanol (0.189 ml) was then added thereto, and the mixture was stirred for another 3 hours. The solution was made acidic (pH about 4) by adding 1M hydrochloric acid aqueous solution and the mixture was extracted with dichloromethane. After filtration of the organic layer with a phase separator (3 ml), the solvent was distilled off to afford 8.2 mg (23 µmol, 100%) of the desired compound 33B (amorphous solid).

### Example 126

### (S) -N-Benzyl-2-(1-(cyclohexylsulfonyl)piperidin-2-yl)-5-methyloxazole-4-carboxamide 34B

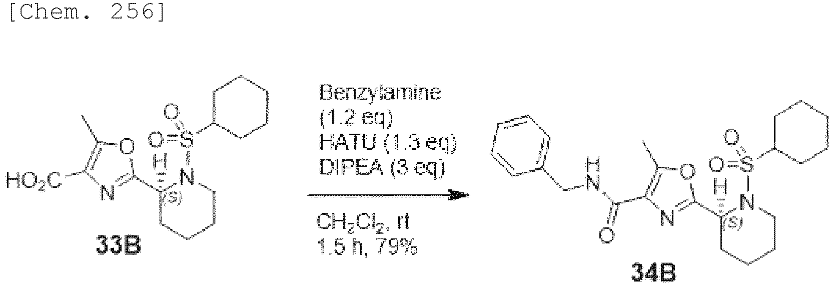

Compound 33B (6.4 mg, 18 µmol) prepared in Example 125 was added to a 10 ml eggplant-shaped flask and was dissolved in dichloromethane (0.718 ml). Diisopropylethylamine (9.4 µl, 54 µmol) and HATU (8.9 mg, 23.3 µmol) were then added thereto, and the mixture was stirred at room temperature for 1 hour 30 min under argon atmosphere. After distilling off the solvent, the residue was purified on a silica gel column Q-pack SI30 size 10 (hexane:ethyl acetate = 100:00 to 66:34) . After distilling off the solvent, 6.3 mg (14.1 µmol, 79%) of the desired compound 34B (white solid) was obtained.

### Example 127

### Methyl (S)-2-(1-(cyclohexylsulfonyl)piperidin-2-yl)-5-methylthiazole-4-carboxylate 35B

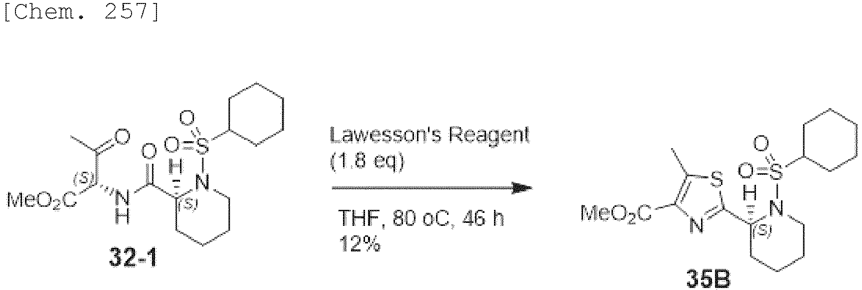

Compound 32-1 (14 mg, 36 µmol) prepared in Example 124-1 was added to a 10 ml eggplant-shaped flask, and then Lawson's reagent (27.6 mg, 68 µmol) was added while washing with tetrahydrofuran (1.2 ml), followed by stirring the mixture at 80°C for 46 h under argon atmosphere. After distilling off the solvent, the residue was purified on a silica gel column Q-pack SI30 size 10 (hexane:ethyl acetate = 100:0 to 60:40). After distilling off the solvent, 1.7 mg (4.4 µmol, 12%) of the desired compound 35B (amorphous solid) was obtained.

### Example 128

### (S) -2-(1-(Cyclohexylsulfonyl)piperidin-2-yl)-5-methyl thiazole-4-carboxylic acid 36B

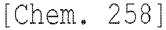

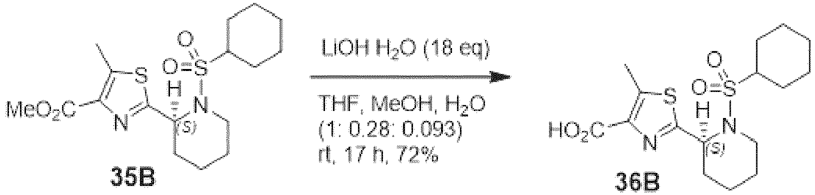

Compound 35B (11.1 mg, 29 µmol) prepared in Example 127 was added to a 10 ml eggplant-shaped flask and was dissolved in tetrahydrofuran (0.84 ml), methanol (0.235 ml) and water (78 µl). Lithium hydroxide monohydrate (21.7 mg, 0.517 mmol) was added thereto, and the mixture was stirred at room temperature for 17 hours. The solution was made acidic (about pH 4) by adding 1M hydrochloric acid aqueous solution and the mixture was extracted with chloroform. After filtration of the organic layer with a phase separator (3 ml), the solvent was distilled off to afford 7.7 mg (23.5 µmol, 72%) of the desired compound 36B (amorphous solid).

### Example 129

### (S) -N-Benzyl-2-(1-(cyclohexylsulfonyl)piperidin-2-yl)-5-methylthiazole-4-carboxamide 37B

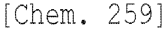

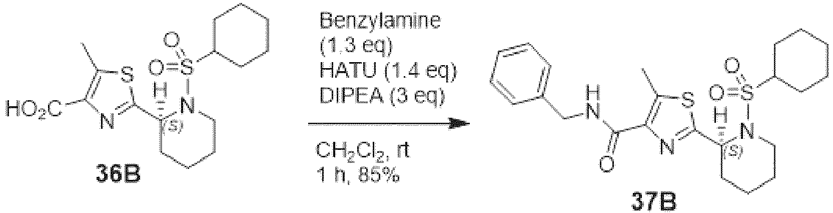

Compound 36B (5.5 mg, 15 µmol) prepared in Example 128 was added to a 10 ml eggplant-shaped flask and was dissolved in dichloromethane (0.591 ml). Diisopropylethylamine (7.7 µl, 44 µmol) and HATU (7.9 mg, 20.7 µmol) were then added thereto, and the mixture was stirred at room temperature under argon atmosphere for 1 hour. After distilling off the solvent, the residue was purified on a silica gel column Q-pack SI30 size 10 (hexane:ethyl acetate = 100:00 to 66:34). After distilling off the solvent, 5.8 mg (12.6 µmol, 85%) of the desired compound 37B (white solid) was obtained.

Physical property data for the compounds prepared in Examples 120-129 are shown in Table 23.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acid-acetonitrile;
0-0.9 min linear gradient A:B (95:5)-A:B (10:90), 0.9-3 min A:B (10:90)

**Table 23**

| [Table 65] | | | | | | |
|---|---|---|---|---|---|---|
| Ex | Cn | Chemical Structure | MW | ¹H-NMR 8 ppm | [M+H] | RT :Min |
| 120 | 29B | 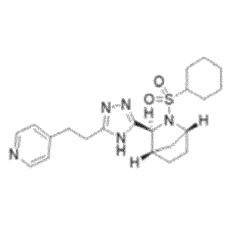 | 415.56 | (CDCl3) δ: 8.48 (2H. d, J = 4.4 Hz), 7.14 (2H. d, J - 6.0 Hz), 4.63 (1H, s), 4.12 (1H, s). 3.08 (4H. br-s), 2.93 (1H, d, J = 3.6 Hz), 2.74 (1H, tt, J = 12.0, 3.2 Hz), 2.32-2.25 (2H, m), 2.11 (1H, br-d, J = 12.8 Hz), 1.93 (1H. br-d, J = 12.4 Hz). 1.87-1.09 (12H, m) | 416 | 0.57 |
| 121 | 308 | 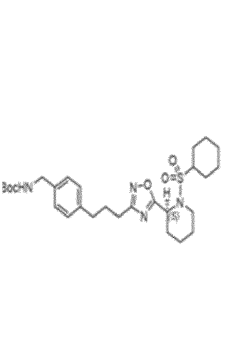 | 546.73 | (CDCl3) δ: 7.21 (2H, d, J = 8.0 Hz), 7.15 (2H, d. J = 8.0 Hz), 5.33 (1H, d, J = 4.8 Hz), 4.82 (1H, br-s), 4.29 (1H, d, J = 5.6 Hz), 3.81 (1H, d, J = 13.2 Hz), 3.26-3.18 (1H, m), 2.95 (1H. tt, J = 12.4. 3.2 Hz), 2.76 (2H, t, J = 7.6 Hz), 2.69 (2H, t, J = 7.6 Hz). 2.29 (1H, dd, J = 14.0, 2.4 Hz), 2.19-1.96 (6H, m), 1.90-1.12 (12H, m) | 547 | 1.88 |
| 122 | 24AH | 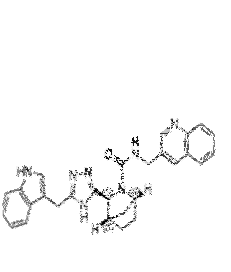 | 477.57 | (CDCl3) δ: 8.57 (1H. br-s), 8.07 (1H, m), 7.89 (1H, s). 7.65-7.52 (2H. m), 7.46-7.31 (2H, m), 7.21 (1H. br-s), 6.90-6.63 (3H, m), 4.76 (1H, m), 4.49 (2H, br-s), 4.34 (1H, d, J = 17.2 Hz), 4.24 {1H, m), 4.12 (1H, d. J = 17.2 Hz), 3.18 (1H. br-s), 2.48 (1H, br-s), 2.00-1.23 (6H. m), 0.91-0.77 (1H, m) | 478 | 1.24 |

**Table 23 continued**

| [Table 66] | | | | | | |
|---|---|---|---|---|---|---|
| 123 | 28AH | 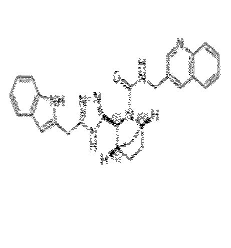 | 477.57 | (CDCl3) δ: 9.74 (1H, br-s), 8.65 (1H, br-s). 8.02 (1H, d, J = 8.4 Hz), 7.90 (1H, s), 7.68-7.61 (2H, m), 7.51-7.45 (2H, m), 7.29-7.24 (1H, m), 7.10-6.99 (2H, m), 6.34 (1H, s), 6.00-5.53 <1H. br-s), 4.62-4.08 (6H. m). 2.96 (1H, s), 2.03 (1H, br-d, J = 10.0 Hz). 1.88-1.53 (4H, m). 1.42 (1H. d, J = 9.6 Hz), 0.90-0.78 (1H, m) | 478 | 1.28 |
| 124 | 32B | 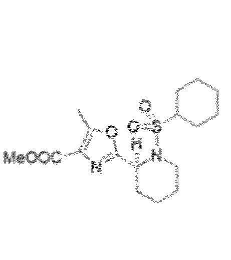 | 370.46 | (CDCl3) δ: 5.18 (1H, br-d, J = 5.2 Hz), 3.90 (3H, s), 3.79 (1H, br-d, J = 13.6 Hz), 3.16 (1H, td, J = 13.2, 2.4 Hz), 2.99 (1H, tt, J = 12.0, 3.6 Hz), 2.63 (3H, s), 2.35 (1H, br-d, J = 12.4 Hz), 2.20 (2H, br-t, J = 11.6 Hz), 2.00-1.15 (13H, m) | 371 | 1.7 |
| 125 | 33B | 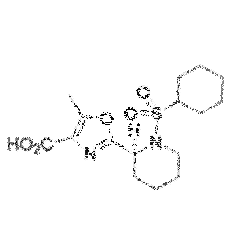 | 356.44 | (CDCl3) δ: 5.18 (1H, br-d, J = 4.8 Hz), 3.77 (1H, br-d, J = 13.6 Hz), 3.19 (1H, td, J = 13.2, 2.0 Hz), 2.97 (1H, tt, J = 12.0. 3.6 Hz), 2.66 (3H, s), 2.34 (1H. br-d, J = 13.6 Hz), 2.19 (2H, br-d, J = 12.4 Hz), 2.02-1.13 (13H, m) | 357 | 1.56 |
| 126 | 34B | 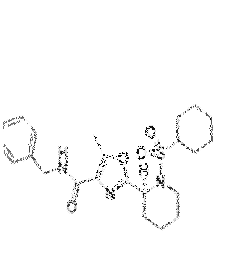 | 445.58 | (CDCl3) δ: 7.37-7.26 (5H, m), 7.18 (1H, br-t, J = 5.2 Hz), 5.14 (1H. d, J = 4.8 Hz), 4.59 (2H. m), 3.77 (1H, br-d, J = 14.4 Hz). 3.19 (1H, ddd, J = 13.6, 11.6, 3.6 Hz), 2.91 (1H. tt, J = 12.0, 3.2 Hz), 2.67 (3H, s), 2.25 (1H, dd, J = 13.6, 2.4 Hz). 2.14 (2H, br-t, J = 14.4 Hz), 2.00-1.03 <13H. m) | 446 | 1.76 |

**Table 23 continued 2**

| [Table 67] | | | | | | |
|---|---|---|---|---|---|---|
| 127 | 35B | 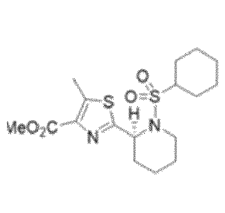 | 386.53 | (CDCl3) δ: 5.24 (1H, br-d, J = 4.8 Hz), 3.91 (3H, s), 3.76 (1H, br-d, J = 13.6 Hz). 3.27-3.18 (1H, m), 2.97 (1H, tt, J = 12.4, 3.2 Hz), 2.76 (3H, s), 2.56 (1H, br-d, J = 12.8 Hz), 2.24 (2H, m), 2.02-1.19 (13H, m) | 387 | 1.76 |
| 128 | 36B | 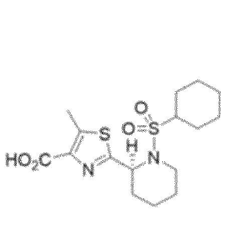 | 372.50 | (CDCl3) δ: 5.20 (1H, br-d, J = 4.8 Hz), 3.71 (1H. br-d, J = 15.2 Hz), 3.15 (1H, ddd, J = 15.2, 12.0. 3.6 Hz), 2.95 (1H, tt, J = 12.0, 3.2 Hz), 2.81 (3H, s), 2.51 (1H, br-d, J = 14.0 Hz), 2.22 (2H, br-t, J = 12.8 Hz), 2.04-1.16 (13H, m) | 373 | 1.61 |
| 129 | 37B | 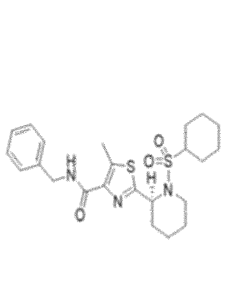 | 461.64 | (CDCl3) δ: 7.71 (1H. br-t, J = 5.6 Hz), 7.37-7.26 (5H, m), 5.18 (1H, d, J = 4.8 Hz), 4.65 (1H, dd, J = 14.8, 6.4 Hz), 4.58 (1H. dd, J = 15.2, 6.0 Hz), 3.70 (1H, br-d, J = 14.8 Hz), 3.17 (1H, ddd, J = 14.4, 10.8, 4.4 Hz), 2.91 (1H. tt, J = 12.0, 3.2 Hz), 2.85 (3H, s), 2.43 (1H, br-d, J = 14.0 Hz), 2.18 (2H, m), 2.02-1.12 (13H, m) | 462 | 1.83 |

| | | | | | | |
|---|---|---|---|---|---|---|
| Ex: Example Number Cn: Compound Number MW: Molecular Weight | | | | | | |

### Example 130

### Methyl (S)-2-(1-(cyclohexylsulfonyl)piperidin-2-yl)-5-methyl-1H-Imidazole-4-carboxylate 38B

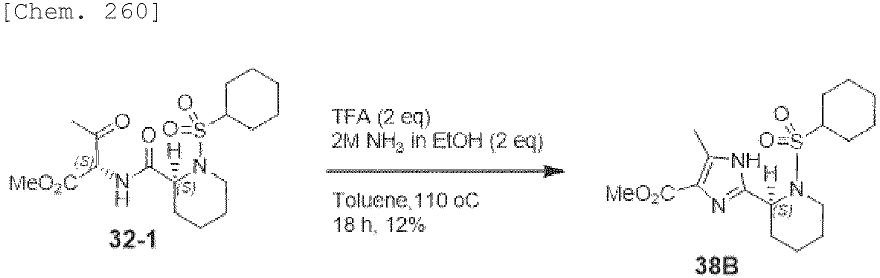

Compound 32-1 (22.1 mg, 57 µmol) was added to a 10 ml eggplant-shaped flask, and toluene (0.25 ml) was added thereto, followed by conducting an azeotropic distillation once. Toluene (0.948 ml), trifluoroacetic acid (8.7 ul, 0.114 mmol) and an ethanol solution of 2M ammonia (57 µl, 0.114 mmol) were then added thereto, and the mixture was stirred at 110°C for 18 h under an argon atmosphere. After the solvent was distilled off, saturated sodium bicarbonate aqueous solution was added and the mixture was extracted with ethyl acetate. After distilling off the solvent, the residue was purified on a silica gel column Q-pack SI30 size 10 (hexane:ethyl acetate = 100:0 to 40:60). After distilling off the solvent, 2.4 mg (6.5 µmol, 12%) of the desired compound 38B (amorphous solid) was obtained.

### Reference Example 1

### Tert-butyl (S)-2-(1-benzyl-1H-1,2,3-triazol-4-yl) pyrrolidine-1-carboxylate 39-1

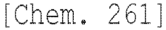

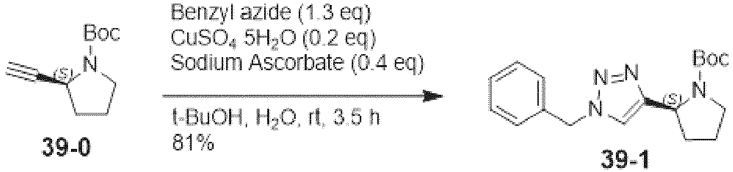

Tert-butyl (S)-2-ethynylpyrrolidine-1-carboxylate 39-0 (32.3 mg, 0.165 mmol) was added to a 10 ml eggplant-shaped flask, and was dissolved in t-butanol (2.363 ml) and water (0.551 ml). Benzyl azide (28.6 ul, 0.215 mmol), sodium ascorbate (13.4 mg, 66 µmol) and copper sulfate (8.3 mg, 33 µmol) were then added sequentially thereto, and the mixture was stirred at room temperature for 3 hours and 30 minutes under an argon atmosphere. After the solvent was distilled off, saturated sodium bicarbonate aqueous solution was added and the mixture was extracted with chloroform. After distilling off the solvent, the residue was purified on a silica gel column Q-pack SI30 size 10 (hexane:ethyl acetate = 100:0 to 50:50). After distilling off the solvent, 44 mg (0.134 mmol, 81%) of the desired compound 39-1 (amorphous solid) was obtained.

### Example 131

### (S)-1-Benzyl-4-(1-((4-fluorophenyl)sulfonyl)pyrrolidin-2-yl)-1H-1,2,3-triazole 39AJ

### Example 131-1: (S)-1-Benzyl-4-(pyrrolidin-2-yl)-1H-1,2,3-triazole 39-2

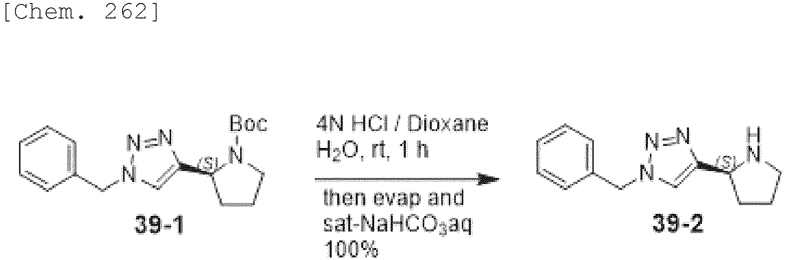

Compound 39-1 (38.6 mg, 0.118 mmol) was added to a 10 ml eggplant-shaped flask, and 4 N HCl/dioxane (1.18 ml) and water (0.118 ml) were added thereto, followed by stirring the mixture at room temperature for 1 hour. After removing the stirrer bar and distilling off the solvent, saturated sodium bicarbonate aqueous solution was added to make it basic, and it was extracted with chloroform. After drying over magnesium sulfate, which was then filtered off, distillation of the solvent afforded 27.3 mg (0.118 mmol, 100%) of the desired compound 39-2 (white solid).

### Example 131-2: (S)-1-Benzyl-4-(1-((4-fluorophenyl) sulfonyl)pyrrolidin-2-yl)-1H-1,2,3-triazole 39AJ

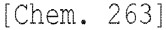

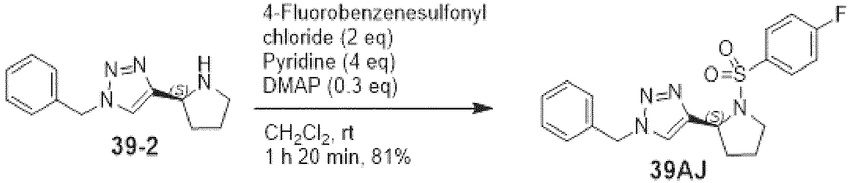

Compound 39-2 (8.5 mg, 37.2 µmol) was added to a 10 ml eggplant-shaped flask while washing with dichloromethane (0.745 ml), then pyridine (12µl, 0149 mmol) and DMAP (1.4 mg, 11 µmol) were added, and finally 4-fluorobenzenesulfonyl chloride (14.6 mg, 74 µmol) was added thereto, followed by stirring the mixture at room temperature for 1 hour 20 minutes. Methanol (20 µl) was added thereto and the mixture was stirred for 5 minutes to quench the reaction. After distilling off the solvent, saturated sodium bicarbonate aqueous solution and saturated brine were added thereto, and the mixture was extracted with ethyl acetate. After distilling off the solvent, the residue was purified on a silica gel column Q-pack SI30 size 10 (hexane:ethyl acetate = 100:0 to 50:50). After distilling off the solvent, 11.6 mg (30 µmol, 81%) of the desired compound 39AJ (amorphous solid) was obtained.

### Example 132

### S)-1-(cyclohexylsulfonyl)-2-(5-phenyl-1H-imidazol-2-yl) piperidine 40B

### Example 132-1: (S)-1-(cyclohexylsulfonyl)-N-(2-oxo-2-phenyl ethyl)piperidine-2-carboxamide 40-1

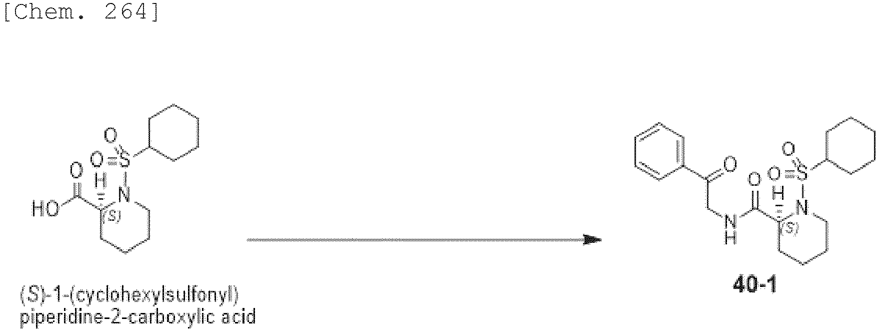

(S)-1-(cyclohexylsulfonyl)piperidine-2-carboxylic acid (50 mg, 0.182 mmol) and 2-amino-1-(phenyl)ethanone hydrochloride (36.1 mg, 0.200 mmol) were added to a 10 ml eggplant-shaped flask and were dissolved in dichloromethane (1.82 ml). Diisopropylethylamine (0.127 ml, 0.726 mmol) and HATU (76.0 mg, 0.200 mmol) were then added thereto, and the mixture was stirred for 1 hour at room temperature under a nitrogen atmosphere. After distilling off the solvent, the residue was purified on a silica gel column Q-pack SI30 size 10 (hexane:ethyl acetate = 80:20 to 50:50). After distilling off the solvent, 53.6 mg (0.137 mmol, 75%) of the desired compound 40-1 (amorphous solid) was obtained.

### Example 132-2: S)-1-(cyclohexylsulfonyl)-2-(5-phenyl-1H-imidazol-2-yl)piperidine 40B

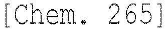

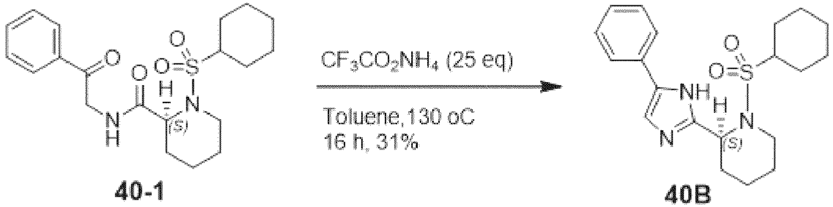

Compound 40-1 (8.4 mg, 21 µmol) was added to a 10 ml eggplant-shaped flask, and toluene (0.2 ml) was added thereto, followed by conducting an azeotropic distillation once. Toluene (1.07 ml) and ammonium trifluoroacetate (70.1 mg, 0.535 mmol) separately prepared were then added thereto and the mixture was heated to reflux at 130°C for 16 hours under an argon atmosphere. After distilling off the solvent, water and saturated brine were added thereto and the mixture was extracted with ethyl acetate. After distilling off the solvent, the residue was purified on a silica gel column Q-pack SI30 size 10 (hexane:ethyl acetate = 100:0 to 60:40). After distilling off the solvent, 2.5 mg (6.7 µmol, 31%) of the desired compound 40B (amorphous solid) was obtained.

### Example 133

### Tert-butyl (2S,4S)-4-cyano-2-(3-(3-phenylpropyl)-1,2,4-oxadiazol-5-yl)pyrrolidine-1-carboxylate 41-1

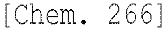

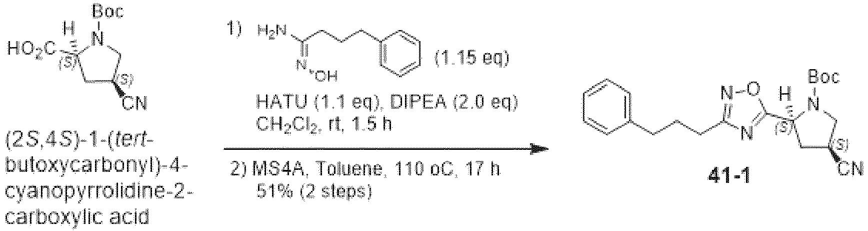

1-Boc-4-cyanopyrrolidine-2-carboxylic acid (92 mg, 0.383 mmol) was added to a 25 ml eggplant-shaped flask and was dissolved in dichloromethane (1.829 ml). HATU (160.2 mg, 0.421 mmol) and diisopropylethylamine (0.133 ml, 0.766 mmol) were then added thereto, and the mixture was stirred for 5 minutes at room temperature under a nitrogen atmosphere. N'-hydroxy -4-phenylbutanimidamide (78.5 mg, 0.44 mmol) was then added while washing with dichloromethane (2.0 ml), and the mixture was stirred at room temperature for 1.5 hours. After removing the stirrer bar and distilling off the solvent, the residue was purified on silica gel column Q-pack SI30 size 20 (hexane:ethyl acetate = 100:0 to 34:66) . After distilling off the solvent, the imidamide intermediate was obtained as a mixture with tetramethylurea. Pre-dried MS4Å (666 mg) was added to the intermediate, which was then dissolved in ultra-dehydrated toluene (3.33 ml). The mixture was stirred at 110°C for 17 hours with a Dimroth condenser attached. After removing MS4Å by celite filtration and distilling off the solvent, the resulting residue was purified on silica gel column Q-pack SI30 size 20 (hexane:ethyl acetate = 80:20 to 34:66). After distilling off the solvent, 75.4 mg (0.197 mmol, 51% (after 2 steps)) of the desired compound 41-1 (oil) was obtained.

### Example 134

### (3S,5S)-1-((4-fluorophenyl)sulfonyl)-5-(3-(3-phenylpropyl) -1,2,4-oxadiazol-5-yl)pyrrolidine-3-carbonitrile 41AJ Example 135

### (3S,5S)-1-((4-fluorophenyl)sulfonyl)-5-(3-(3-phenylpropyl)-1,2,4-oxadiazol-5-yl)pyrrolidine-3-carboxamide 42AJ

### Example 134-1: (3S,5S)-5-(3-(3-phenylpropyl)-1,2,4-oxadiazol-5-yl)pyrrolidine-3-carbonitrile 41-2

### Example 135-1: (3S,5S)-5-(3-(3-phenylpropyl)-1,2,4-oxadiazol-5-yl)pyrrolidine-3-carboxamide 42-2

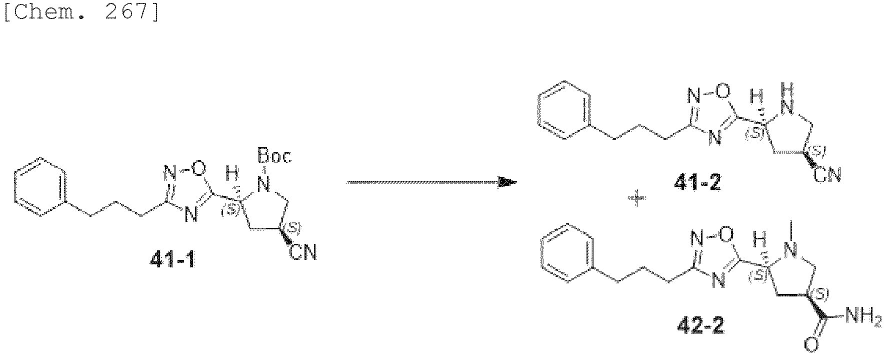

Compound 41-1 (38.9 mg, 0.102 mmol) was added to a 10 ml eggplant-shaped flask, and 4N hydrochloric acid/dioxane (1.02 ml) and water (0.1 ml) were added thereto, followed by stirring the mixture at room temperature for 1 hour. After removing the stirrer bar and distilling off the solvent, then saturated sodium bicarbonate aqueous solution was added thereto and the mixture was extracted with chloroform. The organic layer was dried over magnesium sulfate, which was then filtered out, and the solvent was distilled off to afford 24.4 mg (86 µmol, about 85%) of a mixture of the desired compounds 41-2 and 42-2 (oil).

### Example 134-2: (3S,5S)-1-((4-fluorophenyl)sulfonyl)-5-(3-(3-phenylpropyl)-1,2,4-oxadiazol-5-yl)pyrrolidine-3-carbonitrile 41AJ

### Example 135-2: (3S,5S)-1-((4-fluorophenyl)sulfonyl)-5-(3-(3-phenylpropyl)-1,2,4-oxadiazol-5-yl)pyrrolidine-3-carboxamide 42AJ

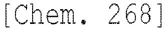

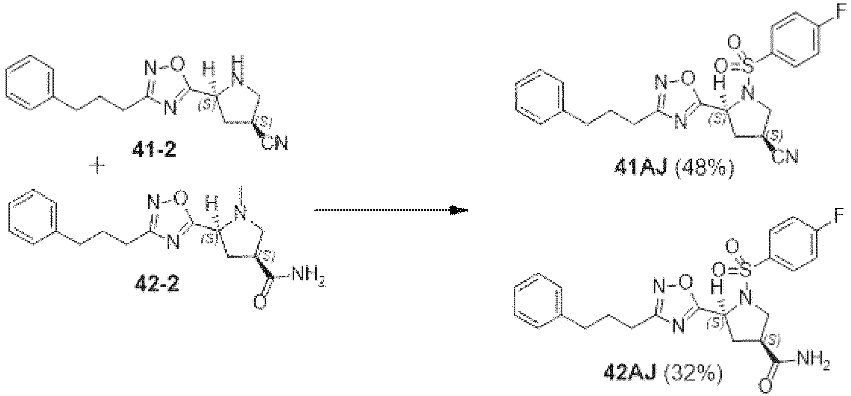

A mixture of compounds 41-2 and 42-2 (11.5 mg, 40.7 µmol) was added to a 10 ml eggplant-shaped flask while washing with dichloromethane (0.718 ml), then pyridine (26.2 µl, 0.326 mmol) and DMAP (1.5 mg, 12 µmol) were added sequentially, and finally 4-fluorobenzenesulfonylchloride (24.0 mg, 0.122 mmol) was added while washing with dichloromethane (0.6 ml), followed by stirring the mixture at room temperature for 1 hour 30 minutes. Methanol (40 µl) was added thereto and the mixture was stirred for 5 minutes to quench the reaction. After distilling off the solvent, saturated sodium bicarbonate aqueous solution and saturated brine were added thereto and the mixture was extracted with ethyl acetate. After distilling off the solvent, the residue was purified on a silica gel column Q-pack SI30 size 10 (hexane:ethyl acetate = 100:0 to 0:100). After distilling off the solvent, 8.6 mg (19.5 µmol, 48%) of the desired compound 41AJ (oil) and 5.9 mg (12.9 µmol, 32%) of 42AJ (white solid) were obtained.

### Example 136

### (2S,4S)-N,4-di phenyl-2-(3-(3-phenylpropyl)-1,2,4-oxadiazol -5-yl)pyrrolidine-1-carbothioamide 7AQ

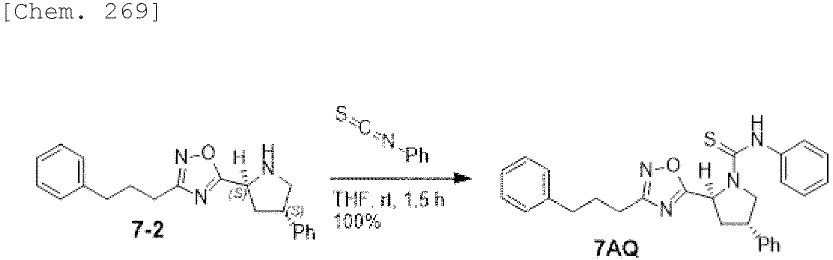

Compound 7-2 (8.5 mg, 25 µmol) was added to a 4 ml vial, and THF (0.34 ml) and phenylisothiocyanate (4.6 µl, 38 µmol) were added sequentially, followed by stirring the mixture at room temperature for 1 hour 30 minutes. Water was added thereto and the mixture was extracted with ethyl acetate. After distilling off the solvent, the residue was purified on a silica gel column Q-pack SI20 size 10 (hexane:ethyl acetate = 100:0 to 66:34). After distilling off the solvent, 11.9 mg (25 µmol, 100%) of the desired compound 7AQ (amorphous solid) was obtained.

### Example 137

### (2S,4S)-N-Benzyl-4-phenyl-2-(3-(3-phenylpropyl)-1,2,4-oxadiazol-5-yl)pyrrolidine-1-carbothioamide 7AR

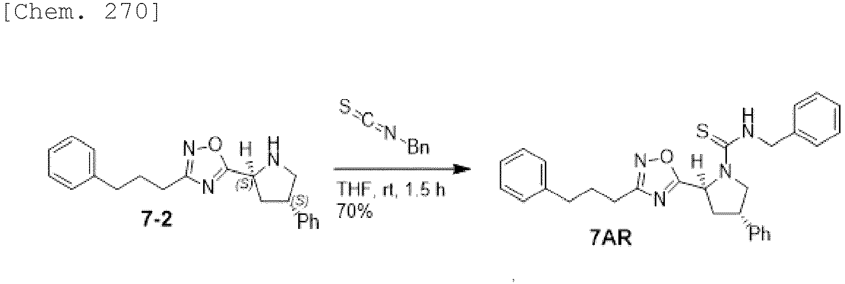

Compound 7-2 (8.3 mg, 25 µmol) was added to a 4 ml vial, and THF (0.33 ml) and benzylisothiocyanate (4.0 µl, 30 µmol) were added sequentially, followed by stirring the mixture at room temperature for 1 hour 30 minutes. Water was added thereto and the mixture was extracted with ethyl acetate. After distilling off the solvent, the residue was purified on a silica gel column Q-pack SI20 size 10 (hexane:ethyl acetate = 100:0 to 66:34). After distilling off the solvent, 8.4 mg (17.4 µmol, 70%) of the desired compound 7AR (amorphous solid) was obtained.

### Example 138

### Tert-butyl ((5-((2S,4S)-1-((4-fluorophenyl)sulfonyl)-4-phenylpyrrolidin-2-yl)-1,2,4-oxadiazol-3-yl)methyl) carbamate 43AJ

### Example 138-1: (9h-Fluorene-9-yl)Methyl (2S,4S)-2-(3-(tert-butoxycarbonyl)amino)methyl)-1,2,4-oxadiazol-5-yl)-4-phenyl pyrrolidine-1-carboxylate 43-1

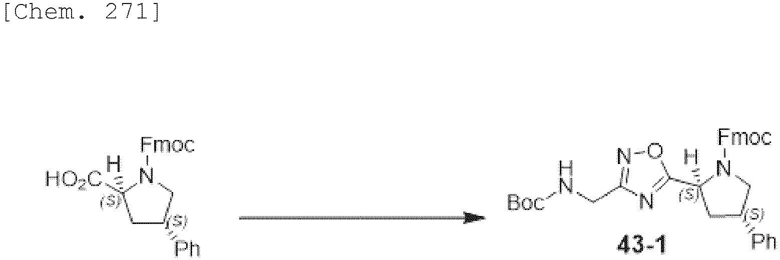

1-Fmoc-4-phenylpyrrolidine-2-carboxylic acid (131 mg, 0.317 mmol) was added to a 25 ml eggplant-shaped flask and was dissolved in dichloromethane (1.97 ml). HATU (144.6 mg, 0.380 mmol) and diisopropylethylamine (0.110 ml, 0.634 mmol) were then added thereto, and the mixture was stirred for 5 minutes at room temperature under argon atmosphere. Then, tert-butyl (n-hydroxycarbamimidoylmethyl) carbamate (68.7 mg, 0.349 mmol) was added while washing with dichloromethane (1.2 ml), and the mixture was stirred at room temperature for 1.5 hours. After distilling off the solvent, the residue was purified on a silica gel column Q-pack SI30 size 20 (hexane:ethyl acetate = 80:20 to 40:60). After distilling off the solvent, 138.8 mg (0.237 mmol, 75%) of the imidamide intermediate (white solid) was obtained. This intermediate (84.5 mg, 0.145 mmol) was added with pre-dried MS4Å (423 mg) and was dissolved in ultra-dehydrated toluene (2.89 ml). The mixture was stirred at 110°C for 14 hours with a Dimroth condenser attached. After removing MS4Å by celite filtration and distilling off the solvent, the resulting residue was purified on silica gel column Q-pack SI30 size 20 (hexane:ethyl acetate = 85:15 to 50:50). After distilling off the solvent, 12.1 mg (21.4 µmol, 15%) of the desired compound 43-1 (oil) was obtained.

### Example 138-2: tert-butyl ((5-((2S,4S)-1-((4-fluorophenyl) sulfonyl)-4-phenylpyrrolidin-2-yl)-1,2,4-oxadiazol-3-yl) methyl)carbamate 43AJ

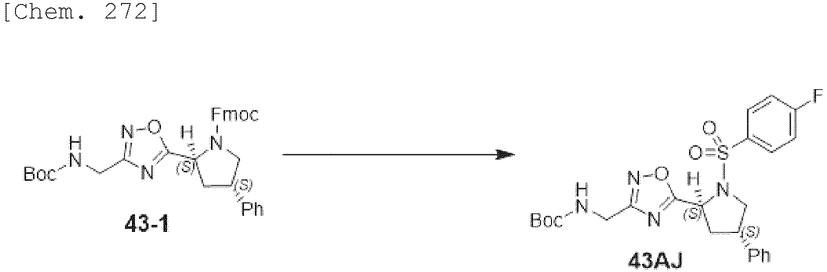

Compound 43-1 (17.4 mg, 31 µmol) was added to a 10 ml eggplant-shaped flask, and dichloromethane (0.61 ml) and piperidine (47µl, 0.461 mmol) were added thereto, followed by stirring the mixture at room temperature for 1 hour 30 minutes. After removing the stirrer bar and distilling off the solvent, the residue was then purified on a silica gel column Q-pack SI30 size 10 (hexane:ethyl acetate = 50:50, and chloroform:methanol = 100:0 to 95:5). After distilling off the solvent, 9.8 mg (28.5 µmol, 92%) of the N-free intermediate (colorless, amorphous solid) was obtained. This intermediate was then added to a 10 ml eggplant-shaped flask while washing with dichloromethane (0.213 ml), then pyridine (18.4 µl, 0.228 mmol) and DMAP (1.0 mg, 9 µmol) were added, and finally 4-fluorobenzenesulfonyl chloride (22.4 mg, 0.114 mmol) was added while washing with dichloromethane (0.6 ml), followed by stirring the mixture at room temperature for 1 hour. Methanol (30 µl) was added thereto and the mixture was stirred for 5 minutes to quench the reaction. After distilling off the solvent, saturated sodium bicarbonate aqueous solution and saturated brine were added thereto and the mixture was extracted with ethyl acetate. After distilling off the solvent, the residue was purified on a silica gel column Q-pack SI30 size 10 (hexane:ethyl acetate = 100:0 to 66:34). After distilling off the solvent, 12.7 mg (25.3 µmol, 89%) of the desired compound 43AJ (white solid) was obtained.

### Example 139

### N-((5-((2S,4S)-1-((4-fluorophenyl)sulfonyl)-4-phenyl pyrrolidin-2-yl)-1,2,4-oxadiazol-3-yl)methyl) quinoline-2-carboxamide 45AJ

### Example 139-1: (5-((2S,4S)-1-((4-fluorophenyl)sulfonyl)-4-phenylpyrrolidin-2-yl)-1,2,4-oxadiazol-3-yl)methaneamine 44AJ

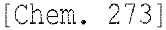

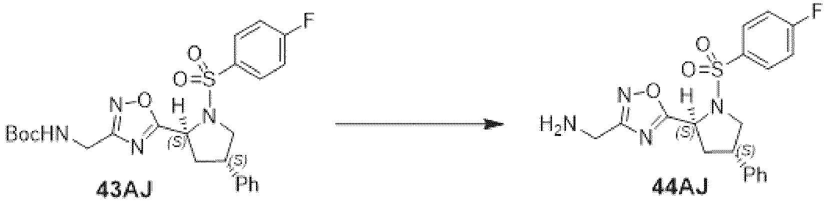

Compound 44AJ (11.0 mg, 21.9 µmol) was added to a 10 ml eggplant-shaped flask, and 4N hydrochloric acid/dioxane (0.292 ml) and water (29 µl) were added thereto, followed by stirring the mixture at room temperature for 1 hour. After removing the stirrer bar and distilling off the solvent, then saturated sodium bicarbonate aqueous solution was added thereto and the mixture was extracted with chloroform. The organic layer was dried over magnesium sulfate, which was then filtered out, and the solvent was distilled off to afford 8.7 mg (22 µmol, 100%) of the desired compound 44AJ (oil).

### Example 139-2: N-((5-((2S,4S)-1-((4-fluorophenyl)sulfonyl)-4-phenylpyrrolidin-2-yl)-1,2,4-oxadiazol-3-yl)methyl) quinoline-2-carboxamide 45AJ

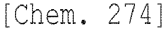

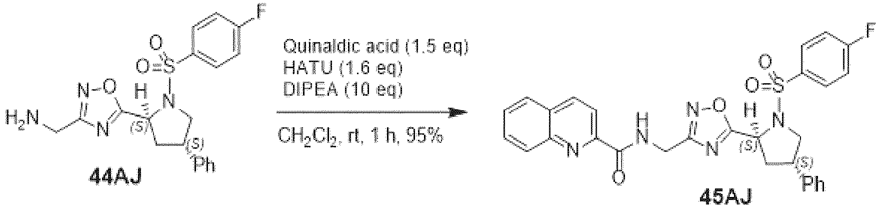

Compound 44AJ (4.0 mg, 9.9 µmol) was added to a 4 ml vial, then dichloromethane (0.398 ml), HATU (6.1 mg, 15.9 µmol) and 2-quinoline carboxylic acid (2.6 mg, 14.9 µmol) were added sequentially, and finally diisopropylethylamine (17.3 µl, 99 µmol) was added thereto, followed by stirring the mixture at room temperature for 1 hour. After distilling off the solvent, saturated sodium bicarbonate aqueous solution (0.5 ml) was added and the mixture was extracted with ethyl acetate (1 ml x 2) . The solvent was distilled off and the residue was purified on a silica gel column Q-pack SI30 size 10 (hexane:ethyl acetate = 90:10 to 50:50). After distilling off the solvent, 5.2 mg (9.3 µmol, 95%) of the desired compound 45AJ (white amorphous solid) was obtained.

### Example 140

### N-((5-((2S,4S)-1-((4-fluorophenyl)sulfonyl)-4-phenyl pyrrolidin-2-yl)-1,2,4-oxadiazol-3-yl)methyl)quinoline-3-carboxamide 46AJ

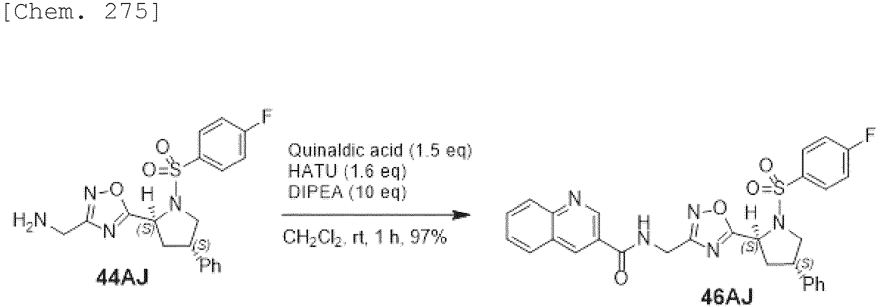

Compound 44AJ (4.7 mg, 11.7 µmol) was added to a 4 ml vial, then dichloromethane (0.467 ml), HATU (7.1 mg, 18.7 µmol) and 3-quinoline carboxylic acid (3.1 mg, 17.5 µmol) were added sequentially, and finally diisopropylethylamine (20.3 µl, 0.117 mmol) was added thereto, followed by stirring the mixture at room temperature for 1 hour. After distilling off the solvent, saturated sodium bicarbonate aqueous solution (0.5 ml) was added thereto and the mixture was extracted with ethyl acetate (1 ml x 2) . The solvent was distilled off and the residue was purified on a silica gel column Q-pack SI30 size 10 (hexane:ethyl acetate = 66:34 to 20:80). After distilling off the solvent, 6.3 mg (11.3 µmol, 97%) of the desired compound 46AJ (white amorphous solid) was obtained.

The compounds listed in Examples 130-140 above are shown in Table 24 along with their physical property data.

In the table, LC/MS elution conditions, Retention Times (RT) and [M+H] are shown under the following conditions.

### Elution Conditions:

Flow rate 0.9 mL/min, mobile phase A = 0.05% (v/v) formic acid solution, mobile phase B = 0.05% (v/v) formic acid-acetonitrile;
0-0.9 min linear gradient A:B (95:5)-A:B (10:90), 0.9-3 min A:B (10:90)

**Table 24**

| [Table 68] | | | | | | |
|---|---|---|---|---|---|---|
| Ex | Cn | Chemical Structure | MW | ¹H-NMR δ ppm | [M+H] | RT :Min |
| 130 | 38B | 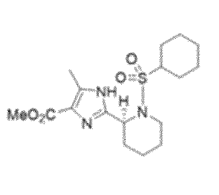 | 369.48 | (CDCl3) δ: 10.15-9.86 (1H. m), 4.94-4.85 (1H, m), 3.87 (3H, s), 3.61 (1H, br-d, J = 12.0 Hz). 2.96 (1H. tt, J = 12.0.3.2 Hz),2.90-2.70 (1H, m), 2.56-2.47 (3H, m), 2.22 (2H, br-d,J= 12.0 Hz), 2.10-1.16 (14H, m) | 370 | 1.51 |
| Re.Ex 1 | 39-1 | 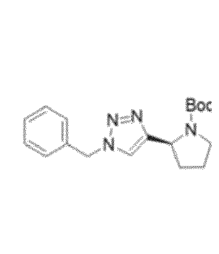 | 328.42 | (CDCl3) δ: 7.46-7.16 (5H, m), 5.58-5.41 (2H, m), 4.99 (0.42H. br-s), 4.95 (0.58H, br-s), 3.53-3.34 (2H, m), 2.43 (0.84H, br-s), 2.23 (1.16H, br-s), 2.13 (0.84H, br-s), 1.92 (1.16H, br-s), 1.42 (3.8H, br-s), 1.19 (5.2H, br-s) | 329 | 1.6 |
| 131 | 39AJ | 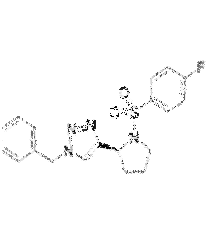 | 386.45 | (CDCl3) δ: 7.77 (2H. m). 7.57 (1H. s), 7.43-7.36 (3H, m), 7.32-7.27 (2H, m), 7.14 (2H, m), 5.49 (2H, m), 4.89 (1H, dd, J = 7.6. 3.2 Hz), 3.53 (1H. m), 3.28-3.20 (1H. m), 2.39-2.32 (1H, m). 2.00-1.72 (3H, m) | 387 | 1.6 |
| 132 | 408 | 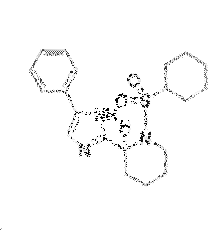 | 373.52 | (CDCl3) δ: 10.26 (0.45H, br-s), 9.86 (0.55H, br-s), 1.76 (1H, br-s), 7.65-7.20 (5H, m), 4.97 (1H. d, J = 4.8 Hz), 3.61 (1H, br-d. J = 12.8 Hz), 3.02-2.84 (2H, m), 2.72 (1H, br-d, J = 12.4 Hz), 2.30-1.15 (15H, m) | 374 | 1.41 |

**Table 24 continued**

| [Table 69] | | | | | | |
|---|---|---|---|---|---|---|
| 133 | 41-1 | 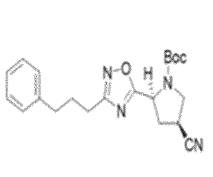 | 382.46 | (CDCl3) δ: 7.32-7.15 (5H, m), 5.21-5.02 (1H. m), 4.16-3.97 (1H, m), 3.78 (1H, dd, J = 11.2, 8.0 Hz), 3.21 (1H, m), 2.90-2.68 (5H, m), 2.56-2.43 (1H, m), 2.08 (2H, quin, J = 7.2 Hz), 1.45 (3.2H. s), 1.30 (5.8H. s) | 383 | 1.75 |
| 134 | 41AJ | 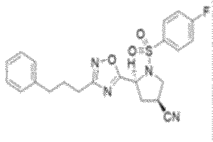 | 440.49 | (CDCl3) δ: 7.84 (2H, m), 7.33-7.13 (7H, m). 5.27 (1H. dd, J = 8.4, 2.0 Hz), 4.08 (1H, dd, J = 10.8, 7.6 Hz), 3.61 (1H, dd, J = 10.8, 8.4 Hz). 3.16 (1H, quin, J = 8.0 Hz), 2.86 (1H, m), 2.72-2.67 (4H, m), 2.56 (1H, ddd, J = 14.0. 8.4, 6.0 Hz), 2.03 (2H, quin, J - 8.0 Hz) | 441 | 1.72 |
| 135 | 42AJ | 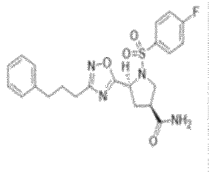 | 458.51 | (CDCl3) δ: 7.87-7.81 (2H, m), 7.33-7.12 (7H, m), 5.88 (1H, br-s), 5.43 (1H. br-s), 5.15 (1H, dd, J = 8.0, 7.2 Hz), 3.90 (1H, dd, J = 10.4, 8.0 Hz), 3.63 (1H, dd, J = 10.8, 8.8 Hz), 2.98 (1H, quin, J - 8.0 Hz), 2.74-2.65 (5H, m), 2.56-2.46 (1H, m), 2.03 (1H, quin, J = 7.6 Hz) | 459 | 1.61 |
| 136 | 7AQ | 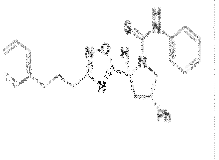 | 468.62 | (CDCl3) δ: 7.79 (1H, br-s), 7.43-7.15 (15H, m), 5.95 (1H, br-s), 4.29 (1H, dd, J = 9.6, 8.0 Hz), 3.92 (1H, m), 3.78 (1H, t, J - 10.0 Hz). 2.81 (2H. t, J = 7.6 Hz), 2.73 (2H, t. J = 7.6 Hz), 2.70-2.54 (2H, m), 2.13 (2H, quin, J = 7.6 Hz) | 469 | 1.83 |
| 137 | 7AR | 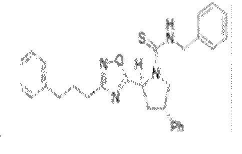 | 482.65 | (CDCl3) δ: 7.36-7.15 (15H, m), 5.96 (2H, br-s), 4.86 (1H, dd, J = 14.4, 5.2 Hz), 4.77 (1H, dd, J - 14.4, 4.8 Hz), 4.15 (1H, t. J = 8.8 Hz), | 483 | 1.87 |

**Table 24 continued 2**

| [Table 70] | | | | | | |
|---|---|---|---|---|---|---|
| | | | | 3.89 (1H. m), 3.58 (1H, t, J = 14.0 Hz), 2.79-2.68 (4H. m), 2.59 (1H, m), 2.52-2.44 (1H, m), 2.08 (2H, quin, J = 8.0 Hz) | | |
| 138 | 43AJ | 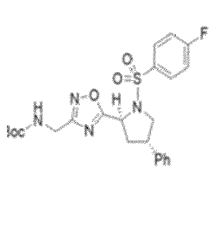 | 502.56 | (CDCl3) δ: 7.87 (2H, m), 7.35-7.19 (5H, m), 7.16-7.12 (2H, m), 5.29 (1H, dd, J = 8.8, 2.0 Hz), 5.06 (1H. br-s), 4.47 (2H, d, J = 5.2 Hz), 3.93 (1H, t. J = 8.4 Hz), 3.78 (1H, m), 3.38 (1H, t, J = 9.2 Hz), 2.48-2.32 (2H, m), 1.48 (9H, s) | 503 | 1.73 |
| 139 | 45AJ | 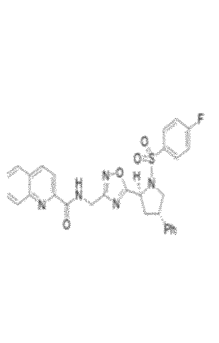 | 557.6 | (CDCl3) δ: 8.83-8.77 (1H. m), 8.37-8.32 (2H. m), 8.15 (1H, dd, J = 8.4, 0.8 Hz), 7.93-1.85 (3H, m), 7.79 (1H, ddd, J = 8.4, 6.8, 1.6 Hz), 7.67-7.63 (1H, m), 7.33-7.22 (3H, m), 7.21-7.12 (4H, m), 5.33 (1H, dd, J = 8.8, 1.6 Hz), 4.89 (2H. m), 3.93 (1H, t, J = 8.4 Hz), 3.79 (1H, m), 3.40 (1H, t, J = 9.6 Hz), 2.51-2.33 (2H, m) | 558 | 1.76 |
| 140 | 46AJ | 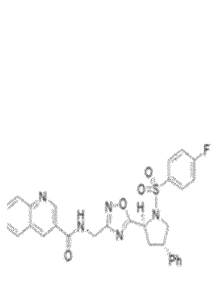 | 557.6 | (CDCl3) δ: 9.34 (1H, d, J = 2.4 Hz). 8.67 (1H, d, J = 2.0 Hz). 8.17 (1H, d, J = 9.2 Hz), 7.94 (1H, d, J = 9.6 Hz), 7.90-1.81 (2H, m), 7.64 (1H. ddd, J = 8.4, 7.2, 1.2 Hz), 7.33-7.10 (7H. m), 6.99 (1H, br-t, J = 2.4 Hz). 5.28 (1H. dd, J = 8.8, 1.6 Hz), 4.90 (2H, m), 3.97 (1H, t, J = 8.0 Hz), 3.79 (1H, m), 3.33 (1H, t, J = 9.6 Hz), 2.47 (1H, dd, J = 12.0. 6.4 Hz), 2.41-2.31 (1H, m) | 558 | 1.63 |

| | | | | | | |
|---|---|---|---|---|---|---|
| Ex: Example Number Cn: Compound Number MW: Molecular Weight | | | | | | |

The present compounds were investigated for an inhibitory activity on RS virus proliferation.

### Test Example 1

### In vitro Test for anti-RS virus proliferation inhibitory activity

A mixture of Vero cells (3.4 × 10⁵ cells/mL) and human RS virus A2 strain (1.7 × 10⁴ TCID50/mL) (EMEM medium supplemented with 10% FCS) was added to a 96-well plate at 50 µL/well, and the 10% FCS-supplemented EMEM medium adjusted to the desired final concentration of the compounds to be tested was added at 50 µL/well, followed by incubating the plate at 37°C in the presence of 5% CO₂ at 37°C. After 3 days of incubation, the supernatant was removed, the cells were washed with PBS(-), and the cell lysis buffer supplied with the SingleShot (registered trademark) Cell Lysis Kit (Bio-Rad) was added dropwise at 50 µL/well to lysis the cells thereby providing the cell lysate.

Human RS virus RNA copy number in the cell lysate was determined by quantitative RT-PCR using iTaq Universal SYBR Green One-Step Kit (Bio-Rad). Specifically, primers targeting human RS virus N gene (Rameix-Welti et al, Nat Commun5: 5104, 2014. doi: 10.1038/ncomms6104) was used to prepare the reaction solution according to the instructions provided with the kit, and a 96-well PikoReal Real-Time PCR System (TCR0096, Thermo Fisher Scientific) was used to detect PCR reaction and signal assay.

The copy number was calculated by the relative method using the comparative Ct method using compound-free virus-infected cells as controls. Results are shown in Tables 25-29. Results show anti-viral activity as the following IC50 indications:
A: IC50 <10 µM,
B: IC50 10 µM - 50 µM,
C: IC50 >50 µM

**Table 25**

| [Table 71] | | | | | | |
|---|---|---|---|---|---|---|
| Example Number | Compound Number | Anti-RS virus Activity | | Example Number | Compound Number | Anti-RS virus Activity |
| 1 | 1-1 | C | | 14 | 3-1 | C |
| 2 | 1A | C | | 15 | 3A | C |
| 3 | 18 | B | | 16 | 3B | B |
| 4 | 1C | C | | 17 | 3C | C |
| 5 | 1C' | A | | 18 | 3D | C |
| 6 | 1D | B | | 19 | 3E | B |
| 7 | 1E | B | | 20 | 4B | A |
| 8 | 2-1 | B | | 21 | 4-3 | A |
| 9 | 2A | B | | 22 | 4-4 | C |
| 10 | 2B | A | | 23 | 4-5 | C |
| 11 | 2C | B | | 24 | 4-6 | C |
| 12 | 2D | A | | | | |
| 13 | 2E | A | | | | |

**Table 26**

| [Table 72] | | | | | | |
|---|---|---|---|---|---|---|
| Example Number | Compound Number | Anti-RS virus Activity | | Example Number | Compound Number | Anti-RS virus Activity |
| 25 | 5A | A | | 36 | 7A | A |
| 26 | 5B | A | | 37 | 7B | A |
| 27 | 5C | C | | 38 | 7C | A |
| 29 | 5D | C | | 39 | 7D | A |
| 28 | 5D' | A | | 40 | 7E | C |
| 30 | 5E | B | | 41 | 8A | B |
| 31 | 6A | A | | 42 | 8C | C |
| 32 | 68 | A | | 43 | 8D | B |
| 33 | 6C | A | | 44 | 8E | A |
| 34 | 6D | A | | 45 | 9-1 | C |
| 35 | 6E | A | | 46 | 9A | C |
| | | | | 47 | 10-1 | B |
| | | | | 48 | 10A | C |
| | | | | 49 | 11A | A |
| | | | | 50 | 118 | B |

**Table 27**

| [Table 73] | | | | | | |
|---|---|---|---|---|---|---|
| Example Number | Compound Number | Anti-RS virus Activity | | Example Number | Compound Number | Anti-RS virus Activity |
| 51 | 1F | B | | 62 | 1K | B |
| 52 | 1G | C | | 63 | 1L | B |
| 53 | 10G | C | | 64 | 1M | B |
| 54 | 1H | C | | 65 | 1N | B |
| 55 | 12-1 | A | | 66 | 1O | B |
| 56 | 12H | B | | 67 | 14-1 | A |
| 57 | 12A | A | | 68 | 14A | B |
| 25-1 | 5-1 | A | | 69 | IP | A |
| 31-1 | 6-1 | A | | 70 | 1Q | A |
| 36-1 | 7-1 | A | | 71 | 1R | B |
| 41-1 | 8-1 | A | | 72 | 1S | A |
| 58 | 13H | C | | 73 | 1T | A |
| 59 | 13-1 | B | | 74 | 1U | A |
| 60 | 1I | A | | 75 | 1V | A |
| 61 | 1J | A | | 76 | 15-1 | B |

**Table 28**

| [Table 74] | | | | | | |
|---|---|---|---|---|---|---|
| Example Number | Compound Number | Anti-RS virus Activity | | Example Number | Compound Number | Anti-RS virus Activity |
| 77 | 15C | B | | 93 | 2S | A |
| 78 | C2-16-1 | B | | 94 | 2P | A |
| 79 | C3-16-1 | A | | 95 | 2Y | A |
| 80 | C1-7-1 | A | | 96 | 2T | B |
| 81 | C2-7-1 | B | | 97 | 2Z | B |
| 82 | C4-7-1 | A | | 98 | 2AA | B |
| 83 | C5-7-1 | A | | 99 | 2AB | B |
| 84 | 17-1 | A | | 100 | 2U | A |
| 85 | 18-1 | A | | 101 | 2AC | B |
| 86 | 19-1 | A | | 102 | 2AD | A |
| 87 | 20-1 | A | | 103 | 2AE | A |
| 88 | 2W | A | | 104 | 22-1 | A |
| 89 | 2X | A | | 105 | 228 | A |
| 90 | 21B | C | | | 22B' | A |
| 91 | 2Q | A | | 106 | 23-1 | B |
| 92 | 2R | B | | 107 | 2W' | B |

**Table 29**

| [Table 75] | | | | | | |
|---|---|---|---|---|---|---|
| Example Number | Compound Number | Anti-RS virus Activity | | Example Number | Compound Number | Anti-RS virus Activity |
| 108 | 24-1 | B | | 124 | 328 | B |
| 109 | 24F | B | | 125 | 338 | B |
| | 24F' | B | | 126 | 34B | B |
| 110 | 24H | B | | 127 | 358 | B |
| 111 | 25-1 | B | | 128 | 36B | A |
| 112 | 25AD' | B | | 129 | 378 | A |
| 113 | 25A | B | | 130 | 38B | B |
| 114 | 25AD | B | | 131 | 39AJ | A |
| | 25AD" | B | | 132 | 40B | B |
| 115 | 2AF' | B | | 133 | 41-1 | B |
| | 2AF | B | | 134 | 41AJ | B |
| 116 | 2AG | B | | 135 | 42AJ | B |
| 117 | 268 | B | | 136 | 7AQ | B |
| 118 | 278 | B | | 137 | 7AR | B |
| 119 | 28-1 | B | | 138 | 43AJ | B |
| 120 | 29B | B | | 139 | 45AJ | B |
| 121 | 30B | B | | 140 | 46AJ | B |
| 122 | 24AH | B | | | | |
| 123 | 28AH | B | | | | |

Next, anti-viral activity of the compounds prepared in Tables 7-3 and 8-3 are shown in Table 30 as well.

**Table 30**

| [Table 76] | | | | |
|---|---|---|---|---|
| Compound Number | Anti-RS virus Activity | | Compound Number | Anti-RS virus Activity |
| 5AI | B | | 6AI | A |
| 5AJ | A | | 6AJ | A |
| 5AK | A | | 6AK | A |
| 5AL | A | | 6AL | A |
| 5AM | A | | 6AM | A |
| 5AN | A | | 6AN | B |
| 5AO | A | | 6AO | C |
| 5AP | B | | 6AP | A |
| 5AC | B | | 6AC | B |
| 5AD | B | | 6AD | B |
| 5AE | B | | 6AE | B |

Next, anti-viral activity of the compounds prepared in Tables 9-3 and 10-3 are shown in Table 31 as well.

**Table 31**

| [Table 77] | | | | |
|---|---|---|---|---|
| Compound Number | Anti-RS virus Activity | | Compound Number | Anti-RS virus Activity |
| 7AI | A | | 8AI | A |
| 7AJ | A | | 8AJ | A |
| 7AK | A | | 8AK | A |
| 7AL | A | | 8AL | A |
| 7AM | A | | 8AD | B |
| 7AN | A | | | |
| 7AO | A | | | |
| 7AP | A | | | |
| 7AC | B | | | |
| 7AD | B | | | |
| 7AE | B | | | |

### [Industrial availability]

The invention is useful in treating or preventing RS virus infections.

## Claims

1. A compound represented by formula (I), its enantiomer, or a pharmaceutically acceptable salt thereof: 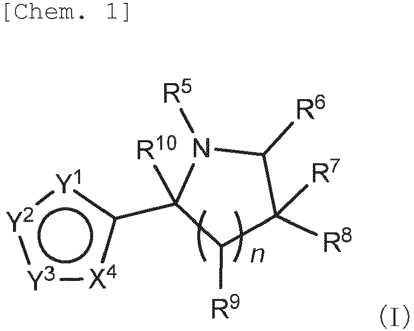 wherein
Y¹, Y², Y³ and X⁴ are each independently -O-, -N=, -S-, -NR¹-, or -CR²=;
in which at least one of Y¹, Y², Y³ and X⁴ is -N= or -NR¹-;
R¹= is hydrogen, an optionally substituted C1-6 alkyl, an optionally substituted C6-10 aryl, or an optionally substituted C6-10 heteroaryl;
R² is hydrogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl;
R⁵ is an optionally substituted C1-6 alkyl, an optionally substituted C6-10 aryl, an optionally substituted C6-10 heteroaryl, an unsubstituted or substituted carbonyl, an unsubstituted or substituted sulfonyl, an unsubstituted or substituted sulfinyl, an unsubstituted or substituted acyl, or an unsubstituted or substituted thioacyl;
R⁶, R⁷, R⁸, R⁹ and R¹⁰ are each independently hydrogen, an optionally substituted C1-6 alkyl, an optionally substituted C6-10 aryl, an optionally substituted C1-6 alkoxy, hydroxy, amino, cyano, carbamoyl or a halogen, or
R⁷ and R⁸ are cross-linked together with the carbon to which they are attached, to form a C3-6 spiro ring,
R⁶ and R⁹ are cross-linked together to form -CH₂- or - CH₂-CH₂-, or
R⁸ and R¹⁰ are cross-linked together to form -CH₂- or - CH₂-CH₂-;
n is an integer of 1 or 2;
in which the substituent in "optionally substituted" is selected from the following:
hydroxy, a halogen, cyano, carbamoyl, amino, an amidinoamino, a carboxy, a C6-10 aryl, a C1-4 alkoxycarbonyl-substituted 5- to 10-membered heteroaryl, a C1-4 alkylsubstituted C6-10 aryl , a hydroxy-substituted C6-10 aryl, a halogen-substituted C6-10 aryl, a C1-4 alkoxy-substituted C6-10 aryl, a (an optionally substituted amino)-C6-10 aryl, a C1-4 alkoxycarbonyl, a C1-4 alkoxycarbonylamino, a 5- to 6-membered heterocycloalkyl, a C3-6 cycloalkyl, a 5- to 10-membered heteroaryl, a (a halogen-substituted C1-6 alkyl)-substituted C6-10 aryl, and a trialkylsilyloxy, an alkylarylsilyloxy, a triarylsilyloxy, or a protecting group;
provided that, when the aromatic 5-membered ring comprising Y¹, Y², Y³ and X⁴ is a 1,2,3-triazole substituted with phenylmethyl, R⁵ is not tert-butoxycarbonyl.

2. The compound according to claim 1, its enantiomer, or a pharmaceutically acceptable salt thereof,
wherein the compound is represented by formula (I): 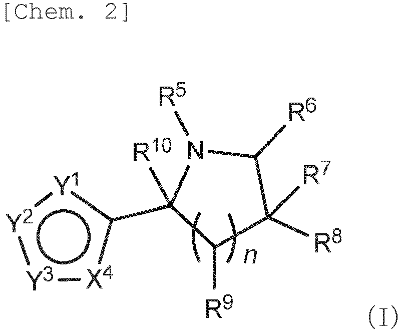
wherein
Y¹ is -O-, -N=, -S-, or -NR¹-;
Y² is -O-, -N=, -NR³-, or -CR²=;
Y³ is -N=, -NR⁴- or -CR¹⁸=;
X⁴ is -N= or -CH=;
in which at least one of Y¹, Y², Y³ and X⁴ is -N=, -NR²-or -NR³-;
R¹, R³ and R⁴ are each independently hydrogen, an optionally substituted C1-6 alkyl, an optionally substituted C6-10 aryl, or an optionally substituted C6-10 heteroaryl;
R² and R¹⁸ are each independently hydrogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl.

3. The compound according to claim 1 or 2, its enantiomer, or a pharmaceutically acceptable salt thereof,
wherein the compound is represented by formula (I): 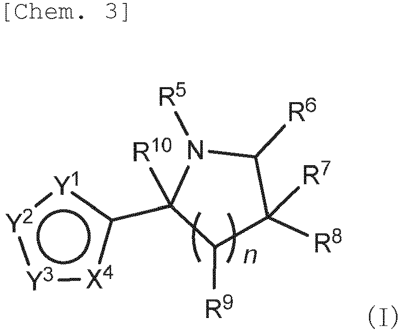
wherein
the group represented by the formula: 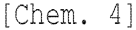 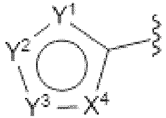 is the group represented by the formula: 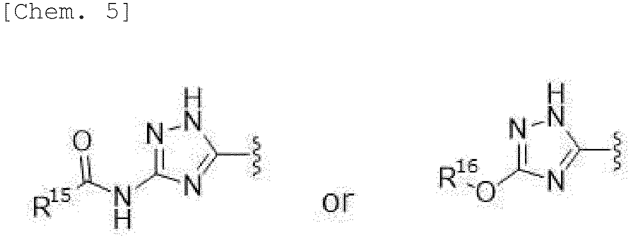
wherein
R¹⁵ is an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl; and
R¹⁶ is an optionally substituted C1-6 alkyl, an optionally substituted C6-10 aryl, or an optionally substituted C6-10 heteroaryl.

4. The compound according to claim 1 or 2, its enantiomer, or a pharmaceutically acceptable salt thereof,
wherein the compound is represented by formula (I): 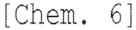 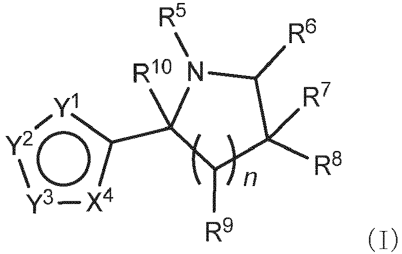
wherein
the group represented by the formula: 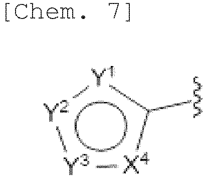 is the group represented by the formula: 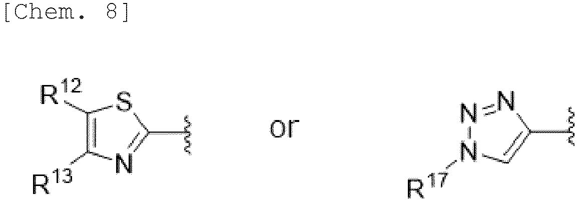
wherein
R¹² and R¹³ are each independently hydrogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl; and
R¹⁷ is an optionally substituted C1-6 alkyl, an optionally substituted C6-10 aryl, an optionally substituted C6-10 heteroaryl.

5. The compound according to claim 1 or 2, its enantiomer, or a pharmaceutically acceptable salt thereof,
wherein the compound is represented by formula (I): 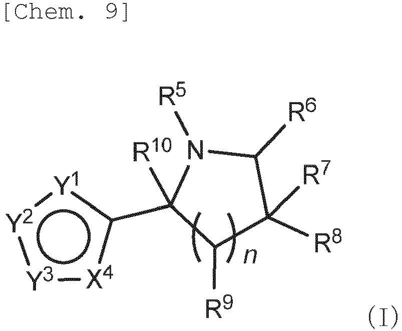
wherein
the group represented by the formula: 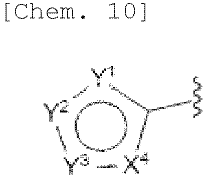 is the group represented by the formula: 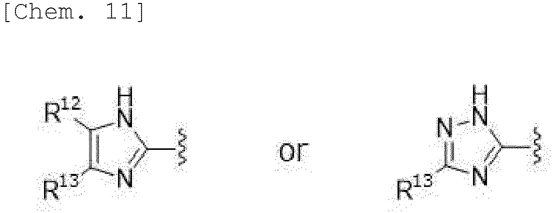
wherein
R¹² and R¹³ are independently hydrogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl.

6. The compound according to claim 1 or 2, its enantiomer, or a pharmaceutically acceptable salt thereof,
wherein the compound is represented by formula (I): 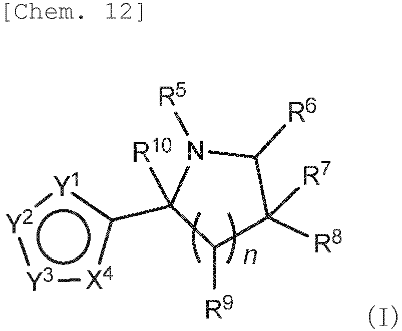
wherein
the group represented by the formula: 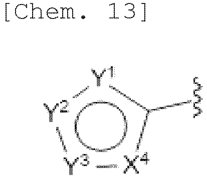 is the group represented by the formula: 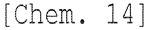 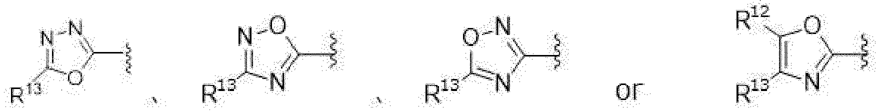
wherein
R¹² and R¹³ are each independently hydrogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl.

7. The compound according to claim 6, its enantiomer, or a pharmaceutically acceptable salt thereof,
wherein
the group represented by the formula: 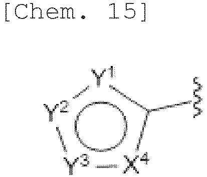 is the group represented by the formula: 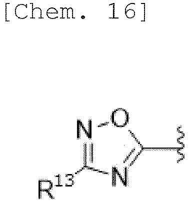 wherein
R¹³ is hydrogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C6-10 heteroaryl.

8. The compound according to claim 1 or 2, its enantiomer, or a pharmaceutically acceptable salt thereof,
wherein the compound is represented by formula (I): 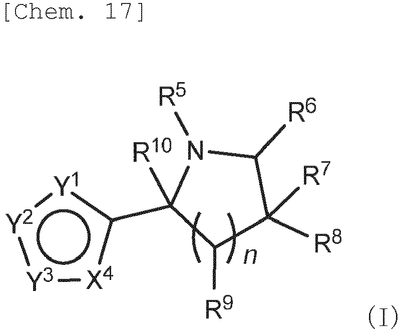
wherein
the group represented by the formula: 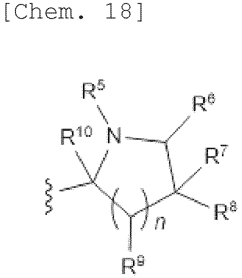 is the group represented by the formula: 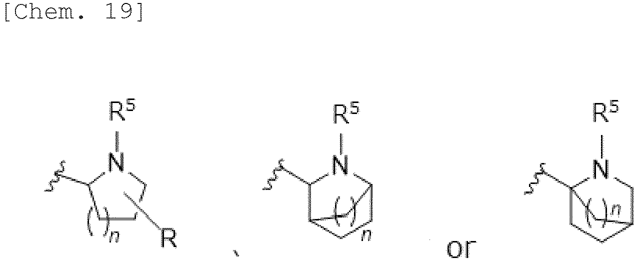
wherein
R is hydrogen, an optionally substituted C1-6 alkyl, an optionally substituted C6-10 aryl, an optionally substituted C1-6 alkoxy, hydroxy, amino, cyano, carbamoyl or a halogen;
R⁵ is an optionally substituted C1-6 alkyl, an optionally substituted C6-10 aryl, an optionally substituted C6-10 heteroaryl, an optionally substituted carbonyl, an optionally substituted sulfonyl, an optionally substituted sulfinyl, an optionally substituted acyl, or an optionally substituted thioacyl;
n is an integer of 1 or 2.

9. The compound according to claim 8, its enantiomer, or a pharmaceutically acceptable salt thereof,
wherein the compound is represented by formula (I): 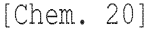 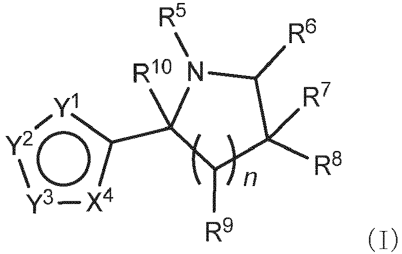
wherein
the group represented by the formula: 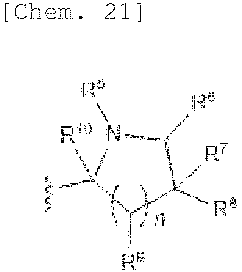 is the group represented by the formula: 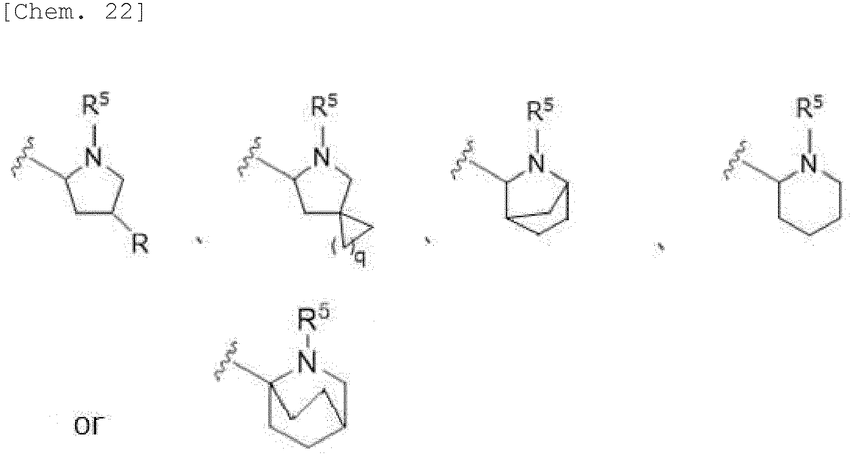
wherein
R is hydrogen, an optionally substituted C1-6 alkyl, an optionally substituted C6-10 aryl, an optionally substituted C1-6 alkoxy, hydroxy, amino, cyano, carbamoyl or a halogen;
R⁵ is an optionally substituted C1-6 alkyl, an optionally substituted C6-10 aryl, an optionally substituted C6-10 heteroaryl, an optionally substituted carbonyl, an optionally substituted sulfonyl, an optionally substituted sulfinyl, an optionally substituted acyl, or an optionally substituted thioacyl; and
q is an integer from 1 to 4.

10. The compound according to claim 9, its enantiomer, or a pharmaceutically acceptable salt thereof,
wherein
the group represented by the formula: 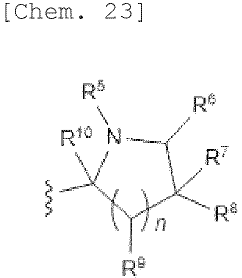 is the group represented by the formula: 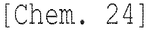 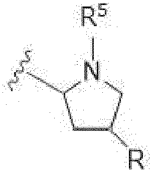 wherein
R is hydrogen, an optionally substituted C1-6 alkyl, an optionally substituted C6-10 aryl, an optionally substituted C1-6 alkoxy, hydroxy, amino, cyano, carbamoyl or a halogen;
R⁵ is an optionally substituted sulfonyl.

11. The compound according to claim 7, its enantiomer, or a pharmaceutically acceptable salt thereof,
wherein the compound is represented by the formula: 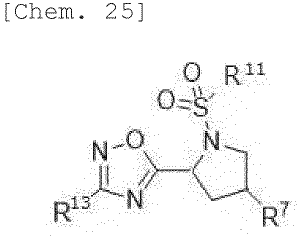
wherein
R¹³ is the formula: 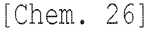 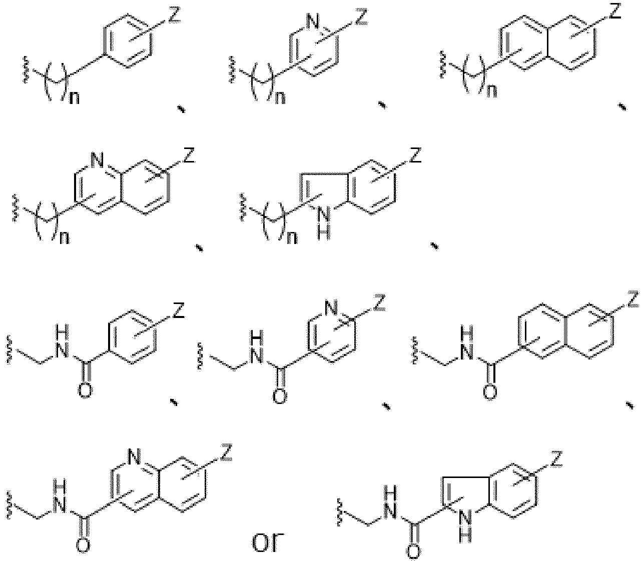
in which
Z is hydrogen, amino, dimethylamino, a halogen, hydroxy, cyano, carbamoyl, an optionally substituted C1-6 alkyl, or an optionally substituted C1-6 alkoxy;
n is an integer from 1 to 5;
R⁷ is the group represented by the formula: 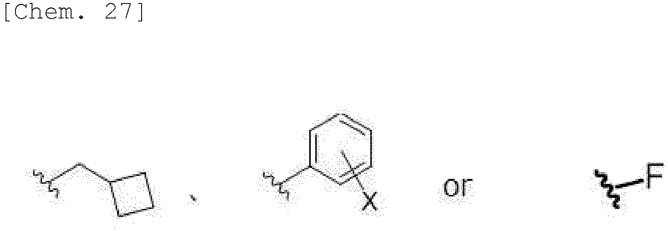
in which
X is hydrogen, amino, a halogen, hydroxy, methoxy, or an optionally substituted C1-4 alkyl;
R¹¹ is methyl, or the group represented by the formula: 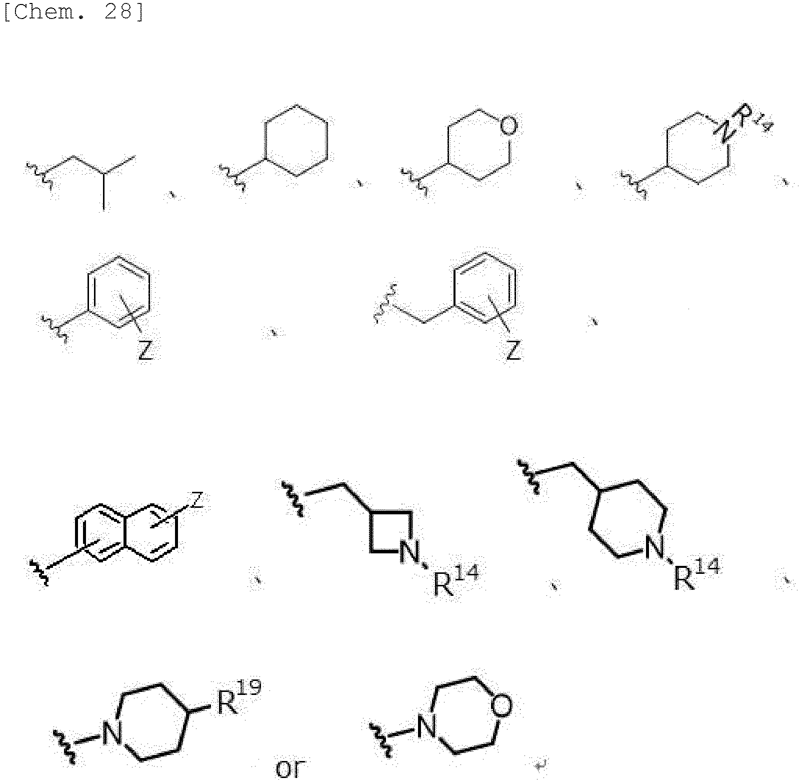
in which
R¹⁴ is each independently hydrogen, an optionally substituted C1-6 alkyl, an optionally substituted C6-10 aryl, an optionally substituted C6-10 heteroaryl, an optionally substituted carbonyl, an optionally substituted sulfonyl, an optionally substituted sulfinyl, an optionally substituted acyl, or an optionally substituted thioacyl;
R¹⁹ is hydrogen, an optionally substituted C1-6 alkyl, an optionally substituted C6-10 aryl, an optionally substituted carbonyl, hydroxy, an alkoxy, or an alkoxymethyl;
Z is the same as defined above.

12. A pharmaceutical composition, which comprises the compound of any one of claims 1 to 11, its enantiomer, or a pharmaceutically acceptable salt thereof.

13. The pharmaceutical composition according to claim 12, for treating or preventing an RS virus infection.
